# EUROPEAN PATENT APPLICATION

(11) **EP 2 085 485 A2**
(43) Date of publication of application: **05.08.2009**
(21) Application number: 08253870.3
(22) Date of filing: 03.12.2008
(51) Int. Cl.: C12Q 1/68

(54) **Method of diagnosis of cone-rod distrophy**

(30) Priority: 08.02.2008 GB 0802456
(71) Applicant: Norwegian School of Veterinary Science, N-0033 Oslo (NO)
(72) Inventor: Lingaas, Frode, 2900 Fagernes (NO); Wiik, Anne Caroline, 0168 Oslo (NO); Wade, Claire Margaret, Como New South Wales 2226 (AU); Lindblad-Toh, Kerstin, Malden, MA 02148 (US)
(74) Representative: Webber, Philip Michael

(57) **Abstract**

The present invention relates to a method of diagnosing or detecting cone-rod dystrophy in a mammal, particularly in a canine species. In particular, the invention relates to methods involving the detection of a deletion in the *NPHP4* (nephroretinin 4) gene and associated biomarkers. The invention also provides primers, nucleic acid molecules, polypeptides, antibodies, as well as kits for use in such methods.

## Description

The present invention relates to a method of diagnosing or detecting cone-rod dystrophy in a mammal, particularly in a canine species. In particular, the invention relates to methods involving the detection of a deletion in the *NPHP4* (nephroretinin 4) gene and biomarkers associated with this deletion. The invention also provides primers, nucleic acid molecules, polypeptides, antibodies, as well as kits for use in such methods.

Cone-rod dystrophy (CRD) is a progressive retinal degenerative disease that causes deterioration of the cones and rods in the retina and frequently leads to blindness. The cone-rod dystrophies are a heterogeneous group of disorders that are known to occur naturally in man and dogs. The disorders are characterized by a predominant loss of cone function, with relative preservation of rod function [1, 2].

Cone-rod dystrophy is classified as a retinal ciliopathy, together with retinitis pigmentosa, macular degeneration, cone-dystrophy, Leber congenital amaurosis, and retinal degenerations associated with Usher syndrome, primary ciliary dyskinesia, Senior-Loken syndrome, Joubert syndrome, Bardet-Biedl syndrome, Laurence-Moon syndrome, McKusick-Kaufman syndrome and Biemond syndrome. Mutations for these latter disorders have been found in the genes: retinitis pigmentosa-1 (RP1), retinitis pigmentosa GTPase regulator (*RPGR*), retinitis pigmentosa GTPase regulator interacting protein (*PPGRIP1*), as well as the Usher, Bardet-Biedl, and nephronophthisis genes [3]. These genes are expressed in the outer segment of the photoreceptors, which is a specialized and modified cilium, where they have a structural role and are crucial for transport function. They also are involved in protein-protein interaction with transport-like proteins (Adams et al. 2007).

Nephroretinin-4 has been found to be abundantly present at the subcortical plasma membrane region of polarized renal epithelial cells, in the centrosomes of dividing cells and, to a lesser extent in the primary cilia of highly confluent cell cultures [6]. NPHP4 is localized in the connecting cilium of photoreceptors, form a functional complex and interact with other proteins at the ciliary base.

The number of genes or loci currently identified for CRD in humans is low, compared to those for retinitis pigmentosa, RP (e.g. RetNet™). A few genes have been associated with autosomal recessive inherited CRD in man including *ABCA4* [4, 5] and *RPGRIP1* [10] and the mapped loci *CORD8* [7, 8] and *CORD9* [9].

Nephronophthisis (NPHP) comprises a group of autosomal recessive cystic kidney diseases, which constitute the most frequent genetic cause for end-stage renal failure in children and young adults. Six genes, *NPHP1-6,* have been identified as being mutated in human NPHP. The disease has additionally been associated with various extrarenal disorders. In Joubert syndrome (JBTS), NPHP may be associated with cerebellar vermis aplasia/hypoplasia, retinal degeneration and mental retardation. Mutations in *NPHP6* and *RPGRIP1L,* a nephrocystin-4 interactor, are involved in Joubert syndrome [11, 12]. In Senior-Loken syndrome (SLSN), NPHP mutations are associated with retinitis pigmentosa, tapetoretinal degeneration or retinal dysplasia [13]. SLSN is present in about 10% of persons with mutations in *NPHP1, 2, 3* or 4 [14-17] and >80% of persons with mutations in *NPHP5* or *NPHP6* [18, 19].

*NPHP4* encodes nephroretinin-4 (also known as nephrocystin-4) that is conserved in evolution [17]. RPGRIP1 and NPHP4 interact strongly *in vitro* and *in vivo,* and they co-localize in the retina. Their interaction is disrupted by either mutations in *PPGRIP1,* found in patients with LCA, or by mutations in *NPHP4,* found in patients with SLSN [20].

At least 35 NPHP4 sequence variants are known in man, all causing nephronophthisis. A number of these display extrarenal manifestations, such as retinitis pigmentosa (RP), loss of hearing, bronchitis, Cogan syndrome, Usher syndrome and ophthalmologic abnormalities (unspecified).

No mutations in NPHP4 have previously been known to cause CRD. To date, only some unspecified ophthalmologic abnormalities have been described as being correlated with mutations in NPHP4, but these are always found in combination with kidney disease.

Mutations in NPHP6 are found to be a frequent cause of Leber congenital amaurosis without any kidney disease. Furthermore, a mutation in NPHP6 (CEP290) has been reported in low frequency Abyssinian cats, causing autosomal recessive late onset photoreceptor degeneration (rdAc).

So far, the only dog breeds in which CRD has been detected are the standard wire haired dachshund (SWHD), the miniature long haired dachshund (MLDH) and the pit bull terrier (PBT) [21-23]. A mutation in canine *RPGRIP1* has been demonstrated to cause autosomal recessive CRD in miniature longhaired dachshunds [22], while the genetic basis of the cone-rod dystrophy found in the pit bull terrier is still unknown [21].

The inventors has now found that a deletion in the NPHP4 gene is associated with the presence of CRD in dogs. This discovery is of significance not only in the diagnosis and detection of CRD in dogs, but also in other mammals, particularly humans. Phenotypic similarities between humans and dogs caused by mutations in orthologous genes make the dog a valuable model for the study of human retinal disease [24]. Dog populations display a number of different inherited retinal disorders and the genetic basis of at least 11 diseases have been identified to date [22, 25-34]. An example where an identical mutation was found for Retinitis Pigmentosa (*PP17*) in man and an orthologous progressive rod-cone dystrophy (*PRCD*) gene in the dog [33] shows the close relationship between the genes in the two species.

The molecular tools described herein can efficiently be used to reduce the frequency of the specific mutation or other mutations in this gene by obtaining a biological sample containing DNA and/or RNA or protein, and using the information about polymorphisms in the gene (e.g. the disease-associated mutation, SNPs in the same gene or the SNPs or microsatellites closely linked to the gene on each side, including promoters and enhancers) to define the haplotype and the disease/carrier-status of the mammal.

In one embodiment, the invention provides a method for diagnosing or detecting cone-rod dystrophy (CRD) in a mammal, the method comprising determining the presence or absence in a sample of nucleic acid that has been obtained from the mammal of: (i) a deletion in the *NPHP4* gene, the deletion comprising all or part of exon 5, and (ii) a wild-type exon 5 in the *NPHP4* gene, wherein the presence of (i) and the absence of (ii) is indicative of the presence of CRD in the mammal, and wherein the presence of (i) and the presence of (ii) is indicative of the mammal being a carrier for CRD.

Preferably, the deletion in exon 5 is one which results in the production, upon translation of the NPHP4 gene, of a polypeptide which is truncated at the C-terminal end compared to a wild-type NPHP polypeptide.

In other embodiments, the deletion in exon 5 is one which results in the production, upon translation of the NPHP4 gene, of a NPHP4 polypeptide which does not bind to RPGRIP1.

In yet other embodiments, the deletion in exon 5 is one which results in the production, upon translation of the NPHP4 gene, of a NPHP4 polypeptide which does not bind to RPGRIP 1 but does bind to NPHP1.

As discussed above, mutations in genes other than *NPHP4* are known to cause CRD in at least some mammals. Hence the absence of (i) and the presence of (ii) cannot be said to be indicative of a normal (i.e. wild-type CRD phenotype) mammal in all cases.

The skilled person will understand that the mammal will possess two alleles of the *NPHP4* gene and that the deletion in exon 5 and the wild-type *NPHP4* gene exon 5 may be present in one or both alleles.

As used herein, the term "nucleic acid" encompasses both DNA and RNA. The nucleic acid may be single-stranded or double-stranded. Examples of nucleic acid include genomic DNA, cDNA and mRNA. In particular, the invention encompasses nucleic acid which has been amplified from nucleic acid obtained from the mammal, e.g. PCR'd DNA, and also cDNA which has been produced from RNA obtained from the mammal.

As used herein, references to cDNA relate to the cDNA which has been obtained from NPHP4 RNA, either wild-type NPHP4 RNA or mutated NPHP4 RNA having the deletion of the invention.

As used herein, the term "cone-rod dystrophy (CRD)" refers to a progressive retinal degenerative disease that causes deterioration of the cones and rods in the retina and frequently leads to blindness. The invention particularly relates to a form of CRD which is not associated with clinically-detectable kidney disease.

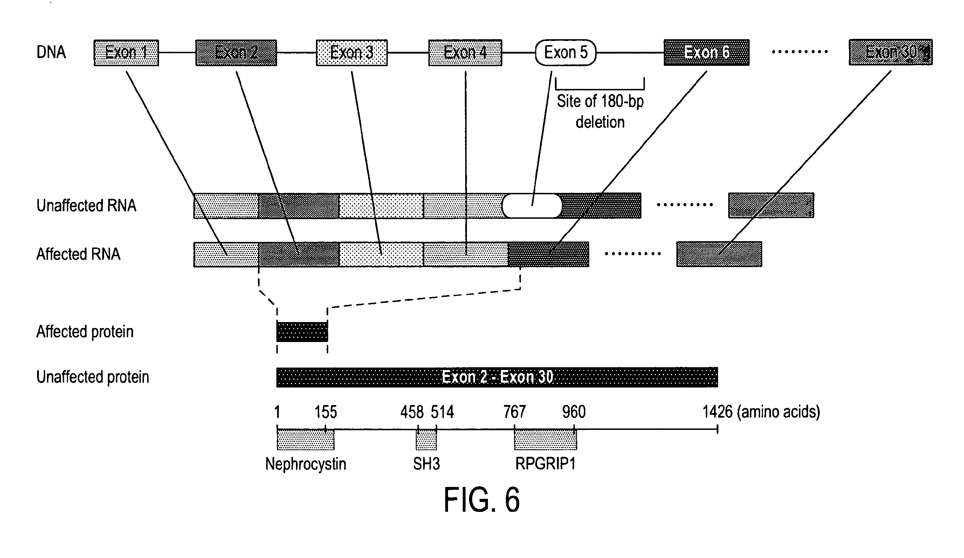

The *NPHP4* gene is well known in the art [17]. Furthermore, the complete sequence of the *Canis familiaris NPHP4* gene is disclosed herein; and Figure 6 discloses a partial intron/exon map of this gene. Corresponding genes in other mammals are also known or can readily be identified. The performance of this invention in mammals other than *Canis familiaris* can therefore readily be carried out.

As used herein, the term "wild-type" *NPHP4* gene refers to the *NPHP4* gene is which is present in most members of the population. The wild-type form of the gene contains all of the exons. In particular it does not have a deletion in exon 5.

The term "mammal" includes, *inter alia,* domestic mammals (dogs, cats, rabbits, etc.) and farm mammals (such as cows, pigs, sheep, donkeys, goats, etc) and well as humans. Preferably, the mammal is a dog or a human. In other embodiments of the invention, the mammal is a non-human mammal.

Preferred dog species include the daschund and the pit bull terrier, particularly preferably the Standard Wirehaired Dachshund (SWHD) and the miniature long haired dachshund (MLDH).

The deletion of all or a part of exon 5 is preferably a deletion which results, during transcription into mRNA, in the skipping of exon 5, i.e. a mRNA is produced in which the 3'-end of exon 4 is joined to the 5'-end of exon 6.

Hence in embodiments of the invention where the nucleic acid sample is RNA, the deletion is preferably all of exon 5.

Exon 5 of the *NPHP4* gene is generally about 65 bp in length, although some variation might exist from species to species.

Preferably, the deletion of part of exon 5 is one which results in the deletion of 1-65, 1-60, 1-50, 1-40, 1-30, 1-20 or 1-10 nucleotides from exon 5.

In some embodiments, the deletion is measured from the 5'-end in a 5'-3' direction, possibly including the (5'-end) intron/exon boundary. In other embodiments, the deletion is measured from the 3'-end in a 3'-5' direction, possibly including the (3'-end) exon/intron boundary. In yet other embodiments, the deletion will be in the middle part of the exon, leaving the both 5'- and 3'-intron/exon boundaries unaffected.

In some preferred embodiments where the nucleic acid is DNA, the deletion in exon 5 extends into the intron between exons 5 and 6 of the *NPHP4* gene (i.e. into intron 5-6).

In some particularly preferred embodiments, the deletion starts at and includes nucleotide 19 of exon 5 and extends in a 3' direction into intron 5, resulting in a deletion of about 180 nucleotides of genomic DNA.

The invention also provides a method for diagnosing or detecting cone-rod dystrophy (CRD) in a mammal, the method comprising determining the presence or absence in a sample of DNA that has been obtained from the mammal of:
(i) a deletion in a first allele of the *NPHP4* gene, the deletion starting at and including nucleotide 19 of exon 5 and extending in a 3' direction into intron 5,
(ii) a wild-type exon 5 in a second allele of the *NPHP4* gene,
wherein the presence of (i) and the absence of (ii) is indicative of the presence of CRD in the mammal, and wherein the presence of (i) and the presence of (ii) is indicative of the mammal being a carrier for CRD.

In some particularly preferred embodiments, the deletion results in a deletion of about 180 bp of genomic DNA.

The invention also provides a method for diagnosing or detecting cone-rod dystrophy (CRD) in a mammal, the method comprising determining the presence or absence in a sample of RNA that has been obtained from the mammal or of cDNA obtained therefrom of:
(i) a deletion in the *NPHP4* RNA, the deletion consisting of all of exon 5,
(ii) a wild-type exon 5 in the *NPHP4* RNA,
wherein the presence of (i) and the absence of (ii) is indicative of the presence of CRD in the mammal, and wherein the presence of (i) and the presence of (ii) is indicative of the mammal being a carrier for CRD.

The skilled person will understand that, in mammals having a nucleic acid deletion in an exon, a mutated polypeptide will be produced. The complete amino acid sequence of the mutated dog NPHP4 polypeptide is given in SEQ ID NO: 7. Hence the presence of all of this sequence or a non-wild-type part of this amino acid sequence in a sample obtained from a mammal will be indicative of the mammal being either heterozygous or homozygous for the deletion.

The invention therefore also provides a method for diagnosing or detecting cone-rod dystrophy (CRD) in a mammal, the method comprising determining the presence or absence in a sample of nucleic acid that has been obtained from the mammal of:
(i) a nucleic acid molecule coding for a mutant or truncated NPHP4 polypeptide whose amino acid sequence consists of the amino acid sequence given in SEQ ID NO: 7 or an amino acid sequence which has at least 70% sequence identity thereto, preferably at least 75%, 80%, 85%, 90% or 95% sequence identity thereto, and
(ii) a nucleic acid molecule coding for a NPHP4 polypeptide whose amino acid sequence comprises SEQ ID NO: 8 or whose amino acid sequence comprises an amino acid sequence which has at least 70% sequence identity to SEQ ID NO: 8, preferably at least 75%, 80%, 85%, 90% or 95% sequence identity to SEQ ID NO: 8,
wherein the presence of (i) and the absence of (ii) is indicative of the presence of CRD in the mammal, and wherein the presence of (i) and the presence of (ii) is indicative of the mammal being a carrier for CRD.

Preferably, the nucleic acid molecule encoding (i) comprises the nucleotide sequence given in SEQ ID NO: 3 or 5 or 9, or comprises a nucleotide sequence which has at least 70% sequence identity, preferably at least 80%, 85%, 90% or 95% sequence identity, to SEQ ID NO: 9.

Preferably, the NPHP4 polypeptide referred to in step (ii) is a polypeptide whose amino acid sequence consists of the amino acid sequence given in SEQ ID NO: 6 or an amino acid sequence which has at least 70%, preferably at least 75%, 80%, 85%, 90%, 95% or 99% sequence identity thereto and which also encodes a mammalian NPHP4 polypeptide.

In other embodiments, the nucleic acid molecule of step (ii) comprises the nucleotide sequence given in SEQ ID NO: 1 or 2, or comprises a nucleotide sequence which has at least 95% sequence identity to SEQ ID NO: 2, or comprises the nucleotide sequence given in SEQ ID NO: 4 or comprises a nucleotide sequence which has at least 75%, preferably at least 80%, 85%, 90% or 95%, sequence identity to SEQ ID NO: 4.

Preferably, the nucleic acid molecule of (ii) comprises the nucleotide sequence given in SEQ ID NO: 2.

In some embodiments of the invention, the term "method for detecting cone-rod dystrophy in a mammal" means a method for detecting probable cone-rod dystrophy in a mammal or a method of determining an increased likelihood of cone-rod dystrophy in a mammal.

The skilled person will be aware that the deletion may be detected either by direct means (such as those described above) or by indirect means, for example, by establishing a relationship between the deletion and a co-segregating polymorphic site (e.g. a Single Nucleotide Polymorphism or SNP). A number of polymorphic sites in the dog genome are already known.

In a further embodiment, therefore, the invention provides a method for diagnosing or detecting cone-rod dystrophy (CRD) in a mammal or for determining the likelihood of a mammal possessing a CRD genotype, the method comprising determining the presence or absence in a sample of nucleic acid which has been obtained from the mammal of:
(i) a polymorphic site which co-segregates with a deletion in the *NPHP4* gene, the deletion comprising all or part of exon 5, and
(ii) a polymorphic site which co-segregates with the wild-type exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD.

The invention also provides a method for diagnosing or detecting cone-rod dystrophy (CRD) in a mammal or for determining the likelihood of a mammal possessing a CRD genotype, the method comprising determining the presence or absence in a sample of nucleic acid that has been obtained from the mammal of:
(i) a polymorphic site which co-segregates with a deletion in the *NPHP4* gene, the deletion comprising all or part of exon 5, and
(ii) the wild-type exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD.

The invention further provides a method for diagnosing or detecting cone-rod dystrophy (CRD) in a mammal or for determining the likelihood of a mammal possessing a CRD genotype, the method comprising determining the presence or absence in a sample of nucleic acid that has been obtained from the mammal of:
(i) a deletion in the *NPHP4* gene, the deletion comprising all or part of exon 5, and
(ii) a polymorphic site which co-segregates with the wild-type exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD.

The presence or absence of the polymorphic site may be detected using any of the methods described herein for the detection of the deletion, *mutatis mutandis.*

The polymorphic site may, for example, be a site having 1, 2, 3, 4, 5, 6, 7, 8, 9 or 10 contiguous or non-contiguous nucleotides, some or all of which nucleotides are different from the corresponding wild-type mammalian *NPHP4* gene sequence.

Preferably the polymorphic site is a repeated motif of polynucleotides, wherein the motif is 1-50, 1-30, 1-20, 1-10, 1-5 or 1-2 nucleotides in length, and is repeated 1-100, 1-50, 1-40, 1-30, 1-20, or 1-10 times. Most preferably, the polymorphic site is a microsatellite or SNP.

In some preferred embodiments, the polymorphic site is a dinucleotide or tetranucletide motif, for example (AT)ₙ or (CG)ₙ.

The distance between the polymorphic site and the deletion may, for example, be 1-10⁷, 1-10⁶, 1-10⁵ or 1-10⁴, 1-10³ or 1-10² nucleotides. Preferably, the polymorphic site is as close as possible to the deletion, for example 1-50, 1-25 or 1-10 nucleotides.

Alternatively, the distance between the polymorphic site and the deletion may, for example, less than 50 cM, less than 20cM, less than 10 cM, less than 5 cM or less than 1cM.

The skilled person will understand that the closer the polymorphic site is to the deletion, the greater the probability will be that the polymorphic site and the deletion will co-segregate in any one mammal.

Preferably, the polymorphic site is within the *NPHP4* gene, for example in an intron or exon or promoter, or a SNP or a microsatellite. Most preferably, the polymorphic site is a SNP.

The coding sequence of the wild-type canine *NPHP4* gene is given herein in SEQ ID NOs: 1 (genomic) and 4 (cDNA); the corresponding polypeptide sequence is given in SEQ ID NO: 6.

The gene and polypeptide sequences of the mutant *NPHP4* sequences are given herein as SEQ ID NOs: 3 (genomic) and 5 (cDNA), and 7 (polypeptide). It will be appreciated, however, by the person skilled in the art that the invention relates primarily to the presence or absence of a deletion in exon 5 of the coding sequence of the *NPHP4* gene. Consequently, the precise nucleotide sequence of the *NPHP4* gene of the mammal being tested is not of primary importance in the context of the present invention, i.e. the sequence of the *NPHP4* gene of the mammal being tested might differ from that of the sequences disclosed herein due to natural sequence variation and inter-species variation. However, these natural sequence variations (if present) should not prevent the skilled person from identifying exon 5 in the *NPHP4* gene/polypeptide in the mammal being tested. It is not a requirement of the invention, therefore, that the mammal being tested has a *NPHP4* gene which has the precise sequences disclosed herein. The sequences given herein are included merely for reference purposes.

The person skilled in the art will readily be able to establish the nucleotide sequence corresponding to exon 5 of the mammalian *NPHP4* gene in a sample of nucleic acid obtained from the mammal being tested. This may be done either by manual evaluation of the sequence by one skilled in the art, or by computer-automated sequence comparison and identification using algorithms such as BLAST (Basic Local Alignment Search Tool; Altschul, S. F., et al., (1993) J. Mol. Biol. 215:403-410; see also www.ncbi.nlm.nih.gov/BLAST/), the Megalign program of the LASERGENE bioinformatics computing suite (DNASTAR Inc., Madison, WI) or using the Clustal method of alignment (Higgins and Sharp (1989) CABIOS. 5 : 151-153). If using the Clustal method, default parameters for pairwise alignments may be KTUPLE 1, GAP PENALTY=3, WINDOW=5 and DIAGONALS SAVED=5.

The mammalian DNA to be tested may be obtained from any suitable cell type or tissue sample from the mammal. In a preferred embodiment, the DNA is obtained from a bodily fluid, e.g. blood, obtained by known techniques (e.g. venipuncture) or saliva. Alternatively, DNA can be obtained from dry samples (e.g. hair follicles or skin) or from germ cells, e.g. sperm or egg cells.

mRNA may be obtained from any cell type of the mammal which ordinarily expresses NPHP4, i.e. cell types which express NPHP4 in wild-type mammals. In mammals in which the deletion is present, a mutant form of NPHP4 mRNA should be expressed in muscle, kidney, lung, eye, nerve, brain, testis, thymus, trachea, uterus, bone and connective tissues.
Preferably, the mRNA for testing is obtained from muscle, kidney or lung.

Numerous methods are known in the art for the detection of deletions such as the exon 5 deletion referred to herein. The invention is not limited to any one detection method. These methods include nucleic acid sequencing; microsequencing; allele specific oligonucleotide hybridization; size analysis; hybridization; 5' nuclease digestion; single-stranded conformation polymorphism; allele specific hybridization; primer specific extension; oligonucleotide ligation assay; and restriction enzyme analysis.

Polymerase chain reaction (PCR) may or may not be used as part of the detection method of the invention. Using PCR, the presence or absence of the deletion in the mammalian *NPHP4* gene may be detected by amplifying a region of nucleic acid from the mammalian genome which includes the deletion, using first and second nucleic acid primers. The primers will preferably have sequences and lengths so as to hybridize specifically under appropriate conditions to the region of mammalian nucleic acid which includes the deletion in exon 5. The primers in this case will not hybridize to the deleted part of exon 5, but will flank this region so that the deletion or wild-type sequence will be included in the fragment that is amplified. Primers may be designed, for example, using the SNP analysis program of the WatCut package found at http://watcut.uwaterloo.ca/watcut/watcut/template.php. The primers are preferably DNA primers. Preferred primers are disclosed herein.

The invention particularly relates therefore to a method as disclosed herein which additionally comprises the step of amplifying the region of the mammalian *NPHP4* nucleic acid which includes exon 5 of the coding sequence of the mammalian *NPHP4* gene. Preferably, the amplifying is by an exponential amplification method, e.g. the polymerase chain reaction (PCR).

The length of the amplified nucleic acid which includes exon 5 of the coding sequence of the mammalian *NPHP4* gene will generally be a length which will allow for the detection of the presence or absence of exon 5 by any suitable method. Preferably, the length of the region of amplified nucleic acid will be 20-3,000 nucleotides, more preferably 25-500 nucleotides and most preferably 50-250 nucleotides.

Preferably, the primers used in the amplification process are between 15-50 nucleotides in length, more preferably 18-35 nucleotides and most preferably 20-25 nucleotides in length. The upstream and downstream primers might have different lengths.

SEQ ID NO: 2 shows the nucleotide sequence of exons 4-6 of the wild-type canine *NPHP4* gene. Exon 5, the start of intron 5-6 and the deletion in the mutant canine *NPHP4* gene are shown below (with the deletion underlined):

Exon5
GTTGAGGCTGTATCACGGCACTCCCCGAGCCCTCCTGCACCCACTGCTCCAGGACC
CTTTCGAAC
Intron 5-6
gtaagagacaaggcctggcctggcctgaggtgttgctgtgaagtcctctcatcctc
cctatgactttcctggtgttatctgctttgactccatcttgttctctttctctgtg
taaaccattatcagcttagctgatttatttaagggattaggacaaaaagtgtcatg
agcttggatggttgacgtaactgaaggaactgtaag ...
(SEQ ID NO: 209)

Thus it will be clear to the skilled person where suitable primers may be located in order to amplify the region of DNA which is shown above. For example, the sequence of the upstream primer might be found within the end of intron 4/start of exon 5, within intron 4-5, or indeed further upstream. Similarly, the downstream primer may be located in the non-underlined region of intron 5-6 or further downstream.

Primers are preferably placed in intron 4 and in the undeleted part of intron 5. This provides a short PCR product in subjects with the deletion. The relative distance between the sizes of the PCR products in the deleted genotype versus the wild-type genotype is therefore large.

Preferred primers are those whose sequences are shown in the Figures. Primers for amplification of cDNA are preferably placed in exons 3 and 6.

The nucleic acid primers of the invention may additionally comprise, at the 5'-end of the primer, the nucleotide sequence of a restriction enzyme, thus facilitating the cloning of any amplified fragments. The nucleotide sequences of the primers of the invention might also be different from the naturally-occurring *NPHP4* sequence in 1, 2 or 3 nucleotides, allowing for the production of artificial restriction enzyme sites within the sequence of the primers.

A further embodiment of the invention relates to nucleic acid primers as disclosed herein.

All nucleic acid fragments of the invention can be prepared according to methods well known in the art (e.g. [35]). For example, discrete fragments of the DNA can be prepared and cloned using restriction enzymes. Alternatively, discrete fragments can be prepared using the Polymerase Chain Reaction (PCR) using primers having an appropriate sequence.

Oligonucleotides and primers of the invention may also be synthesized by standard methods known in the art, e.g. by use of an automated DNA synthesizer (such as is commercially available from Biosearch, Applied Biosystems, etc.).

Using methods well known in the art, nucleic acid sequencing of the region of nucleic acid which includes exon 5 of the *NPHP4* gene may be used to determine the presence or absence of deletion compared to that of the wild-type coding sequence.

Examples of standard DNA sequencing methods include those based on techniques developed by Maxam and Gilbert [36] or Sanger [37]. A variety of automated sequencing procedures may also be utilized, as may sequencing by mass spectrometry.

Microsequencing methods may be used to determine the identity of a single nucleotide at a predetermined site. Hence these methods may be used to determine presence or absence of polymorphisms in the *NPHP4* gene. Such microsequencing methods are discussed in Boyce-Jacino et al. (U.S. Pat. No. 6,294,336).

Single base variations in DNA sequences can be detected by a variety of techniques including Southern blot analysis for restriction fragment length polymorphisms, allele-specific oligonucleotide hybridization [38], denaturing gradient gel electrophoresis [39], chemical cleavage of mismatched heteroduplexes [40], conformational changes in single strands [41], and allele-specific priming of the polymerase chain reaction (PCR) [42-44].

An alternative strategy for DNA diagnosis, the oligonucleotide ligation assay (OLA), employs two adjacent oligonucleotides (20-mers), a 5' biotinylated probe (with its 3' end at the nucleotide to be assayed) and a 3' reporter probe [45-47].

The invention also provides a method of testing a nucleic acid molecule, comprising determining the presence or absence of a nucleotide sequence in the nucleic acid molecule which encodes a truncated NPHP4 polypeptide or a mutant NPHP4 as disclosed herein.

As mentioned above, the skilled person will understand that, in mammals having a nucleic acid deletion in an exon, a mutated polypeptide will be produced. The complete amino acid sequence of the mutated dog NPHP4 polypeptide is given in SEQ ID NO: 7. Hence the presence of this amino acid sequence in a sample obtained from a mammal will be indicative of the mammal having at least carrier status, i.e. being heterozygous or possibly homozygous for the exon 5 deletion.

The invention therefore provides a method for diagnosing or detecting cone-rod dystrophy (CRD) in a mammal, the method comprising determining the presence or absence in a sample of polypeptide obtained from the mammal of:
(i) a mutant NPHP4 polypeptide whose amino acid sequence consists of the amino acid sequence given in SEQ ID NO: 7 or an amino acid sequence which has at least 70% sequence identity thereto, preferably at least 75%, 80%, 85%, 90% or 95% sequence identity thereto, and
(ii) a NPHP4 polypeptide whose amino acid sequence comprises SEQ ID NO: 8 or whose amino acid sequence comprises an amino acid sequence which has at least 70% sequence identity to SEQ ID NO: 8, preferably at least 75%, 80%, 85%, 90% or 95% sequence identity to SEQ ID NO: 8,
wherein the presence of (i) and the absence of (ii) is indicative of the presence of CRD in the mammal, and wherein the presence of (i) and the presence of (ii) is indicative of the mammal being a carrier for CRD.

Preferably, the polypeptide sequence of step (ii) above consists of the amino acid sequence of SEQ ID NO: 6 or an amino acid sequence which has at least 80% sequence identity thereto, preferably at least 85%, 90% or 95% sequence identity thereto.

The detection of the polypeptides in the methods of the invention may be carried out by any suitable method known in the art. Examples of suitable methods include the fractionation of the polypeptides in the sample by one or two dimensional SDS-PAGE, optionally followed by Western blotting with an appropriate antibody; and various chromatographic methods including HPLC and protein identification using mass spectrometric (MS) - based methods [48]. Alternatively, part or all of a purified NPHP4-containing fraction or fragments thereof may be sequenced. Alternatively, the protein truncation test (PTT) may be used [49-50].

Due to the presence of the exon 5 deletion, the mutant NPHP4 polypeptide of SEQ ID NO: 7 is only 155 amino acids in length, as opposed to the wild-type polypeptide (SEQ ID NO: 6) which is 1429 amino acids in length. As a result of this, the mutant NPHP4 will present different epitopes compared to the wild-type polypeptide and hence the two polypeptides may be distinguished on this basis. Similar issues apply to mutated NPHP4 polypeptides from other species.

In yet a further embodiment, the invention provides a method for diagnosing or detecting cone-rod dystrophy (CRD) in a mammal, the method comprising:
(i) detecting whether a first antibody binds to a polypeptide sample obtained from the said mammal, wherein said first antibody is an antibody which binds to a polypeptide whose amino acid sequence consists of the sequence given in SEQ ID NO: 7 but does not bind to a polypeptide whose amino acid sequence consists of the sequence given in SEQ ID NO: 6, and
(ii) detecting whether a second antibody binds to a sample obtained from the said mammalian, wherein said second antibody is an antibody which binds to a polypeptide whose amino acid sequence consists of the sequence given in SEQ ID NO: 6 but does not bind to a polypeptide whose amino acid sequence consists of the sequence given in SEQ ID NO: 7,
wherein the detection of binding of (i) and the absence of binding in (ii) is indicative of the presence of CRD in the mammal, and wherein the detection of binding in (i) and the detection of binding in (ii) is indicative of the mammal being a carrier for CRD.

In yet a further embodiment, the invention provides a method for diagnosing or detecting cone-rod dystrophy (CRD) in a mammal, the method comprising:
(i) detecting whether a first antibody binds to a polypeptide sample obtained from the said mammal, wherein said first antibody is an antibody which binds to a polypeptide whose amino acid sequence consists of the sequence given in SEQ ID NO: 7 or a sequence which has at least 80% sequence identity thereto (preferably at least 85%, 90% or 95% sequence identity thereto), but does not bind to a polypeptide whose amino acid sequence consists of the sequence given in SEQ ID NO: 6 or a sequence which has at least 80% sequence identity thereto (preferably at least 85%, 90% or 95% sequence identity thereto), and
(ii) detecting whether a second antibody binds to a sample obtained from the said mammal, wherein said second antibody is an antibody which binds to a polypeptide whose amino acid sequence consists of the sequence given in SEQ ID NO: 6 or a sequence which has at least 80% sequence identity thereto (preferably at least 85%, 90% or 95% sequence identity thereto), but does not bind to a polypeptide whose amino acid sequence consists of the sequence given in SEQ ID NO: 7 or a sequence which has at least 80% sequence identity thereto(preferably at least 85%, 90% or 95% sequence identity thereto),
wherein the detection of binding of (i) and the absence of binding in (ii) is indicative of the presence of CRD in the mammal, and wherein the detection of binding in (i) and the detection of binding in (ii) is indicative of the mammal being a carrier for CRD.

The protein sample may be taken from any tissue which expresses NPHP4. Many NPHP4-expressing tissues are known (see http://www.ncbi.nlm.nih.gov/UniGene/ESTProfileViewer.cgi?uglist=Hs.4623 48). In particular, the tissue may be retina or lung, particularly preferably a tissue which would be expected to contain NPHP4 polypeptide of SEQ ID NO: 6 and/or SEQ ID NO: 7. The sample will, in general, be a protein sample, which may be purified or fractionated, as desired.

In some embodiments of the invention, the method for diagnosing or detecting cone-rod dystrophy includes the additional step of obtaining a sample from the mammal. The sample will be a sample of nucleic acid, DNA, RNA, polypeptide, etc. which is appropriate for the method in question.

In other embodiments, the method additionally comprises prescribing or administering a medicament to the mammal for the treatment of cone-rod dystrophy.

In the context of the present invention, the term "determining" may mean "obtaining an indication of the likelihood of" or "obtaining an indication of the probability of".

The invention also provides a method for diagnosing or detecting cone-rod dystrophy (CRD) in a mammal, comprising the steps:
(i) sending one or more biological samples which have been obtained from the mammal to a remote location;
(ii) receiving data on the presence or absence of a deletion in all or part of exon 5 of the NPHP4 gene of the mammal,
wherein the data has been obtained by a method of the invention.

In the above context, the biological sample is one which comprises appropriate nucleic acid or polypeptides upon which the methods of the invention can be used. Appropriate sources of nucleic acids and polypeptides are discussed above.

The sample may be sent to the remote location by any suitable means, e.g. by post or by courier.

The data may be received by any suitable means, including electronic means (e.g. by email, text-message, electronic file) and paper means (e.g. a letter or computer print-out).

In some embodiments, the above method additionally comprises the step: (iii) selecting a mammal which has a particular genotype or phenotype on the basis of the data received, and optionally breeding that mammal with a second mammal of the opposite sex by *in vivo* or in *vitro* means.

A further embodiment of the invention relates to an antibody which binds to a polypeptide whose amino acid sequence consists of the sequence given in SEQ ID NO: 7 but does not bind to a polypeptide whose amino acid sequence consists of the sequence given in SEQ ID NO: 6.

A further embodiment of the invention relates to an antibody which binds to a polypeptide whose amino acid sequence consists of the sequence given in SEQ ID NO: 6 but does not bind to a polypeptide whose amino acid sequence consists of the sequence given in SEQ ID NO: 7.

A further embodiment of the invention relates to an antibody which binds to a polypeptide whose amino acid sequence consists of the sequence given in SEQ ID NO: 7 or a sequence having at least 80% sequence identity thereto (preferably at least 85%, 90% or 95% sequence identity thereto) but does not bind to a polypeptide whose amino acid sequence consists of the sequence given in SEQ ID NO: 6 or a sequence having at least 80% sequence identity thereto (preferably at least 85%, 90% or 95% sequence identity thereto).

A further embodiment of the invention relates to an antibody which binds to a polypeptide whose amino acid sequence consists of the sequence given in SEQ ID NO: 6 or a sequence having at least 80% sequence identity thereto (preferably at least 85%, 90% or 95% sequence identity thereto) but does not bind to a polypeptide whose amino acid sequence consists of the sequence given in SEQ ID NO: 7 or a sequence having at least 80% sequence identity thereto (preferably at least 85%, 90% or 95% sequence identity thereto).

The antibodies of the invention may be of any suitable source, for example but not limited to monoclonal, polyclonal, chimeric, bispecific, single-chain or fragments thereof. Antibody fragments include, but are not limited to, Fab, Fab' and F(ab')₂, Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CH1, CH2, and CH3 domains. Also included in the invention are antigen-binding fragments also comprising any combination of variable region(s) with a hinge region, CH1, CH2, and CH3 domains. The antibodies of the invention may be from any animal origin including birds and mammals, or derived from phage or ribosome display libraries. Preferably, the antibodies are human, murine (e.g. mouse or rat), donkey, rabbit, goat, guinea pig, camel, horse, or chicken. The antibodies of the invention may be monospecific, bispecific, trispecific or of greater multispecificity.

The antibodies of the invention may be labelled in any suitable manner, thus allowing for detection in a suitable assay.

Subtraction methods for the isolation of antibodies of the invention which bind to a polypeptide of SEQ ID NO: 7 but not to a polypeptide of SEQ ID NO: 6, or *vice versa,* are well known in the art.

In a further aspect, the invention provides a method for assaying for the presence of a mutated form of NPHP4 polypeptide in a sample, the method comprising:
(i) providing a substrate to which is bound NPHP1 polypeptide;
(ii) contacting said substrate with the sample, and optionally determining the binding of any NPHP4 polypeptide in the sample to the bound NPHP1;
(iii) washing the substrate in order to remove any unbound sample;
(iv) contacting the substrate with labelled RPGRIP1 polypeptide;
(v) washing the substrate; and
(vi) detecting binding of any labelled RPGRIP1 polypeptide to the NPHP4;
wherein the binding of NPHP4 in step (ii) with the absence of binding of labelled RPGRIP 1 polypeptide to the NPHP4 in step (vi) is indicative of the presence of mutated NPHP4 in the sample.

The source of the sample may be any tissue which normally expresses NPHP4, preferably retina or lung.

Preferably, the methods of the invention are carried out *ex vivo* or in *vitro.*

A further embodiment of the invention provides a kit for diagnosing or detecting cone-rod dystrophy (CRD) in a mammal, the kit comprising first and second nucleic acid primers for amplifying the region of nucleic acid which includes exon 5 of the mammalian *NPHP4* gene. Preferably, the primers are ones disclosed in the Figures.

A yet further embodiment of the invention provides a kit for diagnosing or detecting cone-rod dystrophy (CRD) in a mammal, the kit comprising an antibody which binds to a polypeptide of SEQ ID NO: 7 but not to a polypeptide of SEQ ID NO: 6.

A yet further embodiment of the invention provides a kit for diagnosing or detecting cone-rod dystrophy (CRD) in a mammal, the kit comprising an antibody which binds to a polypeptide of SEQ ID NO: 6 but not to a polypeptide of SEQ ID NO: 7.

Also provided is a kit for diagnosing or detecting cone-rod dystrophy (CRD) in a mammal, the kit comprising:
(i) an antibody which binds to a polypeptide whose amino acid sequence consists of the sequence given in SEQ ID NO: 7 or a sequence having at least 80% sequence identity thereto but does not bind to a polypeptide whose amino acid sequence consists of the sequence given in SEQ ID NO: 6 or a sequence having at least 80% sequence identity thereto; and/or
(ii) an antibody which binds to a polypeptide whose amino acid sequence consists of the sequence given in SEQ ID NO: 6 or a sequence having at least 80% sequence identity thereto but does not bind to a polypeptide whose amino acid sequence consists of the sequence given in SEQ ID NO: 7 or a sequence having at least 80% sequence identity thereto.

A further embodiment of the invention relates to a nucleic acid molecule whose nucleotide sequence consists of the sequence given in SEQ ID NO: 3 or 5 or 9, of the complement thereof.

A yet further embodiment of the invention provides a nucleic acid molecule which encodes a polypeptide of SEQ ID NO: 7.

The invention also relates to the aforementioned nucleic acid molecules when present in a vector, preferably an expression vector, and an isolated host cell comprising said vector. Such a nucleic acid molecule may be used to produce the polypeptide of SEQ ID NO: 7, which is useful for producing the antibodies of the invention.

The isolated host cell is preferably a bacterial cell, yeast cell, animal cell or mammalian cell, for example a CHO cell or human cell. In some embodiments of the invention, the host cell is not a human embryonic cell or a human embryonic stem cell.

A further aspect of the invention provides a method for producing vectors or contructs, the method comprising the steps:
i) producing by extraction, amplification or synthesis a wild type or mutated NPHP4-gene sequence for the purpose of gene transfer or research in mammals, most preferably dogs or humans
(ii) determining the *NPHP4* genotype of DNA in a vector or contruct
(iii) selecting a vector or construct containing the wild type or mutated *NPHP4* gene
(iii) transferring the *NPHP4* gene to a mammal artificially, using vector/constructs.

In particular, the invention provides a process for producing a vector, comprising the steps:
(i) obtaining a wild-type or mutant *NPHP4* gene;
(ii) determining the genotype of the *NPHP4* gene using a method as disclosed herein; and
(iii) inserting the *NPHP4* gene into a vector.
Optionally, the vector is transformed, transfected or injected into a mammalian cell.

The deletion in exon 5 of the *NPHP4* gene has been shown herein to be correlated with cone-rod dystrophy. The resulting polyptide has only 155 amino acids (in the canine form of the mutant polypeptide). This mutant polypeptide contains the binding site for Nephrocystin (NPHP1); NPHP 1 interacts with NPHP4 in kidney. However, the mutant polypeptide has lost the binding site for RPGRIP 1; the latter polypeptide interacts with wild-type NPHP4 in retina. The invention therefore provides a mutant NPHP4 polypeptide which binds NPHP1 but does not bind RPGRIP1.

A yet further embodiment of the invention relates to a polypeptide whose amino acid sequence consists of the sequence given in SEQ ID NO: 7 or a sequence having at least 75%, preferably at least 80%, 85%, 90% or 95% sequence identity thereto. Such polypeptides may be used to produce antibodies for use in the methods described herein which require such antibodies.

A yet further embodiment of the invention relates to a polypeptide whose amino acid sequence consists of the sequence given in SEQ ID NO: 8 or a sequence having at least 75% sequence identity thereto, preferably at least 80%, 85%, 90% or 95% sequence identity thereto.

The skilled person will appreciate that an indication of the genotypic/phenotypic status of a particular mammal regarding its susceptibility to cone-rod dystrophy (CRD) may be obtained by testing the parent or progeny of the mammal, instead of the mammal *per se.*

Sequence alignments and percent similarity calculations may be determined using a variety of comparison methods designed to detect homologous sequences including, but not limited to, the Megalign program of the LASARGENE bioinformatics computing suite (DNASTAR Inc., Madison, Wl). Multiple alignment of the sequences are performed using the Clustal method of alignment (Higgins and Sharp (1989) CABIOS. 5:151-153) with the default parameters (GAP PENALTY=10, GAP LENGTH PENALTY=10). Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4.

The methods of the invention may be used to select mammals for specific breeding programmes. In some cases, it may be desirable to identify mammals which are heterozygous for the exon 5 deletion or mammals which are homozygous for the exon 5 deletion and selectively use these mammals in breeding programmes. In other embodiments, it will be desirable to select mammals which are homozygous for not having the exon 5 deletion.

In the following, the first and second mammals will generally be of the same species. In preferred embodiments of the methods of producing a mammal and mammal breeding systems, the mammal is a non-human mammal.

The invention therefore provides a method of producing a mammal, the method comprising the steps: (i) selecting a first mammal whose *NPHP4* genotype has previously been determined using a method of the invention, and (ii) breeding said first mammal with a second mammal of the opposite sex, in order to produce progeny mammals.

Yet another embodiment of the invention provides a method of producing a mammal, the method comprising the steps: (i) determining the *NPHP4* genotype of a group of one or more mammals using a method of the invention, and selecting a first mammal from within this group, and (ii) breeding the first mammal with a second mammal of the opposite sex, in order to produce progeny mammals.

The history of any one mammal and whether its *NPHP4* genotype has previously been determined using a method of the invention will be readily apparent from the farmer's or owner's records on that mammal.

The invention further provides a method for producing a mammal, the method comprising the steps: (i) selecting a male first mammal whose *NPHP4* genotype has previously been determined using a method of the invention, and (ii) inseminating a female second mammal with sperm from the male first mammal, in order to produce progeny mammals.

The invention further provides a method for producing a mammal, the method comprising the steps: (i) determining the *NPHP4* genotype of a group of one or more male mammals using a method of the invention and selecting a male first mammal from within this group, and (ii) inseminating a female second mammal with sperm from the male first mammal, in order to produce progeny mammals.

The invention further provides a method for producing a mammal, the method comprising the steps: (i) selecting a female first mammal whose *NPHP4* genotype has previously been determined using a method of the invention, and (ii) inseminating the female first mammal with sperm from a male second mammal, in order to produce progeny mammal.

The invention further provides a method for producing a mammal, the method comprising the steps: (i) determining the *NPHP4* genotype of a group of one or more female mammals using a method of the invention and selecting a female first mammal from within this group, and (ii) inseminating the female first mammal with sperm from a male second mammal, in order to produce progeny mammals.

In some embodiments of the invention, step (i) additionally comprises the step of selecting the second mammal from within the group.

Preferably, the first and/or second mammal is one which has previously been or is selected as being homozygous or heterozygous for the *NPHP4* wild-type genotype or which has been or is selected as not having the *NPHP4* exon 5 deletion genotype.

In some embodiments, the first mammal is homozygous or heterozygous for the *NPHP4* wild-type genotype. In other embodiments, the second mammal is homozygous or heterozygous for the *NPHP4* wild-type genotype. In yet other embodiments, the first and second mammals are either both homozygous or both heterozygous for the *NPHP4* wild-type genotype.

The invention also provides a method of producing a mammal which is homozygous for the *NPHP4* wild-type genotype, the method comprising the steps: (i) determining the *NPHP4* genotype of a group of two or more mammals using a method as disclosed herein, (ii) selecting a first mammal from the group which is homozygous for the *NPHP4* wild-type genotype, (iii) selecting a second mammal of the opposite sex to the first from the group which is homozygous for the *NPHP4* wild-type genotype, (iv) breeding said first mammal with said second mammal, in order to produce progeny which are homozygous for the *NPHP4* wild-type genotype.

The invention further provides a method of producing a mammal which is homozygous for a *NPHP4* mutant genotype, the method comprising the steps: (i) determining the *NPHP4* genotype of a group of two or more mammals using a method as disclosed herein, (ii) selecting a first mammal from the group which is homozygous for the *NPHP4* mutant genotype, (iii) selecting a second mammal of the opposite sex to the first from the group which is homozygous for the *NPHP4* mutant genotype, (iv) breeding said first mammal with said second mammal, in order to produce progeny which are homozygous for the *NPHP4* mutant genotype.

The invention also relates to mammals (preferably non-human mammals) produced by the above methods and to progeny thereof. The invention particularly relates to germ cells, for example sperm and eggs, from the aforementioned mammals.

The invention further provides a method of producing a zygote, the method comprising the steps: (i) combining germ cells from a first mammal whose *NPHP4* genotype has previously been determined using a method of the invention with germ cells from a second mammal of the opposite sex, and (ii) culturing the combined germ cells under conditions suitable for producing a zygote.

The invention also provides a method of producing a zygote, the method comprising the steps: (i) determining the *NPHP4* genotype of a group of one or more mammals using a method of the invention, and selecting a first mammal from within this group, (ii) combining germ cells from the first mammal with germ cells from a second mammal of the opposite sex, and (iii) culturing the combined germ cells under conditions suitable for producing a zygote.

The invention also provides a method for producing sperm, the method comprising the steps: (i) determining the *NPHP4* genotype of a group of one or more mammals using a method as disclosed herein, (ii) selecting a male mammal from the group, and (iii) obtaining sperm from the male mammal.

Preferably, the obtained sperm is then stored, most preferably under refrigerated conditions. The invention also relates to sperm produced by the above method.

The invention further provides a method for producing eggs, the method comprising the steps: (i) determining the *NPHP4* genotype of a group of one or more mammals using a method as disclosed herein, (ii) selecting a female mammal from the group, and (iii) obtaining one or more eggs from the female mammal.

Preferably, the obtained eggs are then stored, most preferably under refrigerated conditions. The invention also relates to eggs produced by the above method.

In some embodiments of the invention, the selected male mammal will be homozygous for the *NPHP4* wild-type genotype. In other embodiments of the invention, the selected mammal will be homozygous for not having the *NPHP4* exon 5 deletion.

The invention additionally provides a mammal breeding system comprising the steps: (i) maintaining a first population of mammals which are homozygous for the *NPHP4* wild-type genotype and/or a second population which are homozygous for not having the *NPHP4* exon 5 deletion genotype.

Optionally, the *NPHP4* genotype of the first population and/or the second population has previously been determined using a method of the invention.

Alternatively, the mammal breeding system additionally comprises the step: determining the *NPHP4* genotype of one or more mammals in the first population using a method of the invention and/or one or more mammals in the second population using a method of the invention.

Preferably, the mammal breeding system additionally includes the step: (ii) selectively breeding a first mammal from the first population with a second mammal from the second population, wherein the first and second mammal are of the opposite sex; and producing progeny therefrom.

The invention also provides a mammal breeding system comprising the steps: (i) maintaining a first population of mammals which are homozygous for the NPHP4 wild-type genotype and a second population which are heterozygous for a deletion in NPHP4 exon 5 in one allele and wild type NPHP4 in the other allele, and optionally, (ii) selectively breeding a first mammal from the first population with a second mammal from the second population, wherein the first and second mammal are of the opposite sex; and producing progeny therefrom.

The invention also relates to mammals produced by the mammal breeding systems of the invention, and also to germ cells, for example sperm and egg cells, obtained from the mammal.

The invention also relates to processes for producing transgenic mammals (preferably non-human mammals) which are homozygous or heterozygous for not having the *NPHP4* exon 5 deletion. Such mammals may, for example, be produced using nuclear transfer from cultured somatic cells (McCreath et al., Nature (2000), 405, 1066-1069) or by using mammalian embryonic stem cells. The exon 5 deletion may be removed from the mammalian genome by any known means, e.g. homologous recombination using an appropriate vector containing the wild-type exon 5 and adjacent sequences, and optionally a suitable selection marker (e.g. antibiotic resistance). Methods of homologous recombination are well known in the art. Alternatively, appropriate vectors may be used to introduce the wild-type *NPHP4* gene into the genome of the mammal at a location other than the wild-type location.

Mammal somatic cells may be isolated by any appropriate means. Generally such cells are grown in culture and then transformed with the homologous recombination vector. Transformed cells are then selected before the nucleus is transferred to a enucleated mammal egg cell.

A further aspect of the invention therefore relates to a process for producing a modified mammalian cell, comprising the step of introducing a wild-type *NPHP4* gene into the genome of the mammalian cell.

Preferably, the mammalian cell is a somatic mammalian cell or a mammalian embryonic stem cell.

In some embodiments, the mammalian cell is a somatic mammalian cell and the process additionally comprises the step of transferring the nucleus of the somatic mammalian cell to a mammalian enucleated egg cell. In embodiments wherein the mammalian cell is a somatic cell, it is preferably a fibroblast. The invention also includes mammals made using the above process.

The invention also relates to a mammal which is homozygous or heterozygous for a wild-type *NPHP4* gene or non-exon 5 deleted *NPHP4* gene or a functional *NPHP4* gene, wherein the mammal additionally comprise a selective marker in its genome adjacent to the *NPHP4* gene. Preferably, the selective marker is an antibiotic resistance marker.

A further aspect of the invention relates to a process of transferring the wild type *NPHP4* gene to an affected patient in an attempt to cure the disease or in an attempt to learn more about gene therapy for eye disease in general or specifically retinal eye diseas, using dogs with the NPHP4 mutation described herein.

A number of methods would be known for the skilled person in the field, and would often include the use of different vectors, first of all viral vectors. In some embodiments, the process could be performed by injecting wild type versions of the gene, or trying to introduce genes that express the wild-type protein (and maintain the expression of the gene in a long period) in the affected cells.

The process could be performed in mammals, after typing and detecting mutations in the NPHP4 gene, preferably in the first part of the gene that interacts with RPGRIP1, or more preferably with individuals with mutations in exon 5. This could be performed in any mammals, but most preferable in dogs and humans.

The invention therefore provides a method of treating cone rod dystrophy in a patient, comprising administering to the patient a vector comprising a wild-type *NPHP4* gene. Preferably, the vector is administered directly or indirectly to the cells which are affected by cone-rod dystrophy, particularly preferably to the eye or the retina of the patient. Preferably, the vector comprises the *NPHP4* gene in a form which facilitates the transfer of the *NPHP4* gene to affected cells. Most preferably, the *NPHP4* gene is stably transferred to the genome of the affected cells, at a position which allows expression of the *NPHP4* gene.

A number of single nucleotide polymorphisms (SNPs) and microsatellite markers which are found in the region of the *NPHP4* gene are disclosed herein. Individual SNPs/markers disclosed herein or combinations of SNPs/markers (e.g. haplotypes) disclosed herein form part of the invention and can be used as tools for the identification of individual mammals. For example, the haplotype of an individual mammal which has particular value in a breeding programme can be used for the identification of that individual and/or for the identification of the offspring of that individual.

The invention therefore provides the use of one or more SNPs or microsatellite markers as disclosed herein for the identification of a mammal or family of mammals, or offspring thereof.

The invention also provides the use of one or more SNPs or microsatellite markers as disclosed herein for the identification of mutations in the *NPHP4* gene. Furthermore, the invention provides the use of one or more SNPs or microsatellite markers as disclosed herein for the identification of mutations in a gene associated with eye disorders.

### BRIEF DESCRIPTION OF THE FIGURES

Figure 1. Pedigree of the dachshund family segregating crd that was used for genome-wide association. Discordant sibs of half sibs, marked *, were genotyped on the canine SNP-array and sibTDT genome-wide association was performed. Microsatellite markers in the associated CFA5 region was used for identification of recombinant breakpoints and haplotypes are shown for each dog.
Figure 2. Primers used for amplifying all microsatellite markers used.
Figure 3. Primers used in amplifying the exons of the DAB1, AJAP1 and VAMP3 candidate genes.
Figure 4. Primers used for amplifying all NPHP4 exons.
Figure 5. Genome-wide association mapping identified a locus on CFA5.
   5A. The best association was identified by sibTDT analysis (pgenomeTDT=0.00060) but case-control association was also significant (PgenomeASSOC = 5.2 x 10-3) using plink [51].
   5B. Comparison of sibTDT and single SNP association statistics across the CFA5 region.
Figure 6. Illustration of the 180bp deletion in exon 5 of the NPHP4 gene on DNA, RNA and protein level. The mutation leads to exon-skipping of exon 5 and a stop codon in exon 6 which truncates the affected protein at 155aa. The truncated protein contains the nephrocystin interacting domain but not the RPGRIP1 domain.
Figure 7. Location of SNP's in the genomic sequence of NPHP4.
Figure 8. A deletion in exon 5 is visualized on the genomic and transcript level.
   A. PCR products of exon 5 in affected dogs (338bp, lane 1 and 2) carrier dogs (heterozygotes, lanes 3, 4, and 5) and unaffected dog (518bp, lane 6).
   B.PCR amplification of cDNA of one affected (329bp, lane 1) and one healthy dog (392bp, lane 2) using primers localized in exon 3 and exon 6.
   C. Alignment of sequenced PCR product of mutant and wildtype cDNA and D. DNA.
   E. Testing for the mutation in human exon 5 showing a normal PCR-product from a wild type individual.

### BRIEF DESCRIPTION OF THE SEQUENCES

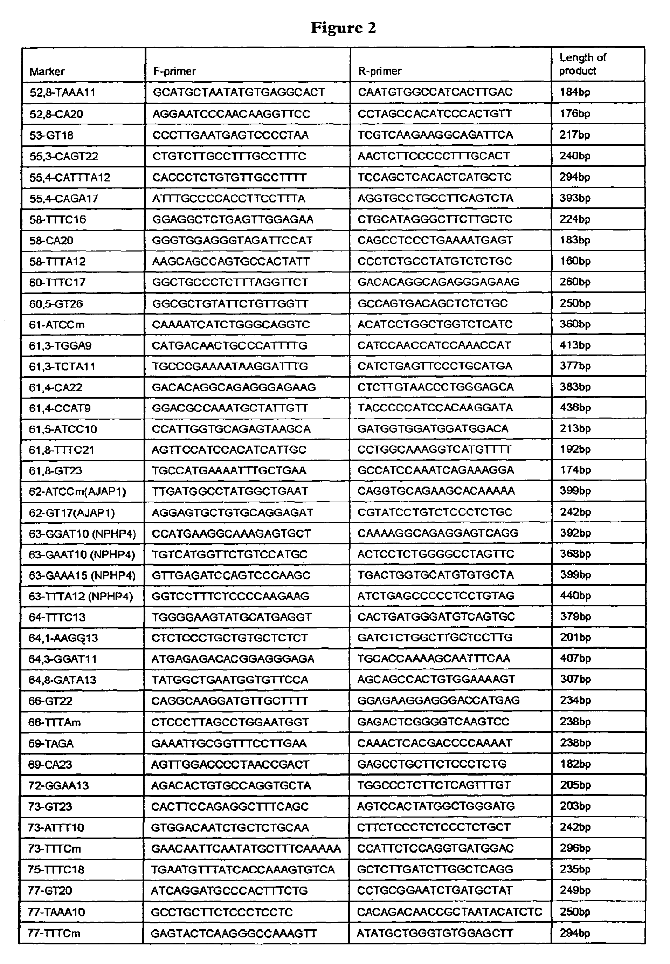

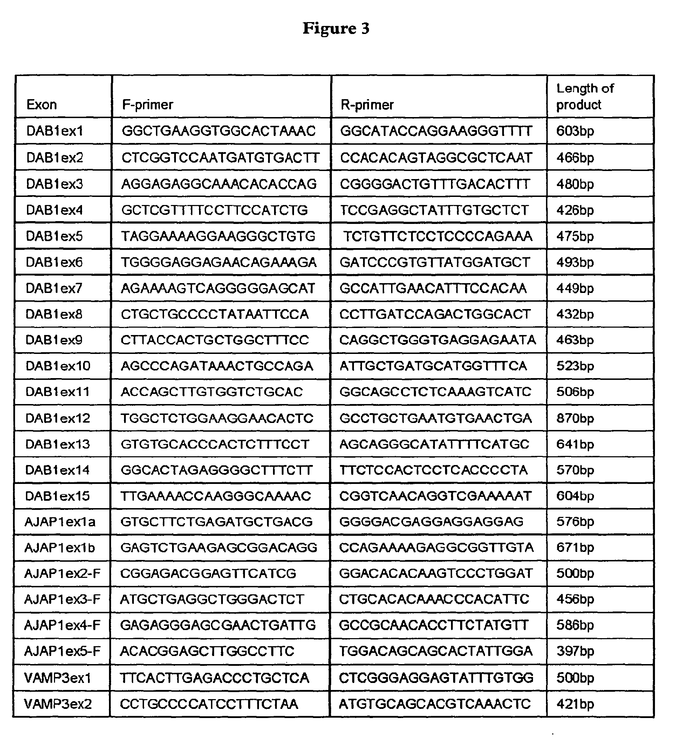

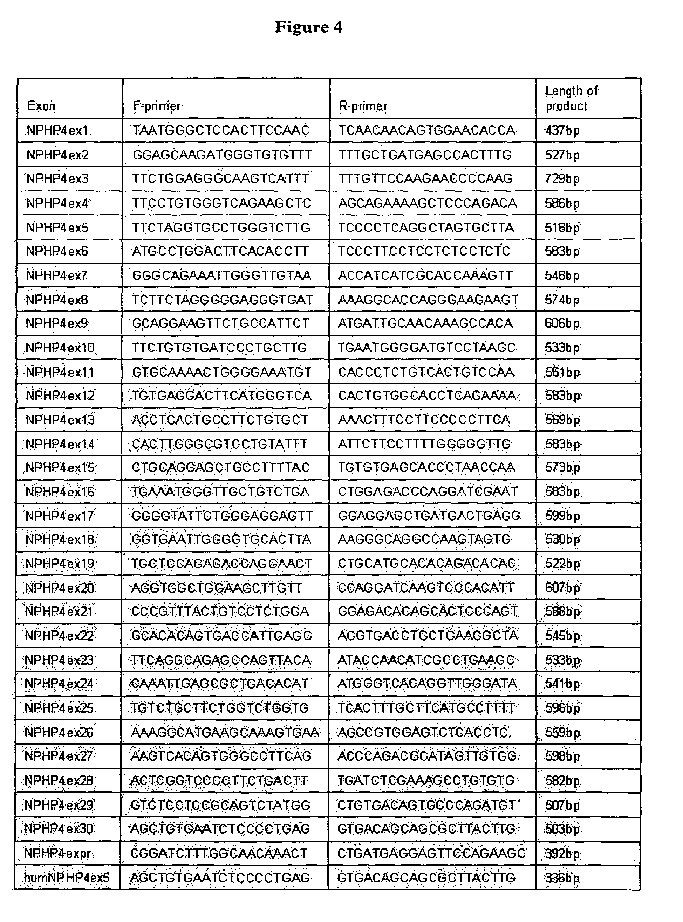

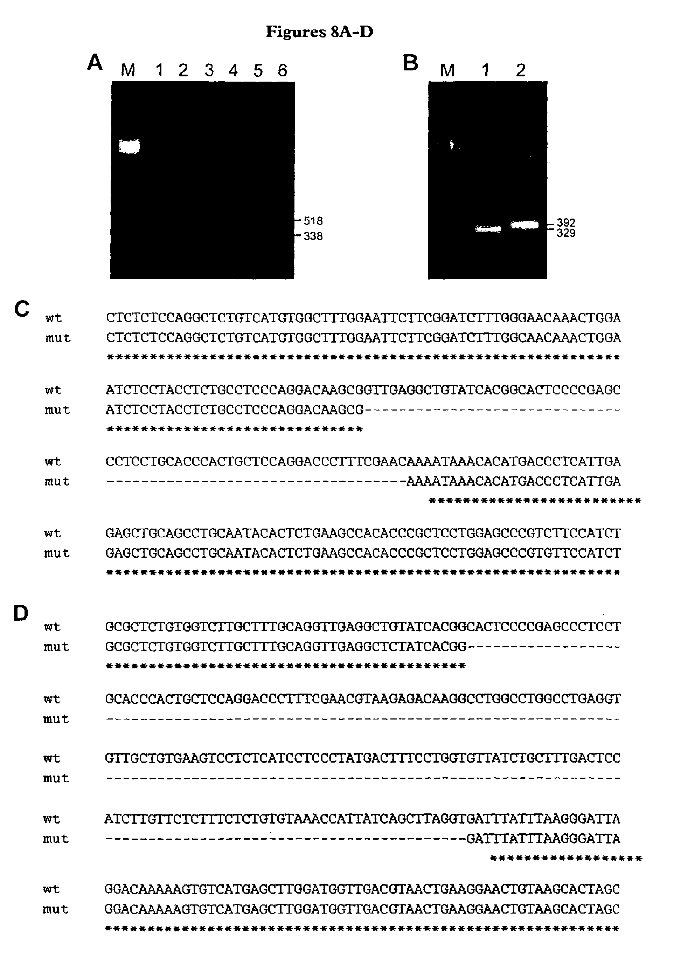

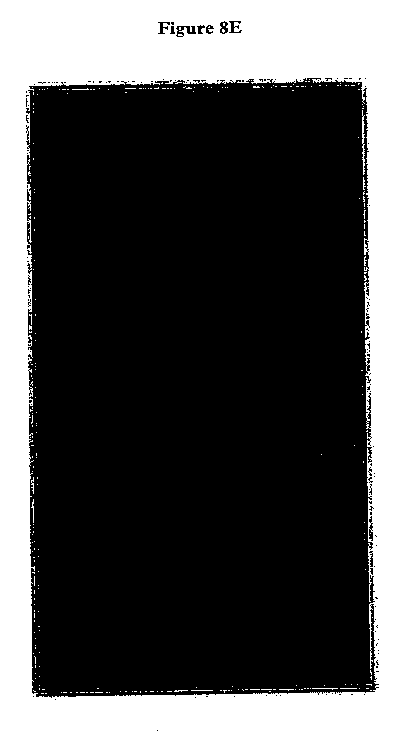

SEQ ID NO: 1 is the genomic sequence of the wild-type *Canis familiaris NPHP4* gene. (5 dna:chromosome
   chromosome:BROADD2:5:62820170:62935638:1)
SEQ ID NO: 2 is part of the genomic sequence of *Canis familiaris NPHP4* gene, from and including exon 4 to exon 6.
SEQ ID NO: 3 is the genomic sequence of the mutant *Canis familiaris NPHP4* gene, with the 180 nucleotide deletion starting from and including nucleotide 19 of exon 5. In the "NNNN" sequence, N can be any of the nucleotides A,C,G or T. (In this sequence, all the NNNN-nucleotide stretches are in the introns). (5 dna:chromosome
   chromosome:BROADD2:5:62820170:62935638:1)
SEQ ID NO: 4 is the cDNA sequence of the wild-type *Canis familiaris NPHP4* gene.
SEQ ID NO: 5 is the cDNA sequence of the mutant *Canis familiaris NPHP4* gene with the deletion of exon 5.
SEQ ID NO: 6 is the full amino acid sequence of the wild-type *Canis familiaris NPHP4* gene.
SEQ ID NO: 7 is the full amino acid sequence of the mutant *Canis familiaris* NPHP4 polypeptide which results from the deletion of exon 5.
SEQ ID NO: 8 is the amino acid sequence encoded by wild-type *NPHP4* exon 5 (only).
SEQ ID NO: 9 is a fragment of SEQ ID NO: 3 which spans, but excludes, the exon 5 deletion. The contiguous sequence is present in mutant *Canis familiaris* genomic DNA.
SEQ ID NO: 10 is the nucleotide sequence which encodes the amino acid sequence of SEQ ID NO: 6.
SEQ ID NO: 11 is the nucleotide sequence which encodes the amino acid sequence of SEQ ID NO: 7.
SEQ ID NO: 12 is the nucleotide sequence which encodes the amino acid sequence of SEQ ID NO: 8.
SEQ ID NOs: 13-94 are the primer sequences given in Figure 2.
SEQ ID NOs: 95-140 are the primer sequences given in Figure 3.
SEQ ID NOs: 141-204 are the primer sequences given in Figure 4.
SEQ ID NOs: 205-208 are the sequences given in Figure 8.
SEQ ID NO: 209 shows exon 5 and the start of intron 5-6 in the mutant canine *NPHP4* gene, as disclosed in the description.

### SEQUENCE LISTING FREE TEXT

SEQ ID NO: 3 <223> 180 nucleotide deletion after nucleotide 93419.
SEQ ID NO: 9 <223> Exon 5 deletion is after nucleotide 132
SEQ ID NOs: 13-94 <223> PCR primer
SEQ ID NOs: 94-124 <223> Primer used in amplifying exon of DAB1 gene
SEQ ID NOs: 125-136 <223> Primer used in amplifying exon of AJAP1 gene
SEQ ID NOs: 137-140 <223> Primer used in amplifying exon of VAMP3 gene
SEQ ID NOs: 141-204 <223> Primer used in amplifying exon of NPHP4 gene
The invention will now be illustrated further by the following non-limiting Examples. Other features and advantages of the present invention will become apparent from the above detailed description. It should be understood, however, that the above detailed description and the following specific examples, while indicating preferred embodiments of the invention, are given by way of illustration only, since various changes and modifications within the spirit and scope of the invention will become apparent to those skilled in the art from the detailed description.
All documents mentioned herein are incorporated herein by reference.

### EXAMPLES

### Example 1

### Materials and methods

### Animals and diagnosis

Day blindness was diagnosed in a wire haired standard dachshund and a purpose bred family of dogs confirmed that this most likely was a simple recessively inherited phenotype. Detailed clinical descriptions of the affected family have been reported previously [52-53], establishing the disease as an early onset cone-rod dystrophy. All procedures used in this study were in accordance with the Norwegian Animal Research Authority ("Forsoksdyrutvalget") and adhered to the Association for Research in Vision and Opthalmology Statement for the Use of Animals in Opthalmic Vision and Research.

### Samples

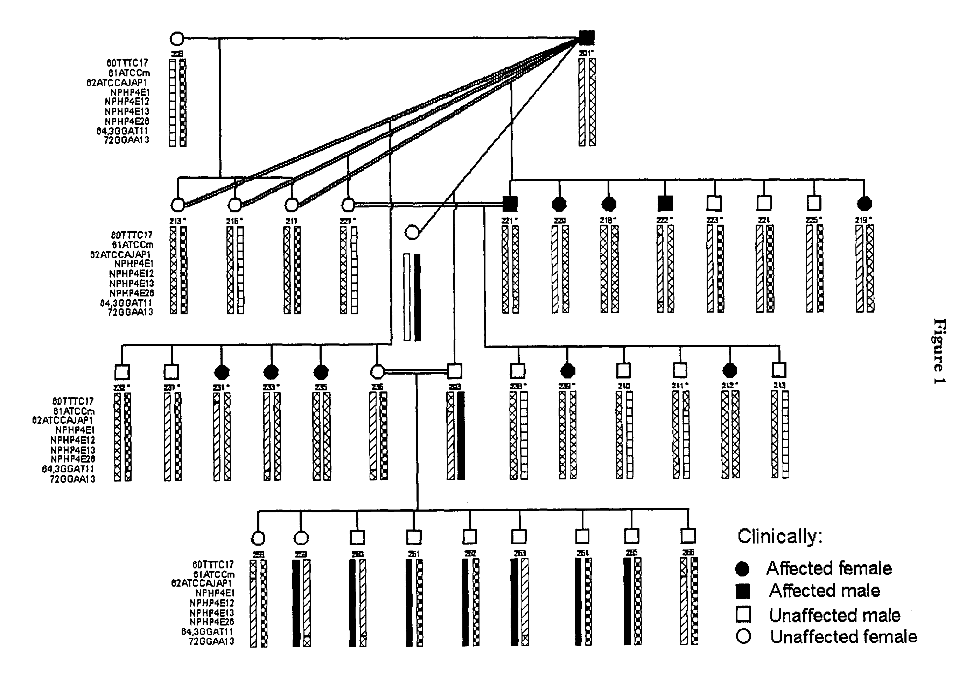

Blood samples were collected in EDTA-coated tubes from all the families segregating the disease and from two non-affected, unrelated dogs. Genomic DNA was isolated from the blood samples using DNeasy Tissue kit (Qiagen, GmbH, Germany). Thirteen affected and thirteen sex- and age matched controls (preferably full sibs) were selected for whole genome SNP analysis (Fig. 1). As some of the dogs were euthanized a number of samples from various tissues (including retina and muscle) were collected immediately post mortem and frozen on liquid nitrogen for RNA analysis. Total RNA was isolated from tissue using RNeasy Fibrous Tissue kit (Qiagen). mRNA for further use in the RACE protocol was isolated from the total RNA using Dynabeads oligodT particles (Invitrogen, Carlsbad, CA, U.S.A.).

### Whole genome association analysis

From the SWHD family 13 pairs of cases and controls were chosen. Where possible, mis-matched full sibs (n=10) were used in the analysis to equally distribute population stratification effects among cases and controls. Where a full sib pair was not available, mis-matched half-sibs were used (n=2 pairs). The founding sire (case 201) and dam (control 208) were included in the analysis.

Genomewide association analysis was undertaken using the Affymetrix version 2 Dog SNP Array "Platinum Panel" containing 49 663 SNP markers (Affymetrix, Santa Clara, CA, U.S.A.). SNP genotypes were obtained by following the Affymetrix protocol for the human 500K array protocol but with a smaller hybridization volume to allow for the smaller surface area of the canine array as described in [54]. Detailed information on the arrays is available at http://www.broad.mit.edu/mammals/dog/caninearray/.

The Sibling Transmission Disequilibrium Test (sibTDT) was carried out on all 49 663 SNP using the "Dfam" option in the analysis package Plink [51]. Data were filtered to exclude individual calls with BRLMM calling probabilities lower than 0.01, loci with low minor allele frequency (<0.05), loci with low genotyping success rate (failed calls >0.1), and individual dogs with greater than 25 % of genotypes missing. After applying these filters, 24 798 SNPs and 25 dogs (13 cases and 12 controls) remained in the analysis. The mean genotyping rate in the individual SWHDs included in the analysis was 96%. Genome-wide significance was ascertained through permutation of phenotypes (n=10 000 permutations) over all 24 798 SNPs in the analysis (EMP2 in Plink).

Case-control association mapping using Plink was applied to the same data with the same filtering parameters to test the efficacy of case-control association strategies in related samples. A region of association spanning from 57.6 and 72.8Mb was identified using the array analysis.

### Segregation studies

Primers for microsatellite analysis in the region of CFA5 (52.8 and 77Mb), were identified by searching selected sequence windows of approximately 200,000 bp for microsatellite motifs. Tetra- and dinucleotide repeat microsatellites with more than ten and nineteen repeats, respectively, were selected. Primers for amplification of selected microsatellites were designed using primer3 (http://frodo.wi.mit.edu/cgi-bin/primer3/primer3_www.cgi) (Figure 2). One primer in each pair was labelled with fluorescein. For genotyping, PCR was carried out as described above with 28 cycles. The sizes of the alleles were estimated with an automated genotyper (ABI PRISM® 3100 Genetic Analyzer, Applied Biosystems, Foster City, CA, U.S.A.) with software for fragment analysis.

### Mutation screening

Chromosomal locations of the predicted canine orthologs of the candidate genes were determined by using the http://genome.ucsc.edu web site. Primers were designed in introns, using primer3 (Figure 3), at a distance of approximately 100-200 bp from the intron/exon borders, to provide optimal PCR products for bi-directional sequencing. The primers for amplification on each of the canine NPHP4-exons were positioned in introns according to the described human sequence NM_015102 (Figure 4). The exon specific primers for amplification of cDNA were based on the predicted canine sequence XM_546745.

PCR amplification reactions were performed using 83 µM of each primer in a 15 µl reaction containing 1.5 µl DNA prepared as described above, 1x Ammonium PCR buffer containing 1.5 mM MgCl2, 83 µM each of dATP, dCTP, dGTP and dTTP, and 0.4 units Taq DNA polymerase (Ambion, Austin, TX, U.S.A.). After an initial denaturation at 95°C for 2 min and 30 sec, samples were amplified for 34 cycles at 95°C for 30 sec, 58°C for 40 sec and 72°C for 50 sec, followed by a final extension of 72°C for 5 min and 30 sec. All PCR products were bidirectionally sequenced using the PCR primers and the BigDye Terminator v3.1 Cycle Sequencing Kit (Applied Biosystems). Amplification was done in a 10 µl reaction mixture containing 2µl BigDye sequencing buffer, 1.5µl BigDye sequencing enzyme, 1 µl PCR product, 0.5 µl sequencing primer (5 pmol/µl) and 5 µl dH₂O. The sequencing reaction was performed by cycle sequencing with the following protocol: initially 95°C for 1minute then 28 cycles each of 95°C for 10 sec, 50°C for 15 sec and 60°C for 2 min. Sequence reaction products were purified by ammonium acetate precipitation and sequenced on the ABI3100.

### Transcript analysis

One affected family member was compared to an unaffected English cocker spaniel (ECS) at the mRNA level. Total RNA was isolated from 30mg of muscle tissue using RNeasy fibrous tissue kit (Qiagen, GmbH, Germany) following the manufacturers instructions. The RNA was precipitated using standard procedures with ethanol, sodium acetate and glycogen. The RNA was dissolved in 10µl RNase free water and first strand cDNA synthesis was performed using Superscript II RT following the manufacturer's descriptions. The final cDNA product was diluted 1:1 with RNase free water. Exon specific primers were designed based on the predicted canine NPHP4 sequence XM_546745. PCR with these primers were performed as described above with 1µl cDNA as template.

3'-and 5'-RACE was performed to amplify the ends of the NPHP4 transcript in both dogs. RNA was isolated from 30mg muscle tissue as described above, precipitated and dissolved in RNase free water. 5'- and 3'-end RACE was performed with GeneRacer Kit (Invitrogen, Carlsbad, CA, U.S.A.) and Marathon cDNA Amplification Kit (Clontech, Mountain View, CA, U.S.A.), according to the manufacturer's protocols. PCR and nested PCR was performed using Kit specific 5'- and 3'-primers with Reverse end Forward gene specific primers and cloning of PCR products was performed using TOPO cloning kit. The inserts of the clones were amplified with M13 forward and reverse primer and sequenced with T3 and T7 primers.

### Example 2

### Genome-wide association analysis

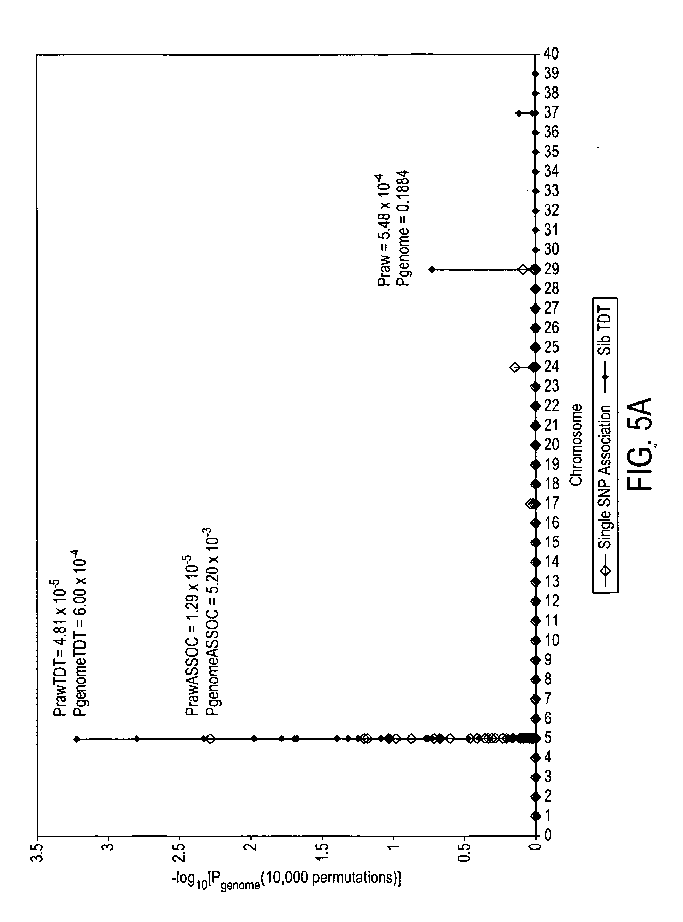

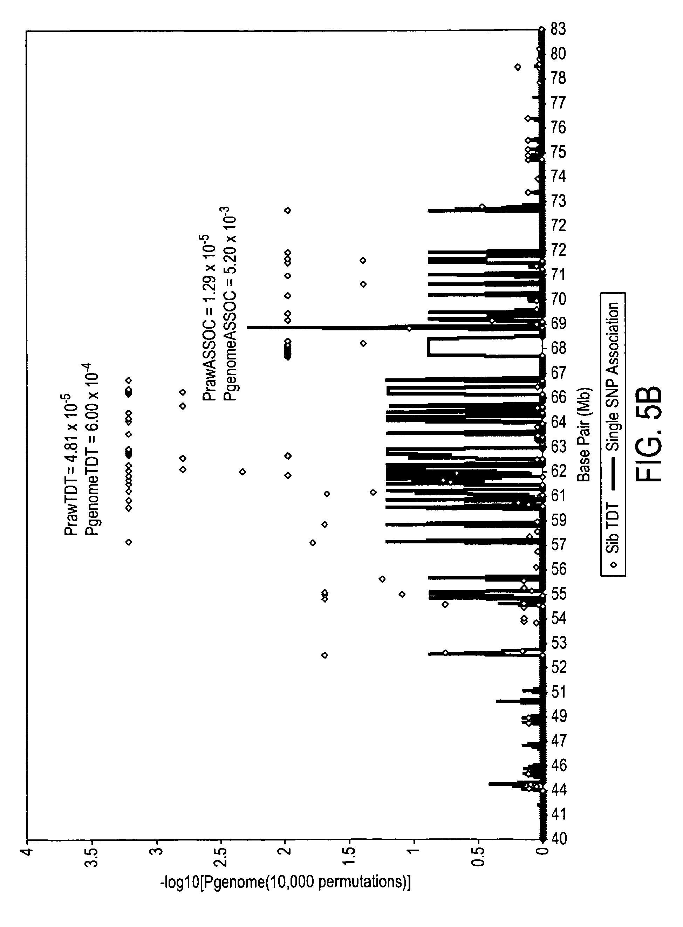

A significant association was detected to CFA5 using sib TDT analysis of ∼50,000 SNPs in 13 cases and 13 controls selected as discordant sib-pairs from a pedigree of standard wire-haired dachshund (Figure 1). All samples were genotyped on the Affymetrix v2 canine SNP array. One control was excluded from further analysis based on low call rate (<75%). Genome-wide significance (prawTDT =4.8e-05 and pgenomeTDT=0.00060) was achieved over the region CFA5: 61,122,335-67,576,960 using a sibTDT analysis as part of the Plink software [51]. Homozygosity was detected in affected cases from 64.82Mb-68.95Mb (Figure 5A and 5B). Case-control association identified the same region, but reported the highest association to a spurious SNP in the "shoulder" of the linkage region (P rawASSOC = 1.3 x 10-5, PgenomeASSOC = 5.2 x 10-3 Figure 2B). In this region, allelic frequencies differed maximally between cases and controls, however one control dog was homozygous for the "affected" allele at the point of association (Chr5:69351798) and the foundation sire (201) was heterozygous in the near vicinity (although not at this SNP).

### Fine-mapping and mutation detection

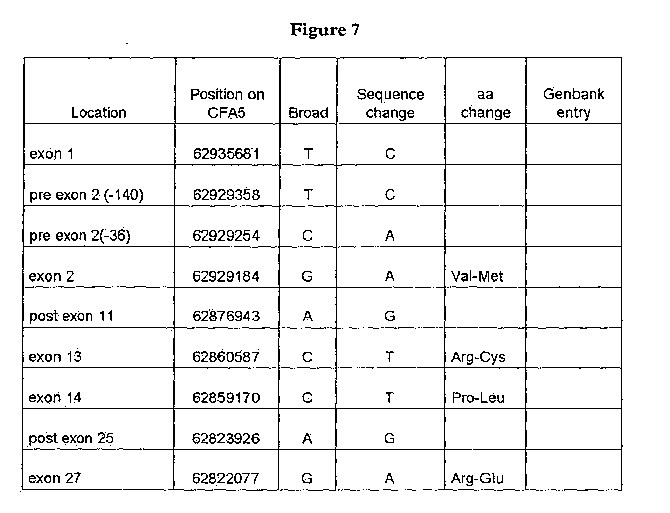

Fine-mapping and haplotype analysis of microsatellites of the region between 52.8Mb to 77.0Mb revealed crossovers at 61.4Mb and 72Mb (Figure 1). This region contains 130 genes that were examined to find possible candidate genes expressed in the retina. DAB1 (55.3Mb, outside region), AJAP1 (62.0Mb), VAMP3 (64.4Mb) and NPHP4 (62.9Mb) were identified as potential candidate genes. Exon sequencing, including exonintron boundaries, showed no mutations in the first three genes. PCR products from exon 5 of NPHP4 showed a different size in unaffected (519bp) and affected (339bp) individuals and with both bands seen in carriers (Figure 8). Exon sequencing showed a deletion of 180bp in exon 5/intron 5 (location 62,913,591-62,913,770) in the affected animal, where only the first 18 bases of exon 5 were retained (Figure 6). Screening of exon 5 in 72 unrelated SWHD revealed 5 carriers, giving a gene frequency of the mutated gene of approximately 4% in the SWHD population. The exon screening also revealed 4 SNPs within exons and 4 SNPs were found in intronic sequence close to exons. All of the expressed SNPs give rise to amino acid changes, but all are present as polymorphisms in multiple breeds and are not associated to disease within the dachshunds (Figure 7). No mutations were found in the exon/intron junctions.

PCR products from human exon 5 of NPHP4 using the primers in Figure 4 ("humNPHP4ex5"-F -primer and R-primer) show the testing for the presence of a normal PCR product in a human. This shows that the method of the invention is easily adapted to the testing of human sampled (Figure 8C).

### Transcript analysis

Comparison of cDNA from affected and unaffected dogs, revealed complete exon-skipping of exon 5 in the affected animals. Translation of the 4819 bp long cDNA sequence of the healthy dogs gives a 1429 amino acid (AA) protein, while translation of the cDNA of the affected dog contains an early stop codon in exon 6 resulting in a short protein of 155 AA (Figure 8). The RACE procedure of the 3'- and 5'-ends gave a complete mRNA sequence, with an extra untranslated exon 1 of 73bp located at 6312bp upstream (CanFam2.0 62,935,566 - 62,935,638) of exon 2. In addition exon 2 was found to have a 5'-UTR of 35 bp and exon 29 a 3'-UTR of 420bp (CamFam2.0 62,819,750-62,820,169). The amplification of the transcript ends revealed no differences between the affected and the non-affected dogs. The amplified cDNA also revealed a difference with the previously predicted transcript XM_546745. The cDNA sequence, amplified in this study, contains an additional CAG-triplet in front of exon 20 in all dogs.

### REFERENCES

[1] N.Udar, et al., Identification of GUCY2D gene mutations in CORD5 families and evidence of incomplete penetrance, Hum. Mutat. 21 (2003) 170-171.
[2] M.Michaelides, et al., A detailed study of the phenotype of an autosomal dominant cone-rod dystrophy (CORD7) associated with mutation in the gene for RIM1, Br. J Ophthalmol. 89 (2005) 198-206.
[3] N.A.Adams, A.Awadein, H.S.Toma, The retinal ciliopathies, Ophthalmic Genet. 28 (2007) 113-125.
[4] R.Allikmets, et al., A photoreceptor cell-specific ATP-binding transporter gene (ABCR) is mutated in recessive Stargardt macular dystrophy, Nat. Genet. 15 (1997) 236-246.
[5] R.Allikmets, et al., Mutation of the Stargardt disease gene (ABCR) in age-related macular degeneration, Science 277 (1997) 1805-1807.
[6] Mollet G, Silbermann F, Delous M, Salomon R, Antignac C, Saunier S. Characterization of the nephrocystin/nephrocystin-4 complex and subcellular localization of nephrocystin-4 to primary cilia and centrosomes. Hum Mol Genet. 2005 Mar 1;14(5):645-56.
[7] M.Ismail, A.Abid, K.Anwar, M.S.Qasim, S.Khaliq, Refinement of the locus for autosomal recessive cone-rod dystrophy (CORD8) linked to chromosome 1q23-q24 in a Pakistani family and exclusion of candidate genes, J Hum. Genet. 51 (2006) 827-831.
[8] S.Khaliq, et al., Novel locus for autosomal recessive cone-rod dystrophy CORD8 mapping to chromosome 1q12-Q24, Invest Ophthalmol. Vis. Sci 41 (2000) 3709-3712.
[9] M.Danciger, et al., CORD9 a new locus for arCRD: mapping to 8p11, estimation of frequency, evaluation of a candidate gene, Invest Ophthalmol. Vis. Sci 42 (2001) 2458-2465.
[10] A.Hameed, et al., Evidence of RPGRIP1 gene mutations associated with recessive cone-rod dystrophy, J Med. Genet. 40 (2003) 616-619.
[11] H.H.Arts, et al., Mutations in the gene encoding the basal body protein RPGRIP1L, a nephrocystin-4 interactor, cause Joubert syndrome, Nat. Genet. 39 (2007) 882-888.
[12] E.M.Valente, et al., Mutations in CEP290, which encodes a centrosomal protein, cause pleiotropic forms of Joubert syndrome, Nat. Genet. 38 (2006) 623-625.
[13] F.Hildebrandt, P.Jungers, C.Robino, J.-P.Grunfeld, Nephronophthisis, medullary cystic kidney disease and medullary spong kidney disease., in: R.Schrier (Ed.), Diseases of the kidney and urinary tract., Lippincott Williams & Wilkins., Philadelphia, 2001.
[14] G.Caridi, et al., Renal-retinal syndromes: association of retinal anomalies and recessive nephronophthisis in patients with homozygous deletion of the NPH1 locus, Am. J. Kidney Dis. 32 (1998) 1059-1062.
[15] J.F.O'Toole, E.A.Otto, J.Hoefele, J.Helou, F.Hildebrandt, Mutational analysis in 119 families with nephronophthisis, Pediatr. Nephrol. 22 (2007) 366-370.
[16] H.Olbrich, et al., Mutations in a novel gene, NPHP3, cause adolescent nephronophthisis, tapeto-retinal degeneration and hepatic fibrosis, Nat. Genet. 34 (2003) 455-459.
[17] E.Otto, et al., A gene mutated in nephronophthisis and retinitis pigmentosa encodes a novel protein, nephroretinin, conserved in evolution, Am. J. Hum. Genet. 71 (2002) 1161-1167.
[18] E.A.Otto, et al., Nephrocystin-5, a ciliary IQ domain protein, is mutated in Senior-Loken syndrome and interacts with RPGR and calmodulin, Nat. Genet. 37 (2005) 282-288.
[19] J.A.Sayer, et al., The centrosomal protein nephrocystin-6 is mutated in Joubert syndrome and activates transcription factor ATF4, Nat. Genet. 38 (2006) 674-681.
[20] R.Roepman, et al., Interaction of nephrocystin-4 and RPGRIP1 is disrupted by nephronophthisis or Leber congenital amaurosis-associated mutations, Proc. Natl. Acad. Sci. U. S. A 102 (2005) 18520-18525.
[21] J.W.Kijas, et al., Cloning of the canine ABCA4 gene and evaluation in canine cone-rod dystrophies and progressive retinal atrophies, Mol. Vis. 10 (2004) 223-232.
[22] C.S.Mellersh, et al., Canine RPGRIP1 mutation establishes cone-rod dystrophy in miniature longhaired dachshunds as a homologue of human Leber congenital amaurosis, Genomics 88 (2006) 293-301.
[23] E.O.Ropstad, E.Bjerkas, K.Narfstrom, Electroretinographic findings in the Standard Wire Haired Dachshund with inherited early onset cone-rod dystrophy, Doc. Ophthalmol. 114 (2007) 27-36.
[24] G.D.Aguirre, G.M.Acland, Models, mutants and man: searching for uniquephenotypes and genes in the dog model of inherited retinal degeneration., in: E.A.Ostrander, U.Giger, K.Lindblad-Toh (Eds.), The dog and Its Genome., Cold Spring Harbor Laboratory Press, Cold Spring Harbor, NY., 2006, pp. 291-325.
[25] G.D.Aguirre, et al., Congenital stationary night blindness in the dog: common mutation in the RPE65 gene indicates founder effect, Mol. Vis. 4 (1998) 23.
[26] G.Dekomien, M.Runte, R.Godde, J.T.Epplen, Generalized progressive retinal atrophy of Sloughi dogs is due to an 8-bp insertion in exon 21 of the PDE6B gene, Cytogenet. Cell Genet. 90 (2000) 261-267.
[27] K.E.Guziewicz, et al., Bestrophin gene mutations cause canine multifocal retinopathy: a novel animal model for best disease, Invest Ophthalmol. Vis. Sci. 48 (2007) 1959-1967.
[28] J.W.Kijas, et al., Naturally occurring rhodopsin mutation in the dog causes retinal dysfunction and degeneration mimicking human dominant retinitis pigmentosa, Proc. Natl. Acad. Sci. U. S. A 99 (2002) 6328-6333.
[29] C.S.Mellersh, L.Pettitt, O.P.Forman, M.Vaudin, K.C.Barnett, Identification of mutations in HSF4 in dogs of three different breeds with hereditary cataracts, Vet. Ophthalmol. 9 (2006) 369-378.
[30] S.M.Petersen-Jones, D.D.Entz, D.R.Sargan, cGMP phosphodiesterase-alpha mutation causes progressive retinal atrophy in the Cardigan Welsh corgi dog, Invest Ophthalmol. Vis. Sci. 40 (1999) 1637-1644.
[31] D.J.Sidjanin, et al., Canine CNGB3 mutations establish cone degeneration as orthologous to the human achromatopsia locus ACHM3, Hum. Mol. Genet. 11 (2002) 1823-1833.
[32] M.L.Suber, et al., Irish setter dogs affected with rod/cone dysplasia contain a nonsense mutation in the rod cGMP phosphodiesterase beta-subunit gene, Proc. Natl. Acad. Sci. U. S. A 90 (1993) 3968-3972.
[33] B.Zangerl, et al., Identical mutation in a novel retinal gene causes progressive rod-cone degeneration in dogs and retinitis pigmentosa in humans, Genomics 88 (2006) 551-563.
[34] Q.Zhang, et al., Different RPGR exon ORF15 mutations in Canids provide insights into photoreceptor cell degeneration, Hum. Mol. Genet. 11 (2002) 993-1003.
[35] Sambrook, J. Fritsch, E. F., and Maniatis, T. (1989) "Molecular Cloning: A Laboratory Manual", Cold Spring Harbor Laboratory Press, Cold Spring Harbor, N.Y.).
[36] Maxam and Gilbert (Proc. Natl Acad Sci USA (1977) 74:560)
[37] Sanger et al. (1977) Proc. Nat. Acad. Sci 74:5463.
[38] Conner, B. J., Reyes, A. A., Morin, C., Itakura, K., Teplitz, R. L. & Wallace, R. B. (1983) Proc. Natl. Acad. Sci. USA 80, 278-282.
[39] Meyers, R. M., Maniatis, T. & Lerman, L. S. (1987) Methods Enzymol. 155, 501-527.
[40] Cotton, R. G. H., Rodriques, N. R. & Campbell, R. D. (1988) Proc. Natl. Acad. Sci. USA 85, 4397-4401.
[41] Orita, M., Iwahana, H., Kanazawa, H., Hayashi, H. & Sekiya, T. (1989) Proc. Natl. Acad. Sci. USA 86, 2766-2770.
[42] Chehab, F. F. & Kan, Y. W. (1989) Proc. Natl. Acad. Sci. USA 86, 9178-9182.
[43] Newton, C. R., Graham, A., Heptinstall, L. C., Powell, S. J., Summers, C., Kalsheker, N., Smith, J. C. & Markham, A. F. (1989) Nucleic Acids Res. 17, 2503-2516.
[44] Wu, D. Y. & Wallace, R. B. (1989) Gene 76, 245-254.
[45] Landegren, U., Kaiser, R., Sanders, J. & Hood, L. (1988) Science 241, 1077-1080.
[46] Alves, A. M. & Carr, F. J. (1988) Nucleic Acids Res. 16, 8723.
[47] Wu, D. Y., Ugozzoli, L., Pal, B. K. & Wallace, R. B. (1989) Proc. NatI.Acad. Sci. USA 86, 2757-2760.
[48] Rappsilber and Mann, Trends Biochem Sci. (2002) Feb;27(2):74-8.
[49] Roest, et. al., (1993) Hum. Mol. Genet. 2:1719-2 1;
[50] Van der Luijt, et. al., (1994) Genomics 20:1-4
[51] Purcell, S., Neale, B., Todd-Brown, K., Thomas, L., Ferreira, M.A., Bender, D., Maller, J., Sklar, P., de Bakker, P.I., Daly, M.J., et al. 2007. PLINK: a tool set for whole-genome association and population-based linkage analyses. Am.J.Hum.Genet. 81: 559-575.
[52] Ropstad, E.O., Bjerkas, E., and Narfstrom, K. 2007b. Clinical findings in early onset cone-rod dystrophy in the Standard Wire-haired Dachshund. Vet.Ophthalmol. 10: 69-75.
[53] Ropstad, E.O., Bjerkas, E., and Narfstrom, K. 2007a. Electroretinographic findings in the Standard Wire Haired Dachshund with inherited early onset cone-rod dystrophy. Doc.Ophthalmol. 114: 27-36.
[54] Karlsson, E.K., Baranowska, I., Wade, C.M., Salmon Hillbertz, N.H., Zody, M.C., Anderson, N., Biagi, T.M., Patterson, N., Pielberg, G.R., Kulbokas, E.J., III, et al. 2007. Efficient mapping of mendelian traits in dogs through genome-wide association. Nat.Genet. 39: 1321-1328.

## Claims

1. A method for diagnosing or detecting cone-rod dystrophy (CRD) in a mammal, the method comprising determining the presence or absence in a sample of nucleic acid that has been obtained from the mammal of:
(i) a deletion in a first allele of the *NPHP4* gene, the deletion comprising all or part of exon 5, and
(ii) a wild-type exon 5 in a second allele of the *NPHP4* gene,
wherein the presence of (i) and the absence of (ii) is indicative of the presence of CRD in the mammal, and wherein the presence of (i) and the presence of (ii) is indicative of the mammal being a carrier for CRD.

2. A method as claimed in claim 1:
(A) wherein the deletion in exon 5 in (i) is one which results in the production, upon translation of the *NPHP4* gene, of a polypeptide which is truncated at the C-terminal end compared to a wild-type NPHP4 polypeptide;
and/or
(B) wherein the deletion in exon 5 in (i) is one which results in the production, upon translation of the *NPHP4* gene, of a NPHP4 polypeptide which does not bind to RPGRIP1;
and/or
(C) wherein the deletion in exon 5 in (i) is one which results in the production, upon translation of the *NPHP4* gene, of a NPHP4 polypeptide which does not bind to RPGRIP1 but does bind to NPHP1;
and/or
(D) wherein the deletion of all or a part of exon 5 is a deletion which results, during transcription into mRNA, in the skipping of exon 5;
and/or
(E) wherein the deletion of part of exon 5 is one which results in the deletion of 1-65, 1-60, 1-50, 1-40, 1-30, 1-20 or 1-10 nucleotides from exon 5.

3. A method for diagnosing or detecting cone-rod dystrophy (CRD) in a mammal, the method comprising:
(A) determining the presence or absence in a sample of DNA that has been obtained from the mammal of:
(i) a deletion in a first allele of the *NPHP4* gene, the deletion starting at and including nucleotide 19 of exon 5 and extending in a 3' direction into intron 5,
(ii) a wild-type exon 5 in a second allele of the *NPHP4* gene,
wherein the presence of (i) and the absence of (ii) is indicative of the presence of CRD in the mammal, and wherein the presence of (i) and the presence of (ii) is indicative of the mammal being a carrier for CRD:
or
(B) determining the presence or absence in a sample of RNA that has been obtained from the mammal or of cDNA obtained therefrom of:
(i) a deletion in the *NPHP4* gene, the deletion consisting of all of exon 5,
(ii) a wild-type exon 5 in the *NPHP4* gene,
wherein the presence of (i) and the absence of (ii) is indicative of the presence of CRD in the mammal, and wherein the presence of (i) and the presence of (ii) is indicative of the mammal being a carrier for CRD.

4. A method for diagnosing or detecting cone-rod dystrophy (CRD) in a mammal, the method comprising determining the presence or absence in a sample of nucleic acid that has been obtained from the mammal of:
(i) a nucleic acid molecule coding for a mutant NPHP4 polypeptide whose amino acid sequence consists of the amino acid sequence given in SEQ ID NO: 7 or an amino acid sequence which has at least 70% sequence identity thereto, and
(ii) a nucleic acid molecule coding for a NPHP4 polypeptide whose amino acid sequence comprises SEQ ID NO: 8 or whose amino acid sequence comprises an amino acid sequence which has at least 70% sequence identity to SEQ ID NO: 8,
wherein the presence of (i) and the absence of (ii) is indicative of the presence of CRD in the mammal, and wherein the presence of (i) and the presence of (ii) is indicative of the mammal being a carrier for CRD,
preferably
(a) wherein the nucleic acid molecule encoding (i) comprises the nucleotide sequence given in SEQ ID NO: 3 or 5 or 9, or comprises a nucleotide sequence which has at least 70% sequence identity to SEQ ID NO: 9, or
(b) wherein the NPHP4 polypeptide in step (ii) is a polypeptide whose amino acid sequence consists of the amino acid sequence given in SEQ ID NO: 6 or an amino acid sequence which has at least 70% sequence identity thereto and which also encodes a mammalian NPHP4 polypeptide, or
(c) wherein the nucleic acid molecule of step (ii) comprises the nucleotide sequence given in SEQ ID NO: 1 or 2, or comprises a nucleotide sequence which has at least 95% sequence identity to SEQ ID NO: 2, or comprises the nucleotide sequence given in SEQ ID NO: 4 or comprises a nucleotide sequence which has at least 75% sequence identity to SEQ ID NO: 4.

5. A method for diagnosing or detecting cone-rod dystrophy (CRD) in a mammal or for determining the likelihood of a mammal possessing a CRD genotype, the method comprising:
(A) determining the presence or absence in a sample of nucleic acid which has been obtained from the mammal of:
(i) a polymorphic site which co-segregates with a deletion in the *NPHP4* gene, the deletion comprising all or part of exon 5, and
(ii) a polymorphic site which co-segregates with the wild-type exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD;
or
(B) determining the presence or absence in a sample of nucleic acid that has been obtained from the mammal of:
(i) a polymorphic site which co-segregates with a deletion in the *NPHP4* gene, the deletion comprising all or part of exon 5, and
(ii) the wild-type sequence of exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD;
or
(C) determining the presence or absence in a sample of nucleic acid that has been obtained from the mammal of:
(i) a deletion in the *NPHP4* gene, the deletion comprising all or part of exon 5, and
(ii) a polymorphic site which co-segregates with the wild-type exon 5 of the *NPHP4* gene,
wherein the presence of (i) and the absence of (ii) is indicative of the presence of CRD in the mammal, and wherein the presence of (i) and the presence of (ii) is indicative of the mammal being a carrier for CRD;
preferably wherein the polymorphic site is a microsatellite or SNP.

6. A method for testing a nucleic acid molecule, comprising determining the presence or absence of a nucleotide sequence in the nucleic acid molecule which encodes a truncated NPHP4 polypeptide.

7. A method as claimed in any one of the preceding claims, wherein:
(A) the presence or absence of the deletion or polymorphic site is determined by nucleic acid sequencing; microsequencing; allele specific oligonucleotide hybridization; size analysis; hybridization; 5' nuclease digestion; single-stranded conformation polymorphism; allele specific hybridization; primer specific extension; oligonucleotide ligation assay; or restriction enzyme analysis;
and/or
(B) wherein the method additionally comprises the step of amplifying the region of the mammalian *NPHP4* nucleic acid which includes exon 5 of the *NPHP4* gene.

8. A method for diagnosing or detecting cone-rod dystrophy (CRD) in a mammal, the method comprising:
(A) determining the presence or absence in a sample of polypeptide obtained from the mammal of:
(i) a mutant NPHP4 polypeptide whose amino acid sequence consists of the amino acid sequence given in SEQ ID NO: 7 or an amino acid sequence which has at least 70% sequence identity thereto,
and
(ii) a NPHP4 polypeptide whose amino acid sequence comprises SEQ ID NO: 8 or whose amino acid sequence comprises an amino acid sequence which has at least 70% sequence identity to SEQ ID NO: 8,
wherein the presence of (i) and the absence of (ii) is indicative of the presence of CRD in the mammal, and wherein the presence of (i) and the presence of (ii) is indicative of the mammal being a carrier for CRD, preferably wherein the polypeptide sequence of step (ii) above consists of the amino acid sequence of SEQ ID NO: 6 or an amino acid sequence which has at least 80% sequence identity thereto;
or
(B)
(i) detecting whether a first antibody binds to a polypeptide sample obtained from the said mammal, wherein said first antibody is an antibody which binds to a polypeptide whose amino acid sequence consists of the sequence given in SEQ ID NO: 7 but does not bind to a polypeptide whose amino acid sequence consists of the sequence given in SEQ ID NO: 6, and
(ii) detecting whether a second antibody binds to a sample obtained from the said mammalian, wherein said second antibody is an antibody which binds to a polypeptide whose amino acid sequence consists of the sequence given in SEQ ID NO: 6 but does not bind to a polypeptide whose amino acid sequence consists of the sequence given in SEQ ID NO: 7,
wherein the detection of binding of (i) and the absence of binding in (ii) is indicative of the presence of CRD in the mammal, and wherein the detection of binding in (i) and the detection of binding in (ii) is indicative of the mammal being a carrier for CRD;
or
(C)
(i) detecting whether a first antibody binds to a polypeptide sample obtained from the said mammal, wherein said first antibody is an antibody which binds to a polypeptide whose amino acid sequence consists of the sequence given in SEQ ID NO: 7 or a sequence which has at least 80% sequence identity thereto, but does not bind to a polypeptide whose amino acid sequence consists of the sequence given in SEQ ID NO: 6 or a sequence which has at least 80% sequence identity thereto, and
(ii) detecting whether a second antibody binds to a sample obtained from the said mammal, wherein said second antibody is an antibody which binds to a polypeptide whose amino acid sequence consists of the sequence given in SEQ ID NO: 6 or a sequence which has at least 80% sequence identity thereto, but does not bind to a polypeptide whose amino acid sequence consists of the sequence given in SEQ ID NO: 7 or a sequence which has at least 80% sequence identity thereto,
wherein the detection of binding of (i) and the absence of binding in (ii) is indicative of the presence of CRD in the mammal, and wherein the detection of binding in (i) and the detection of binding in (ii) is indicative of the mammal being a carrier for CRD.

9. A method for diagnosing or detecting cone-rod dystrophy (CRD) in a mammal, comprising the steps:
(i) sending one or more biological samples which have been obtained from the mammal to a remote location;
(ii) receiving data on the presence or absence of a deletion in all or part of exon 5 of the NPHP4 gene of the mammal,
wherein the data has been obtained by a method as claimed in any one of claims 1 to 8.

10. A method as claimed in any one of the preceding claims wherein the mammal is a domestic mammal, a farm mammal or a human, preferably wherein the mammal is a dog or a human, and most preferably wherein the dog is a Standard Wirehaired Dachshund (SWHD).

11. An antibody
(A) which binds to a polypeptide whose amino acid sequence consists of the sequence given in SEQ ID NO: 7 or a sequence having at least 80% sequence identity thereto but does not bind to a polypeptide whose amino acid sequence consists of the sequence given in SEQ ID NO: 6 or a sequence having at least 80% sequence identity thereto; or
(B) which binds to a polypeptide whose amino acid sequence consists of the sequence given in SEQ ID NO: 6 or a sequence having at least 80% sequence identity thereto but does not bind to a polypeptide whose amino acid sequence consists of the sequence given in SEQ ID NO: 7 or a sequence having at least 80% sequence identity thereto;
and wherein the antibody preferably comprises a detectable label.

12. A method for assaying for the presence of a mutated form of NPHP4 polypeptide in a sample, the method comprising:
(i) providing a substrate to which is bound NPHP1 polypeptide;
(ii) contacting said substrate with the sample, and optionally determining the binding of any NPHP4 polypeptide in the sample to the bound NPHP1;
(iii) washing the substrate in order to remove any unbound sample;
(iv) contacting the substrate with labelled RPGRIP1 polypeptide;
(v) washing the substrate; and
(vi) detecting binding of any labelled RPGRIP 1 polypeptide to the NPHP4 polypeptide;
wherein the binding of NPHP4 polypeptide in step (ii) with the absence of binding of labelled RPGRIP1 polypeptide to the NPHP4 polypeptide in step (vi) is indicative of the presence of mutated NPHP4 polypeptide in the sample.

13. A kit for diagnosing or detecting cone-rod dystrophy (CRD) in a mammal, the kit comprising:
(A) first and second nucleic acid primers for amplifying the region of nucleic acid which includes exon 5 of the mammalian NPHP4 gene; or
(B) an antibody as claimed in claim 11.

14. A product wherein the product consists of:
(A) a nucleic acid molecule whose nucleotide sequence consists of the sequence given in SEQ ID NO: 3 or 5 or 9, of the complement thereof; or
(B) a nucleic acid molecule which encodes a polypeptide of SEQ ID NO: 7 or 8; or
(C) a vector comprising the nucleic acid molecule of (A) or (B); or
(D) an isolated host cell comprising the nucleic acid molecule of (A) or (B), or the vector of (C); or
(E) a polypeptide whose amino acid sequence consists of the sequence given in SEQ ID NO: 7 or a sequence having at least 75% sequence identity thereto.

15. Use of:
(A) one or more SNPs as defined in Figure 7 for the identification of a mammal or family of mammals, or offspring thereof; or
(B) one or more SNPs as defined in Figure 7 for the identification of mutations in the *NPHP4* gene; or
(C) one or more SNPs as defined in Figure 7 for the identification of mutations in a gene associated with eye disorders; or
(D) one or more microsatellite markers as defined in Figure 2 for the identification of a mammal or family of mammals, or offspring thereof; or
(E) one or more microsatellite markers as defined in Figure 2 for the identification of mutations in the *NPHP4* gene; or
(F) one or more microsatellite markers as defined in Figure 2 for the identification of mutations in a gene associated with eye disorders.


SEQUENCE LISTING

<110>  Norwegian School of Veterinary Science

<120>  Method for identifying progressive cone rod dystrophy in dogs

<130>  489.96926/01

<160>  209

<170>  PatentIn version 3.3

<210>  1
<211>  115469
<212>  DNA
<213>  Canis familiaris


<220>
<221>  misc_feature
<222>  (10684)..(10929)
<223>  n is a, c, g, or t

<400>  1
   ttactggtac acgaccttga cgcagaaggc ttcttcattt ttgtcttcgt ggtcattgat      60
   gtaaatcaga atctcctcct ccccggggct ctgccgaggt gcgaaccaca ggccgatggt     120
   gtaggtctct cctcccccaa tctggaagca agcatcagat cctagcccta ctgcagccct     180
   gctctccatg ccagcatggt caacacatgc aaacccaggc tgacagagca gggtaaaccg     240
   aggctctggc ccccactgct gccaggcacc tgaggttctg aacacaacct ccccgtgtta     300
   caggacacca ctgctctgcc cacgcctcag gggagattca cagctgaaga aacaccttcc     360
   aatgacagac agactgccat ctgctgtact gtcgttaaaa tacttgcaac agagaaaagg     420
   ctaacatgca ggacacttgc gaataaaatc gctgataaac tgcagggtaa attggccacc     480
   agaagccctg tgacagtgcc cagatgtggg ggccctagcc gcatgggcag aactgctgag     540
   agaggggtgg ccgcccctac tcccagtacc accggtgcag atcagcactg ccccctcccg     600
   gggctctgga gacccctgtg cctcggggcc cctgggacat gccgaggctg ggtgtcccac     660
   atgcagtgcg cacaccctgt acgcagcgct tcccgcacct ggaaggagtc ctctctgaac     720
   tgcagcaggt cagggtggtc gctgtacagg tgataggtcc tctgggaggg gtaagggttg     780
   gtgtaggtga tccgcttgtt ggcgcctctg cctttccctg cagccagtgt gatctcgaaa     840
   gcctgtgtgg gcaggggtgt cagccttcct cagcctgctc tgcacgtaca gcccgaccta     900
   tgtctccata gacatgacaa gacacccgcc gagggggctc actgtacagg agcaaccccc     960
   gcccccaccc ccaggccata gactgcggag gagactcggt cacaggacca ggagacagcg    1020
   aggtgacaca gcacccatac cacacacgac ccagcaggtt cccctccaga ccttggagat    1080
   gagaggcggg cggcaggaga ggcacacgag ccatgaggcc accagctggt ggtagtccac    1140
   gtcgaccagg ttgaggtgga tgaaccggct ccctgccctg cggggcctca caccgacatg    1200
   caggtcctgc accccatgtg gtggcagcac gaacacacct ttggggtcag tctttcaaaa    1260
   caacagcaga gaggcgggtc agtatgcagt ccctcatgct gtgcagaggg gttccctgaa    1320
   cacctgcagc cagtgcacga gaatcccaag ggacctcagc tattcagagg cagggggcac 1380
   gtggcctgca ggaagtcaga aggggaccga gtgacaatgc caggtctgaa gccagacgca 1440
   gccacacagt cagggttgca gtgcatctca gtccgcatca ccacccgcat gggcaagtgc 1500
   ccctccatgt ggcaacagtc cccttagcac cagagctgcc tcagcagagg gaaatcttaa 1560
   caggctccag ctgtaagctt cagcaaggag tctgcattgc cctcattacc cagacgcata 1620
   gttgtggcta aattaattcc aaaattctat taatatttac cttccaataa ttctaaaaat 1680
   tggcctatag ggtttatatg tcactcattc catgatggca gatccaataa aaggcagttt 1740
   gaatgaagac tctgccaaga gtcttaaagg agctgtgagc cgatcctcag ccccacactg 1800
   gctgcagcca gtctccacac cgtccgtgcc ccaggtgggg tgaggatggg gagaggccag 1860
   agaacaccca gcgacctacc ttcagctcct gagggtgtga ggtgaaagcc tgcactctcc 1920
   taaccgtccg tgtccctcga aggacaaggg ataggcgtgt ccgctggcct gtgacacagg 1980
   agacgtccac acgctgcaag gagtggaggt agacctgcca tatttggatg ggtgtcgcca 2040
   gccaacgatc cctgtgtagg acagaaaccg tgatcaatgg tggtgaggac agcagcactg 2100
   accaggaaga ggccaagaag gtggccagga caactggggc aggctggagc ccagctgcca 2160
   tcatccctgc agccaccttc cccactctga aggcccactg tgactttgca ccacaacacc 2220
   ctgtgtgagt ggtaatgtgg gcttctacca gcaacaaaac agtcccaaac caggtggtct 2280
   gggacaaagc ctgtggaaag agtgccgtgg aagagtatgt gggttcagag gccacccaga 2340
   cctgagctca caggccccgt cctagctgcc tggtgctgga ggccactggc ccttccctgg 2400
   aaatggggct cgataaccct gcccctcggg cttctccaca cggccagaga ccagaaccga 2460
   gcatggctac acctgcccca atcccagatg tcctattcag aagcccccag cctaccccag 2520
   ttttaaaaga tcctcaaggg ggacacctgg gtggctcagc agttgagtgt gtgcctttgg 2580
   ctcagggagt gatcccaggg ccctgggatc aagttccaca tcagtctccc tgcagggagc 2640
   ctgcttctcc ctctgcctat gtctctgtcc ctctgtgtat ctctcatgaa taaataaata 2700
   aaatctttaa aaaaaaaaaa aaaagaagat cctcaaggga ctggaagagg agacttccca 2760
   gaacaaggta aaggaacagg gttatttcaa ctgagtggga gaggcaagaa ggcgatgtga 2820
   tcaccatctc aggcacggag gatgaagact gccaccctcc ctgtcccctg aacggagcca 2880
   ggcactgctc tggggcagac gccaccccaa gttcctttcc ttggtgggct ctccatgcct 2940
   ctgcaagccg ccgggccact ctgatccgat ggcgtccttt gtgaggcccc tgccagccag 3000
   ccgtggagtc tcacctcccg tggctgaagg ccccacgaca atgtgcccca tcaggaactc 3060
   agcatgagcc caggcggcct ctgggatgct gtggctgcct cccagtcccg cccaccctcc 3120
   agacctccca ggtttgggct cacagactca cgcgtaaacg gcgacaaaga agtctctgct 3180
   ctctgggctt ggaccgctgg ccaccttcag gaacacgtcc cgaggctccc tggggccctg 3240
   gcggacatgg gcgccctgag aacaccacct gcctcccccc caagcccctg gcaggtgaaa 3300
   gcgggcagga actggctccc tccctcccaa caggtgttgc attttcagta ttaggcagaa 3360
   ttcgtttttt tcttcactgc aaaaaagaaa atcattaact tcttgatttc atattgagtt 3420
   caataattat attctgcagg agattttatc cagtttccta accagtcata acataattat 3480
   tgcctttatt ctttacacaa tggcaattaa taagtggctt aattaccact cactttattc 3540
   actttgcttc atgccttttt tttttttttt catttgaatt caggtaatcc aagaactgat 3600
   ctgccagcag aaaagatgaa aactcatgca taaagctccc acaaggatgt caaaatagcc 3660
   ccttagcacc ttagaaagac accacttaac tcaaaagccg ccgtgaagag gcctcaggag 3720
   gccaggggct cagatgacca ggcactgact gggagacgct cggcagaact taccacattc 3780
   tgggtctcgc agatgacgtt ggggtcgcta catcggacat agactggggg ctcctcacca 3840
   ggtcttccca cgggagcacc tgctgcaggg aatatcatag caaacgtctt ctggacaggc 3900
   ccgggccaca tgatgttgct ggcccatcac aggtacccca cagacaccag aagacagcaa 3960
   tgccaccctt cctactgtcc cgcccacgtg ggcactggca ctcccgccct aagccacacc 4020
   cgcaggcact tatccgaaga gagtgaccaa aagtaaagag cggggggggc tatggtgcag 4080
   ggcgccagac acggggacgc tgctgcactc taaagcacca gaccagaagc agacaagtga 4140
   cgcggcagca gaaggcgcga gcgcactcca ggcacagccc tcggggccgt gggctttagg 4200
   aagtgggatt agccagccaa gggcataccc tcgcatctca gaggacgcga aaaggtcacc 4260
   atttcaaacc ataagggcgg gtggcagact ttacagatgg gtcacaggtt gggataagag 4320
   catcacagga gaatgtgatg atgccacttg tcaccctgag ggaaaaacct ttagccatct 4380
   gtggaacctg caaaatgcca gccgcaggga cagatgggac ctgcctggaa gcgtgtgcca 4440
   gggaggcagg cggatggcct tcttcaggaa ggtgagctct gggtggtaga agcggaaaac 4500
   ctggtccacc acatggggct gggtctccac ggttaggcaa agcacggcga tgggcttacc 4560
   gccactcccc cggaacaaga cctgccagga gagcagaggt tgccagcatc agggctggca 4620
   cgtgggctca tgaagagctg cctgcaccaa gccagcatcc acagggccca gtccccccac 4680
   cccccgcacc gtgacgtggg aagcacaggt gttgtcagaa atggtgggct gaatctgcag 4740
   cagcaggtcc ccacagaggg gatggcaccc agcacaggga ctgcatgtgg acatgttccc 4800
   gtgacacaca tgcctcaaat gtgtcagcgc tcaatttgac agccccaatg tcaaatactc 4860
   cttcagccac cgacccatct cccaacatgc ctgccatgtg catgctggga agctgcccag 4920
   agggtctgat gttaacctct ccaagtcctc atgggtggta gtggagcaca agctctttaa 4980
   ttctgaacct aaacaagaaa ggctagagat acaaacttcc tttctaaaaa aaaaaaaaaa 5040
   aagttactgg gacacctgag tggctcagtc agttaagcat ccaactcttg gtttcaactc 5100
   agggtcatga gatggagccc cacggcgggc tctgcactca gcaaggagtc tgcttgagat 5160
   tctctctgcc cctgcagccc tcccaagtgc atgcacatgc atgctctctc tctcctccgc 5220
   ccctttctct ctctctctct ctcaaaaata aaaaatcctt ttaaaagaaa aaagaaagag 5280
   agaaagggaa agaacgggta tctccctctg gccctgagcc ctccaggccc tgcatgaggc 5340
   caggcatgca gcctgacacc caaggcaacc ttcccacagc aacagaaccc ggtctgcaaa 5400
   ccttccacac gggtaagacg ttcttctgct ctggtacgtt ttattgtgaa acctttcaga 5460
   tgtacatatg taaaaagctc tatgtataag agcatgttta agtcataaat cataacagga 5520
   cagccagcag gagcccaaat ccccacttgg gatgaaccca ccatccacac tgttgagctc 5580
   tgggtgcccc accagccccc caccaaggaa gccacccttc aagattctgt gggcctggtc 5640
   ccatcttttc ttggctgtct tcccaccttt ggatttcacg caaggtcaca cttcacctga 5700
   aaggtcttcc cccatcacat gtgctccctt ggctgggaga gaagctccca caaacacacc 5760
   caccacccca tcatctgtgc cttgacactg gccacatttg caccgctagt ctgagacttc 5820
   gggtctaaca ggagcacgtg tgtgttcagc tgctatgccc actgcagacc aggcttctgc 5880
   cccaccccac ccacctgctc tcatggctgg gcaccccctc agtccacatg tctgctcccc 5940
   ctggccactc ctggaagtgc agggaagagc caggcttggg aggacaagga ctctctggtc 6000
   agcctcaccc agtgacccgc cccggtccag cagtccctcc caagtggggc ttgggtcctg 6060
   gggcttattt cccacactac agacccagca ccaacaggtg acaccctcct tcagatggag 6120
   tcttgggctc aaccgagtcc tctagcacaa gaaggggcag acagccactt cctgacattt 6180
   ccaccacctt aaaagcttgg gagtgctggt tttacccttc cagtgacctg gttagcacgt 6240
   tagcagttaa cctaggtatc agtgtcagag agcgggcagg tctagcgtcc aatgcctgtc 6300
   acaggagaag cagcaaacct gaaaccacgt gttcataatg agactccaaa aacatgacct 6360
   cctctggagg ttgctagggt atagtttatt atcttgaaaa ctgataaata aaatcaaaga 6420
   atacaacatt gtgatgcctg ggtggctccg cagttgagca tctgcctttc actcaggtcg 6480
   tgatcccagt cccaggatca agttccacat cgggctcctt gcatctccct ctgcctatgt 6540
   ctctgcctct ctctgtgtct ctcatgaata aataaaatct taaaaaaaaa aaaaaaagaa 6600
   tccaacatct atcctgactt tctgatataa catatatttc agggcaacca aactgctggt 6660
   ggggaaattc tcattcattg ccaaatcaca gctaacaagt acagaaagaa tgaaaatagt 6720
   tgaatgtccc catttcccag ggtgaaaatg agcacaggca atgaacccga ggtagaccac 6780
   aaacacgggg agccagggtc agtggacccc aactcacaca caccaaacat gagaagtgag 6840
   cagtggggac accaggagat tcagaaggcc tgctgctgac cagggcaggg acagcggcat 6900
   gtactaggct gaaagtcctc atcggccagg gacacatcct ggggtcatta tgggtgaact 6960
   catgagctct ctggggtttg ccttcagact ccagatccat atatggcaaa tgtaccaggc 7020
   tctcagattc cactgatagc cctacataga gagcggacct actccttgac caaggcaaag 7080
   ctgtgcctct acagtaagtg aaatacagca ggttccacta gagatggggc cgtgggcaca 7140
   agctgacacc accgtgatgt gggaacacag gcctgcaatt gggaaccgtt tcttatctgg 7200
   gacccactaa taaaacccat tctcaggggg cagggacaag gcaaagtctt gttgcttttg 7260
   tttttacttt caaactactt cacaaaggca tttgtctacc caaaaaagag cttcagaaat 7320
   tcctaaaaac aagtgtccta agctctccct caggatatcc taaaggcagg ctgcaggtgt 7380
   gtgtgcaaac atgccctccc tcccaggcag taatgaaggt ttccagaagt aactgcacgc 7440
   cactcccact ggtgctatgg gatgtgggat gtggtggacg gggtcagagg gctagtctag 7500
   ccccctgtgt ggtggccacg tgctgggaca gctgtcctcc cagggccatg gggcaggcac 7560
   gcagaccacc aaaggctaca aggcggccag tggctgggga taggcttcca tgcaggacac 7620
   ccacccagag gccataccaa catcgcctga agcagtcact gccctatcag gacatgaaaa 7680
   gtccaccctg gtaggaggtg caataaagga cagagggtca ggtcctaggg gcctgaggaa 7740
   caacagaagg caggaatgct tcaccgggtc tgctcagagg acacctggct ctcaggcaaa 7800
   accccaggag tgtctgggat cagagatctc ctacccctta ccttggcatg cttagtgggc 7860
   attgcactgg atttctgggg caatccggta tccgtgtcct tctcagatcc gagctcagca 7920
   gagggctgtg agaaaccaat cggtcatgag atgggagtcc gagggtgatg gccactacct 7980
   gcaggacatc tctttgtagg aacagctgcc accagggcag agggctcccg tgcgccgtct 8040
   catgggttct cactgggtgg ctgtctgggt gaccactgtc atgaaccaga ctcacagccc 8100
   caaagaggga acaggggtgg ccatgaacta tgaaggctca ctggacatgt aactggctct 8160
   gcctgaagtc aaagacctga cgtcttcact gtctttacga tctcttatcc caaaagtgca 8220
   tctaaagagc ctgttgagga ttctgtgtgt ctctgagagc tcctcaagcc catttaagtg 8280
   acaaccatcc tacagaactg actccaaaag ctcaggatga aatgcatgcc ctgcaccctt 8340
   acccacccat cccaccagtc acacgggcat gtaccactca cacccatcac tggaaacctc 8400
   cacgcctgca tttccagcag aggagcaaat gctacagaag acgtgggcac acagcattaa 8460
   ctagtgaaca caaaatgcag caaagtttca acccatcatc tcttctaacg taagggagag 8520
   gtcctatacc cagagcactc agaggagaca gtccagcctt tcctgaagcc caatgcatag 8580
   gggctgggag agcaggtgac ctgctgaagg ctaacgggtc tgagcagcac agggggacac 8640
   atggaaatgc cccactatta tttagccaaa tgggatttgc ccacgtgcct gacaagtcca 8700
   tctggcattg gaagaatggc ctccccacct gcgccagggc cagttgccct acagagaagg 8760
   tctggtattt gaaggggatg tgagcggtct ctcgtgggcg caggaagatc tgcggggcaa 8820
   ggctgccccg cagatggaac atgtcctcct ccaccggcgt gtgcagcttg gtcgcgtctt 8880
   tgaagtatct ccactcctga ctgtcctgga tgatgctaga tagcagggga gacacagcac 8940
   tcccagtgag tcctccgccc gcccactcag agctggcacc taggaccagg ggtgaaagcc 9000
   aggaccagtg ggaccccttc agggatgaga gcagcccggc tttcagggag aacctgtaga 9060
   cacccccagg gaccctgtcg gggctgccac tcaagcctac ctgagctcag gattgtccac 9120
   ctcaatggtc actgtgtgct gtgtgttgtg ggggttccta agggcaaact cgaagaactc 9180
   ggcagtgccc agtgtgacgt ggattgtgtg catcgtggtg atggccaggc tcagcgcact 9240
   ggtgatgctc tctgccttcg tacgctcccg ataggcagca atgacctgca ggtcacgtgc 9300
   gtgctgtgcg cggatattct gctgaatctg tgggacacaa atgccaaacc aggccaccat 9360
   catggggctg gctctccttc tcaagggctg tttggccacc tcccccactc ccatgctgaa 9420
   taccaaattt cttctggaat caccacctcc tccttcccca gacacccggg ttggatggga 9480
   ctggcccatc ctcaggtcca gaggacagta aacgggtcta cacggcactc ctggctccaa 9540
   tcagccctgc agtggtcttc cctgtagaag gtggcatctg ccctgcaggc tcccaagaga 9600
   ctacaaccac ccatgaggcc acctcttccc atgcctcaag gctggcatca gcatgatggg 9660
   gaattagaag caaaggtttc taacaaaagg gggggaaatg acctcaaagc tactaagact 9720
   agttatgtgc tcattggcca ggtccccagt gtggcgccgg gagccccgtc tgtgagcagg 9780
   cgcctgtgta acctgagcca ggccacggtc cagacagctg gcacgctgcc ttagggggct 9840
   cagccacagc tcagcctgcg gtccttcatc cacctccttc ctccatgccc tgattcactg 9900
   ggcttggcca acacaaagtg ttctagaggc tggagactgg cttaggtttc ctgtgtgact 9960
   tacactgaaa ggtgaggtcc ttggaggctg ccctgatcta ggacctagga ggggatgccc 10020
   cgtgctgggg aatgagccag gccccgtgct aagaccctct gaaccctgag gaggcccaga 10080
   catcagcagg cagaggatgt ctgaatgtct ctggacttca actcaagaag taaggatcca 10140
   atatggtttc tgacttaaat cactttggat attgacttcc aacatccaaa actttggcct 10200
   tcccctagca agagagttga ggccaagtct ttaatttctc cctggggatt tgaaaacggg 10260
   atggtatttg tgcttatatt tgaggcctcc ccacacagac agagatgatt agagcttctt 10320
   gatggagtgc gggtcactcc cagtttacca tggaccaggt ggtgaacagc ctcagggaga 10380
   gtcaacatga tcctgggggt tgtgaagggg agactccttg cctgtcctgt gtcctgccac 10440
   accacagagg aggctgtcgg gaagcctggc accccacagg acatggggtg acttctccga 10500
   tatgaaccag ccagacatag ccctccctct ctcaaccctt cctttgggga tccacctgcc 10560
   tggaacaccg ggtgtttgca ctgctcctca ccagtggtgg ggttagtggg aaggatcctg 10620
   cctgtggagg cggcccacag ccatatggtg tcacccatgc agcttgggtc tggcatttgc 10680
   tctnnnnnnn nnnnnnnnnn nnnnnnnnnn nnnnnnnnnn nnnnnnnnnn nnnnnnnnnn 10740
   nnnnnnnnnn nnnnnnnnnn nnnnnnnnnn nnnnnnnnnn nnnnnnnnnn nnnnnnnnnn 10800
   nnnnnnnnnn nnnnnnnnnn nnnnnnnnnn nnnnnnnnnn nnnnnnnnnn nnnnnnnnnn 10860
   nnnnnnnnnn nnnnnnnnnn nnnnnnnnnn nnnnnnnnnn nnnnnnnnnn nnnnnnnnnn 10920
   nnnnnnnnna cacttattgg aatcctcagg tcccaacagg cagcagagcc cagtgatcag 10980
   agcagtgaga ctgctgagcc ttctgctggg atttctggaa ataaagctct gtcttctgtg 11040
   gcacctggca tggaatggtg tgaagcgggg ttagtgctac tataagagac aacctggggg 11100
   ctgcagggag tgggcaagtg gcagaggtag gggcatgacc ccaggggcct gaagcagggc 11160
   cccaggcctg cctgctcagt accaggctcg gagtgctagg gcacctgctc caacatggaa 11220
   tggctggtcc agagcccagg cccaggccct ggggaccccg tggctgcaca gacctgagtg 11280
   catggggctt tgtcactgca acctcagagg acccccctcc ccgccggaca gggagatgtc 11340
   ctacaagcct tctcccaaag ctgggtgggg ggtcagggtc cacagagaag gtgcccagca 11400
   cacagtctcc ctatgggtgg ggctctgctg gcataggggc tgacgctgac cacagcagaa 11460
   ggtgggacct gccatcatcc cgagtgccag acacagaaag cctagccacc ctcatgagaa 11520
   cactgagact gctgcaaaca tatgtcgagc ccaactctgc tccaccactt ccccaagaag 11580
   cccccagatc atcaccaccc ctcgagctgt tcctgactgc tgggggagtg agccacgcac 11640
   agcgctcttc tctgtcttcc aatgagcagc ccccagggcc cgagaccacc agactgcacc 11700
   agcatgtcag gggccaagga ccagccagag gctgagcacc acttccagct cccggagggc 11760
   caactgaccg cgtaaattcc caaactctcc tgaaggctga gagagctcta ggtgcggaga 11820
   aaccaccatc tcctccataa aaacaagtca ctttcacctt aaattctcca ggccgctgac 11880
   ccagaatacc atgagaattt agtggaacat ttccttgtat ttagaaatcc aattacttcc 11940
   caattcactg ctgtcatcag aatgagggct gcgagctgca gaatctctac caggacacat 12000
   caacaggagg aagcccacca aggtaagaat cgtcaggaca ccagcaccaa actcatcaag 12060
   gactggcagg gtgctgggcc caccaccctc caaggcgggg ttgctgggga gcccgggggt 12120
   gggggtgggg gcagcattac ctaggggtct ctggggccac tagccccagg ggcctcaccc 12180
   tcagcagctg cctggctctc tttgaggagc acctgacagc aggaagcctc cccagctgct 12240
   gtgagcacga agggccgagc aggttttgcc tctattcaga tatctgcctg ccctaatgta 12300
   aagtgtgatc ctcacccgtg tggcttcttg attccataaa accatcaggt acaaccaccc 12360
   tctcctccca catctaagct gtaaatatgg tagggtcttc tttagttttt tagccaaaaa 12420
   tgggaatttt tccagtcaga atggttactt gatttgcact gataattcat gatacggaag 12480
   gcattcacag cactgacccc aaacctgggg gaccctcaga atcccctgct gagctttctt 12540
   aatagcccag gtaaagaccc aggtgcagca aactggacac agaattccct ggggtagagc 12600
   caagaatcgg cttttgtaaa aactctccag acttgaaaac gttgcttggg ggatgcctgg 12660
   gtggctcagt ggttgagcac ctgccttcag ctcagggagt aatcctggag tcccaggatc 12720
   aagtcccaca ttaggctccc tgcatggagc ctgcttctct ctctgcctat gtctctgccc 12780
   gccccccccc cccccccccc cggtctcatg aatacataat ctttaaaaaa aaaaaaaaaa 12840
   aaaaggaaaa cctcccttgg tggctgagag ggtccgtttc ccatacattc cacaagtagg 12900
   atttgggaga gagagagcga gccacagggg aagagaagag agagcacagg ctgctttcat 12960
   tcacaatgac tgtggctctg cacggggaca cccgggggca gaggggaggc ggatggagcc 13020
   ctcaccaaca tgctggcccc ccagcagctg agctctcctc tggactcttg aaggcgcacg 13080
   gacctcatcc gctccagctt gcgcctgcgg acggcatccc cctcacggca agcaccttgg 13140
   ggccccttgc tcccatgttg catgtgggtg aacagcatgg cggccagctc acggtccacg 13200
   tcagccagct tctgtgcttg gacaacattt tttactgcta gaaggttcaa agagagaaca 13260
   agaggttaag gcagagtcta cattctttaa acagacctgg caacaagctt ccagccacct 13320
   cacccaagga gggagctcag caggcaacct gccctttcct gcaccttcta agatgctgat 13380
   aaagatggta atcctatagc ctggtctata cagtgtcccc cccctttacc agcctttcaa 13440
   ataaaaggat tctgatcctt agagacaaat tcatacacca ttggataact ttccagagga 13500
   aaaaaaaaaa gtcagtgttc tagaagctga tgctttctgg attcccaagg gccttgccac 13560
   cctgacccag gaccactagc cctgtcaccc tcacccagga ccaccgaccg tgctcacaaa 13620
   agcccccacc agcgagagcc tcctgataca ggcaccactg agcaatcagg cccacggccc 13680
   caccagggcc tcagacgcac tcgtgtgctc aggtcaggag tcaggggctc tgatgagctg 13740
   ctcacttggc ctgaagcccc ctgagcagct caggggggtg gggtccagac taggcctggc 13800
   tcccaagctc atgcccctgt ctctgccacc agctgcctct agacagcctg gccccccggc 13860
   actgactcct ctgggagact atgctcaaat cctggacaca cacaaaagcc atgtgggcaa 13920
   acagaagctg aagcaggcca gacaccagca ggcagaggag ctgcattttc ttcaggtgat 13980
   gaagccacac gcggcacatg gccatgtcac aagctgacac acgagattca cagaccacca 14040
   ctcaagccag tgcaggcagg cagaaggtgc accccaactt agcactggcc cttcctgacc 14100
   ctgaggtacc agcagtgcct acaggaaatg ggttgccgtc ctgaatctgc caaggggttg 14160
   cacatggtca ccgcatctcc agcctcctac tgaaggctcc atgtggtggt aaagcagtcc 14220
   ttcccccggg ctcgcccacc tcgtcctctg tcacatggaa caacaagtgc tatttccatg 14280
   ttcctcacca ccattttaaa gctgttcaac tgcttttcag ataccatgag tcagtttaga 14340
   caagtaaaat gccaccccaa aaagactgtt ttcacgtctc aggtgcaagc atgctgagtg 14400
   ggcaaccccg cctaggttgg gtttgagagg cccgggggaa ggggccaggg caggagacaa 14460
   gcaaggctag gggtaagcca aggagctcac atgtcagatt gcatcactct gctccaacaa 14520
   aatcagaaat gaaatctcca cactgctcag agcctggaag cactttctaa aactctgtac 14580
   aacttcctac ttgccaagcc tgccctgtgg gggaagacag gctctctgat ggagcacccc 14640
   agcctctgtc tttgaccaaa cagggagaga ggaaccaatg actccagaga ctcaattctc 14700
   tcaacaaacc ctcacaggtt acagcaccct gcatttcggg cagagcccct gacctgctgg 14760
   gccctgaggc cgtggctttc acaaatactc cctgcctgtt cttgggacag ccagggatgg 14820
   atgggccctg gccccaaagt gggaaatcca gattctcacc caagttcacc tccctctgcc 14880
   acacaattag gcaagtcacc tgaccgtctc tgccttggtt tccttaacac taaacaggag 14940
   aacagactct cagcctgccg ggatcatgga ccttaggccc gtctgagcat ctgagcctcc 15000
   agggccatct cccagaagaa catttgagta cccacaaagt ctgggctgga gtttcagaga 15060
   actctcgtgg ggtcctggag gcctcgcggc tctcaccagc cacgtccagg ggcctcccct 15120
   ccctgggcac aggttcaagg acaacagtat ccgaggacca tcaacacagg aagctcgagg 15180
   tagctgcaga tccacatgag atggcctgca acatggtgac actacccaaa agacccaaag 15240
   ggcacacccc actttgcatc ctacctacaa ctactttggg tcatttaatg ggatgtaaat 15300
   ctttggatgc cccctgctgt gaaattctcc aagcccaggc ctttatctct caggctccgt 15360
   gactggttta ctcctcctca ccccaacata gggacttcaa atcggttttt aaaaaccagt 15420
   gtttgttcat cctaaccttt tcttcatttt ttgctcccct gcagatgggc agctaaccaa 15480
   aaaccgatcc cctcgaagga ttattgtcag cccagacagc ttccacagag cccgctctag 15540
   agtgtcccgg gtatgacaca catgcttaac ccacccagcc caaggggcca ctgaggcagg 15600
   gcgctgagct ggacacaaca gcccccactc ccgcccccag atcacacgtg tacctgcatg 15660
   cacacagaca cacacgcagc cttccacctc ttactccacc ccacccccat gtcaatcaca 15720
   gctacacatg gcccacctac cccaacccgc tccccactca catctcggac ccccgcgcga 15780
   gatgagactg cctccctcga agtgtctggc tccatcattc gaaatgaccc gtgacctgga 15840
   aggtggcaat gagctggaat ctctcactct ctgctcacac acatgaccta aattaaaaac 15900
   atcccagtat aaggtattaa caccaaaaag aagtgtgatg ggacacatcc atgtcccctg 15960
   gagggagttc tgggtttcca agggcctgtc cataccccct ggcctgctaa caccccaaaa 16020
   ctgcactctg gttctgagtc tgcttggcag ttgagaggtg acgggcaggc gggtcctctc 16080
   gtacatgggc agtggtgtct gctttcccac gggggttaag gctgcaggta ctcaggacac 16140
   tttttcacac acactcagtt cctggtctct ggagcagcct agcagccctg cgacgtgggg 16200
   cagctcacac attcccacgc cagcctttcc aatgtcaccc tgctcatgga aggcccttgg 16260
   gcatgtccgc aaatgtctga ctttaaacat acttgaaggt aaaagagcct ctgcaagcta 16320
   gaatcttcag gagctgtttt cacacagaag ccttggagag aagcatgcag cctagagggc 16380
   caagcacaag tccagaggca gagcacacag cacacctgcc ccggctgaac gggaagcagt 16440
   gtcccactaa ccagccaaca tgtgcaaggc tcaggggata agaactgttt gcctgaagaa 16500
   atgcctctcc taggaggaca gaatcatacc ctcaggaccc gtgacctctc tctgtggaca 16560
   tgactcccaa gagcacagct gaagtcagat tccaaaatga acccaagttc caaacacaaa 16620
   tgctcatgct gtgggcttcc atgattccag caggatggtg gtgatctttc tgggagctat 16680
   tagaacaggt accccttccc ctgggtgctc gtctcccgcc tctctgcgtc tcctcccacc 16740
   tggcctccac tctgccctgc tcagcttcat ccgcctctgc ctctgtcctg tgggacagtc 16800
   tgggtgcaag agcaagcccc ccatcacagc cgtgggctgg tcctcagcag tggtgaccaa 16860
   ggtgacattt tcatatccag ctgcctagca gaaatgtctt cctcccgact ggttctgagc 16920
   tgagctgggg aagggaatgc ctatgctgcc agggaggtct ctgccctgct gccaatgggg 16980
   gccgcccctg cagaccccag ggaatggcag ctggagcccc ccaagacatg ggtgtgacag 17040
   ctgaccgtcc cttggcattg tggaaaatgc tggattcaaa aatgagagac tgacaccacc 17100
   tctggccaaa ccaagaagtg atttacttgg catggcttaa agcctggcca caaacaggtg 17160
   agcagagtcc tcccctaggg acttccattc tcatcatttt caaactccct tggtcatctt 17220
   gagcgttttc tgagcatctc aaatcaggct ggccttcccc cagccttcct gcaacaagag 17280
   ccactcctgg ttatctgccc ttctgcctgc taaatggtgt tcacagctca gccagtagcc 17340
   ctggcaagcg ccagagcatc agctgtcatc cacgctcccc gtcaggaaga ccactgtcga 17400
   gggccttccc tgacacagca ccacactccg ggaatgggga agcatggagc ccaagcaccc 17460
   acccaccaca ggcagagaag tcggctccaa agcccaggtt cgccatgaga ctccagatgg 17520
   caggcctgtc tcctctgtag caggttcctt acctgcctgg ccactgtggc cagtgaccat 17580
   cctacctggg gtctctgcct agggtaacct cggtgccagc ctggtctctt gctcttctgc 17640
   ccctacctgg acgtcggctc tatatgtaca gcatacactt ggacctgcag gcccagactt 17700
   ccgggcagac accctgtcgg ccactcccac ccctccctct agccctgtgt cttgagaaca 17760
   agcttcttga gcccagatgt tctcagcctc tgctctgttg agaagcacgg aacacttctc 17820
   cacaggggtc tgcccgccac ccacccacag ctgcttctct gctcttcccc tccagatgca 17880
   ggggctgcag tgtcttctgc caaacatggt catctcagca tcacaaggta ataaagctcg 17940
   tccccaagag tcgagcctgg tgttaacacc ccttcactgg gatcagctgc tcacacctcg 18000
   gggcctgctt tgtcacctga agcccaagag gagcagaatt caaggcaaga caggctctca 18060
   tacttgctca tccacccatt catgaacctc tcatcaccag gccaggtcac tgtggctgct 18120
   gctgggtgag catcacagca ggaaccagga tgtccctgag aaagccccca ggtccctagg 18180
   gggacagaca agccagtagg tggacaggca gggacagaga gcctggctaa gtgggcagga 18240
   gacagccagc tagtgccagg gcccaagtcc cagcactgac actacttgct ctggtcctac 18300
   cctgatttcc aggtgagagg ggaacaaagc accatctggc cccagctgca ctcagttcct 18360
   gccatgagct cagagacagt ccctgctcct ggctctcagc tgcttcttcc cagtgagggc 18420
   aaacagggcc tgcaagctct ccatcccacc ctgaggaccg gctccatctg tcacctggca 18480
   gggggcacat accacctcca ctcactgttg gggacagcgg attcatgcca cacctgtggg 18540
   aacctgggga ccctccgtag ccttccctga agcacaggca gggaggcatt caccatgtca 18600
   agctgaaatg cacgcagcct acccttagca ggcaacagct ctagggggag ggacaggaac 18660
   cagggaaatg gctgcagatc cggatctggt gcagacgggt gcactgccag ggcacacaac 18720
   cggggtgcat ggccggcatt gtcagagccc aggctctcag gactcacggc tgtcatcgca 18780
   gcatgtggga ccagcaggac catgtagtcc cctccagaca gacaagggcc atccctgagt 18840
   gtcacagttt cctctaacag tatttgcact cagagcgtgg gttcgtgtgg aaggctgcac 18900
   aataagctag atcagggcaa catacacctg cttactctgg gcctggatgt ggtgaaggtg 18960
   ctcacttctt cagggcacca tctgcaatct caagttgctg tgggctttct tacccttcca 19020
   tgacaccggg gttaagctgg ggacccacac atttgctgtg ggagcggaga ctccctccag 19080
   atgagcaggg ttcctgactc agctgtgcag ctaccttcat tccacggtgg cagcagagca 19140
   catcaccccg tctgacccca aacccaggct ccctgtccac accacccagc tctccttcaa 19200
   ccctgagaat atggtgccag gggtcagggc agccccttgc tcccctgagg agccctggaa 19260
   gggctgcaga tacaagtgac aatagctccc cgatgaacca cagccacacc gggatcccgg 19320
   ggacagaaaa gcaccagaga cactgcagtc ttgtctgcag ggaccatcgc ggaacctaga 19380
   agccaccagg cacaagcacc ccgccgcccc cttcctatgg cagattcatg ggagatctgg 19440
   gctcagagag ggcacctcgt tctgcagcct cacacttgac agcatacact gggagtgcag 19500
   agccatggag cgccgtccaa ggcaaggcca cgccaggccc atgatgctgt cctgaccctg 19560
   cttctgtccc ctgaagctca cacagcctca tccaatatgc aggcctgcac ctgctgcttg 19620
   gttctaaaca cccagtttta aaaaagaaat acaagacaca atgccatttt ttgttttttg 19680
   tatttttttt caaaaaatgc aaaatttaca gaggaaacca gaccatggct gtatggacag 19740
   tgagctgctc gcgctcgtcc tttgtcaatc cccctccgct ttggcgcctc tgggcgggtg 19800
   tctctgactc tcccccgtgc gctatcgctc ccttgcaggc aaagtatgga aatgacaatt 19860
   taagataacg tataatagag ctaatctttc ttctgaagat aagatttaaa gcgcggagtc 19920
   actgcacatt tgtgggctgt gaataaatat aaaaataacc aatgcaatct cagaagcaga 19980
   acgcttcttg cagctccctc actcatagga gcttctgaat gatgtctgct tctcacatac 20040
   agttcctttt tttctccaga acctaattga gttttgctgt ttccatattg tctgttttca 20100
   aggcggcctc ggatcttaca attgtcaaca agcaggcaga aacctccagc ctaacttaac 20160
   gagtatgcac gcctcctgct gctgcggccg ccccagcctg cccgcctcca agctgcccgc 20220
   acgagtgcat gtggcccatg gggctccagg ctttaaagcc agcctgggct ttgaggaatg 20280
   gaaaattccc ccataaacca tgagaggttt gagtctcatc tagacagaca tggctccaga 20340
   aacctcaaat ggcttggttt cgtcctgctt tccatttcac cccaaaaaac ctcaatcctt 20400
   ccatagtctc ctcaagagag actgagtggg cttgtcccct ggccccacag gggctcactc 20460
   caggccccta cccagtggga agcacaggaa gacatctcag gccccatatc ccctgtcgat 20520
   caggacctcc gtggagcccc cagaatggcc agccaactcc actgtagcca cagccagccc 20580
   cataccctgc cccaggcatg tgaacaacag cgcacagggc tgcactcagt gtctgaagag 20640
   tgaccctcag acatcattat atgtatggtc agagtatagg ctctaaagaa gtgtgtttct 20700
   ctgttccctt atgcccaagc tgcatcaaag ttgagaaaca ttttgctttc ctgtcctatg 20760
   atgaagtagg ctcataatct agatgaaaat ctctcctgga accagtcatt ttatctgagg 20820
   attttcatag agatgtcaca tagacaacac caagaaacaa aatgtggaca agactgagat 20880
   gactaatgat tccaagtcct gatcagaaac caagaacacc aaacaactag tgccaagaca 20940
   ggctcagggg tcaaggacag atctagtggt caaagacagg tccagtagtc aaggacagat 21000
   gctcagtggt caaggacagg tgctcatggt caaggacaga tgctcagtgg tcaagggcag 21060
   gccaagtagt gaaggacaga tgttcagtgg ccaaggacag atgccatatg atcaaggaca 21120
   gatgctcagt agtcaaatag taagagtttt tctgaattcc ggttgatcat atgatgccca 21180
   caataggacc actgccctga gggagctcat gcaccaactg ggaaaatgag gggccaccca 21240
   ggagacagca taagaccagg taaagctgcc agggactctt acccacgttg gccaacgtca 21300
   ggtgcagcca gccctttacc actgtgtaga caccgatggg cttgacacag ccaaatctgg 21360
   tcacatctcc actcaccacc acatcctgtt catattcagt cgccacgacc tcaagctcat 21420
   gcaggacctg cacagccggc cggccttggc ggaggagatg ctgcagagaa aaaggcacca 21480
   gtcagagggt agccgaggta gagcccatct tctactgaaa atctacactc ttgatattgg 21540
   gtctttccca caaattcttc aattaagtgc accccaattc acccactgag aaaatatcat 21600
   ggatgcacaa acaggcctgg cagccaccct cctggaaccc cttctatggg gagaacaatg 21660
   ggaaacgagc aataggagga ggctgggaat gctgagcaga ccccatctcc cagtcagtga 21720
   gagcttcaag aaggaggtat gaaccaggcc ggggatgcta agggcacact aggcccaggg 21780
   tacagagcag accccccaca cccggggcca ggagcatcag gcttcagcat ggctgaaaga 21840
   caggtgaggc ctggttgtgg aggtccacat aaggcccagg ttggtcagag ccggcaagag 21900
   cagatccagg acaggcctcc taaacccagt gctttctgct aatccagttc acccctcagt 21960
   gccctgcctg agcctaagtc atagtgaggc ctgcaatggc ctcttcctgg gaggccttgg 22020
   gaaaatcacc tgacctttca actagtgctt cccactcaca aatgggcatg tggaggtcag 22080
   acaagcatcc caacttctgg aattcttgca cttctactac agcaaacatg cccagctctg 22140
   ggagtggaag tgccccaaat gacccctctg tagtggctct ggcagcttct ggttgcttca 22200
   ggaggatgag gacctggccg gactccccag ccacccaccc gcctactctg cgtgccgaac 22260
   ctcccacaca ggcacacctc tgactcagaa ccacacttct gatgagttct catctgggga 22320
   gggcagagag tgacggtaat aagcacaagc ttccaagtca gaactgggct aaaaggctgg 22380
   gtctgctgtg gtccgcgtga ccgtgggcct gtctctcagc agcacatgaa gacagtgcag 22440
   tacaactggg ctgaggacac cgtccacgaa gttcaagagc aatcagatgc cgtggcaact 22500
   gtcactattg tcctaacaac acccttgtag ccttgggaat gagctgaaga ttgggcaaaa 22560
   tgtacgtcca gatgttcact gcaacattgc tcatggggaa aaaaaaatca ctcagtgccc 22620
   agcaacgaga gcaagtgaga cgagtcctag tgtatccata ccttccagaa aatgtgccta 22680
   cccaaggcac acagctagtt cagcacctgg agttaacagg cggttctctc ttcttacctc 22740
   atctcccatc tcttctatat aaattattaa ggtaaagacc ttatttttaa aaagtaaaat 22800
   ttaattttag tttatccaag ttaagtgtca ataatcacaa cagcatttgt ttcaggggct 22860
   ctttgtggag gacaatctag agtaacgtca tctccaggga cacagggaca ccgggctccg 22920
   caagggatga ggcaggagcc acagcgggtc ccaaggtaac atgggctcct ctctgcccct 22980
   gcagtgccct tcctcccccc actcctacct ccctcttccc tccactccca cttcccccac 23040
   tccctcctcc ctctatccct ccccatccct cctcccccat tccttcctcc cttcactccc 23100
   tccatccacc ttccctcctc tctccatccc tcctccctct atccctccac tccctcccct 23160
   cattccatcc tccctccatt caccctccct cttccctcca tctctcctcc atccatccct 23220
   cctcctccat ctatctctca tccctcctcc cccactccct cctccttcca tccctcctct 23280
   gtttatccct ccactccctc ctcccgtttt tcctccctcc atccctcctt cctctatcct 23340
   tcctctccct cctccctctt ttctctcctc cctcactccc tcctccctcc attcctcacc 23400
   cttcccccca cccccatccc ctcaatgctc cttccatttg ctcattccca gctacacagc 23460
   accatgccca ctgtgggggt catgactgca ctgaagatga agagcagtcc cattcctacc 23520
   ctctagaact ttcaccagcc agcatcagag acagacaagc aatggccacg atgaccccct 23580
   ggagttgcac ggtgacccag aaaggcccaa gggaccaggg tgaggaatga gaacatcagc 23640
   caatatctac tgggtgctca ccacggggga cccctgccct cactcctcct gctcccctgg 23700
   gggcatcact cctgaggggt agacactgaa tcgggtggag gagggatggt acaatccagg 23760
   ttagccctca gagggaagag gggaagctgt gtcctctccc acctccgtcc cacagccggg 23820
   acatcgcgtc cccagtccct agcccaggag ctgcggtgta attacactga ccctctgggt 23880
   agaagcccca ggaagaatct taaattaaca tatggattta ggttttagtg taggaggaag 23940
   atggaaagca caattagaga aagaattaga ttctaattac aattagaaaa gcagagttga 24000
   agtaacagat agcaggcacc aggtgatctc tgcacaccca atcgatgact catcctaccc 24060
   tctgtgggcc aggcccctgc tctcactggt ggggggcaga ggcagaaggg gactgcacct 24120
   gtggtcagtc tgtaggagga gctgatgact gaggaataat ctcgcattac acagctgaga 24180
   ctctgggaaa atcttttcct ttggggaagg acggacttta tgaacaaaga catcagatga 24240
   cagcccacac tggacgagaa ccagcaactg agcagacccc tccccccccc aggagggaca 24300
   ccccacccta accagcccgc tgcagagcac tgccaccttc atctggacgg ccgcagatcc 24360
   cacgagcagc aaggagtctc catcccagat gtcgatctgg agggtctgag cagccatgta 24420
   gtgggtgaac cagcgctgtt ctccgggttt caggaaccca ggttccacca ggtacttgag 24480
   ttgaaagcca ggtgtccctg ttcagagagg ccccgaatga gcccaaaagt gatttgagtc 24540
   attatcatct tcacattttg aagagactcc tgctgtggtc acaatcacta atttctgctt 24600
   ccgtcataac tacagtttaa taaacacatt aatcaaaaat atcataaaac atccacattt 24660
   acaaaataca gagtcgtatg cagaagtcta catttctttc caatacatac aatgaactcc 24720
   tcccagaata ccccagggaa gaatcttgtg aaacagtcta tcctgaatgg ggacagatat 24780
   cctgacgggg ctgcccaggg aggtcattat tctgtgtgca gggtttaagg ggaaacatgg 24840
   tctgccgtca aaagtgggac agcacacatg aaagcacgcg cacacacatg gagctcaggc 24900
   taaggtcagg ctgagctgcc ctcaacgcat tcccagcggc cactaccagg tcatctctga 24960
   gacagcttct ccaggtggca tcaccctcga ttctaaactg acaaaaacac cactaaaatc 25020
   attagttaaa catttccccc ttcccatcaa taacctattt tcaggggcac ctgggtgcct 25080
   cagcggttga gcatctgtct ttggctcagg tcatgatacc ggggtcccag gatagagtcc 25140
   cacattgggc tccctgcatg gagcctgctt tttcctctgc ctatgtctct gcctctctct 25200
   gtgtgtctct catgaataaa taaataaata aaatcttaaa aaaaaaatat tttcagagcc 25260
   tctgacgagt agaatactac catgtttact cagcattggg aagcgcgacc caagtcagtc 25320
   tctgcctcta gccacctcag cccctgggga gcggggtggg ggtggggggc acacagtcat 25380
   gaacgtcccc tctcctctca agactagaac agtgcttcta gttgaaggag ggaagcgggt 25440
   tgggatggga cacagcacca ctgagggcct cgcatgggcc tacgggacta agccaggacc 25500
   agttgccata ggcctggctc cgaccacagt gagaattcac agggacacct cattaaggga 25560
   gcccagcaat aaggaatcag gaaaaaggct cagagagggg gtggtaatga catcatgcag 25620
   cggcaggcca gtccttagta tcacatatcc agtgaccaga caaagtcaca gggccaatgg 25680
   aggccaagga cacctaccac ccttgcccca gtgccaccca ccagctgaga cctaaagtgg 25740
   cctgtgacaa ctggtcattc tcaggacacc gcctaaataa tgggagccaa ggctggctcc 25800
   ttagatgcat ggtgaggacc agacaaagac ctataaacac ccaagaccct gctcatggcc 25860
   cgaatggtat ggcaccccat accaggccca cctgggctgc aggagaagga agagctcagc 25920
   cactggcttc tacactggtc atacttctga ttcgaggaga cagaacaggt ttgacctctg 25980
   tgggccaact tcccaaacga tgcctacaga caccagcctt gggagctcca tactccacct 26040
   ccagcatcgc ccagatccac agaccctcca tccagagttc tacctgctcc ccagtgacgc 26100
   atgtcccaga tggaccctcc atccttccca caccagctcc ttgttacatc gtcccaaagt 26160
   acttccttgg caccagaccc ttgcaacgcc catgctaggc tggctcccgc tccaccacca 26220
   ctgcatccac tcagctgcct ggccctttgg cacaccatta actcacactc ttcaaataac 26280
   tgcagtgccc ccagcatgtg caccaagcac cacgcaggga taacttgaga gagaaggacg 26340
   gacagggagg gtccccacct gatggagctc atggtcacag gataacacac tcagcccaga 26400
   gctaatggcg tgctgagtac ctgggggagg catgtggggc tgtgggggcc atcctggtct 26460
   gggcccagcc tcagggctct catgctggag gctcccagtt cctcctgcca caggcttggg 26520
   cccctttact tctatccagc tctgacaacc ggctcctcag tggccttccc tgaggtccac 26580
   actccctgca gtccatccca ctacacagcc aaagcatcca caaagggaac cctgtgtcct 26640
   ccatcctgct cagaagtgag caatgatact gaccatctac tcacacaaga cagacctcag 26700
   gggcagtggc acctccccag ggtccactca ggttcccaag cttacctccc tcaccccctc 26760
   tcaaacacca agccccagcc cagctggctc cattctgagc ctcagtccag ccacaccccc 26820
   acatccaagt gtggtaggta ctcttcctcc ggttggatgg tcctcagtct ccatctctcc 26880
   ttcatgaccc acaaacaccc ctactctgcg aaggcatcca caactttcac agatgacagg 26940
   cactttatac cactatggtc tacaatccac aatgaatcca caatgaatat acagcagctg 27000
   ggaatatcta cacaccatat gacaaagcaa aaatctagag acgcaaggag aaacagataa 27060
   aaacataata ataggagaga tcaactaatc actttgtttt cagggcaggc caagagggca 27120
   ataaacaaca gcatggaagg ccagccaaga taatccatca catagagcgt atacatgtac 27180
   atatgacacc attccctgct aacattgaat atatctttcg agtctatctg gaacattaat 27240
   aaaaattaac tatatacctg gccacgaaga aaacctgaat atgctccaga aggtgaaaat 27300
   aacacaaaca atattctctg aatacaatgc aacaaattag aaactggaaa caatttttta 27360
   aaaactaatt tacagcttac ttttaaataa cttgatgtca aatggaaaac aaactccagg 27420
   gcagcccagg tggctcagcg gtttagcacc accttcagcc cagggtgcaa ttctggagac 27480
   ctggaatcga gtcccacatc agactccctg catggagcct gcttcctcct ctgcctctgc 27540
   ctctgcctct ctctctgtct ctcatgaata aataaataaa atcttaaaaa aaaaatccaa 27600
   acagattttc tacagaataa tgaaaatact acttatctga atcaccaaag cataaagctg 27660
   taatcaaagg aaaattcata gccttgaata ctttgcatcc acaaaaacaa aagaattaaa 27720
   gtaagtgaat taagcaactc aaattagaaa aataacactg aagaatacaa aaaaaaaaaa 27780
   aaggcaagaa agataaagat gaattttaat tggttagaaa ataagaaaag agcaaaacct 27840
   gcatgaatgt gtctttggag agtcagggaa gcaggagtga ggacatgaga ggatgcccat 27900
   atccctgtcc catgtcccat tagggcagtt ctcccgaggt taacctggat tcagcagaac 27960
   ccccataaag ggcaaacagt ttcacatgga aaaccaaccc agccagaatg tggggtggtg 28020
   gtggggctgc agggaagaag agcatggtgg agtggaggga aagctgtaag agcaaagttg 28080
   cacctctagc tccacagcac acccactgag tggacaggtg gtctaatgca gagcttggca 28140
   aaccacagcc cataggctga ttcagctgta agctaagaag agctttcaca tttttaaagg 28200
   gttgttaaaa aagaagattc cagagagaac atatataacc cagaaagtct gaaatattta 28260
   ctatctggcc cttaacagaa atcagtctgc caacctctgg tctaatgaaa caaaatataa 28320
   aatccagaaa taaatctgag ccataaaaaa agggtataag gtaagggtgg ctctcaagtc 28380
   actttagaaa aagatggact ttcttctaat aaatggcact gggtcaaaag aacagacgtt 28440
   tgaaaaaaaa gataaacctg gactcctatg ttccatagca caacaggaca aactacaaat 28500
   ggaccagaag tctcatgtaa aaaatgaaac ctataaggcc cctggagaaa acgtgtgtgg 28560
   atctctttat gaaccaagga gaacaccttc gcatccatga ctcaggaatt tagaagcaat 28620
   aaaactaaag accaccacat taaatggcat aacagcaaaa ttttatgggg tacctgcaag 28680
   tctcagtctc agggttgtgg ttttgagccc taccctggat ggagagatta cttaaaaata 28740
   aaaatcttgg gatccctggg tgactcaaag gtttagcgct tgcgttcagc ccagggtgtg 28800
   atcctggaga cctgggatcg agtcccacat ggggctccct gcatggagcc tgcttctccc 28860
   tctgcctgtg tctctgcccc tctctctctc tctctctgtc tctcatgaat aaataaataa 28920
   aatcttttaa aaaaaatctt taaaaataaa actttacata gcaaaacaca ccaagagcaa 28980
   agtaaaaatg accaaaacag gagaacatat ttccaattta tatcacaagt aatagctaat 29040
   ctccccaaga aacagacttc ccaaaataga ggacagaaca agaattggat ttaaaaaatg 29100
   ggaaaaaata tgaatagagg tttgcagata gacaaatatc caagatgctc aatttcactc 29160
   attataagag aaatgcaaac taaaacttca ttaagatcta tctcacatcg atcagattat 29220
   acaaatcccg aagtttatgc cacattttgt gggcaaggcc ctgaggaaat aggcagcccc 29280
   atgcgctgca agcggagctt ggaaggccat cgaatgacag gggagtagct gacggctaca 29340
   atctgattgt tagtaacgcc ccccaaacac atgtccacac acagagaaca tataaacaag 29400
   gttacaagct actacattat ctgaaactca aagcctgaaa gcagcctgaa cgtccatcag 29460
   aaggaggttg acctgaatca atcacagggc acacgcccag tgccagacga tgtagctgtg 29520
   aaaacagtgc agggggtccc cacatggagt caagctcagg acagaatgtt aatggaaaaa 29580
   agcaaagtga taaaagagca tatttggtat gctatctatt gaataaggaa ataaggagaa 29640
   ataaaaatac atttgcttac ttgctgaaac aaacagaaac atcagaagga gaagccagaa 29700
   actggaagcc tggttacctc caggaggccc tgcagggggc acatggtcta gagggtacaa 29760
   cggttttaga ctgtgagcca tgtaaatatt ccaagaaatt ataccagaag ggaaagaaag 29820
   ccaataaaaa attgataaca aattgaaata aatgaatctg actgaatatc aaattggtaa 29880
   cagctccaca gaggaaagca tgagctgcag ggattctgac acgctgaact aactatgtcc 29940
   ccagcgggat gtggctccgc cctagagaat gccagaaacc tcaaccctct tcactcagca 30000
   cgcgtgccct tagcagtaac actggcaatg aaattctaaa agtacattat agaaatcata 30060
   aggaaagcaa gccatatgat caggaagcat gattttcagt ataagatgga aaagataaaa 30120
   ataaattaag gaaaaaaccc taaatctgaa ttagaaatat caatgtgaac tcatgtttga 30180
   atatatattc tccagccctg cccagggaaa ggcctggaag caaggccatg caggaccaaa 30240
   agtagcccag ccctcagtct ccaaaagcaa atcccatgca ggagaagcgg ctggtccagg 30300
   tcaaaggcag aggaagtcca caggagccga ggatgctggg ccacccagaa ggcaaggcca 30360
   tgcccaggct cctaggttaa ccaggccacc agcagccaaa cagaggggaa gtagtagtaa 30420
   tgaatatatc acagtgaata gatatcagag tgattcaaaa gccaacacaa gaaacaaaga 30480
   aaaaagacat ataaaacctc aattgttacc accagagtaa ctgtcacagc ttctgactct 30540
   gaaaactgga aattaaaggg aaagtatcaa acatttgctc tgttttggag gatgtataca 30600
   tagttcactc ttggttgata agggaaaact ggttttttaa taaaagaatg tcagtgaata 30660
   aagggaaacc aaatgacaaa ttaagaaaaa ttaccattgc aaattccaat taaagacctg 30720
   gcccagggga tccctgggtg gctcagcagt ttagtgtctg cctttgtccc agggcgcgat 30780
   cctgaagtcc cgggatcaag tcccacatcg ggctccctgc gtggagcctg cttctccctc 30840
   tgtctgtgtc tctgcctctc tctctctgtg tctctcatga ataaataaat aaaatatttt 30900
   aaaaaaaaga cctggcccag gacgtaaaga agtgggtaaa aggttgttgg gtggatattc 30960
   acatggagct aatgactggc ctgagtagtc catgctgaga gccaagggga atggccccct 31020
   gccccccaga gggatgaggg gtagatggag ccagtgccct gagaataaga ggcctgacct 31080
   ccacactgct gatgaaatcc aggcgtgggg gagtgtcagc cctacctgcc cgtgcacaag 31140
   cccttcgggg agaaaccagc agatgcacac agaatgagcg acgttctatg tgacaattgg 31200
   tctggtacct tccagtggcc actgtccctg gagagagaat gcaggggact attccagatg 31260
   aaaggagctt ggggaggtaa ctcccagcac aacccaggac ccagactgaa tgttggagtg 31320
   aaaaagtcac tgtcacaaac atacaaggaa aaaccaggaa aggaaacagt gagatcccgc 31380
   tcattgtcag tatgatggag gctggttttc aatagagcct gttgctatgt tcagaggcaa 31440
   accatcacct cacctgctac tgactttcaa atgattgagg gaaaaagatg aacagttaac 31500
   tctcaagcat gtccaatggc ctagacacga tctccatggc agaggacatg ggactggatg 31560
   cggagatcca ccgaagaaaa agcaggcaag gcagcaccag atggacccag aggaggactg 31620
   gcaagcccca gatgagactt tagcccaggg gggagggggg tctctcagat gacagctcaa 31680
   tgactccagc gcccttggta ataaggaagg tcactgaaac accaggagga acaagttaga 31740
   aaaatccaac tccaggcctg aaagcaaaag acacgaaagg aaggaaggta ccgtgtgtgt 31800
   ggaggacggc ccactaacac ctggacccct ggaaggagca ggggcaccag cgagcatgga 31860
   agccctaggg gcacacccca gtcctaagga gaggagataa caagaaacaa ttctactcac 31920
   aaccaggaac accaagaaac aggagcctcg ggcagaggcc accagaccca gggcggcagg 31980
   ttccagaagc ttctgttcag gaggccacca agtctcctgc tggaccagat ctactccagc 32040
   agatgtgccc tgggagaaac tgcttggcaa attgagtaaa gatgaacatg tgggacccta 32100
   tagcaatttc actcctgggc acacccccaa caccagtgcg ggcacatatg caccagaaga 32160
   atgctcacca cagcattatt caaaagaaat aagctgggaa cagggggatc cctgggtggc 32220
   acagcggttt ggcgcctgcc tttggcccag ggcgcgatcc tggaaacccg ggatcgagtc 32280
   ccacgtcggg ctcccggtgc atggagcctg cttctccctc tgcctatgtc tctgccaatc 32340
   tctctctctc tctccccctc tctctgtgac tatcataaat aaataaatat taaaaaaaaa 32400
   aaaagaaaca agctgggaac aatacaaatg tctatggaca accaatcaga taaatggtgt 32460
   gcaatactca gagacttcta tacagccaag ataaaggaca aactactgcc ccacacaaca 32520
   acatgcgtgg acctcataga cacaatatcc agtggaagta agactgggaa aggcacacac 32580
   aagatgatcc tatttcagtt caaaactgca ggtgcactgg tctacagggc cagaattcaa 32640
   agagtggtca cccctgggga agccaccgat ggtgggaaag gagaaggctg ggagaggcct 32700
   ggggatgggt ctgcggccct atctgagtgg gctggtgatc acacagatgt gttcactgtg 32760
   tacaagttac agctgttata aaaattaact caaaatgggt cacggaccca gatttgaaac 32820
   acaaaactta gatactcttg aaagaaagta gaggacaaaa ttccaggtgt ggtgacgagt 32880
   gtttacctct aacaccaaag acacgaacga tccatgacag aaataattga taaattggac 32940
   tccattaaaa ttacaaacct ctgccctccg aaagacactg gtaagaccgt gaaaaagatg 33000
   agccacaagc tgggagaaga tattcgcaaa gcacacatct gataatagtg caggcaggca 33060
   aaatataaga aggatctctt aaaaaataca ataagaaaat ccgttctaca aacaggtaaa 33120
   atatctgaac agacacctcc ccaaagaaag actcatggca agcaagccca tgagaagatg 33180
   ctccacatca cacgtcatca gggaaatgga aataaaaaca acagtgagat gtcaccatgt 33240
   acatatgaga atgggcacaa tccagaacgc catcaacacc aaatgctggg aggatgtggg 33300
   atagcaggga tgctcactca ctgctggcag aaacccaaca tggcacagcc actcgggaag 33360
   acagttgggc agtctcctac aaaaccaaac atactcttac catacaacca tgcagccatc 33420
   cttctagatg ttacccaaag gagttgaaaa tttatgtcta cacaagatcc tgagcaaaaa 33480
   tgtccatgat tgctctagtc ataatcacca aaaactggaa gcaacgaaga tgcccttcag 33540
   taagtaagcg aatggatgtc catctagaca gtggagtatt attcagcact aaaaagaaat 33600
   gagctaccaa gccacaaaaa gacatggaag agtctcaaat gtgtactact aagtgaaaga 33660
   agccagtcgg gacaagctat atactatata attccaagtg tataacatcc caaaacaatg 33720
   aggacagagg agggggtgat ggggcaaaga ggtggagctc agggcatatt tgggtggtga 33780
   aattattctc tacgatattg cacagggata cagatgctcg gcatttgtca aaatccatag 33840
   aaggtacacc accgagaata agccttgtga actctgagct ttagctaaca acaaagcatc 33900
   aacactggtt cagcaactag ggaacttctt ggggcaaggg gtggatggga actctgtacc 33960
   cccagctcaa tttctccata aacctgaaac tgttctaaaa agtaaaatcc ataaattttt 34020
   taaaattcgg ggaactgaac actgtgttgc atgcatgtta catttcatta acaaaagaaa 34080
   acacatccta agtgagactc tagtggggct aggcactgag ctatggggac ctgagctggg 34140
   gctgctggag gaagcctagc cccagccagg tgcccaggca gtggggtggt ggacctttgg 34200
   ctgagcctca tcaggtaaga cactgaaaag aacttgtttt ggacttcaac ttaaggtttt 34260
   tggacttcga gtcttaatcc aataatgaca aaatgtgatt ttggagttta atttctttgg 34320
   acttcaactt acaaggaaat ttaatttttg gactaatttc tctctgggaa attcctaatg 34380
   gaaaggattt atatttgaat caggagtacc tctccatggt tggccttttg aattaaatta 34440
   cctgaacact attcatttaa cctcatcaca gaacctctgg tgaaccactc cagatagagc 34500
   agatagatgg ggctgggaac aggaaatgaa agaggagaca gtttctcagc aaaggtgcct 34560
   gggagccttg aaatcgatgc actattctga tccatttcag agccttctga gggcatgagc 34620
   taaactgtcc accaatggtc cccaggagca tcagcaaagg gaaggcagtt ggtttgggac 34680
   aagtggtccc aggattgtta ggaaacccag ggagttgaga aaagaaaacc tagagtattg 34740
   caagagagca tccttagctg gccacacact tcatgtagtg gggaccccct acagtcaccc 34800
   tctgcccagc acatgcctct cccagaccaa aagcagctca tgggctggag ggtttgggag 34860
   aaggaacagg gcctgagctg tgcccccatc tcctctctct ttccagattt gtctgcagga 34920
   atcgagggct ctaccccagg gtctgtggcc aggtggtcct acctctgtcg gcagcaaagc 34980
   ccatctgatg aggtgagcac cagcaagcac acccaactct tgggctgacc agtgcctgca 35040
   atagctctga ccctcttgac tgattctgac cttggacaag ggacagcagc ataacctgct 35100
   gtacttatga ctcccctacc ccaagcccag acaaactctg tctactacct gttttctaag 35160
   catttatgct tattttaaaa tttctctcct tctacttata ttgaatttgt tctggttgtt 35220
   ttcttcaaac ttctggagtt gaacaattag atcagaattt ttaaaaatac tttcttcttt 35280
   cccaatacaa gcacttaaag ctgtaaattt ccctataaat aacattttac ctggatctca 35340
   tttgttttgc aatatttgca acatgatttt gtgataccgt tttcaataac cattcttatt 35400
   ttgatttctt cattgaccca cagaataaat aaaagtatgc tttaaaatta aaaaaaaaaa 35460
   actagcagtt cggagccttt ttaggggagg gagtggttga tttctgacat gtggtattac 35520
   agtcagagaa tgtcatgcca ttcagatcag atactggtct cctggaacat ggtgatgcct 35580
   gcacggtggc ctaaaacatg cacacgtata cgtgactgat cagttcttgt aaattagtct 35640
   caagcgtgct tggaaagaat gcacatcctc taaattggtt ctataggttt tataagatta 35700
   tttttttaaa ttgtatctta ttacatcctt gattattttc tgcttgatgc gtcaattcac 35760
   ttggaggttc atcaccttcc acaaccatag atttgtcaat ttccccttct aaagtctgtc 35820
   aatgtgtcaa catttctttt gtgttttgaa gctgcttgtt ggcagttcac aggttcagaa 35880
   ttatttattt tcctagtgat tattctcttg attatattta ataggcttct ttatcctcat 35940
   taccgcattt gccttttgtc tcttttgtct gatgctaatg tcaccaaact actttcactt 36000
   gattatcatt ttaatatggc atttcccacc tgttctttct actgaaggag cagcaagggt 36060
   tcccagctct gcccctcaaa aaccctgcca ctggccaaag aggggtaact catcattgat 36120
   aaaaacaata tatttggtat gttttgatcc attgcagtgt ggtttataat gatgccagaa 36180
   acaaaagccc ttggccccac tttcaaacaa tgctagggaa ctggctcatt attttgaaaa 36240
   tttgtaaata aatgggaaga aatgaacacc attctgcctt tcctacatgg actacacctc 36300
   aaagtaaccc catagttgag gagggcaagt ttctctatat ggaaggattc cagttgagaa 36360
   acaaaaaggt gttccaggtg gggatccctg ggtggctcag cagtttagcg cctgcctttg 36420
   gcccagggcg cgatcctgga gtcccgggat tgagtcccgg gatcgagtcc cgcgtcgggc 36480
   tcccagcatg gagcctgctt ctccctctgc ctgtgtctct gcctctctct ctctttctct 36540
   ctacgtctat cataaataaa aaaaaaaaaa aaaaaaaaaa ggtgttccag aagtacaaca 36600
   tcaccactgt tcaaccttga gcaaaataac cagcctatgg gcaaccatcc taacagccac 36660
   tgactatcca cagaaagaag ggaccagccc ctgcacagcc ccagccccag agagttcctg 36720
   cccaaaacaa aaacatccct ctgaccgtgc atagagatct ccaatgtcca ggaaaaacag 36780
   gtgacagaga agggcactgt aaatgtcacc actgggaggc aaaacccaag tgtagatggt 36840
   gcgaagctct gaaggatgaa gcaatgtggc tacttcaaca aataacacaa caaccaaggc 36900
   caggggcggc aggggagctg cagacacaca cactggagag tcatgtcacc gcaactcaca 36960
   ccttcccaag atcttgatct gaacaaacta ttaaaaactg tatcctccat aaaaacaaac 37020
   aaacaaaaat ccttcttcca tcaagttcga aaaagttttt tataaagttc ctgggaaaat 37080
   ttgaacagta gctaaatatt tgataacatt taagggatta ttattaattt taagcaggat 37140
   aatactgagt ttatgtcctc tcaaacaaga ctattttttt tcagatatct tttttttttc 37200
   ttttcttttc ttttctaatt tgttcaagat gtttcctctc tctggcatcc cactaccctc 37260
   tatttcttct tcttcttttt tttataaatt tattttttat tggtgttcaa tttgccaaca 37320
   tatagaataa tacccagtgc ttatcccgtc aagtgcccac ctcagtgccc atcacccagg 37380
   catccccacc cccctcccac ctccccttcc accaccccta gttcgtttcc cagagttgac 37440
   tctttacttt ttaaaaatac ataaggaaat agtcacagat tttgcttcga gggatctagg 37500
   gtctgggggg gcggggcaca gttgacagct gctggcaccc actggcagaa gcactggttc 37560
   attacactat tccttcaaat tctgcacgta gggatccctg ggtggcgcag cggtttggcg 37620
   cctgcctttg gcccagggcg caatcctgga gacccaggat cgaatcccac gtcgggctcc 37680
   aggtgcatgg agcctgcttc tccctctgcc tgtgtctctg cctctctctc tctctctgtg 37740
   actatcataa ataaaaaaaa attaaaaaaa aaaattctgc acgtgtttga atattttcac 37800
   catacaatat tttgtaagta atacaattta taaacaaggc tcaatgctgg atttctcaca 37860
   tctttgccag acacataagc acttaccagc atcagaggag ccatctttgt cagtgagaac 37920
   caggagatac aacagggaac cagagcctgt cctgcccgtc tcgtccagcc tgatcagctg 37980
   caagcgtggc gttgtcacgg gtgggaagcg atagaactgg aaggtgaaat acacggtctt 38040
   tggccacggt gctccctggc agtcctggga cactctgaat ccaaaaaaag ccccaaatta 38100
   atcaagtatc catagcagag aacctgggtt ttcaagagcc cagcaccaca ggcaagacac 38160
   tgcctcatcc acccacatga gcagctctac tgggatctcg gggagcccgt cagacagcaa 38220
   cccatttcag ccccagctgg agaaccctgc tctcagagct acatgaatac caagaattgg 38280
   ccaacaacac tgtccatgtc aatgacagaa gttcaggcaa agccagtttc acagcatcta 38340
   gagatggctg tgtgagcacc ctaaccaaat aaaaacaaaa acacatagca ttccttaatc 38400
   caggaagacc tcagcataga gagtcctgcc aactcagagc catacagagg ggtttcttca 38460
   atgcaaggaa agtgaaagcc aggtaaagag taacataaat agaatattta agatcgcacc 38520
   tgctaaaggc aagaaaatgc agcactatct cattgctttg tagacaatct gattcttcct 38580
   tttgggggtt gaatttcacg ggatctgtag gatttacagc ctcggctggt tgcttattgg 38640
   catccagaat ctcaggaaag ccgcaggctt gcaggagcac cattgaggct cgtgagagct 38700
   tacttccaga gctaaaacag aaaagatcat ggtggccggc ctccagaaaa gggccggcaa 38760
   agaggtagga cctgccgaca acccctctct ggggaaagcc tgttgttcag agtcagtctt 38820
   cccagcacag catgccaggg gcctcagggc cacctctcac ctcctggtct gggctcccac 38880
   aacaatcggg gtgtgcaccg gcgtaaaagg cagctcctgc agctgttcag caacagaggc 38940
   tcccaggacc aaggacgcct ggctcagctg ggcctccagg tgagagatcc tgccctccga 39000
   ggggggctcc tgctgagcaa aatgcggccc caagtgaggc caacaaggat cccagcctgt 39060
   aacaactgag gaggctgaga tggggggggt gttcattgat gagccacctg tcggactgct 39120
   agcttcagca aaggaaaata caggacgccc aagtgcctct tacactttag gcaaacaatg 39180
   aataattatg gtataatgta agtatgtccc atgaaatatc tggaacttac actaaaaagt 39240
   caccacttat ctgaaattct aatttatctg ggccaattcg tattttaggt gagtcaaaaa 39300
   gcaatgaggg aggtatcatc aattttatga actggcacca ttgagagatc tgctgtctga 39360
   gaaggtcaat taacataaaa accacattga tagatcagaa aaatgaaccc tccttcaacg 39420
   ctgcaagaca gaccactact cagctcccag ctcgcctgta tagacatttc tctgatccca 39480
   tcacctgaat attctagtca ttcctttgta ttccaccttc cacatccctc cagagcagaa 39540
   gacgtgtcta aatgaagaga ctgtttggaa atcagaatgc tctctttcac accagctcac 39600
   ctagctttct gtttcagctc aaaaggaata ccacaaaacc agcgataact ccgacatgtc 39660
   tcagctgcct ataataagaa atgcaggctt ttcataaata cgtgagccct agacatgttc 39720
   tagaataagg catctcacgg tactgttttc aagtaaataa tttaatggcg ctatttttcc 39780
   tcaatagact tccagtacag gactgaagat acagtttgca ggtgagccag ccaacttttg 39840
   aaaccaaact ctatgtgaat tccccaaggt cagtggaaac acaggcctgt tttccccact 39900
   agatgcatcc aaatccagga cggtgggctt cactacttct ccttcagctg ctgccaacaa 39960
   ggagagtgtc ctggagcgct ggaggcttgg atcagcctct atttagaact ctaacaaaac 40020
   tttccttccc ccttcatgta cagagatctg aggggggtct aactttgtct tgtcaggcag 40080
   agcaaaagcc taagtaagcc tcatagcagg gagagctccc acaccaaatt cccaggtctg 40140
   caggacagaa aggtggcaag tacctggggc atggaagtaa gccatgccca cccactcccc 40200
   ctgccaccct gtccctacac ccatgtcaca gagaacccac gaaggtctca gccacctccc 40260
   caaggaccag cacagctcac aaagtcaaag actccagcag atgtcaggag ttcagagcca 40320
   ttccctggaa ggaggaaggg tggccatgct tacctcctgg gccggcgggg gcttagagct 40380
   gtcgggttgc cgtggagggg gtgtggctga gcgggagcgg gtggtaggct gtggggaagc 40440
   tgcgagctgg gaaagggaca actagagggg gaggaagagt gggtgagagg tggaacacag 40500
   cccccctaca ttccagcgcc cccacacaca cacatacact cacactggca taaacccagg 40560
   agacatagca cagaaggcag tgaggtctgc aaccgcttgc tgggtggctg ctcctcgttc 40620
   ctttactagt tactaagagg caagattgtg gtccctacca cgtggtaaca ctggcataaa 40680
   aacaatagta acaaggaaca gcaggtagaa aagaggaagg gccgaggaag acaggttctc 40740
   tgccacttat gggctgtggg ggcagctggt ctttgaactt ctctggattt cacatccagc 40800
   cagggactga acaggtgcaa ggtcccttcc aggtctcacc accgctgtgc ctgggacaca 40860
   atcccgtcac tgaggcagtg actggaatgg gagccgacat gagctctagg gacagctgct 40920
   gagactgaat ccccacacca ctattactgt gtgactgagg gcaggccatt gagtcctttc 40980
   ttttcctctg tctcctcatc tgcaaaatgg gggtgaaaat agtacctaaa tcacaggact 41040
   atcacaggaa ttaaatgaga agtcgtggaa agggcttggc cagaacctgg tgcatgtaag 41100
   cctcctgtca cgtcgcagat gtctggaaag tgaatgcagc tgtaaggagg gcctacgctc 41160
   catggaagac ctgcctctca aatcttggac tcactttatg acatatacag ttagctggcc 41220
   cagcacagta ctgggacaga ggaacccaac acgagacaga tcaaaggagt ggctgggata 41280
   ggcatgtggc aatgggggac cccagcagca gccccaaggg cagagaagag ctgtggggtc 41340
   tgttcagctc cattcagacc cacaagctgt tttgagactt tcctgcctca ggaagtgggc 41400
   agcactgttc tgtggccctg aacacttagg gtgacatgga aactgctgta acgggccagt 41460
   agggaagctt gggaggctgt ctccagacct gtgtcttcag gcaaataaaa cgttcacttc 41520
   tgagcgagtc tccaagctgc ttggtacacg ttaaacccac ttccttgaga cagactttga 41580
   tagtgagtta ttcgactttt ctagataagt cattttccag ggtgatgctg gcaaagtcat 41640
   caggtagaca agccaactct gaccctgaac aaggtgatgc cggaaccact ggcaagcact 41700
   gtgacaagct tggggttaca aaggggtgga ctcaatggcc agccaacaac cagcacctct 41760
   cttcctatgc aggagaccct cgcacacaga actatctgct gtctgacagg cggtggccca 41820
   tctttaccag caccagctgt tcagcctatg ggcctggctt tggccccact gcccagggca 41880
   gtggtcagtg ccaggcaccc tctcctggta gagacaagac aggaggaaat gaagaccaca 41940
   gggcagcctg ggtggctcag cggtttagcg ccaccttcag tccagggcgt gatcctggag 42000
   acctgggatc gagtcccaca tcgggctccc tgcatggagc ctgcttctcc ctctgcctgt 42060
   gtctgtcttt ctctctctct ctctctctgt ttctcatgaa taaataaaat cttttaaaaa 42120
   actttaaaaa ccgaacttaa aaaaaaaaaa aaaaaaagga aatgaagacc acactgtggc 42180
   acctcagaaa aaaacaaagt ctactcggcc ctgaaagatt ctcagctatc agacataact 42240
   gccagagaga gcacatcatt ttttaaaagt actgaatgtg tcccagcaaa acaaaagaga 42300
   caccactgtt cttaatgatg cagagaatgt tctgcccctg gggcctttaa ttaatggatt 42360
   ttggaattct gaaacaggcc tctcagccca gacttacccc tggtcccacc ggcgagtcct 42420
   ggagggcagc gagcccctgg ggcggcagtg ctggcaggtc tgggggggaa gcagacagag 42480
   gtggggaggg gcactcagga gccttgggag gcctgactca gggcctgacc catctgcccc 42540
   cggctgcgac tggtgttgcc gctggcgttg ggcagtccaa tcactctgca gtaaggtcca 42600
   gagccgctcc cccaggtcac agcctcagag gacggagctg agctcgggct gctgtcactc 42660
   cagttcccct acccctggcc accccacctg gcctcatact ttctgactgt gtggtgtcac 42720
   caagaatggg cttcatgacc catgaagtcc tcacacaccc atcctgtgcc tttccatctt 42780
   cccatctgct catactcccg actctggaga gctggggccc tgggtcagct ggcctggcac 42840
   ccccactcct ctgaccaggg tggacttgct gttgtgctaa gtaagcaact ttgaagcaga 42900
   tgcacatgcc gcaccctgct gttcctgcct ccacccccgc agatgcccag cgcacacaac 42960
   ctgtcctgag tccagcgtac tctagatgtg ggcatgctgg gcccaggagg ggccggtggc 43020
   catgcatgtc cttgaccttg gtgatgccaa ggctggctcc tgcaggctcc aaggtgccat 43080
   cctcctgcca agtccagggg ccagccccat cctcacaagc caagtcaggg cgcttcccag 43140
   aaactgagaa acactctggc ctgcaagcct tcacccccga aaccagctca cagcagatca 43200
   tgaatccagc tgtgctccca agaaaggtgt catctcacca gaagcaaagt gtggaggagg 43260
   attccagcag ccaagaacct ctccctccac ttgagagtgc tggcggcagc tcactgccca 43320
   tcccaaggcg gaaagcatta cagagccact gagtcctccg tatcccaccc tgataccggg 43380
   actctggaaa gccccacaga ctgactctgg tcacaaggat gaaactgcag gaaatgggaa 43440
   gggaccagac cagtggctcc cccaccacca gggctaatgc tgtgcggaca ggtccccact 43500
   gactgcctga gacaaaggag cagctcacat ttctgctcca gcaagattca ccacctgtct 43560
   gaagacactc tgttactccc ccatcagcct caactcgctg aaaatcacag tcaacagaag 43620
   ctcaatcgtc caagcctccc tcctgtcaat tcagcttcta gaagcccaaa ctgcctccac 43680
   tgacacctgg gctatctgtg tcccacaggg tcacataaag acctgtgacc aggtgttttc 43740
   tctgctggga gggcacacct gcagcatcac acagatggga aacatcaaca cggcccagaa 43800
   agcactttgg tgaggggacc agacaggatc atagaggata taaagacaag tgcacaggtg 43860
   aacagaaggc caggcagtgg tcttcctgga gaaaactccc aggctggagg gcacagaagt 43920
   ccccccagaa gcactgggga gcccacagta caacttcctc cctgcccatc atcaccaacc 43980
   atgggccaga cctgagtagc atggagcact gaagcccttt gtcccaaatg ccagctatct 44040
   ccctaggagt gcttctctga aacatggctg tgcattctgg ggagaaactg aggcaggcct 44100
   gcggaagggt gctcacaagt ctgcctaggt ctagctgcag gtcaggcctt cactggcaga 44160
   agcctcatcc tgggagcaag gggtctggcg atcttagagg atgggggaac agctgcccca 44220
   aaaggcagag ggaagaaggg ctcatcgttg ctcaagacag agtcactgtg acttaaagaa 44280
   agatggggaa aaataagtgt cccactgggc cctgaaaata aggctttaat cactatttaa 44340
   aggttcttta gacactaaga actcatatgg caaatccacc tgaagaccct catttctaac 44400
   tatttggggg taaaaagcca tcctccctaa gacactgatc acaggaaatg agggacataa 44460
   acctaatctg tgtagagata aagggacagg cagctcagtg acggggtggc catgggcctc 44520
   gccgcagaag ttgctcagag tgaaggtggc tgaggccctc cagcacacag gatgccgggt 44580
   ggcccagggc actagggggc tgtagctgga gaggggctgg acttgcacac agaagagccc 44640
   aggcctgcct gcagcatcca cacctccagc tgtaccagcc ccaccacacc ggccacctgt 44700
   tgctcaggca ggcagggcag gcagggacag tgaggaacat gtgtcccaag cacacctgcg 44760
   tgtcccaagg tcccatcgct gcccacacca gccgcttcca gaatactgcg aagttaggcc 44820
   cagaacaata tgcactctgg cacctgggaa atggcattgc cctctggacc tggggggtgc 44880
   atggggctct ttggggcaag ggatgcgtgc acctgataaa gctgctgctg tacccagatg 44940
   tttcctaagc agaggacaca ggagaagatg cacgtcccag gtgctgataa gggagcgtca 45000
   ctacaaaggt gtcatggttc tgtccatgct gctgctggca gtactataaa acccagacat 45060
   agggcagccc ctgtggcgca gcagtttagt gccgcctgca gcctggagtg tgatcctgga 45120
   gacccgggat cgagtcccac atcgggctcc ctgcatggag cctgcttctc cctctggctg 45180
   tgtctctgcc tctctctctc tctctctcta tgaatgaatg aatgaatgaa tgaatgaatg 45240
   aatgaatgaa taaataaata aataaatcta tctttaaaaa taaaaataaa aataaaaata 45300
   aaaataaaaa taaaaataaa aataaaaata aaataaaacc cagacatgca aggtgccctg 45360
   cctcggcgaa ctaggcccca gaggagtggg gaatcccatg gggcacccaa agcctaaggc 45420
   tggctgaagc agtgactcgt ccccatcaag gcttctgctc tgagccactc actaacatcc 45480
   tacagcagag cacacacacc ctgggatccc tttaaatctg cccattcaca tggctccagc 45540
   tgggcggttc aaactcccgg tcctgcatga ctgcctgggt gacacgactg ccccgggggc 45600
   agggagcctt ccattggaga ggacgtcccc gcagcatcga cacgcagaag gtcacaacca 45660
   tgccaagaac ctgactcagg atattccggg aaaggccatg gtgacatatt tccctcgacc 45720
   cagctatttg tcaaatctac tgaagcaaac ataccaacat tctgacacca ccagaacact 45780
   gaagagcaaa atatgggtca gggaaattct gagtttccaa aaaccaagac cgagtgccct 45840
   ctagtggctc tgtgcactgc ttcctggagg gtgaggagtg gacctctgct ccctcattaa 45900
   ttctagagag gacataaaga tcattcatga cagagagccg aagtccctca cttaacatga 45960
   cagcctcatg tcatcaagat ttccaacaga accgactaag ttggtaaata tcaaaggaga 46020
   aaaagtttcc atcaccaatt aaacacgaat gaacctggga tgaaggcaat cacatcaatg 46080
   taacacaaat gcagcaataa aatccaaatt aaataaagca aatgcaatca ggatgggtcg 46140
   gtgcagacct gctggcccca aatggcagtg gagaatggag gacgtcggta ctagatcctg 46200
   atccctccag caacctgtca atgggtggaa tggcaagtct ctttgcaaag aactcaagct 46260
   ctgcacaggg gagacacact ctccccccaa ccatccaggg agacacctgt cccgctggcc 46320
   aatggatgga tctggagccc aaagtgagct gtctttgtgg ggaggggtcc ccttccccct 46380
   gaacctccac aacatgccct ggagcctaag aatgtctggc ctcccatggg cgctgctctg 46440
   tgcccagctg cccacgtgga gctggaggtg gccagcttcc aggggacagg ccagctctcc 46500
   ctggtctgca tcctgatcat acagcacagg aggggcatga agtgttggta tctgcccgtt 46560
   tgagcatctc aagatgcagg agcagctgcc aagcctgggt gccaccacac acagaagcag 46620
   cagagtgcca gggatgacct tggacttggg agtcccgagg tcaggcactc aatccacggc 46680
   tgacacatct gctagctcca tgccctgact ccacactctc tcccgagagc agcagcaatg 46740
   gcctccagtt ggagaagcac cctcccagct cttaaccaca cgatgaatgg tgggggacac 46800
   agcagcgtga aggggctcca tctcaagacg ttcatggaac acctgaacag gacttctctc 46860
   tgctgggctt cttaagcccc agggtgacct cagagctcct ggggtcgcta tggggagaaa 46920
   cccttcctgg aaggaagcta acaatgggag agctggatgg taaggggacc gggtgtggaa 46980
   ggcagacaga gagacagaat ccgagtgcca ggacacagcc agcctcacat cagctctgca 47040
   aacatgtgcc cagtgctttc ctggcccagg accccggtgg gcaatagaaa gaggagtaca 47100
   cacagccccc attcgtgaga tgctcctagg acagtgctca gggttggaca agtgcccaca 47160
   ccaaagatct gcacctcttc accatcaggg cacaaattag ggcacttgcc actagatgga 47220
   gccacccggc tcactctcag ccaggagctg ggaggggtcc tgtgtgtcac ttccatgcca 47280
   cagcaactga ctgtggggca ggtacctccg acatgtccct ttcctcgtgg tgctatggcc 47340
   agcagaactc agactacgcc ggccatctgt gaccacgaaa gacagagttg ttaccaacct 47400
   gcaggggatg tggagcaaga acaggaaata aaagcctttt gttttaaagc ggtgagatct 47460
   ggggattatg tgtcaccatg gcaaactgga ctgattcatt ctgagggcaa cagaggaagg 47520
   aggagacaca caacagtatg gaataaacaa caggtgtccc aagatcagag agttgtggaa 47580
   gagcacacta tgcctgggag atgcctgcgc cgggctccaa acatgagcag tttactctgg 47640
   gtatttcaat gcaagactat tttttctaaa cttctaaatt caaggtcaaa ggaaaataga 47700
   aattgaaacc aataatttag aaaataagaa cactgtagat caatatgtca gaaatagagt 47760
   caaagaagta cttggaaaga aaccacagga cctgaaatgt tttattaaac agacaaatgg 47820
   agggacagtg actccatggt tgagctgcct ttggctcagg ttgggatccc agggtcctgg 47880
   gatcgagtcc cgcattgagc tctccacagg gagtctgctt ctccctctgc ctatgtctct 47940
   gtctttctct ctctgtgtgt gtcatgaata aataaataaa atttttttaa aaaggcaaat 48000
   gaaaataagc taagcattca acttctttaa aactttaaaa aaaatgaagg gattaataaa 48060
   ggcaaaagca tataaaatta aattataatt aattacatta aaattaaaga cacaattaga 48120
   aacttcaaaa cagcacagga attaatgaag gtatgcaaga gccgactccc tacagaagat 48180
   aaaaggtaaa cacctctgat gaatctgccc aagagaagga gacactcacc aggagaccag 48240
   gaatggcaaa ggaatccaca cagagataga aattaggata tttacattac atacaagttt 48300
   atcccaggat ccattcatct atagactata tatatcccca aagttaacct gagctgagat 48360
   agaaaaccta aatagagcca taaccataat aaaaacttga aggtagggga tccctgggtg 48420
   gctcagctat ttggcacctg ccttcagccc agggtgtgat cctggagccc cgggatcaag 48480
   tcccacgtcg ggctccctac atggagcctg cttctccctc tgcctgtgtc tctgcttctc 48540
   tctctctctc tctctctctc tctctctcga tctctcatga ataaataaaa aaccttaaaa 48600
   aaaagaaact cgaaggtggt gaaaacgagc taaggaaacc ccactgagct gggagaagga 48660
   gtggagggtc agcaggtgcc atcccccacc gggtcccatc tgggtaccag cagggaccca 48720
   gaaccaatga gaagccgcca catggagttc tcttttcctc acgaactcct gttcaaaacc 48780
   atcccttcca gcctcctcct tccctctatc ccctctatta gtggaccctt gctctctggt 48840
   ctctcaactg gcctattggt tcaccatggt ctgcacatct cctttttttt ttttaatttt 48900
   tatttattta tgatagtcac agagagagag agagagagag agaggcagag acataggcag 48960
   agggagaagc aggctccatg caccgggagc ctgacgtggg atttgatcct gggtctccag 49020
   gatcacgccc tgggccaaag gcaggcgcta aaccactgcg ccacccaggg atccctccac 49080
   atctcctttt gtaattcctc tgccgttctg gaataaacca agttgctact caataacttc 49140
   gttgttttca aggttggcat gatcagaaaa tccttcccaa aggcactgag ttcctgatgg 49200
   ttttatagac aagttttacc aaaccttcaa gaaacaagtc ttgccttttg ttgaaattat 49260
   tttaaagccc agaaaaagag agaaagcatc ctaactcact gtgatgatta atttttttta 49320
   agatttattt tatttattca tgagaaacac agggagagag gcagagacac aggcagaggg 49380
   agaagcaggc tccatgcagg cagcccaacg tgggactcga tcccaggtct ccaggatcag 49440
   gccctgggct gaaggcggca ctaaaccact gatgattaat tttatgtgtc aggtagacta 49500
   gatgaatcac aggggcccag acaggtggcc aaccatcatt ctgagcgtgt ctgtgaagat 49560
   gattccagat gaaattccta tttgaatact ccccctgagt gacgcgctct gcccccccca 49620
   accctgcccc cgtgtgggtg ggcatcatct catgtgttga gggcttgagg aacaccagag 49680
   gggaggaagg aagaactggg cgttcctgcc tgtcaatggg ggatgcacat ctccttccct 49740
   ccatgctcct gctccgcagg ccttcagacc cagactggaa cctgcatggt ggctttcccc 49800
   cactctcagg cctctggatt tggactaatt ccccagcagc tttcctgggc ctcttctcag 49860
   cctccactga tggtgtgagc caatttctta taataaatct cttcctacat ggatggccca 49920
   ctggttctgt ttctctagag agccctagct acgcactcat tttgcaaaac gagcactcca 49980
   aataccaaag cctgataaag acatgacaaa acagaaagct atagatgagc gatctgcagg 50040
   tgatggccca ctcatcaaat tccacccaca acctgtgttt gtgagtaagc ttttattgta 50100
   acacagccaa gctcattcat atggtttcca ctacacagca ctggagatga ggagctacaa 50160
   cagggcatcc ggcccacaaa gcctaaaata tttactatct ggccttaaag tcagctgacc 50220
   tctgccacag atgcagctca attatggaga tgagacagaa atcctgaaca caggttaatc 50280
   aaatcaagac agacatgatt ataaagcttc ctaaaagtca ttccctctgt ttcttttctt 50340
   aaacagactt tatttttagg gcagttttat tatattcata gcaatattga acagaaagta 50400
   cagagaattc ccatgtactc cctgccccag atatgcatgg cttccccagt atctacatcc 50460
   cgcaccaggg tgctacacta ttctttttgc tacctaaaat atttggtagt tttttattgt 50520
   ctctttttaa aatcaatctt ttaggtgcct gggtggctca gtcagttaag catctatctg 50580
   ccttccactc gggtgatggt cccagggtcc tgggatcaaa tcccacatca ggctccctgc 50640
   tcaggggaga gtgtgcttct ccctctgctc gctgctcccc atgctggtgg gtatacatgc 50700
   tctctctctc tctctctcta ataaataaaa tcaaaattaa ctaataaatt aaattaaagc 50760
   ataagctctg tgagaacagg agcctaaaaa gtacctggca catcacagag tcccagtaag 50820
   gatttgcaga atggatgagg gagggaggag agaaagagag ccactaagca aaccctatct 50880
   tagtaagtgt tgccttaatt tctggctaat gtaaaatgat agaaacaaac aaaaataatg 50940
   gtagaaacag agttaacact gaatgtgcgg ggcccaatga ggacccactt ccctggatgg 51000
   tgtcatgggc actggtatgt gtccactgaa gactctgggg tcaaactctc catggagatg 51060
   tgctgcattt gcacctgccc tggggaccat cttgtttcac tgggccaacg gctaaaaggg 51120
   gaatgagcag gtggcactgc ccagcgatgg tgccagccag agatgacagc ggcagccgtg 51180
   aagcgtgccc agtgccaggc catcctgagc gcatccacag atgtttgccc actcaatcct 51240
   ccctacaacc tctggtggac catgtctgtg tttctttcca gttgtttcct tcttactgat 51300
   ctgcaggagc tcttcctata atattgcttc tttctgatat tctaattcct tcaattaact 51360
   tctcatggtt gtaggcactg taactgtctt tcaatttaag gtttgtctat cgacttgttt 51420
   gtggagtttt ctctcttaca aaagttttat attttaatat aatcctattt cttagtcttc 51480
   tttgtttatg cattgtctag cttgtttaag aaagctttcc taggtggctc agtcagctga 51540
   gtgtctgact tttggtttca actcaggtca tgatctcggg gttgagatcc agtcccaagc 51600
   agggctcagt gctcagtggg gagtctgctt gtctctctcc ctctgccctc cctggatcat 51660
   gcaaatgtgc tctctcactc tctctctcta aaataaataa ataaaatatt taaaaagaag 51720
   aaagaaagaa agaaagaaag aaagaaagaa agaaagaaag aaagaaagaa agaaagaaag 51780
   gaaagaaaga aaaagaaaag aaagctttcc taaccccaaa gtcacaaaaa tatcctctaa 51840
   ttttttttcg aaggcttttc aagttttatt ctttcctatt cagttctttg ttgcatccca 51900
   ttttacagct gaaaaaattg aggaagagag acagtcagca aggggtccgg ggtcatccaa 51960
   ttagcacaca tgcaccagtc aatgacacat gcaactgttc tcaaagttac tgaaagccgc 52020
   cacgttgtac acttccttcc agaacttgtc ctcactcctc acatggtctt gaagattcac 52080
   ccctgaggac atgtaaagac cttcttggtt cattctgact gctatacggc atctggtcat 52140
   acaaacatac cagctcgttt atccaccccc agggggagga gtgagcaggc caccatgtac 52200
   ccaggaacca gctgctctca acaacaggtg tctagaaaca gcacttctga gtagcagggt 52260
   atgaactaaa tattatgaaa ttgctcttca aagcagctgt ccatatagcg ttcctgcttg 52320
   cgtggtaaga cccttcttcc ccatgagcat cctcatgaac cttgtatatg aggcagctac 52380
   atttctccag ttgtgaagta tgagatgatt cctcatttta ttgtgcattt ctctgatcac 52440
   gactgatact gagccctgtc tcatgcttac gggcccctct ggcttcttcc atagaaatcc 52500
   cctattcatg tgttttgccc atttttcttt cctgttaccg ttatttctta ttgatttgga 52560
   acagctattt ctgtaacctt tcttctttct aatcttctaa ttctagagtt ttctaaaatt 52620
   tctaaagttt tatttcataa tccacctaca atttattttt gcacaagata tgaaattagg 52680
   atctaatttt atatgttcct tatggataag cattcctgca ctgttaagtg actggtccat 52740
   ccctattcct ggtggtaagc agaacgatgt ttcctacccc agaaataccc acatcctaac 52800
   ccccagagcc tggcaatgtg atgttgcact gccggtaatc atggctgctg atcggagaga 52860
   ccatcctaga tgtttgggtg ggcccaatgc tatcacagca gtcatagcgg tcctggtaag 52920
   tgccagggag ccaggaggat cagagtcaga gggagatttg gaaactgcac tgctgacttc 52980
   gaagagaaga gaatgagcca aaggctacta gaggctggaa aggcaaggag gtggagcctg 53040
   ccctggagtc tccggaagga accagcccca ccaaaacctc taccttagcc tgtgagaccc 53100
   cttttggact tctgacctcc agaactgtgg gacagcaagc tcatgctatt ttaagccatt 53160
   ttacatttat ggtaatttgt tacaacaata agagtaaagt aatacactta tctataatgt 53220
   gtccagtcat cacactctgt cttcttatct gtccatttct gccccgatgc cacactgttc 53280
   tagttactat aactataaat ctgccaatta ggaaaactga agcctctttt ctacttcaaa 53340
   attgccttgg ctattcttga ccatgtaatt ttttaattaa aaaaaaaaaa gaatctattt 53400
   caaagtaaaa taaatgcagt gcacgaggga ctacactgca gcatagagca tgagcccaac 53460
   tcagaagtca tgaaaacaca caacatagct ggtgaatcac agtagcacag agcagccagg 53520
   aaaactttca aaaccactag accatcaggc ctgggtggag gatctcagca gcccaccgac 53580
   atcaggcaaa gacatctgtc tcccaccaac gtagagtaag caaataggga ccagactgac 53640
   cgccccatga aacaaatttt tttaaaggga caaaatatat gaaacaactg gcctcaagac 53700
   attagaattc aggcaacaaa ggacagtgat cctcagggac aggaaacaaa agaagtgggc 53760
   cctgaaaccg ccccaactct cagcagctaa tgagtctgtg ggccacagta cagggagacg 53820
   gggcaccagg ggcccccagt ggagaaaaca gagctgaatg tccaagaaga ccgaggaagc 53880
   tcccatttgc agggccaagt gccagacaag acaaggtgca cagaaagaga gctctggtca 53940
   tttacagagg ctcccctcta gaattcagta ggtgactggt caggaaagta cccaatgctg 54000
   gggccagagc cattggaaca gtccccagtg cttacatggg gcagaaatag acttactgca 54060
   cagacaggcc aaactggcaa acctcataat tcaggagatg tcaggcagag cacacaaagg 54120
   ggtcttgtcg tggggaataa ttagccctgg actaaaagct ggtctggtcc cacctaacaa 54180
   cccttaaggg caagagccca aaggatcaaa ctgtttccaa agctcaagag tattcatcaa 54240
   atacaaacct atttagcact ctcaatcaaa gaaaattcac aatatctgca atccaataaa 54300
   aaatactgat ggcagataaa caggaaaata caacccaaat taggataaaa tgactcaatc 54360
   aaaactgagc cagaattggg gcacctggtt ggctcagtta agtgtctgac tcttgatttt 54420
   ggttcaggtc atgatctcag ggtcctggga ttgagttcca agtcgggctc cctgctcagc 54480
   ggagggtctg cttctctctc tctccctctg cccaccgcct acttatgggt gcactgcaca 54540
   ctcctattct ctccctctca aataaataaa atctttttaa aataacttaa aaataaaata 54600
   aaaataaaaa taaattaaca taattctggg aagcagagca agcagcagac tgggtttaaa 54660
   gcccagatct gctcacaaca gtcaaggcaa ttgggaaaat gagacccaag cctgcagacc 54720
   gcagagccac agcgggtaca gccggtgttc gccccaagca gccaagtgat aaggcaattc 54780
   catcattgtt tagaattaat ttgattagaa ggtcttcaaa caaaccagct gcttgcttcc 54840
   agcaccacag cggcaccata aactaaccct tgaaaactcc tttccaggaa aagtcagtat 54900
   atcttcccca taaaagctac ctgtacaggc atgtccccca aggacatgat ctattctggc 54960
   atttccacag acacaggtga actacacata ctacatttca ctagtgtcct ttttgagtag 55020
   cttaatccaa aatatttaat cataacattt acctattttt gtaatctgga atactatctt 55080
   tcatatttac ctttctcaga gcttactatt tgttgtattt ttatttttat ttttttaaag 55140
   attttattta tttatttatt tatttcagaa agagagagag caaaagcagg gtgaggggca 55200
   gggggagaag cagacgcccc actgagcaga aagcccaatg aggggctcaa tcccaggacc 55260
   tgggattatg ctctgactca aaggcagatg cttaaccgac tgagctgcct aggtacccct 55320
   tgttttattt tttaaatgta actaagaata tatgtgaagg aagaagagac gctagattta 55380
   caggagagaa tgagtgggaa ataaattgta ccctttgttt agtattttta agcgatatgt 55440
   tctatgcctt caatgcattc gactttatta agtaaaactg gaaacactct aaacaaaccc 55500
   tgagagaaaa tccatgtctt ccgcccacca ctgcagggac tccatcagat atcctggcaa 55560
   cagcctcggg gctgtggtgc tggacgtttt cagggctaca ctggtaagag tggttattag 55620
   cggacacact ggtggtaaac agcaatccca agagcaatgt gacaagcaca atattcaaaa 55680
   attgaaagat caaaacaact aaaaccatca aatgcactgc ctttttgaga agaaggaaaa 55740
   tactcaatga tgtggtttgt cccaaagcta cggattgttt ggcacctgaa tatgttccga 55800
   tacctcagaa aaccttggaa gatcatgaag acagtggcca gcatgtccag gtccaggcca 55860
   aagaactaag gggactgctt ccaccaacag taaattaaat tcaacaatac caccttccat 55920
   taactagatt ctcaactcag aaaaaatcag tcccaaatga gtcccagacc cactagtgcc 55980
   tccgctccag cagccaggac ctcagtaagt cctccctggg gacagagaag cacccatggg 56040
   gaagatggat aggattaagc tccccactgt gggcttatac attagttcag tccctacctt 56100
   tctaactttc taagagacta acttcctata tctagaacat tcttccagct ggagcagtct 56160
   ctagaagcct caggtgcctg aggctcagtg ccctgccaag tgaacttcac caacacccct 56220
   ctgacatcag ccctgcaggg acctgaccca tgggcttctc agtcagatgc tggcacaggg 56280
   gtccttaggg gtccttatgg tccttagggg tggtgaggca gaagccaaga ggccctgacc 56340
   agcaggtgct aggccagtca gacacaaggc tgacagcaac gttcgctgct ccgccacctg 56400
   cacacatgca gacacatcac tgcccccagg aagatgttgc tctgagggta gccctcactc 56460
   tccccaaagt cgccagctga gatcagacac cccattcttg gaatgcttca agggagtaaa 56520
   aagcaccttt tctaaacaac aaatgtacag cacgttaaca acaacaccca gagatggcag 56580
   cccatggctc tcggtcaaac tcttactctc aacgctgcgt ctataggact cccatgccaa 56640
   gtgcccatta ggtgtcactg gcagagcctt ccacaagcct ctagtcaggg ggctgcaaga 56700
   cacaggtgac aacacacttg gccaccctct gtcactgtcc aattctacat ccccgtatta 56760
   agcaaaagct caaccaactg actccaatta aagcaatctg tagcaattat ctgataattg 56820
   ttattgtact ggactaagta tttttcctta agaacagatg gaagtcatgc tatgtgctca 56880
   aaaagtcagt tctctgcaaa cacttacttg caggagatgt gggtggcttc ctggagggct 56940
   gccgctccgc ctcagggctg tggaccagcc ccgtgggtgt gtccaggtgt ccctcagggc 57000
   tcagcgagaa gtggaactgg acagtccctg actccacttg ctgcacctac aatcaaaaca 57060
   agaacaatgg cagctgcctc ctttcatcgg tgcccttcta ttacaatgca accagcccca 57120
   aagtctacat aaaaccaggg attcctgaag ccccgtgttc tcagatcata atcaagtcac 57180
   tttctgagca gaaacaatca gccgtggcat tgaaatccca gaagaccaca ttagcacaga 57240
   atggctctta ctcaaggaga aattacattt ccccagtttt gcacgttttt aaacaaacct 57300
   atgcaaaact gaccttttaa atgttggtat gtagttctgt tcaacacatg tactcactac 57360
   cggagttggg gtgcagcgtg gctccatcac ccaaacaatc cctcgtgttg ccccagtctg 57420
   actcccccaa ccccctgttt ctgagctgct cctgtcatgg cagcctcggc tctccagaat 57480
   gccacagaca cggaacccaa ggatatataa ttaagcctag cttcttacac tccacataat 57540
   gctgatgaga ctcatcccag taagtagtac gtatcaatag ttggttcctt ttcctgctga 57600
   gcagtattct atggtaccat gtgcatggtt tattcatcca tttaagcaca tttaggctcc 57660
   tttcagtttg ggtggttaca aacaccgggg tacatgtgtc catgtgaacc taagtcttca 57720
   tatctctagg aacaggattg ctgggtcacg tggtacgtgt atatttaatc ctataggaca 57780
   ccatggttta cttctagagt actctgctcc caatttatat gccaatcccg cagcaacagc 57840
   accttacagt ggttgtcgca gctctactcc aagtgtcaca ctgtggggaa ggattcctcc 57900
   aacatcactg ctatcgtcac agttctttgg actcttctaa ttcctgggac tttctgtata 57960
   aattttagga tcagtttctc tacatgtaga ccaaaaaaaa aaaaaatcct actggaattt 58020
   tgaataaaat tacattatct gtacatgaat taggggagaa tttctatctt caacacactg 58080
   aatattccca tgcataaaga tgctgtgtct ctccatttac ttagagtcct ctgtgatctc 58140
   acccaagagt aaccacacga gaactgtgac atgcaccaaa gggaatatgc tacagttcac 58200
   aacagacaga tcctgtacac actgtgttag atttatctta ctgtgggaag gccttgataa 58260
   gtaatactgt ttttttttta ttttgttcat caactgccct ttcttaatat acagaaaaga 58320
   ttgatttttc tatcttgatc tcctattgtg tcttgcaacc tcaataaagt cactaattca 58380
   ttctagcagg tttttgtaga tcccttggga ttttccacac aatcatgctg cctgcaagta 58440
   agtacagttc tgtttctctc tttccaatct atgtgtcttt tattttgtct tcttgcctta 58500
   acgccctggc taggactttg agtatgatgc tgaacactga gtgcacatcc ctcacttatt 58560
   cctgatcttg gggcgggggg gcacccaaac tcttaccact ccataggctg ctcactgatg 58620
   ggtttgtgtg gatgcccccc aagcaggaag agggactgct cttctactgt tagtatgctc 58680
   acagttttta tcataagtca gtgttaaata aatcttatca aatgcctttc atgtgtcata 58740
   cagttcatct cccttaggct attactatgg tgaattatat ttattgattt cttgcatttc 58800
   tgggataaac ccccacttgg tcatggccta gtcttccttc cacattgttg ggttctattt 58860
   gctagcattt tgttggttgt tttgcaactg tgtccatgaa ggacacatgt ccatggtctt 58920
   cttatgctgt ctctggtttt attatcaggg cagcacaagc ctcattacat gaattaggag 58980
   atgttcccat ccttctattt cctgggggag accgtattaa actactcttc tgtctcttcc 59040
   ttaagtcatt ctgtagaatt cacagtgaat aggcctggat gttctcaaca gtgttttagt 59100
   catgaattca gtttctataa tggtcatagg actactcagg ttgtctacat catcctgagt 59160
   gagttttgat catttctgat ttaagataaa ctgatccatt tcatcgaaat tgccaagttt 59220
   acaggcacag aattgctcac agtgttcctt tgctgtctgt ttgatgcctg tggggccagt 59280
   agtgctggtc cttcttccat tcctaacatc agcagtttga gtcctcctct cctgttgtcg 59340
   ttcttgctag acatttatca attttattga tctttccaaa gcctcagttt ttggtttcat 59400
   tgatttcctc tgttttcgtt ctctatttgt tctgttctgt ttcttctgct ggatttaggg 59460
   ttattttgtt cttctttttt ttaattttat ttatttattc atgagagaca caaagagaga 59520
   gaggcagaga cacaggcagg gggagaagcg ggctccatgc gggcagcccg atgtgggact 59580
   tgatcccagg actccaggat catgccctga gctgaaggca gacccttaac tgctgagcca 59640
   cccaggcgtc cctgtttctc tttctcaagc tcctaaaagt agaagataag attactgaat 59700
   gaagaccatt cttctgttcc aacacaagcc cttaatgcta tcaatttccc tggaagcaca 59760
   tgtcaagtgc atcctccacc ttttgatgta ttttcatttt tgttcagtcc aaaacatcct 59820
   ctatttcctc ctgagtcttc ctctttgatc tatgggttat ttagaaatgt attatttact 59880
   tttcaaatgc ctatactttt taatcttatc tttgctaccg acttctggtt aaattctgtt 59940
   atgaccgaaa aatgtaccct atataattcc aattctttag aaatcatgaa gatttgtttt 60000
   atggcccagg atgtggtctg gctgagtgtt ccctgtgccc tagaaaagac tatgtgttct 60060
   gctgctgttg ggtgcagagt tctgtagacg tcatgttgat ccagacggtt catgatgtcc 60120
   tttagctgtt tcatgtcctc agtgattttc tctatgttcc ctggtttact tagagcgatg 60180
   ttaaagtctc caactataac tatgagtttg tctatttgtc ctttaacttc tagtaattag 60240
   tttttttgct ttatgtattt tgaagctctg cctatactgg atgcacacac ctttaggact 60300
   gctatgtctt cttggttaat tgactttttt atcatatgtc atatccttct taattcctga 60360
   aaatttcctt tgctccaaag tccactctgt ctggtgttga tacagccact cctgtttctt 60420
   tttggttttt gttttcgttt ttttttaaag atttatttat tcatgagaga cacacacaca 60480
   gagagagagg cagagacaca ggcagaggga gaagcagact ccatgcagga agcccgatgt 60540
   gggactcaat cccggggctc caggatcaca ccctgggtca aaggcagcgc taaaccgctg 60600
   agccacccgg gctgccttcc tgttttcttt tgattggtgt ttacattgaa tgttttggtg 60660
   tctgttgagt gtcttttctc tgaaagttga gattttcctg cctctttgca tctcaattat 60720
   gtttagatta tatcacagac attttgaata ctatgttagg agactgtgag tctcatttaa 60780
   attctgtgga gaatggtgat atttttgttt cgtcacgaat cagctcaatg ggttcaggcc 60840
   acagtttcca accagccttc tatgcattgt cagcccctgt gccatgcttt tcagatccct 60900
   cctgcatgtg atccacccag ggaaactctg gaacggggct ggaggcctag ccacagtcag 60960
   ttctcagggt ctttggaact gtagttcagg gtcagactca ggcagcctga gggttaaccc 61020
   agcatttctt aatgttatgg gattgctttc caagcctctc cctctccata gcctccccaa 61080
   acctgtctgg gactttacaa tcctccagcc agaccccact gcgctgcaca cttctgggaa 61140
   caaacagtcc ctgaagccaa gacccctcag ggggaggaca gagtagtttc ttaaaagagc 61200
   tcaggagtgt atcaagcaag ttctcattta tcattattgg aagtcaatat ataatgtcta 61260
   aagttgataa acctaagaaa tagcagaagc ataatatttg gaaacaaagt aaatatcata 61320
   agtcagagca aagagttgaa agtggctgcc tctggagata gaaatttttt ttaaaagaca 61380
   attatttgct caaaggaaaa atacaggagt taagaaagaa aacataatca taatgtacta 61440
   cttagcttca cagtaacatc aaaataataa cagaaacccc cagatttacg tagccaaaaa 61500
   ttatgggatg tttctaatat ttatactttt ctctgattag tatctgccaa ttccccttaa 61560
   ctgtaagttc acaggatcgg ggatcttttt ccaagggtat aagtgctcag taaaagttca 61620
   ctgaatgggt aagtaagaca cagaatgacc tggcataggg gcagagacaa ggtgcacctc 61680
   actcaggggc atctctgccc ctaagcctcc acactacctg cccagacatg gcactgctgt 61740
   agaagacaca atgggaagaa gcagaggagg aagcagaatt aagtttatgg ccagttttag 61800
   ttcattattt taattagtta tgtattttag aatagtgatc ctcgactaga ggcaattttg 61860
   cccttgagag acatttggca acgtctgcag acaactcagt ctatcatagt tgggggcacg 61920
   ctactggcat ccagcagaca gaggccaggg ctggtaacga acatcctcca atgcacagga 61980
   caacccccac agcaaagaat tatctggccc aaaatgtcag cagtgctgag ggtgagataa 62040
   cccaattaga agtgatattc cttgtgaata atggcagaat ttgatatatt tgtgtcctca 62100
   agatagtcaa gccttccata ctcatcccca atgcagttta cacactcagg gagtgaagga 62160
   gatgtagtgg catgacaagg ctaaagttaa atagaataga tatatattta aatacataca 62220
   catatacatc cagagaggga aagacagagg caatggctcc aggatctctc acggcagttt 62280
   tataataagg gctcagacta gtccatgggg gaaggcagca aaatgtgctc ccataatgag 62340
   atatccttat aagaagggaa gctgcacaat cagctgttcc tgatccaggt ccctaaagca 62400
   tcacccacat cataaataac attgaaaata acatttgtgg cgagagtcag cacacggatg 62460
   atgcatggaa taccagggat ggtcctttca cctgagaaaa atgagacaac tgagctgtag 62520
   acggctccca gccacactgc tgaaacagca gagtgtggta caggagtgca actgggagaa 62580
   ccaagcaccc ttgcgtgaca ctggcgtcag gatgggccag ccagagcagt gctttcctgc 62640
   gcggggtacc tgcagctccc tggcaggtga cccctcactc acacttcaac tggccctgcc 62700
   accgtttacc ggtcttctcc accggggttt tggagatagc ttcattccaa acatggattt 62760
   taaaagctcc tgctgtggcc cgccctccat gcaaagctgg aaggagccaa gggccagtgg 62820
   gcaagagtaa caagtcacag agatgcaccg ggctcaacac caactgccag gcactgacca 62880
   caaccctggt accaccaaag ctcattctcc atagcaaaag gttccaagag tcctttttga 62940
   aaagacacat atagcacaga agcagatgct gtttgcacag tgcatacaaa taaaagccac 63000
   aggccccaca ggccaccttg ttgagactta tgtgtgaatg gggatgtcct aagccccgag 63060
   ataaaacctg cctcagtgag ttcacagcca actatgactc agcaatcatg cttgtccttt 63120
   aagggcacgt ccatgacatg aaaaaaattc tgttcatcac atatccacaa acatatctgt 63180
   caacttctgg ttttgaaaat gtgtgaactt gctggaagct cacaggcaaa gattcatact 63240
   tataaagtat ctgaacaact tacctcttca gagctcatgc tggcaggggt tactttatag 63300
   accaaacagt gtgaggggtt gggccggatc ccaccctgca gaggtaagat caccttccca 63360
   gagccggatt ccagcaaggg gttccaaaca gcccagcgaa ctatgtgcat acacgccaca 63420
   ctggacaaag aggtgaccga ggctgcctaa gaagggaaat gaggcacatt ggtacatgac 63480
   ttaccaccaa acccaccgtg acttaccacc aaaccaaccc tgcaaacgtc actagggagc 63540
   agtgcagcca agcagggatc acacagaagg aaccagacca ggaggagggc aacatggaag 63600
   cttggggcag ggggcaggga agcagcatcc aacctaacca gtaccctttc ctatactgat 63660
   ctttcttctg gctcaaggaa gtagcagctt ttcttttaaa gaccaacacc atccttctga 63720
   tccctcctgt tcccccgagg ctcttgtgca gatctataag catgctgtat cttgactaca 63780
   tctgcccaca tccattagga aaatactagg aatgacactt ggtgttagtg acaggaaact 63840
   ggaaaataat gaagaatgcc catggaagtt caatttggta ttatttgcac acattttctc 63900
   caaccacctt ttgctcataa tgaatggtac atttcatata cgtgactctc catggatctg 63960
   ggaagttggc caggtccaat tttctgcacc gtcccttctt ccgccttctc tatcactgac 64020
   caagtgaaag caatgaggac actaaggaaa ccggtttcag ggctcaactc agcagtttta 64080
   atggcaccac cttcatccat tcacccagaa agaattaaga ataaagtcac actccaggcc 64140
   tggaacccgt gctcagctgg gcagtgagac ttgcgattgc cgccttgggg gaggcaacat 64200
   cagagacccc agctctgact gacagatgcc gcaggttcct tgggaggcag cacgtcagga 64260
   ccccaggaag aggccatggg gcacttggca agagtccagg caggtgtttt ccagcttcag 64320
   agcacagctg tcctgaccag agtcacattg aaactatcca gcccaaggag gcagcagcca 64380
   cacctgtcac cctgactccc acctcagtgg tgatgtggga ggaggaaaac ctgaaggtgt 64440
   aggcagtact tggagcatgc acacagcatt ccacctctta atgccctctt cacaggtggc 64500
   agacagctgt ctgtgcccct ctgggatgag gacagcaaag gaactgggtg actccgtgtg 64560
   gtatccaggt ggctgaggga ccaaagccct ctgatgctct aaaccaccag ccacatctgt 64620
   gaacccagac agccctctca atgggggcca caggctaggg aggctgggca gcctacaagg 64680
   agggggcctc gagctcttag aaaggttgga agcccatacc tcatctgatg gtaaaggaca 64740
   tcatccccaa ggagaagaga gctgtatcag aagccacaga gtaaggcaag actccagcag 64800
   agctgctaag gctcccaaca gaccccgctc acttgctggg cgtgggtgag ctgccgtgcc 64860
   attgtggcac ggacttgtgg catgggcagg actgactgag cttgagtcca aggcagactg 64920
   tggcacacat acacaaaagg actctggcca gaacccaatg cagatggttc aatggtcagc 64980
   caggccacac cccagccgag catatccatg agaagcatcc aacatggaaa aggaagcaca 65040
   ttcatctcac ccaagtcaga ctatccagac tgttccttag tcaggtagtc tcccagcctg 65100
   ctgtatccct agtcacatgg ggataaatgt ccagagtcag tgtgtggtct tgaattaatc 65160
   cctttcctga agccccattg caagtggccc aacctcactt cagcagggcc tgttctcatt 65220
   ctttactgga gcccaacttc agatcatagg ctaggctccc atcacatatg cctgggtcac 65280
   aggggtaacc aggtccctgc cagttcaatg acaaaaaaca ccaatcctga aagtacaaag 65340
   aaccagcaaa gtaagaagcc taagcatatg tctctgccac acttcaaacg tgtcaaatac 65400
   gtagccaatg cctgccgctc ttcatccggt tagctgatga cagtgacata aaatggtact 65460
   gagcaactgt gcaaagttga ctataaactc actgctcttt aaaaacaaaa ttactataaa 65520
   tgtattggca aaactggaca ctaaaaaacc atgtgattcg accatttctg gactactttt 65580
   ataaacagcc atctcctata aaatccagtt tattactgga tcaaaaataa aatggcacac 65640
   ctgggtggct cagcggttag gcatttgcct tccagctcag ggcatgatcc tggaatccca 65700
   ggatcaagtc ccacatcggg ctccctgcat ggagcctgct tctctctctg cctttctctc 65760
   tctgtgtctc taataaataa ataaaatctt tttaaaattt tttaaattta aaaaaaataa 65820
   taagataaag tatgtatcca agcataacac atgctattcg tgcaaatctt ctggtttagt 65880
   ctttcaaatt aggcgattct gatatgcagt tttccaattt cacagtccct ctggtttcct 65940
   agttctcaat cgataatggc tctcccctac gcggagtacc tgtcagttat acacataccc 66000
   atttcccctc actttcgtct ctaacagcta caacaagaag agatggctag cgagtcactg 66060
   atcactcctt tgcaaagatt tctgtgacaa ttaggacttg ttggcaattt atcagcctgt 66120
   gccctcttct gccaggctgg ggtgacacct gcaggtgaga acctcctcaa tctcacacac 66180
   ctcgaatccc cccagctagg tccctggctc tgagcaggtc aggtgcatcc aagcaggtgg 66240
   gcagaagaaa agcaactatt taactggtca acaaaacaag gctcttcctg tcagtgaatc 66300
   tagtggttga gataaaatca gttgctttct aacatcttat ccgcccaatt gggtttggct 66360
   tcaagaaaag aaatacaaac tgaaaagaga aaaaaaatat tgacttgttc cccatttaga 66420
   cttttattta taacaccaca taatatacaa gaggccaaaa gagtacacct gggttatata 66480
   taaaaagcca ggttcttcag gaacagaaga actgcaccaa cagtcaccag gcaaagccca 66540
   aacagacaag ggaccctgac ctctgggaat gctgtaaacc aggttcccta gggtgggtgc 66600
   catggtggct tcaagaaggc aagtcctcag cctaatccct ggaccctttg tgacctgatt 66660
   tggacaaagg gtccacgtaa acgtaattaa gtattatgag attaggagac ctagattatc 66720
   caggtaggcc tcaaatccaa cgacaagtgt ccttagaaag caggatagag atttgagaca 66780
   gagggaaggc cctgtgaatg agaaggcaga agctgaagca acatggccat gaacagaggg 66840
   aggacagcag gcgccagaac ctgttagagg caggagggac cctcactgga gcctctggag 66900
   ggaaccccac cctgtaggcc cactgctttc agcccccatt tgcagctgat tgtcaatctg 66960
   tggcctccag agctgagaga ataaatgtgg attgctttaa gacacatcca gttcatatca 67020
   atgaacagca gacactcatg caccacgacg aggcaccgaa accacagtgg catgcgggat 67080
   ctccccagta agtgaccctg ggcggcagga tggcaccaac gcgctgagca taaaggtgaa 67140
   agtggacaca atgaacccct gcaggaacag gatgggctct cctaggacac ctcaaatcga 67200
   gacataacag acatcaaggc aggcagatct gcctacagga aaaatggtgc tgaggtggcc 67260
   aagacaccct gggccaaggc ccatgggtga aaaccctgag ttccagtggg acaccacttg 67320
   cggtggggtg gggggtgcct ctgggaggtg acacaaatcc gatgaaagtg ttggggggtg 67380
   tttttagtgt ccttgcaaga aggggaggag gggagagaga cctcccgcct tccacacaca 67440
   cacaccaaca cactcctaca cacccctaca cacacacacc agggcagcag ctgcaaggtg 67500
   gggccccact gggaacccat ctgcggctcc agcacctgga gggataggag tctgacgttc 67560
   acatggcatt tccttcggct gccacagcag ataaagacaa ggccaaacac aggggtgtgg 67620
   gtcaaaggca gaccgcagag caggaagagg tgtcacacac tgtcccgagg gagaacaggt 67680
   accctcactc acaaaaatca ccacagaaaa acgggttaaa gaagtgacca gacagacaac 67740
   ccacaacccg actgcacatt ccacttcacg aactgtccct caccatgagg ccatttctcc 67800
   cctccaagta gcagaaccca aagtatgaga ggccactcct gcaaaggatc atgctcttgc 67860
   ccctggaccc agcatctcct tggggcgcac gcgcacagcc ctcaatccag cacacccttg 67920
   gggcacacac ccctcgatcc cgcatgccct caggcgcatg ctcacacccc tcaatctagc 67980
   atgccctcaa ggtgcatgcc tacacccctt gatccagctc accctcgggg cacatgctca 68040
   cacccctcaa tcccgcacac cctcagggta caggctcaca ctcctcgatg tggcatgccc 68100
   tccgcgtgtg tgcctacacc cctcgatcca ggacctcctc agggcgcaca gccatacccc 68160
   tggacccagc acccctcaga gtgcatgctc acacctctca atccagcaca cccttggggt 68220
   gcatgctcac accccttgat cccacatgcc ctcaaggcac atgctcacac ccctcaatct 68280
   ggcaccccct cagggcacat gctcacaccc cctcaatctg gcacctcctg ggggcgcacc 68340
   ctggagacac cccagagcac aaggtgagcc aacacacaga cagttccacc cacacctggt 68400
   ccacctatag gccctgcaaa ttgctgagct atccaaccac aggaaatgca tgcacaagct 68460
   ggggggcaca agcagagtac atgccgttgg aaaaggagga gacacgctct gtggccagcc 68520
   ccgcctgatg gccagggcac cccaagagtc acccacgcac atggccactt ccctccctca 68580
   ccccaagaag cagcccagga gggatgcact gggagcaaca gcagcggctg gctccagaat 68640
   gaacagtgga agggacacag aggagcaatg cccttctgaa accagctttg ggtagaattt 68700
   ttactgaaaa gcctatcagt gtcctacatg ttccggaagt gaaatgaaat cactaggatg 68760
   ggagggaaat aactctgaac tagagcacag ataaaaacag gatggtgaaa gactcctaac 68820
   tctgggaaac aaactagggg tggtggaagg ggaggtgggc gaggggtggg ggtgactggg 68880
   tgacgggcac tgaggtgggc acttgacggg ctgagcactg ggtgttattc tatatgttgg 68940
   caaattgaac accaataaaa aataaattta taataaaaac aggatggccc actgtgaccg 69000
   agtgagcagt gaggctacac cattcgggcc aggcctaggg gctctggaca ctcttcccct 69060
   ccatctgggt ggccagagcc ctgaacggct gggagggcca tctggcagca gcagtgtggg 69120
   tgaggcaacc ctgcgctgag gctgatggtg ccctgggctt ctccctgggg aaggaggctc 69180
   ggagagcctt gccagtcggc tgggatcggc aagaactggg tttttttttt gttttgtttt 69240
   gtttaagaac tgggttctta aggtcaatct gtgtacatgg caagtgcaca tgtgtgtgct 69300
   cacatgtgtg catatgcgca tatgtgtttc tagaggtaag tctcccagct ctgtccactg 69360
   acatcagatc tgggaaacca aaacacacca gcacccagat ctggattcaa atacatcccc 69420
   tttaaaaaga accagggtgg gagcagccca ggtgacgcag cggtttggcg ctgccttcag 69480
   cccagggcct gatcctggag accctggatc aagtcctgcg tcgggctccc ggcatggagc 69540
   ctgcttctcc ctctgcctgt gtctctgcct ctctctctct ctctgtctct tatgaataaa 69600
   taaaatcttt aaaaaaaaaa aaaaaaagaa ccagggttca tcaaaaacaa ggaaagtcag 69660
   aaactgtcac agtccagagg tgtgtgagaa gacacgatga ctacatgatg catgacatcc 69720
   caggtggggt cctagaacag aagaggactt taggaaagga aattcaaata aagcatggac 69780
   ttaggttggt aacaatatat cgatactggt tcattagttg tgacaaatgt accatagtaa 69840
   ggaggctaat aagaatggaa ctggggacag gtatgtggga tctctgtatt atccacacag 69900
   tttttctgta aatctaaaac ttttctgaaa taaaaagttt attgagaaaa ggcaggagtg 69960
   gggagaccac agctccccac agaaatggct gatgtcgagg ctggaatggg gaagctgtga 70020
   gatgagcctg gggcaccttg ttctaccaga atgtaagaaa ctgctcggaa aacagaacac 70080
   catggaggat gtcacaagga cacagcctga agggactacg gacaactcca ggacaatctg 70140
   agcactaaaa taatcaggaa cagtagattg gtttttttaa aaggtgtcct tgagtccatc 70200
   ccaatgtaga gctggaaagc ctcactcaga ggcagcaagg atgccaaccc cggcagatgt 70260
   ctgtgtggcc tgaagggtct ccccacaaat ttcttgtaag gacaaaatca gttactgtcc 70320
   agtggggaca tcacatgaca cccgactggt gatcaaaaca ggccaaaaag gtgggagggc 70380
   tgccacgcac atctgatgtg ggactctgcg ggacacagcc caccccactg cagtgttcta 70440
   gcagagatgc agaggctgac cccattggga ggaagcatca ctaaatccaa caggggaaca 70500
   tgtactttaa aacgttttta ccaaaatcta taaagaactt attaaattca acacccaaaa 70560
   aacaaagaat ccagtcaaga aatgggcaga agacaagaac agacatttct ccaaagaaga 70620
   catacaaatg gccaaaagac acatgaaatc agggaaatac agatcaaaac cacgagatac 70680
   cacctcacac tggtgagaat ggctaaaatt aacaaatcag gaaacaacag atgttggtaa 70740
   ggacgtggag aaagaggaac cctcttacgc tgttggtggg aatccagact ggtttagcca 70800
   ttctggaaaa cagcgtgggg gttgctcaaa aagttaaaaa cagggttacc ctataatcca 70860
   gcaactgtac tgccctaggt atttacccta aggatacaaa cattgtgtgg cctgaagggg 70920
   cacctggacc cgaatcttta tagcagccat gtccataata gccaaccaac agaaagagcc 70980
   cagatgccca tcgacaatga atggataaag aagatgtggt gtatatatag aatggaatac 71040
   taatcagcca tcaaaaaatg aaatcttacc atttgcaatg acatggatgg aaccagaggg 71100
   tattatgtta ggtgaaataa gtcagtcaga gaaagacaat tatatgattt cactcatatg 71160
   tggaatttag gaaacaaaac aggggatcat aagggaaggg agggaaaaat aaaataagat 71220
   gaaatcagag gggtagacaa accataaaag actcttaact ctaagaaaca aactgaagaa 71280
   cactggaggg aaggggggat aggcattaag aagggcatgt gatgaaaaaa aaaaaaaaag 71340
   aagggcacgt gatggaacga gcactgggta ttatacatgc aactgatgaa tccctgaact 71400
   ctatctgtaa cactaaaaaa taaattaatt aaaaataaaa tgtttccaaa aggagggaaa 71460
   atgtcctata aaaataaaat cagtaaatcc aaaaaaaggc tgaggagctg ctccagatca 71520
   gaggagaccc tgaaggcagg aaccgtacca ccatgagccc tgcactggag agaaatgcca 71580
   ccaaggacac accaggtcgc tggcaggtct ggattacagt ggagattatg gtcagactca 71640
   gtgctgagcc aggcaggctg ctgtgtgtgc ccatggaagg gcatctcagc tctgcagaaa 71700
   tgcacactga agcatgtgaa gtcaggggct gtgacgtctg taagttaccc ccaaagagct 71760
   cacaaaagat aattgtatgg atctgcacac acacgtacac atgcacagag gacaggtggg 71820
   aggaagaaat actggccaga aggtatggat aaagcaagtg tggcaggtgc gaacatctga 71880
   atctgggtta agaatagacc agtagtccta gtaattttgt aactggtctg taagtttaaa 71940
   atcatgtcta aataaaaagt ttttaaagaa attatgcaat atctactatg ccttttcttt 72000
   ttccaagaac tatgttaata tgaaaaggca agtgacagac catggaaaat attcacaatc 72060
   tatatatctg atgcaagatt tatagcaaaa atatataaag gaaattccca tctatccaat 72120
   aataaaaaga cacacaatcc aaaacatgga ctggccactt catgaaggaa gaataggaat 72180
   ggccaacaaa catgggaaca tatattcttg cctggtcatt aaggaaaagc aaattaagac 72240
   cacattcaca cccactagaa agactgaagt caaaaaatag aaaccaaaag ctggtgagga 72300
   tacggagaaa ctgtaagctt caaacagtgc tggtgcaagc accttgggaa aatatttaac 72360
   atgtatttac ctaggaccca gaacatggtt ccatagcact caataacaag aaatgaaaat 72420
   gtatgtccac aaagacttgc ccaagaatgc tgaaagcagc tttatttatt agctggaaaa 72480
   gcctaccaat ggtaaaatga aaaaacaaat ttgcaaccca cattatctga taagagatta 72540
   acatccaggg acacctggat ggctcagtgg ttgaacatct gcctttggct caggtcatga 72600
   tcctggggtc ctgggattga atcccatatc acacttactg cggagagcct gcttctctgt 72660
   ctgcccatgt ctctgtgtct ctcatatata aataaaatat taaaaaaaaa ttaacatcca 72720
   gaacacagag agaacttcct aagcctgcaa caaaacaaca aaccacctga ttcaaaacca 72780
   ggcaaaggac tcaaacagac atatctccta tgaagataca ggccaacaag catgcaaaaa 72840
   gatgctcagc atcattgacc atcagggaaa tgcaaatcaa aaccacaggg aaaccccacc 72900
   tcacacccat tcactgacta ttatcaaaaa acaaacaaac aaacaatgag tgatggcaag 72960
   aacgtggaga caccagaacc ctggtgtact atcggtagga acacaaaatg gcacggctgt 73020
   ggtggaaaac agtgtggggg ttcctcaaaa aaattaaaaa tagaatcacc acatgattca 73080
   ataattcttc tggtatatac cccaaagaac tgaaagtagg atctcgaagg gatatgtgta 73140
   tacacatgtt catgttaaca ttattcacaa tgtttgaaat gtggaagcca cccaaatcca 73200
   tcaatcgatg actaaatgag caaaacgcag catatccatt cctcagtcat aagaaggaag 73260
   gaaattccta aaatgcaaca acgtggatgg gccatgagca cacaattctg agtgaaagaa 73320
   accagccaca aaaggacaaa tactatatga ttccacatac atgaggtcct tagagttgtc 73380
   ataaccataa agctgtcaaa tatacggtgg gagccagggg ctgggggtga ggaatgagga 73440
   gtcagtgctt catggggcca gagttccaga ttcacatgat gtgaattctg gggacagacg 73500
   gtggtgacag ctgcgtaaca aatgtaaatg tccttgaagc caccaaattg cacacctaaa 73560
   aatagttaag atggtaaact ttgtgttatg tgtattttta caagaaaaaa gagacatgaa 73620
   aaaaaaaaac aaaggtaagt cagaatgcca ctaatctata cactttaaaa ttattctagg 73680
   ccttgtgggt ttcttagtaa aataaactac agttgaaaaa gataaaccaa aaaaaaaaac 73740
   aattttcaaa aagtgagttg agggcagcac cggtggccca gcggtttggc gccgcctgca 73800
   gcccagggtg tgatcctgga gacctgggat cgagtcccac gtcaggctcc ctgcatggag 73860
   cctgcttctg tctctgcctc tctctctctc tctgtctctg tctctgtctc tcatgaataa 73920
   ataaataaaa tctttaaaaa aaaaagtgag ttgatatctt tcttaaagct ataaggggag 73980
   cctaagtctg aaagacttcc agaaaaccct atagatatct atatttctaa gttttgtctt 74040
   tttaaaaacg tttttttaag attttattta tttattcatg agagacacag agagagagag 74100
   gcagagacat agacagaggg acaaacaggc ttcaatgcag ggagtctgat atgggactca 74160
   atcccaggtc tccaggatca ggccctgggc tgaaggcggt gctaaaccgc tgagccaccc 74220
   gggctcctct atttccaagt tttacaagtt taactttaca tcattttcag aataaatcga 74280
   gacataactc ttatgtgcca aatatgcaac atcaaaatat tcagttctga ggattcccag 74340
   gcacctggca agcactcaga tctttgttat atggacttac attgtccaaa gaatgctaga 74400
   aaatacataa ataataaatt agttaatctt caccttctag agatctggga gttattttgc 74460
   tatgtgactc cgaaaaaaag gactgttttg accgctagca gccctgtggg gatttgcact 74520
   tattctgaca taaaaatgca gcagaagaca ccgggcctgg ttatttccac agctaccgtc 74580
   attctgtgtc actctcttct gctccaggga atctgcttta tcaactttca caatcagtca 74640
   atgctgatgg agcccacacc tctgtggctt ccagcatttt caggcctcct aatgcaggtg 74700
   cactgtcccc atgtattcaa attcctggga aggaagctat ctggatggga agaaatactt 74760
   tctctgtctc tttacagttc acccagagac aaattacaaa tggctttagt tgttctaagt 74820
   aaaagatgca aacattacca acagattacc aacaaggagc atgtcttctc aaaatggtct 74880
   ttctagtttg ttttccatga ctaccaaaat actcaggacc aaaaaagttc cctcagactc 74940
   aggtaagcag gtctaaatgg gatacactca acctggatgg tttctcccat ttaccactca 75000
   tcggtgacca atctcacaca gggccccagg acactgaggc ttttggctcc tagtccaccc 75060
   cgtggacaag gcctggccca ttctgtgtct ccaagcgtta ggcacaaaag aacacaagac 75120
   ataaggaaca tggcaaggtc atcctgagaa caggtcatgg cacaagaggc atccgtgagg 75180
   gagcatacag ctgccccact taggaagatt ggttcagaaa tcatttttca aagaaaagta 75240
   catttacttt ttttggctct tctgattctg tacttgagta cttgagaaac atccaggcag 75300
   gctgactaaa tactgggtga cactggacaa atctgtgtca gtattttagg tgtgactatg 75360
   gtactgcagc agcacgctta agccccattg ttagagacac agagggaccc gttcacagag 75420
   gtggcagcgg gctgtctggg actgacccag ctggactggg gaggagtggg ggtgggcacc 75480
   tccttctctc tatcttcatt tgaaatttta agtaacaaaa aggttaaaag aagaaaagaa 75540
   cctatgtgcc acaatgttca cagtaatcaa agcagaaaat gactggaatt tgcttcaaaa 75600
   taaatcaaaa tgggggatcc ctgggtggcg cagcagtttg gcgcctgcct ttggcccagg 75660
   gcgtgatcct ggagacccgg gatcgaatcc catgtcgggc tcccggtgca tggagcctgc 75720
   ttctccctct gcctgtgtct ctgcctctct ctctctgtgt gactatcatg aataaataaa 75780
   taaaatcttt aataaaaaaa aaaataaaaa taaaaaataa ataaataaat caaaatgggt 75840
   cgtgggagat ggtacagaca atataggcct gcacatgcag ttggtaactg ctgagccagc 75900
   tggttcattt tactacacgg tctgccttgg tttacctctg aaatgtccat cgtatgaagc 75960
   tctgacacag ggaacaaagg ggagaaagac tggcaggcgg gatgcaagca aagagtgcgc 76020
   tccacggctc tcctatctgc caagggatcg gggtgtgctg gctcagcctg tccccacacc 76080
   ccgggctgcg cccccacaac acacattcct ggggaaggat gggggcccag ccagcagcac 76140
   ccctgtgccc tcccctctaa tgtggagacc tgtctgtgat gggagtgccc tagtcaggcc 76200
   agccccatgc ccacagcagg cagcctgggc ggatcaccac ctcacagcca ggaccctcct 76260
   tcccagactg cacccaagaa agtgcaccca atggcagtcc agtagaatcc agtattctgt 76320
   gtgaagagaa acctccccac cactaagaat cgatgagcta gataagccct gtttcgtaag 76380
   gcagcaacac acagtaaaac cacctgcagg ttaagcactg cagggaagag ttaaaaaaat 76440
   aataaagcat catcaatgaa acgccgggac acctgcaccc cgctgtttat agcagcaatg 76500
   tctacaatag ccaaactgtg gaaggagcct tggtgtccat caaaatgcat ggataaagaa 76560
   gatgtggttt atgtatacga tggaatatta ctcagccatt agaaatgaca aatatccacc 76620
   atttgcttca acatggatgg aactggaggg tattatgctg agtgaaataa gtcaatcgga 76680
   gaaggacaaa catatatggt ctcattcatt tggggaatat aaataatagt gaaagggaat 76740
   agaagggaag ggagaagaaa tgggtaggaa atatcagaaa gggagacaga acatgaagac 76800
   tcctaactct gggaaacgaa ctaggggtgg tggaagggga ggagggcggg gggtgggggt 76860
   gaatgggtga caggcactga gaggggcact tgacgggatg agcactggat gttattctgt 76920
   atgttggcaa attgaacacc aataaaaaat aaatttatta ttaaataaat aaataataat 76980
   aataataata ataaagtgtc atgtgagaca caccagtttc tgtatacata ctctgtgatc 77040
   accagcagac cgtaattcaa cattctagag tggatgctaa tgacacgtcc acagacgcag 77100
   agagcgccaa acgcacacag caacttggcc ggctccctcc ctgtctcctt ccaagggcag 77160
   cacagggggc caccccagga gccccctccc tcctggatga gtcccccacc caagcccaga 77220
   gtgccctccc cacgcagcca ggtgccttca catacagttg catttcagtg gcaatttctg 77280
   gtaataaata aagcagagct ggaaacgacg gcttgattaa aacaactgtg accaaaaaat 77340
   aaataaataa aacaactgtg accatgtgtt cagccataat cagaacacct gcttctagat 77400
   ttgttcagct gatcaggact ctgaattata ctgacctgag gtgtggacgg cagagagcag 77460
   atgagaaaat aaaacagcac ctccactcag cacccccttc accttcctaa ggctcccctc 77520
   aagaccatac ttggtcctgg tcctccagca gcaaggctgc aatgctggca gaccgactgc 77580
   catggaaggt gccttgtcat ccttttattt ctaatttaca tgttaaattg atatttactt 77640
   attcaaacag aaaattgaat cttaagggta gtatgatttg agtcttctaa agactctcca 77700
   tcagtgatac aatgtctaat aaataaataa ataaaattaa aaaaaaaaaa aaaccaatgt 77760
   ctatatgcta tacagggacc catgaataac ccggggctga aggacagcaa cccccacgca 77820
   gtcaaaagcc tgcatgtaac ttctgacttc tccaaaaact taactactaa caccattcag 77880
   ttgaccagag gccttaccac taacagcaac agtcagtgaa cacatatttc gtatgtcgta 77940
   tgtattatat gctgtgttct caccataaag tcagccagag gttagaaaat gtcattaaga 78000
   aaaccatgag aagaaaaata cacttacagt actgtatcta acaaaaaaaa aaaaaaaaaa 78060
   tctgttgtaa gtggccccac gcagttcaaa cctatgttgc tcaagggtca accacatttc 78120
   ttctaagtga atcttacaaa gtcttagcta tgatacaggc acacacaaag ttcactgtaa 78180
   gaaaacattt accacttagt aatggtaaaa acagtaaagt gcatagtaaa gctttactat 78240
   ccttaacatc atacgaagaa tacattaaaa tagactcatt tttcacaatt atcaacttga 78300
   tatttgtgaa ttaatgctat tatgcatgcc tattgaacta ctgattacca tcggccagca 78360
   gaacattcca tcaccagaaa gaggggcaat gattgcaaca aagccacaac caggaagaag 78420
   cgttttctat aatctcattc tacacccgtt atgtacactg ctgataatca cgctgctctg 78480
   aacagaaaca atgttagtgg tgactttgtt tctccttggg gcacggagcc cagggccttc 78540
   tcaccttgct gtccactcca gaagggctgg tgaacacgta ctccagttga aagactatgg 78600
   caaacgcagg gtgatggacc atctcaggga gtcggaggcg gcttctcaaa actagagcct 78660
   ggtttccaga gctgggtcaa gaacacagag cagcagcaca tcaggcagtg ttcctgccac 78720
   cctgcacccc ccagggtgcc aggccctgct gctgtggcag gttcccccag caatgctgca 78780
   gagctctgca ctgtgtctgg cccccaaggt agatcctaaa ccagagcctc tcatgcttca 78840
   gtcgacactc attccaaatc gccagagctt acatcaggag gacagaacca catggccagg 78900
   tctccgagga ggctcgtcct ccagggcacc cctcggagag gtctctgatc ctgacttggg 78960
   aatagaggac tgaacagaat ggcagaactt cctgcctgct gcttctccac cacgtgagag 79020
   actgatcaca gacacagtgg gggtataaag tgtgctaggg tcccctcaga tcctaagcag 79080
   ccacacctgg agacacctat aatgttagct ccagaactgt cacagcacag ttcaggattt 79140
   ttccaacctg ataacactga ggtctgctcc taacagatat aaatcagcaa gaggaaacaa 79200
   aggcaccagg gaagaagtcc aggtggacaa gtgcatactt cacatgcaaa accagctcca 79260
   tttccctttg atccccagta atgtgcaccc tagtacctcc taattccacg aggctggaca 79320
   tcacgaccct agggagaata cctggtcttc gaagaggagc caactttcct gctgaaactg 79380
   gcagagcgcg tcaaggccac atccatctcg ggcaccagca ccaccacgtg tggcctctgc 79440
   acgaagccca gaccattgtg cacgcccaca cacaggcggc gctccaagat ctccagcgtg 79500
   ctgccatcta gtggactgtc cccctagaag agcacatgtc acacacagta aatccctgtg 79560
   cagaagggca gaagctccca gtggagaggc aaacaaagag aagaaactgt aactgaagtc 79620
   tcaggcattt ccagatcaga tggtgctcag tgaaaatgaa ttcaggcaaa taatcctatg 79680
   agaaccgcca gcctcagggc attttctagg tagttctggg tctttacaca tagcacggtc 79740
   ttctcagagc aatatcaccc tccccctaga agatacctga atcaccaaag tcttactcag 79800
   gtttccatgg ctgagctcaa ttcttaggat gcagcaaatg agcaaccagg gccctagcgc 79860
   cccaaggcag agaagcccac tgcccccaag gtgcctctat cctcccccag actcagacac 79920
   cagaggcttg gggtggaagg ggcagcagga aaagggagca catttatgca gtaagtcata 79980
   aaccaataaa cacagaatct gccccaaagt gatcgctgga tattaaagtt tttcatctta 80040
   acaaaatatg tacaggcact ttaacaaatg agtcaggata gtcctccagg ctattactgt 80100
   ttattaaatt acgtgatcgt ggtgaaatga ttcctgaatt ttcaagtttt cttcactgcc 80160
   cattaatcaa gctgaacaga cctaaattca gcctattagt ctctgagtaa tgaaaacaga 80220
   gcgatggagg aaacagagta gctaccagtt ggataaggac aaaaatacaa agtagtaaaa 80280
   atatgtaata aagtgatttt ggggggatac acaaaattca acaatgatac tcctaaagca 80340
   tctctaccct tatgtgaccc actctgagca ataagaacac agcaggaaac acagtaagaa 80400
   aatgtgctcc gtcaatcctc ttttctgtgt ttctacttca catgagacat cgtatagaac 80460
   taccttgtca agaacgggca gcaagcaaaa aaaagtccat ctcatctatg tgtgctgtgc 80520
   acatgcatct atgcatacat gtgcatgtgt gcatgtatgt gtgtgcttgt gtgcatgtgt 80580
   gtgcagctaa gactttgctg acttcccaga ctgctaggat gggcacctac cagtggaact 80640
   gctcgttgtc acgatggaga gatagcaaag gcctgtgaca ggctgacact gaagcacctt 80700
   gggcacagca gagaacccaa ccacatccct gttttcctgg tgcaaatact ccagacagac 80760
   aggcaacaga gcaaggaata ggtaaggaac ccaggtgctc gtgggcaggg ccaagcaccc 80820
   ggagaaagga agcaggtgac atacttacag agacttgggg ggtgggcctc atcagcacat 80880
   tccagtggcc tgcaggactg tcgagtttgc acagagacct gaagcctaag gggagccatg 80940
   caagggtcta gacgcagagc atccaggaaa atgagctacc agcccaaagg ccacagtggg 81000
   gacacactgt gacctgtgac caccaagact tcatggcagc acatcagatg ggtaggcaga 81060
   cctggaccct gtgaggggag tacctttcac agaaagtggg tcggccggga taaaatgact 81120
   aagacttcta gtcgtctcct tggaatgagc caaatatctt gaaaatgtcc tttataaatg 81180
   ctgataattt tattggtctc cctaagctca tttggtgatt taatttactt ttacatgaag 81240
   actgctttaa gatacaacaa aactaacatt agggtctcac ggtaatagca ccatcatcgc 81300
   accaaagtta aaacaacggg ttagacatcc tcttctgcca ctaaaagagg aagcacgcaa 81360
   gactgtggct ggaccgtcca gcctggtggt gtcagagacc ggcagcgtcc ccacctctgc 81420
   cgtccccacc tctgccctgc ccacatctct actgttcaga ttaagctcca ccatgtagga 81480
   cagtacctcc cggaagttat cactgatgag gagttccaga agctcttcct caaacttctc 81540
   aagggaagga tatagcgtga agaataaatc atccaagtac catgtgacag acttctgaag 81600
   gcgaggcttt cggagggtgt cccctgaaaa aatgaggggg ttcgtgtaga gtatccgtgg 81660
   ggacaggacc accagaagca cctagggcct ctcacccacc taggtcaagg ccatggtgcc 81720
   aggccctcca ggcagagaag aaaccacagg cacagcactc agagccctaa atcagaagcc 81780
   acccccattc acaccaagac gccaagccca gatttcaatt acaacccaat ttctgccccc 81840
   accctcctcc aggaacacat tttataattc agcaatccaa agaacacaga tcactgaatg 81900
   cagggtaaca tctgttccta tcagcttggt ttccctttat cgctaacttc ctctctagca 81960
   agaacacagc cagtgatgcc gtttcttatt tttgtacgtt taccttgaac catttggaaa 82020
   catgaagaaa tactcatgag cacattaagc caacaagcaa aaacaccgtc tgggctggca 82080
   ccacaagaaa agtccccatc tgggtggggg agctgccctg gactgtgagc tccatgagga 82140
   cagaaggggc ggtagttcct ggcacacagc tgggtgcccg gcacagagca ggtgcatcaa 82200
   ccctgggctg tgaaaggcat ggtccagtct gagcagagac ctcccctcca actcgggcac 82260
   gaaggccaag ccaaccacct ccacaccagt acacaccccg cgctctgccc tcccatcccg 82320
   tcactgccct attgccctgt ctcttcccca gctttcaccc gtaagaacta aggagcaaag 82380
   ccttagattt taagaactat ttccaaagtt agagaaagga aacatcagcg gaagactcag 82440
   cgcagaggga actgtcatgg taagagccct tggtcgtcct gctaggaggt caatcagcac 82500
   agccagaggc gatcccatct tcgcctctgt gtgcactgag acccttcaga atctcctcag 82560
   ccgctctctg ccccactaat tccacatttt gaacctattc agcaaaatta atcgaatgtg 82620
   ggagcaaagc tttatgcgca agaatactta ttaaacatta tttataacag aaaaaaataa 82680
   gaaacaacca aatgctccct aggatgggga atgttaaaca agttaccatg cctccgtata 82740
   aaacaggaat gaaactccta ttgtcaaaga tcacttaatg attgaaaaat aatcataata 82800
   ttgcaggtaa aaacgatgtg tgtaacactg cgtatgagat catttaaagc caattttatt 82860
   tgaaaatcta catttacatg catacatgga tacgtatgag aagtaatcac agatgacttt 82920
   atttccttct ttatggtttt ctatcccttc ctcatcacaa atatgtatta gattctcata 82980
   tttcacaatt tcttatgctt ctaagaaacg ttgcacgttc ctttctgaat ttaaaaagaa 83040
   gtgttgaagc tgctctgtgg aggaatcaga acccacgcaa agtataggta tagttattca 83100
   ccatggccaa gggttaaaga caacccaagt gaccagcaac agatgaaaat aaggaaaatg 83160
   tggtccatcc acacacagaa tcttattcag cattaaaaag gaaggaaatt gacacctgga 83220
   tggacctgga agacatggcg ctcggtgaaa tgagatagtc accgaaaggg caaatcctag 83280
   acaatctact cacaagaagc cctaaggtgg taaaatcaga gtcagaagat ggaagggcgg 83340
   gtgccagggg ctggggaggg gctgggcact agtgtctcat ggggctgagc tgcagttttt 83400
   gtgagatgag acattctgga ggcaaacggt agtggtggcc gcagactgca tggtttacat 83460
   ggttcaagtg gcaagtgtga cactacatct atgttaccac aatttttctt taaagagcaa 83520
   aacatagagt ccaaactttc atgaaatggc caacaagtgt ctgaggcatg tcctgaattg 83580
   agatcctcca ccagagaaag agaagggacg cctgggtggc ccaggggttg agcatctgcc 83640
   ttcggctcag ggcgtgatcc tggagtccca ggatcgagtc ccacatcggg ctccctgcat 83700
   gcagcctgct tctcccactg cctatgtctc tgcctctctg tgtgtgtgtg tctgtcttga 83760
   acaaataaat aaaatcttaa aaaaaaaaaa aaggagaaag aggaccgaca aggaaagaga 83820
   ggaaggagct aaagcagcac acttccacca ccagtgccaa gggcagcaag ccaggtcctc 83880
   catcatgggc ctccctagca acaggtcaca gggcacacag cagcatgggg gtgctcatgg 83940
   aggattttgg ggatgacgag acagggcagc tgtggccctc tgggcacaga gaaggagcga 84000
   gggaccatca tcaaaccagg gcccatcagc aggaagaggc caggcccgag ccaacaccac 84060
   tgcctgctgg gggtgctgag tacatcctac ccgcaggtag aaggcgtgtg tcctgaaaca 84120
   agggaagaca acgggtggtc aggcaccctg gggccagggc ctaggggcca accccagttt 84180
   ctaaacctgt gtcctcagct gtggaagcca ggtgacagcc ccccactcag gacagtgctg 84240
   tggggacaga gctaatacca ttcacagcca tgaacacagg gtcatagctc ccgaggaaag 84300
   gcacacacat gtacacacat agctcatcag actaatctta aagcctcggc atggctcatc 84360
   cctgtactgg ctcatccctg tacgttctag tctctacttt acaagagaaa cagggctcag 84420
   acattctaag tgacttgcct gaggatacca cacccacaga atgtgagtga gggagaccca 84480
   aatgttgaag ctgctaatac acatccccaa atgaccagga aagtactgtg agaacttcag 84540
   ggagtgaata gattcacaga tacagaaccc aagagaaaca aagacgggct gctctgggag 84600
   caggggactc aggtgggacc ccaggggcgc cagccaatgc cagcccttcc agagatgggg 84660
   cagcagcaga gacagacttg ccggtatgct ggggccactg cctgtccagc agggaagcca 84720
   cccgcccctc gccaatctcc actactgtgc tgttgcctgc ccctctagac aacagtacag 84780
   taggatgagc aaatctgggc atccagaagg tggcggagtc cagggcttct ggaagaggct 84840
   cagtcctggc aggaagcagg ctctgcccag ctccccattg acaggtggcc cccaggccca 84900
   cagccattag caaaaaggag ccttgactct agtctcacag agatgtgagg aagggagccc 84960
   cacacacaga agtcagacag ggagaggaaa gatggttcct ccaccaggag atcagactaa 85020
   ggagccacat atgcacaggg cagaaccttc cagatcctga ggggcccagg aggcagggca 85080
   gggccaggtt gcatctggcc ggctttctcc aggccatgtc ctgtgaaaat cacaggcact 85140
   ggagaatggc tctcactcag aggacagagc aggcctccca ggatgctcca ctgcagagga 85200
   gcggccaggg tgaggacagt atcccatgaa taaagaacaa aggtcctggg gccggtgctt 85260
   cctaagttct cagtgctgag ctccatcggc ttacttcttt gctcctcaca tgcatctcca 85320
   ggggcaggtc ccaagaaagt ccccatttta tgcaggaaag ggcagcagca gaggctggga 85380
   gaggttagga gcctggaggc ccactccgta ctcagaacct cttccctgct ccctcctgca 85440
   tccccctcac aacagcttca ccatctgaag tgtcaccctg aaatgtcacc tcattcactc 85500
   tgaagggtca ccccgttcac tctaccaccc aacctcagca accggggttg gggaactggg 85560
   gtgaggcggg ggacggggac tcatggcccg cacactctgt gactggggct atttttcata 85620
   tggttgttgc caaggctctg ggcaggaatg caatccccaa aaacagcaat gttccttcag 85680
   gaatccagac tccctgccct cagggatccc cacacatgca ctgccgagaa accagggatt 85740
   acaggcgcag acaggcaaga actccctgta gtgacatcct taagagaagc gttcagaaat 85800
   gccctccagg tgcccaacaa gctctccacg aggtcctaac agggtcccac agcctcccca 85860
   ggacacagct ttcctggaag ggcagtggat tccccagaat gcaccccacc aaacagtgtt 85920
   gcaagaggaa caacagaatg agaccagtta gagggtgacc caggcaggag cggaaagagt 85980
   ctgagccatc cacattgcta aaacacctgt tgcctttgat ggctgtggca agacgtgatg 86040
   ttgcccttgc ttccatctgt ccaagacagg agcactaaga gccccatgct gtgccctcac 86100
   cctggctcag tgtctgtgca cacggatcca gccaatgagc cttccgcgga tgaatcatgg 86160
   acaaacccaa gcatcggtga acaagggaga ggataaaagg atacaccgaa gtcggagcac 86220
   tgctttctcc agggaggttc cccggggagc aagcctggaa ggaggacttt cagcaaagat 86280
   gacctctgtc gtattaaaac tggagctcac acagggcagg agtcagctct cctcttgtca 86340
   ccccaccacc acccctgcct acccccacct acccacaacc cacagtaacg tggcacaatc 86400
   cagaaggtga atcccagcaa ggaccgagcg gcccagagaa gcctaggaga aggcccacag 86460
   ccactgagat acttggatgg agggtgggta gggtggggga agcagcattt ttaaggagca 86520
   cctcaggaga ttcctgagca gggacttggg aggaccatgt gggataccac cagagtgggc 86580
   cacctcgtgg cagactggcg ggcatgaggt tctgacaccc cacttctcag ctctaccgag 86640
   ctctggtgta acagatgagg aggcagacct ggaaaggaga gcccacggca aaggccgctt 86700
   tgtctgcttc agacgcctgc gtggtgtcag ccagctcagg ggagtgtgag gccctctgct 86760
   tcctccagcc ctacagcact gtgagaagtg ggcccaggag ccctgcactc cctgagcacc 86820
   cagtcaggaa gtctcgctca ggactccagg cagtcctggc attcacctag ccttcagggc 86880
   cactgttaga aaacaggctt ccaacttata tttacaacga agcctccagt gcactcagct 86940
   actttatgca aagctcgcac ccactccagg gcaggatgag caatctgagc cttcccccaa 87000
   acgacagaca tctttctggt cctgatctcc tgagaccatc cccccaatga cagaggcagc 87060
   atgcagatcc gcaaccaagg agcctccgtg cggagactca ctctgagcca ggccacggga 87120
   gctctccttg aaagaagcag ctttcaggag ccacggcatg tgtgcaacga ccctggtgct 87180
   gacacagctg tccaggtctg tcaaaccaaa gcaagtgaaa cggtcagaac caggccgagg 87240
   agaccacgag agggagggtg cgtggatttc caagatgcat tggcaggacc tgctaatctg 87300
   gccagatgct gagggcctcc ctctgagagc cacccgaggg gaaaggcctg cctcccttct 87360
   ggagagtgac aaagagccac cagcacaaca cagctaagac cttcatttcc agccattccc 87420
   aggctgaaaa tcactcttcc aaaatcagcc ttcacagaac tgagtgagtc aaagtggaag 87480
   acaggaggaa ggttggagac tgtgtcaaca tcggtatcct ggtcgtgact ttgtgctaca 87540
   atttggggga aaccaggcaa aggacacatg gagtctctct gcgttattaa aaccacatgt 87600
   gaatctagac tgacctcaat ataaatttca gttaacaact caatcttcct gtattgtgtg 87660
   atattttaca aatgaacaaa tatccacatc cccatatggc accaaaataa aatcagaccc 87720
   tctccaaatt gcacgtgaat tccaccactg aagctggtag cagtcactgc cttggccctc 87780
   tcagggctat aaaatattat tcagcaaaga aacacttttc caagagtgag tcacaaaagc 87840
   ccagaagacc ctgggcagga tttataacac agacaagaac ggcgtcagaa agcagggaca 87900
   gtgcctgcat ggctgagagc acagatcagc ctggcgtcct gcacatccag ggaaggtgga 87960
   gcaaaaccag ttccttttct cagatggtca ccggaggact tggtgagaag ctgtgttccg 88020
   gtcctttctc cccaagaaga gggagtatgc tcctgcccac ccgggcactt tccggctcca 88080
   cttctagggt ccctggtcat tagcttttat gcaaaatatt agggacacct aggtggctca 88140
   gcagtttggc acctgccttc agcccagggc atgatccagg agacccggga tcaagtctca 88200
   cgtcaggctc cctgcatgga gcctgcttct ctctctctgc ctctctctct gcctgtgtct 88260
   ctgcctctct ctgtatctct catgaataaa taaaatcttt tataaataaa taaataaata 88320
   aataaataaa taaataaata aataaataaa taaaaattaa caacagatta aaacaacagc 88380
   aatttcccag acgctgcctg cacccagtaa gtgtcccagt gaccaactgg ccgctctaca 88440
   ggagggggct cagatggctt ctgaagacat gacccttcag aatgaggagc tggagtatgc 88500
   cccactctgc agctggcccc tctgatgccc caggctgaca caagacactg agaataaact 88560
   cggggccacc agagctgccc attgtcacca caggaatgac ataggaccag gctccagatg 88620
   agcttcggca ggtccaggtt ttatctgcag ctctactgac acccactggc aagtcataga 88680
   gtgagggaca cacctgagcc cccaccaaca ctctgctcac ccagaagccc aggtcagaag 88740
   gactcatgta agctgagttt taggcaggtg tgcacctagg ttcttgacat caggacatag 88800
   cacagtggca caatgggccc tctctgatcg agagggccga ggcctcagag tcacacgcgg 88860
   attgtcatgt tttcaatctc gaaggcagtg accacgcaac attctatctc cctgcttgac 88920
   cccgtcttct ctttcgagag gacataagca aattctggag ggtgaaaagg cttcctatat 88980
   ggggctggtg gcagggggca gggggttggg ggggctgggg gagggtggtt gcttggtaag 89040
   ccatggcaaa tccagaggtg ggaccctcct cagacttgct cctgttcata gctatcattt 89100
   ctggtgtgtc atcatctctt cttagcaaca ctggaaaaat agtgaccttg taatgggctg 89160
   accaccatgg gcccatccca gaaatttacc aactcagata acaatgtctg ggccccagta 89220
   ttgactagaa cactgagcca tgtgcaggga cgttgttact gaaaacattc ccatactgga 89280
   gagctgagct acttcttttt tggggggtct tgagtattcc tattctaagt caaaactcgg 89340
   cccttccagc tcacgctcat gcccaatcct gctctcccca gaacagaaca gggagccagc 89400
   ccaccccaca caagaggttc cagcagcacc aggtggggaa ggatgggtgt ggcaccttct 89460
   cctggatacc tcagacagcc agacggagag cctccagaag tgcctggaag gagcatgaag 89520
   ttcagatcga ggcccgtcag cagaacccag gagcagagca tatctctgca gagacctcct 89580
   ggctaatcca ccactggaga acctagttct aaacacacca agacagagcc cggaggcttt 89640
   cacgtggagt tcggttttca aaaatactca agccatggct cacccgcatt cacagcagca 89700
   ttatgcacga cagtcaaaaa gcatcctgtc cattgaaagg taaacagaga aacaaaatgt 89760
   ggtccatccc tgcaatggaa tattattcag ccttacaaag gaaggaaatt ccgacacatg 89820
   acccaacaag gatggacctt gaggacacga tgccaagtgg aataacccag ccacagaagg 89880
   gcaaatatgg actatttccc tcagaggagg agcctggagg agtcaaattc acagaggcag 89940
   gaagcagggc agtggggtgg ccagggctgg ggaggggaca cggggagtca ttattgaggg 90000
   attgacagag tttcagtcag gggacatgag aaagttctgg agtggatggt ggtggtggtg 90060
   ggtgtacaac aagatgaaca tccttaatgc ccctgaactg catacttaca aacggttaga 90120
   acagtagatt tatgttatgt ctattttgcc acaatttaaa gagaacgata atcaagcctc 90180
   tgatatggca gcccagcaag ggtgggaact aacaacagtg gttttcccgt tttcccgacc 90240
   ttggtcatga aaatgcgaac gatggatctt atgacagcat ttcccaagta aggacacaga 90300
   agcaacttga gggatgagag acaccgctct gaagggacct gtgccctgcc ctcctccctc 90360
   ccctccctcc cttcccctct cctccctcct ctcctcctcc cttcctcctc tcctctctct 90420
   cctcccattt gctaagggta cctagccaca cccagcccac ctcatagcac agagcctgat 90480
   ccctgcaagg tctgaccacc aaactaatac agactcacca aatagaggga gacagagcaa 90540
   cccttaccct tttctccatg agctggaacg aggccaggga tgtgctgcag accagacacc 90600
   agaaggttct ggggaagaag atggaacacg ggctccagga gcgggtgtgg cttcagagtg 90660
   tattgcaggc tgcagctctc aatgagggtc atgtgtttat tttctgtagg agaaaacacc 90720
   tatatgagag ttggcactaa aggccctttc agtcaaacca gccaataaac tgagcatcta 90780
   agaaacacca caaacaggaa acatattttt ccatggagag gggggaatcg ctctgttagt 90840
   tcagcctaaa aagcaaacta atccttacta aatccttagt aactctctac taaaactgca 90900
   ataacagaga tacttgcttt taatccacat agtgaaatct ctcctgcttg cctctccgcc 90960
   caaggtgtga agtccaggca ttgcctggtc ctcagctcag cgggggactg caagggaccc 91020
   tttcccctca gcacctgcac cctggagtcc cacagagcat cgggactagg ccctgggaga 91080
   ggcaggagac caagacagag ccccgcctcg aggacatgtg ctcatctggg caaccgacac 91140
   acaaacacag atacccacag cacaaaggac aataggatcg gaccctgaga aaggtataca 91200
   taacgcacag cgagctaggg gaagaaggag agcaccctga gaaagcagca gctgagaaag 91260
   gcccagacac agtgctcagg gagctggagg gaacaaaacc aacagaacag accctggggt 91320
   gcaagaggca gagtccagaa tcacagggcc cagaagctga gagggaccag gacatgccct 91380
   ctactcatcc ccgccaaaac cttgcttggc cttgatttct tccactgaca ggagcctcac 91440
   cacctcctga ggcaagctac atgatttcat aagaaaagag ctaaagtctg aagcttaatt 91500
   ttattgataa agattggctt ttaggttatt tctcaatatg accaactgca ttcattagga 91560
   aaagcaatta atgaaaacta tatttttatt acctaatgac tacacacata gggtctgcta 91620
   actttaaatt tttaagtgcc ctgagaggta ccctctcgtg tggccggctg gagagacact 91680
   ggttgggcct ccatggaagg caaataggag aaatgttaca aactagcccc cagaccgagc 91740
   tctgcagctt ccaagagctc gacccacagt tattgggcac atatacaaaa gagaggtgtc 91800
   cacaccagca acgtacccag ccccagggcc ctggatctgc ccggtatcca ctggcagggc 91860
   cactcagtca acacagaaca cacactcaac aactcaacaa cataccatga ggtcataggg 91920
   agaacgaggg gctctctcaa caccaagacc aaacaaacct tctctaaaat gagaaaaatg 91980
   agatggacat gcggctctgc tcctgtcctg gtacaaagac cccccagggt ccaggagact 92040
   cagaggggct gcctggggta tggaggaagg agagcagaac actgcagggg caggggagct 92100
   cactgaacct tgttacactt ttttatgcta tttttttaag attttattta tttgctcatg 92160
   ggagacacag agagaggcag agacataggc agaagaagaa gcaggctccc tgcagggagt 92220
   ctgatgtggg acttgatccc aggactccag ggatcacaac ctgagccaaa ggcagacgct 92280
   caaccgctga gccacccagg catcctttta tgttattctg aatcatgtga atatattgat 92340
   cattcaggca agaaatgtgt acatttctgg aaatgcccac gagaaggtga gctaccagcc 92400
   acatagctcc tcaggcactg ccacagccac acaatcttcc aatgctgccc gggacaaggt 92460
   cctgatggtg tgtgtccttc caaaggtcct gaccctgggg cactgcacta actcagtcaa 92520
   tggagcacgt gactcttgat ctcagggttg tgagttcaag ccccacattg ggcatagagc 92580
   ctacttaaca taaaattaaa aattaaatga aaaaaaaaag ccctgattct aaggatgctg 92640
   taggacacaa atactggcaa agtaacgatc agactcaatt gagaaataat gtaaaagcca 92700
   atctttatca ataacattaa gcttcaaact aattgctctt ctattgtgac atttccgtca 92760
   aattcagcag gcagtggaag ggatggggct tgctcaaaat tcccatcaca gaatccaggc 92820
   aacaactcaa agacacagaa agtcaccctc cccaacagat ctacctccaa ccactggacc 92880
   ccctccacat cctggccctg ggccaggtca ggtctccacc aggggcctcc aagatggctt 92940
   ccacccggtg ccccctcccc atcgttcaag tggccaggag ccagagtagg tcacatgccc 93000
   ttcaggtcac agaggcatgg caggaagccc cctgctctgc ctaagacacc ccccaccccc 93060
   acccctgcag ctgctgggcc tcatggaaga tggcaggcat ccctgcagga cgttcctgtg 93120
   catttaggct ttgccgtgct ggacataacc aatgggcctc acagtgaaag gtttctggta 93180
   ctagcatggc catggccagt gatagacaac aactacttgt agtggttgtc aggtggacag 93240
   gccaccaccc cagaggccag gaggcccaca tccctcccac caggaggttc tggggctgcc 93300
   tctcccaggg ccgtctgcac aaagcaacac cttcccctca ggctagtgct tacagttcct 93360
   tcagttacgt caaccatcca agctcatgac actttttgtc ctaatccctt aaataaatca 93420
   gctaagctga taatggttta cacagagaaa gagaacaaga tggagtcaaa gcagataaca 93480
   ccaggaaagt catagggagg atgagaggac ttcacagcaa cacctcaggc caggccaggc 93540
   cttgtctctt acgttcgaaa gggtcctgga gcagtgggtg caggagggct cggggagtgc 93600
   cgtgatacag cctcaacctg caaagcaaga ccacagagcg catgtcaacc ctccgcatcc 93660
   tgcatttagc caagaaacat cagtgatgct tctcactcca cacctactag agctgtgtca 93720
   acaccataca atcacatcct ctgcaggaga gacaggtgag caaagcagca gccccccatg 93780
   cccccgggcc acatgcttcc aacacacaga cagtactcct cccgacacac gcaagaccca 93840
   ggcacctaga aaaagtacac cagggaaatt tctaatatct gaaatgtccc aagaaagaca 93900
   gcatttgagg aatcttcatg gttgtgtatc tagagcactg ttctcaacca gggatgatac 93960
   catctccaag aggatatctg gtaatgtcca gagatatttt tgattgtcat ggctaggggt 94020
   gggtgcagcg gggagagggc tttggcatct ggtaggtaag gcctgggatg ctctcagaca 94080
   ccccacaggg cacgaggacg gcccccacag caaagacttg tcctggcccc catgtcagca 94140
   tgccaaggct gaggaaccct gtccaaagtg acagtgtaaa cttcagcact ggctgacatg 94200
   gggacacact gtcttctccg tcctgctata tagccagctc ctaaacagag cccggcacaa 94260
   tgtgtctgct gaacagatgc ccactgctta ctgggtgcct gttggtgaag aacatattat 94320
   ttagcaccca cacaacctta tgacatgtgt agcgttctct ctcatgcccc gatgagtgaa 94380
   atcagtcaag agtttgggtc actggcctaa aaccctacag gaataagtga gagctcactt 94440
   catcctacat cagttcctac atcagcctga agaacacttc cttggctcac tgagaaatgg 94500
   caaacagtta ccacacagca gaaaagctcc cagacaacac acggttagga agaaaccaca 94560
   acacaagctg acaattgcaa agttttctgt tacagccaat gagggagcat gaaatctcca 94620
   caaaatgatg ctggaaaact ccaaggatga agaagacgag gcaggtaccg cttgtcctgg 94680
   gaggcagagg taggagattc cagtttgttg ccaaagatcc gaagaattcc aaagccacat 94740
   gacagagcct ggagagagcc atcctgcttc cggccttcgg tgaccacctc aaccacagcc 94800
   accacgttag ggtggttcaa ggacgtgtga aagtacaagg gctgcaaaac agaaaccaca 94860
   acttcggtga cagactgacc atccaccagc tgcagagagc atacagaaaa ggtacaggcg 94920
   ccctcgctct gccctcctat gaatgtgtgt gcccgtgtgg aacaccgcgt ccctcccagg 94980
   gcaccgcagg agagaacagg catggtaaac ttgctccctc ggagtcaaga aacattccag 95040
   agcacccaac acccaagaga gcagactatt tgcttcgctc ctggagcttc tgacccacag 95100
   gaaatgacag atggcaggcc ctccacacac cacctgtcgc acacaccacc ccattgccca 95160
   acaaggaccc agtcactgca aacacacaca cctgctgagc tacttcacag agttctcacc 95220
   acgtaaaact tcaaaacaac tcgccaatat tggatctaac ccgttttgtg ggtaaccatt 95280
   atgaagttcc acctaaacta gttttgttta aactaagcag gatccctaga ttttaggaga 95340
   tgctttgtaa ttatacccag ttcagagccc gactaacatc tttttatgac aaaaagcgcc 95400
   taacatcatt ttcttacaag atttttttct ttttgaatct aagccaagta gatagtattc 95460
   aagtctctct taaaaggttt tccacctgta acgttccctc ggatgcaaat ggacccccag 95520
   ctggggcggt ggaaggggtc agatccacct gcgtccagcc tccctgcgat gtgcactcct 95580
   gctcaggaga gacagtgcct ctgctcagcg gtcgcttcct tcaacttcat catgcccggt 95640
   ctggctgctt ttatttaaaa ctaataaacg tcgtaggcac agaagatggg gacagaggcg 95700
   ctgctggggt ttgcagaaca aatctagatg gaggcactga gctgtggctc tggacacgga 95760
   aggtccccag aatcgcatct gcactggggg agcatgggtg tggcacccgc ccccaacaca 95820
   aagaccaccc ccctccaagc aggtagcggt aaggccgggc tgctgcgatg tgatttaaac 95880
   gtggactaat cccccattaa cgagtcatta ccgtggtgtc accaggaaaa cgtttactgt 95940
   aatcacagac tgtcagccca ggataacaga gagacacatt aaaacatcag ccatgagata 96000
   taaatcgtta ttctattatc ttttattgca aaatgatgaa ataatgacca cttacagctg 96060
   acaggtggtt ggaccaaaag agaattatgt ttctgtttct aagactcaag aactgttttg 96120
   gagagcccca ctgcagagtt gggtttgtca ctgtctcacc tcttatattt atacctgtgc 96180
   acatccgtgt taggaacact acccactttt cctttggcag tgaaacaaat accttaaacc 96240
   gtgatgcaaa gccacagtca tctcctaccc agaggtcacc ttgaaattgc tctgcctgga 96300
   ggccctggga ccagctgctt ccacacgtcc caggttcaaa tttatggctg ctccctctcc 96360
   tttccgttta ccccaaaaac aactgctctg agaccaaaac aacaggagca gagccctgct 96420
   gttgtccatc tgcaaagacc ccatcttcaa gtgggagggt cagtggtgga agtgccactt 96480
   cctgaacacg acccgaggtg tgaggggtgt tgccctggca ctactggcca tgaagggcac 96540
   atggtctctt ccaaatataa tttatggacc ctttggtgtg ggaacagcat cgtgctaaag 96600
   aatttctaag gagcctgaaa acacgatccc accctcaagt tcagagttca aatgtaaagg 96660
   ctagattagg cagatagaaa tggcaaattc tgctcaggac cgaccagaat cacgtggaag 96720
   taaaagcctc ccacgggtga gagcacaggg cagctccaca gcttgttagg aaaacccaac 96780
   aggccctata gtcacctatg ctaagcccca gtcagcaaac caagggacac agacccaact 96840
   gcagttccca ctctcccggg cttggagctc tgagtgggtg gatctggaat tccctggtca 96900
   gcaccagtga ggtgatctcc tggtagaccc tctgcacccc cgcacacaca ggaaggtacc 96960
   ttgcctgaaa aagtttctct ttgctagaaa cttcttcctt ccccattgta tctataaaag 97020
   cctttcactt tgtacagctt cctatagatc ctttctggag gctaaatggg atgctgccca 97080
   aattatgagt cgtgcataaa gccaattaga tcttcaaatt tactcagttg atttttggtg 97140
   ttgtgggttc atttagattt tatttatttg agataagatc gagggagcag gaggaggggt 97200
   gggggtgtgg agggaaaagg aaaagcaggc tccccgctgg gcagggagcc caactcaggg 97260
   ctgacatgga gcttgatccc atgaccctta aatcacaacc tgatccgaaa gcaaatgctt 97320
   aacccactga cccatgcaga tgcccctgat ttttggtttt tgtctttttt tttaagattt 97380
   tatttattca tgagagacac agagagaggg agagacagag acacaggcag agggagaagc 97440
   aggctctatg caaggagcct gacgtgggac ttgatcctgg gactccagga tcacaccctg 97500
   aaccaaaggc aggcactaaa ccacttagcc acccagggat ctccatgatt tttggttttt 97560
   aacaagcttt aatcccctgg tcaccttgag gggaccgtcc ccaggagggt aaagttgggc 97620
   cagtccagct cacctggtac ctaggcatat tcccacctta cagcccagga acaagctaca 97680
   ctacaaatcc tgccagcatt cagctgagca aaaactgcaa ataagccctt cagtctaagg 97740
   acatttaatt aacaacatgc agcaagcttt gagaggaaaa ccctatgcat caactgaaat 97800
   tgcatgatta aaagtttgag tcaagcccat caaattacct gcaagagaag gcctctccag 97860
   acagaagagg ggaccctcgt tcaccccagt cacatcccac tcatctacat ctgacaggtt 97920
   tcctccctga caggataaac agattaataa atcatggaaa tgctcctgtc ttaatgcctt 97980
   gatcttcaaa aacatggaca ccattttgcc tcggggccag gtccccattt ctcacccaca 98040
   tgacagaact gaaattgggc ctctgatgat gtgcctctgt ggagagactg cagccaatgt 98100
   gtccccaacg gcattgccac tcgacctgcc cccttcagtc cccgagcaga aagctgatgg 98160
   cacggccagg gaagagcaga gcagagattc taacagtgcc ttccaaacaa tttttttaaa 98220
   taaaccttta atagaatatc ttcaggaagg cagaaagata acacttcctg cagctcctac 98280
   ctgctagctc agcctcccac ctgagggctt gggtggttcc tgtacacttc ccacctcaag 98340
   actgaatgct ccaggcctgg ggaactctgc tacatgaaac tctgctgaga gatgcaaagt 98400
   ctctagttcc agagtcagaa gcttcagtcc actgttaccc acaggaaaca acacgataga 98460
   tccagcccca ctaggagact cgtccagccc cccatgtcag cgtgccaagg ctaaggagcc 98520
   tgtccagagt gacagtgtaa acttcagccc tggctgacat ggggacacac tgtcttctcc 98580
   aacctgctac agccagctcc tgaacagagc ctggcatgat gcatctgctg aacagatgcc 98640
   cactgcttac tgggtgtctg ttggtgaaga acatattatt tagcacccac gtaaacctta 98700
   tgacatgcgt agtgttctct ctcatgccca gatgagtgaa atcagagtca agagtttggg 98760
   tcactggcct aaaaccccac aggaataagt gagagctcac ttccatccta catcaacccc 98820
   tatatccaag gccagagcca atcccactcc aacctaaagg acacttcctt ggcttactca 98880
   gaagagtaag caaacgcaaa taacccacag tatatattat tatgaataga caacacaagc 98940
   tgacaaattt aaagctttct atcatccccc atggtagtta aatctccgta cgactaaggc 99000
   ccagtgctag aaacatccag gaaagtgatc attcctccat atctggctcg gcgtcctggg 99060
   ggtactttcc gtagagctgg agaggaaggg gccacggtga aggacactgc cctagggctc 99120
   ctgccatggt cacatatgct tgggagctgc atagaggaca catgagaggc tggctattga 99180
   gggagaacca gcacgtgttc aagaagtaag ggattaatca agtcacttgg aagagcttcc 99240
   aggactttcc ttgggattac atggatattc cttaacagca tccactttta aagagggaat 99300
   tacatggttc tcattactct aacaaataag aacatttgag aattgtttct catggtgtca 99360
   cttaagggaa gcagaagcca cccaattagc tgtaccttga gctcaaacac ctgggttacc 99420
   tactcctcta acaacacccg gggccaaggc aagctctggg taggtgcaag cctggtctgt 99480
   gtggcggtgg tcagtggaga cccctaggac tcctgcccag tcactcccat aacataagag 99540
   gaggaagaaa aatgagactg tcacaggaag cccgtgtgcg gttagttaag ggaaaatttt 99600
   aaaggagaat tatgtccatc tcagaaactg cacggtactt tggcttccca gattattttt 99660
   taaaagatac tttctgagag gctgaacaaa ttattaacca tctcagcaga agtgaaaggc 99720
   aattcctctg cacgagaatg gtctggaagg acggcccaga actgcgggct gggagctgag 99780
   aggctccagc aggcagcacc taggaggccg agcctgagag gggaggaatt catcattcac 99840
   ggaaacatca gctcaagttc aaggcaaggt ctttctgcag gacagtgttt gccaagtaat 99900
   aaaatcaggc ccacatatga cacctaacga ctcagtaaaa accctgagca caggagctgg 99960
   gttcattaat ctacatttga ctggacagcc atggtgattc caaagatgca gcctgcccac 100020
   ctccagtcag atctccccac gccactgctg gccagtaaga ccagaaaaca caaccaataa 100080
   aatcagagag agaccttcac caaatttcag aatccccctg aaaaggggca cctggtattc 100140
   acttccacgg gtatgcgggc agagcgcaca gaccctcctg tgcatcagtg ccccgtgtgc 100200
   gtggaagtac ggatgtgcac ataagcttgc gtgcatgcgc cacacagggg ttttgctttc 100260
   cttaactcaa ctcaagaaga acctatttcc ctgtgtgctg agcaccaagg aaaggagagt 100320
   agtgctgggc tcggtgagga cgcagctgaa gggcgcggcc gaccggagct cattcctggg 100380
   ccgccactgg cctctgctgc tcgccttggg catagcagct ggacggcagg gctccctgag 100440
   caggtccggg ctctggcctc acctcccagc cgaagacact agggccagcc cccggcccta 100500
   atgcacactc cacttcagca actccaagac tgaagggtgc agaattttcc agaaacatta 100560
   aaccctgaag tcctcactaa cccagaatcc cctatcaccc tgcccagctc agcagaagac 100620
   aaaatgtgag ggcagccgag gcaaagcagg gtggcacttg gcaccctgga cctgggactt 100680
   ggccgtgagg gctcaggact gggagacacc tgaagtgttg tggtcccacg agtggcacgg 100740
   cagccaaggt ctctgggggt agcaatgcta accctagaca agtccaggag gagcaggtct 100800
   gctctgaggg ctgttttcta ccgtgactgc agttctagag ggtcaccata gccaagccac 100860
   cctgggcaga ggacatgacc ctatgacagg gacgtcccac cactcaccat gggccaggga 100920
   agggtgggct gaggggagga tgtagaagag gaactcattc caaaactaca accgcaaagc 100980
   actcagtact gatttcagtg cacgcctccc tccctctacc cacaagactc ttccaaacgg 101040
   aaagcccagg agcagacact ggtcagggga ggctgcagag caggtgcgct gccccaggca 101100
   ctcccagccc cctaatgtgc tgggttactc ttcaaggaag gacatggaac accagcctct 101160
   ggaggagagg ggtgggaatg gaatcacata aatgtgctgt gagccaacgc tcagcccaca 101220
   gttaactccc tggtagggga cagccatgtc accatggcct tcatcattca cttggtaact 101280
   gtcacaacac caggcttccc agggccagac tgtacccacg acaaagagaa atttgaacag 101340
   ccatgccctt gagggccacc ctcaaaggca gcagaggctg agctgaagcc aggtgatcag 101400
   ccacaccggg gccatgctgc tcctggtgaa ccctgacctg ctggatcaga ggcgtgaagg 101460
   ccactcatca cctgtgctga tgaaaccctg tagggagtca ccgacaggcc cacaggtgac 101520
   tcacactcgg cacggcaggc cagaggcctt tcagcagcac aacttctgtc cccaggacaa 101580
   cattcctgac gtctaagggg taggagcacc ctccactacc ctgtgatggg agggctccca 101640
   tcagaatcaa tccaagcccc aaccacaatc caaccataat ctggactctg ggtggcagcc 101700
   aaatgctgag agcagcacat gctcagctta ctttgtttca cgtctgggcc gggtcagtcc 101760
   ccagcagcaa agggctacct tcggctctgg ttacaggcag tgacacctcc aagaatggct 101820
   atggtcacag ccacctccat tactcacagc actacctgcc cctcgagccc actctgccca 101880
   tgagaaagat cacatcaacg gggcctcagc cctttccata cctgtgacca tcacccacat 101940
   caatcaagca gaccaactgt gttgggaagt gtgccccagc gctgggctct gcccctggtg 102000
   tttgcagcct gtgagcgtgc aagccctgct ccactcacag tgcctgtgct cacagacatc 102060
   caaaggacta gtccctcttg ctgcaggtct ggcttcaatg ctcgctgcca gggaggaccc 102120
   tccttctttg ccctcccacg tcaggtctcc ccgttcaccc ccagggacag ttctggtctc 102180
   cttcagaggc tcaccacaat ttgcaatgac cctgtcacct gcctttgtgt atcacccctg 102240
   ctatacacca tgagtgccac ggccatgcca cccaccatca ccactagacg ctcagtccaa 102300
   gggctgagta gaggctcgta ttgggtgccg ccaaacaaag acaaaccatc tagctgcagc 102360
   cagacacaac catgcgggca agaccatgtc accaagcaat gccaatgcag ccactcagga 102420
   tggaaacaga aggcatgggt tcgagtccag gctctgccac ctgctacctg ggcaagccac 102480
   cctccttcta agctgcaagt gctcatcttt aaaaacaaag aaaaaagcca caatgaaaga 102540
   aggcagacaa ggagagtgag ggaaaacagg agaatcagat gagatcaata atttcctaac 102600
   tttgttccaa gaaccccaag ggtccttgaa ggcgagaaga cctccaggag ccccctgtcc 102660
   taccgcttcg accagagcca tgtgctctca tctgttcagg atgtttggcc tccacacaga 102720
   ttttgtttag aaatgaagtg ccccagtgtt aaaacaagct ttgcaatgac ccatctgctg 102780
   ctcagtggca tttctcgggt ctgagatgca ctggtcgtgt ccccaggtca taagccccac 102840
   acacctgctc ttggccacac agctgccctg cctgccacca tggcccacct catcaaagat 102900
   gatcctagac ggctgcctgg aaagctgctc agccggcctc accgcggtct tccatgtcct 102960
   cccgaaaaag tgtcggtagg tgacatcaaa gagagacaca cgcagatggc agcccacctc 103020
   cgaaagcacc tccagcactc cctaggagac agaagggtac aggtcctgaa gggttcttga 103080
   catcctgagc catcccatgt gtgctgactg accagcttgg tagaattgga tcacttagca 103140
   tcttttaggt gtcaatatcc ttggggggaa cttttctctc ccatggccag cggtcttcag 103200
   gttatgcatg aaagtgcttg gccctcccgg ctacttagag cactgaagcc ccgaggcaca 103260
   gacacgggca tatatgcccc tcctggggcc agctaattcc tatacagagt aaatgacttg 103320
   ccctccagaa catctaggat gagcaaacca accaatccag agttcacact cccacctggc 103380
   cctccatctg gctcttagac tcggggagac cactgagatt actcacacca gccatcctaa 103440
   gcctggtcat ctgctcagcc tgccttgcac attcctcccc acagaaacaa caatgaaggc 103500
   tttcagccac agtctctcca gctgctctgc ctcccgatcg accccatgcc accccgcgtg 103560
   gccctatgtg gcctggtaca cccccacctc ttgggaatgg caataaacgg tcttctctag 103620
   gttgctctca ctgggcctga acaaacaaaa tcccacacct gcctgtgtcc ctacagcagc 103680
   tgtgagaggt agagggagaa gccaatgtgc catggaactc agcaggggtg cctcacaaag 103740
   cacatgggtg tcaaatcccc gcactgcatg cttacatagc tctcaactat tttatccatt 103800
   acacctccct ccagctacca catggaggtg actgggtgac accagtgatg ggccagatgt 103860
   ggggaggtga ggaacaacag ctggaaggac ggggtggcca gctgcctcca ggtgcccagg 103920
   cctcacctgg gcctagtgct gcaagtccta catccctagc gaaggatgat ggcatgtttt 103980
   ctgccccggg ctgctgggaa tgaggcagtg ttgggtacag aggtgtcaga cagctgacag 104040
   gtctggaaga ggcatctgag ccccgtttca atcacagagg tatgggggcc ccttgggact 104100
   ttgcagacct ttctcagacc tctctcgaca cacgggctgc ctctctaccc atatgttttc 104160
   tcaggaacct ccccagctca ctcccatgcc ttccctgagc actgccatct agacacctgc 104220
   ccatcctctg accctggttt tttttttttt ttttttttaa gattttattt atttattatt 104280
   tttttttaag attgtattta ttttttaaaa gattttattt attcatagac acacagaggc 104340
   agagacacag gcagaggggg aagcaggctc ccctacaaga gcctgatgcg ggacttgatc 104400
   ccagaatgac aatctgagtc gaaggcagat cctcaaccac tgagccacct ggttaaactg 104460
   tggtttctgg ggccaatttc tttcagggat gtttgctatc atctaaagtt gtcaattctc 104520
   aaagtgacta aactcgtaca aacattgtag gactaaactt tttcatgtgt ttcctacttg 104580
   gtcttttttt tttttttttt tttgacttgg ttttccttta aatgctatct tttcactgat 104640
   tttgtacgta cagctgaccc ttgaagaaca caggtttgaa ttgcacaggt ccacttctat 104700
   gtggtttcat tctgttttga tgatgttttg aggatgatgg tacagaaccg tagatgtgtt 104760
   ttctcttcct tacaccttaa caggattttc tttcctatag ctgactttat tataagaaca 104820
   taggatatga tacatacagc ataaaaaaat acatattagt caactgttta cattcccagg 104880
   aaggattctg gaataatagt aggctattat ttgttaagtt ttgggggagt caaaagttat 104940
   gtgtggattt ttgactgcgt tgcggggtca gtgcccctaa cctcacattg ttcaagagtc 105000
   aacaataaaa cttgtaaaat ttagggttcc tgtgttaaat cccatctgat tgtgaggtac 105060
   tatcctttac gtatatcact agatctgatg tcctcatagt ttaagaactt ttgtgttcat 105120
   attcatgagg gatgtctgta ctttcttgtg atatccttgc ccaaactggt tatcagttat 105180
   ggcagcctca aaataacagc tgggaagcat tctctcctct gttttctgaa tgagtttatc 105240
   aaaaattcaa attaattaat cttcctctta tactttataa aatccaacaa tgaagtcatc 105300
   aaagtctgga gtttctttgt aggaagctgt ttaattatga attcatttat ttaacaaata 105360
   taggactttt tatattttct atttctttta gtgtcacttt tggcaagtcg agcttttcaa 105420
   gaaatgtgtc tatctcaact aagttatcaa atttgttggc acacaagtta ttcatatccc 105480
   tcgacttttt aacatctgta ggatatatag caatgctcct tctttccttc ctaatattgg 105540
   caatctgtgt gttttcttgc tcttttttca tgatcagtct tgtaggagtt tatcaatctt 105600
   attcatcttt tcaaagaacc aacttctggc tttgctgatt ttctctatta tttgcccttt 105660
   ctttactgat ttctgctaaa tgagcagaaa tctaccattt tcttcttcta ttctctttgg 105720
   gtttaacttg ttcttctcat gtgatgctta aagaggacat agaggtcatc gaatttataa 105780
   ctttcttctc ctcttacata gtcatataaa gatgtaaatt tccctacaag tactaaacta 105840
   ctcacacccc ataaattttg gtctgttctt ccattactca gttcaaatta ttttctaatt 105900
   tccctatgaa tccttctttg acccaaggat tagttgttag tatgctgttt aatttcctcc 105960
   tgtttgagga ttttctaggt atcttcttgt tcatttctaa tttaacatca tacaagtcag 106020
   tgaacatata tatactcttt ataaattcca atccttttaa atccattgag acttattttc 106080
   atggcccagc atatggtcta tcttggtgaa tgtcccacat gcacttgaga aaaacgcgta 106140
   ttctatttgt tggttatagt gttttagaaa tgacagtcag gtcagagtgg tggatggtgt 106200
   ttcaatcttc caaatcctta tgagtgttct ggttcatttg ttctatcaat tattgagaga 106260
   ggagggctaa aagtctccaa ttgtgatggt gaatatgaat ggagctccct ttagtcctac 106320
   acaattttga ttcacgtgtt tggaagctgt gtcgctgggt gctgcgcaca aaggcgcaat 106380
   cacagcttgc ccaaccatta tgccaaacta ttgccaaact attgtgcttt gaaaggccag 106440
   tataggggca cctgggtggc tcaattggtt aagtgtctgc catcagctta ggtcatgatc 106500
   tcagtgtcct gggatcaaga gccgcattgg actccctgct cagtggggac tcggcttctc 106560
   cttcttcctc tacccctccc ccctccactc acacttgctc tctctcagat aaataaataa 106620
   ataaaatctc ttaaaaaaaa aaaaaaaaaa gccagtatag gaggctggcc cttggatgga 106680
   ggctgggcac ttagcattca ggagggttcc catcaccata ctgacaacgg caccctgtgc 106740
   ctaaactgta tacagtggtt tacgctaaac acctcctttc ttgctgggag tgtggaatct 106800
   ggaaacatgc taggcagagg atacccgcct gaccagtcct caaacactgt ggccactgag 106860
   tgtctaacga gcttccttgc tgggaacatt tcatacgtgt tgtcatgact cgttaccgag 106920
   ggaactgaat gtgtcctgtg aggacccttg aagcacatgc ctagcccccc cacactttcc 106980
   cccatatgcc tttcctattg ctgatcttac tctatatcct ttcactgcag taaacctttt 107040
   ctatgaggct gattatatgc tgacacatgt gaagtcttcc caaggaatca ctgagtctgg 107100
   ggggtggtct cacaggtctc aacagaggta catgcacatt tagaactgtc ctatcttcct 107160
   gactgcatca ttatgaaatg ccccttcatc tctggtagta ctcctcacct tgaaactgac 107220
   tgtctgacat cacagcacaa aggcttctta caaggtctac atggcctgta ttttctccat 107280
   cctttaactt tcataggtac agttcatttc tttctgctta aagtgtgttt ttttataagc 107340
   aacctataaa tggtcttaac aataatttga aacacgtaac tctgagtcag gaactccatt 107400
   tggaggaagt attccacaga catatatgta catatgtgta aatgaagtta atcactgtag 107460
   ctgtgtctgt attgcaaatg actgagaaaa aatctacatg ttcatcaata aaggtctgga 107520
   taaatacagg caggttcatc cagagaaata ctatgttgtc ttaagagaga ttgttcatgt 107580
   cattacatga acaatctata ggacatatta ctaagaatat tttttaagca aaataataca 107640
   ggtatattta cctgctgtgt ttgcttagag tttccagaaa gttacacaaa aaagcaggtc 107700
   acactagctg agtctagggt gggacactgc tgccttaggg attctgtgta attatacact 107760
   ctcctcacgt accctagtga attctgaaca ctgataatta ccttttcaaa agtaaataaa 107820
   tacaaataaa caaaataaaa taggaaaata aataaaattt ccttcctaaa attcctaaga 107880
   gagaggacag cactgtccac tactctgtga gctcctatta ggtggaagtc actggcgctg 107940
   ggtgaccagg caggagccca gctggccacc tggattgggg caccccacgc acccggctgc 108000
   agtcccctca ggctgtttcc cgctctgtga aatccagtag cgacggaccc cacttcagag 108060
   ggctgctgca aggagcaggc caactggaac aggaaagtgt tgggctacta gtaaggctgg 108120
   aagagcagca gccaccatgg ggcaagcaca gtggccacac acagcagtcg ctggattaga 108180
   gggcttacaa gcgaatgcta tagggtgtga gtgatatcaa agaagtgagc tcttacagat 108240
   ccaggccccc aacaactaaa tacatggaga atcacttgtc tatctgcaga ggagaacata 108300
   gccttgccaa gccaaaccct atgaaagtca catgttcaca ctggcaggtc tggatccttc 108360
   tcatgcgtcc cactgggtgt cccactgggt ctcactagaa ccatccaatt acatgggtgc 108420
   tctgtgggca gcgcagaagt accagcttct ctagcagttt tcctgcccct gaaggacaca 108480
   gatgaagcca gagccacaga gcaagggcct gagtgtgcag gacgctatgg cacatccagg 108540
   cacacagctt cagccagagc tctgcccttg tggttctaac tgccaggctg caagcaggct 108600
   gcttgagtcg tgcatgagcc cacctcctgc aaaccaccca gctcagaaat aatgcaggaa 108660
   atcatattac ctggatttta aaatggcctc actgtgggca gaaagaaacc ttcacccagt 108720
   gtcaaaagtg ctgaccgagt tgctgaatca cgcatgtgtt ctctttgctg atgagccact 108780
   ttgcctccaa ataatgccaa ggcccaaggt aatgggtatg gtggtaagac tcaaataaat 108840
   aagtgaaaat caataccctc tcagctgaga gcaggagcaa catatatagg aaaataaata 108900
   gcaaatttct atacctgctt aattagtggc ccgtcaagcc acttcaggac acactgaaat 108960
   gctgttgatt cctttaaaag ctggcgtgct ctctgtgggt ggggagggac cagcacgttt 109020
   tggtcaaaaa tcctgagcca gtcattcatt ctgccagtcc cgagggtcac ttcggcctct 109080
   aggagtacct gcacaatttg tgttcaaata aataaaggag ttacgaaggc catagtgcac 109140
   agagcaattc tgaaagtttc ctacctcccc ttgcatccca gaggaaccac catccccaaa 109200
   acccacactt tgcaattcct cccctgcatc cccagtgcag cacactggta tctgtgccac 109260
   cttcccctgg taaacacacc catcttgctc cagatcatct ctctacccct gctttcagga 109320
   gccaactaca gcatgaccat gctggcctca gtgactagtg cagaaatagg tatatgcagg 109380
   gctgctttgt acagctgcag aggttgagca ctacatgaca gtgccaggcc ttggggactg 109440
   gggctatctt ggaagggact aggaagcatg acccaatcag agccaaggag atgcaaatct 109500
   cacagtttct gctggctctg ctgagggaag ggaatactct tctgcagccc tcgaaagaga 109560
   ggtcatcagt atgaagctgt ccggaaccac cgtgggagca gcttccttga gacacacatg 109620
   aaaaaagcaa gcagagcaaa agcacaagca agggtcagtg gatctaggtg tcacagttag 109680
   agccctggcc tgaggagacc ctaaagcacc ccaggactgc tcagctacat taatacagtc 109740
   tctcgttgcc aaagctggtt tggaacagat tttgagtgtt atgggctttt tggtgacctg 109800
   gctcactgag gacatcatcc accttcccac cagatagaga tctgtacctt ctctagcctc 109860
   ttttcggctg acatacaatc ttaagatctt ggctttgata ggaggctgaa ttctgcacac 109920
   taaaatgaca agcaagataa ggcagcagat ttggtctcct aaaacagagg tactttaaat 109980
   gggttcccca gactctgcgg ttccccggag ttgtcaaaag ctcaatgtca aagctatttt 110040
   cataatattc agatgttatt tgcctttttc accgtgtcga tgtttgggct gatgaagcaa 110100
   agggaagagt gggcacactg gcctcagtgc accgaggcaa acctctggcc gtcgtgcatt 110160
   cttcaccact cacagcaagg gcagagtccc cagtgaagct gcaaaaacta tgaattttat 110220
   tacatcttga tccttgagac tggcattttt aatactctgt gatgagaagg gaaagacagg 110280
   gtagccctgg tggcgcagtg gtttagcgcc gcctgcagcc cagggcgtga tcctggagcc 110340
   ccgggatcaa gtcccacgtc gggctctctg catggagcct gcttctccct ctgcctgtgt 110400
   ctctgcctct ctctctagct gtgtctctat gaataaataa acttataaaa aaaaaaaaaa 110460
   gaaagaaaag aaaaaaagag aagggaaaga cacagcaagc attgctgctg caaaatgaag 110520
   ggtactggtc atctcagggt gaggctgctg tgagcctctg tggcacactc aggacccact 110580
   gctcctgttg tctggtgatg ctatcttttt tttttttttt tttaagattt tatttgagaa 110640
   agagagagag agagagagag agcatgagca agggaagggg cagagaaaga agcagacgcc 110700
   ccacagagca gggagtccga ttcaggactc aatcccagga ccctggaatc atgacctgaa 110760
   ccaaaggcag acacttgact gagccaccca ggtgcccctg gcaataccat ctttactaaa 110820
   agagtagctg agagacagac tgagcttact caaactcggg cactttgcag tctctcaaac 110880
   gtgaactaaa tgagtctgtc actttcaagg aaaactggta ataagaattg ggctttcagg 110940
   caaaaatcag aattgggaaa atctgtatcc tccactgtga ttctgacagc ttcctcctcc 111000
   ttaaagacgt gcttgacaag atggatggtg actgtgacaa aagtgatctt ctgaaactac 111060
   gattaaatgc acactatttg gaagatcagc ataccttaga agcagtgttc tccaagtgag 111120
   cagtgcatca tgatataaaa aagcctgcat gagtaaaaga tccatccaca gtgcaggaca 111180
   gggcagccct ggtggtgcag cggtttggcg ccgcctgcag cctggggtgt gatcctggag 111240
   acccggaatc gagtcccaca tcgggttccc tgagtggagc ctgcttctcc ctctccctgt 111300
   gtctctgcct ccctctgtgt gtctatgaat aaataaataa aatctttaaa aaaaaaaaaa 111360
   aagtgcagga cagaccaaag gactttaaag taccggagca cgcaaacttc actggcaggg 111420
   tgccaaactc cacactgcaa caaactttta agaaactacc actcgcagtg gcgctggcgt 111480
   tgcatttgga tgaagaatca aaggagaatg ccacaattac ctgagactct gctcccttct 111540
   ccgcatgagg ctgtttcttt caaatacttc aaccaaaaca actttgctca acaggttaaa 111600
   aacagaagcc taagggcgcc tggatggctt agatgagtat cagactcttg atttcagctt 111660
   aggtcacaat ctcagggttg cactcagtgg ggaatctgct tgagagtatc tccctcttcc 111720
   tctccctctg cccctcccat tgtgagtgct ctcaaataaa taaatcttaa aaacaaaaag 111780
   aaagaagcct atatgagacc cagctgtcca ttaagccaga cattacagaa atgtgcaaaa 111840
   atgcaaaacc accccttttc ctgctaattt ttttattctg gaaaacagct ggttttcata 111900
   aaaaatacat tttatgctaa catgtaacag gtctattgtt atctctacat gagcaataac 111960
   attgctaaca tctctcagtt ttcatttcca atacagttaa agctggtaga cataataatt 112020
   aaagtaaaca aaaggtctct ggagtcctcg gcaatttttt aaagggtaaa aggtacgaga 112080
   ccacaaagtg tgagaaaggc tattctaaca ggacccaaaa gatgagggct ttgtacagcc 112140
   aggccaccct ccacctttgc aggctgtgtg gggagcggag aggatccccc agagcacgga 112200
   catggctggc gtgaaaagct ctcccacacc cagcatgccc aggcacaaag gcccagcacc 112260
   cacaccccac agcccctgca ggcaggcatg cagtgcagca tccagatcta gactgtcacc 112320
   atgtgagcag tgaggcacac atgcattgcg ttttctgttc tgagcagtta cgtctgctca 112380
   gtatcccctt gttttaagga atgaccctac aatccagtgt gggaaggctg tccacaaatc 112440
   taggcagagc actgtagtga tgggctcaag atgaacacgt gacccacact gtgccaatca 112500
   tcatctcccc caggactcca gccgcggttc cctctgaaga ggctgtctct gctgagaggg 112560
   caggagctgc tgggccagcc tgaattccag gggctaccct gccacttcag ggagagcctg 112620
   aggtcaaagg gtgtcctgcc cacagagccc agacccctag ggggagagag cctgatggaa 112680
   ggcagccccc actcccatag cagccgaggt gtagcagcca agaaaagccc atggaggctt 112740
   tccctggtgg ctaaatggcc cccaaagaag tcccggtcct catccccagg atctgtgaat 112800
   atgggacttc gcatggcaaa agggactctg ggagtgtgat taagtgaagg gtcttaagga 112860
   ggggaatgga tgacaggagc cacctagatc cacctggatg acacaggtgg gcctgatgta 112920
   gtcacaagga tcctcagaag agggaggcag gagggtcaca gaacaggtcg agtgaccacg 112980
   aaacagacag agaagggagg gagggagggg tcacaggcca atgaccagct agaaaaggca 113040
   aggaaggctt ctcccctgga gctttcataa ggaacacagc cctgcccaca ccttgatctg 113100
   agcccagtga tattgaattt ggacctctga cctccagacc tggaagaggg taactgtgtt 113160
   gtctcaagct gcccagattg tggtatcatt acaaaagcca caggcactca cacaggtgtc 113220
   taccactagt aagcagcaga ggcctgatac ccagcaggag ggccacatgc ccaggagagc 113280
   ttcttggagg ccctgggggc cagtgactgg gcccacctgg cccctgtgct tcccatggtt 113340
   gcagcactcg ccacctgtcc tgtgagggtc tgcctgctgg ccaatcactt gcacgagggg 113400
   atctattttg ttgtgtcctt ggtacctggc tcaccatctg aactcaacaa attttcataa 113460
   aatcaaagtc tcaataaaat tgactcccaa gcatttttta aagagaaatt tttgggatcc 113520
   ctgggtggcg cagcggttta gcgcctgcct ttggcccagg gcgcgatcct ggagatccgg 113580
   gatcgagtcc cacatcgggc acctggtgca tggaggctgc ttctccctct gcctatgtct 113640
   ctgcctctct ctctctctgt gtgtgtgtga ctatcataaa taaatttttt taaaaaattt 113700
   taaaaaattt taaagagaaa tttttaaaag aaatgttttc tcattgactt tcattaaaaa 113760
   aaaagggggg ggggtgttaa ttcatacaaa gtgagtgtca ctacggattg aaagtccctg 113820
   tcaggaagtc cccctgccta gggagcaccg tagccaagca tcacctctga aaggggcctc 113880
   tgtgcaaggc ccgcactgtt tccccggctc caggactggg tctcttttgc aggcccttcc 113940
   agactttgct cattgggccc agattcctga gccagcacct acctccccac cccctggcta 114000
   ccacacttgg ccagcagatg ctgaaggacg ctcctgttct agtccaccta gcagaggaag 114060
   catgtccaca ggactgcccc aggctgtacc cagtccccat aggatgcacg ctgggaagat 114120
   aggcctccac tctgaatggg acagtcaccc aaccaaaaca gtgagccagg cagatggagg 114180
   cgggtagcaa cagtctgagt gggcgataga gaagagggca cccgccacat aggctcctgg 114240
   gagtgggaga cctagtgggc acagagcatt tccctcccaa cctcccaacc cccacctaac 114300
   actctgtcct ctctgtggcc actgtgcccc cgctcggccc ctgctccaat tcctcctgga 114360
   caccatgccc cctaggatcc tgtcacagaa agtcaccaca attgctcagg ctccccattt 114420
   ctgttgtcct gcgagaatga gctcagccag gtcagggcta gatcctcagt gaaaatcttt 114480
   tgatgactac gtaaataatt aaattatgct gtgaaggagc taagactgct acagagagag 114540
   agcagaaggc ctgtagggct agatgaacag ggagaaaaca gtgaggcccc atgcccaaca 114600
   caaagggctc tccaggggag aaacaacatg agaaaacaca ccagctccca cagagagcct 114660
   gctgcgagct gggtgggcac tcacagaccc attcatgtgt attagtccaa caaatgtgca 114720
   gataagtatc acagacgagg acaaggagac taaggctaaa cagccaggcc aaagccacat 114780
   ggctgctcat gaccaggctc aaagccagga gaggctccgg agcctacagc atgacactgg 114840
   ctggagcacc caggctaagc cccactctcc tcagacttca gggaagtctc ctgccctccc 114900
   tgaatctgct cccacctaca ggatgagagg ggactgtgag gtctcccaga gggctctgct 114960
   ctgcacacct cttctgttcc actgtgtgca atggtgaaca atgccaaagg ccaggtcttt 115020
   aagaatctaa aaatccagtg gggaaaagac aagggttcga gtgtgagcag cacacagaag 115080
   acaagcataa ttcagtctcc tttttgggag gatgcggggc agagagaaga ggaaatcccg 115140
   gcactgaaga agcaagctgg gataaaatct caacaacagt ggaacaccag agaaagaggg 115200
   gggcaggagc agctgtgcag gcagggggat gtttccttcc aagaagtgaa caagatctgg 115260
   gatgcctgag accgaaaagc tttgggaact ttactgggag taagtggcag ttatctcaac 115320
   cccgcctcga agttttttgg tctctcccac cccccccccc cccccccctc cgagcaagca 115380
   atcttctttg cacacctacc aggtgccagg acctggagaa accatcctca acatagcaga 115440
   caagctccct gccctcaggg aatggatct 115469

<210> 2

<211>  4294
<212>  DNA
<213>  Canis familiaris

<400>  2
cccttgtact ttcacacgtc cttgaaccac cctaacgtgg tggctgtggt tgaggtggtc      60

accgaaggcc ggaagcagga tggctctctc caggctctgt catgtggctt tggaattctt     120

cggatctttg gcaacaaact ggaatctcct acctctgcct cccaggacaa gcggtacctg     180

cctcgtcttc ttcatccttg gagttttcca gcatcatttt gtggagattt catgctccct     240

cattggctgt aacagaaaac tttgcaattg tcagcttgtg ttgtggtttc ttcctaaccg     300

tgtgttgtct gggagctttt ctgctgtgtg gtaactgttt gccatttctc agtgagccaa     360

ggaagtgttc ttcaggctga tgtaggaact gatgtaggat gaagtgagct ctcacttatt     420

cctgtagggt tttaggccag tgacccaaac tcttgactga tttcactcat cggggcatga     480

gagagaacgc tacacatgtc ataaggttgt gtgggtgcta aataatatgt tcttcaccaa     540

caggcaccca gtaagcagtg ggcatctgtt cagcagacac attgtgccgg gctctgttta     600

ggagctggct atatagcagg acggagaaga cagtgtgtcc ccatgtcagc cagtgctgaa     660

gtttacactg tcactttgga cagggttcct cagccttggc atgctgacat gggggccagg     720

acaagtcttt gctgtggggg ccgtcctcgt gccctgtggg gtgtctgaga gcatcccagg     780

ccttacctac cagatgccaa agccctctcc ccgctgcacc cacccctagc catgacaatc     840

aaaaatatct ctggacatta ccagatatcc tcttggagat ggtatcatcc ctggttgaga     900

acagtgctct agatacacaa ccatgaagat tcctcaaatg ctgtctttct tgggacattt     960

cagatattag aaatttccct ggtgtacttt ttctaggtgc ctgggtcttg cgtgtgtcgg    1020

gaggagtact gtctgtgtgt tggaagcatg tggcccgggg gcatgggggg ctgctgcttt    1080

gctcacctgt ctctcctgca gaggatgtga ttgtatggtg ttgacacagc tctagtaggt    1140

gtggagtgag aagcatcact gatgtttctt ggctaaatgc aggatgcgga gggttgacat    1200

gcgctctgtg gtcttgcttt gcaggttgag gctgtatcac ggcactcccc gagccctcct    1260

gcacccactg ctccaggacc ctttcgaacg taagagacaa ggcctggcct ggcctgaggt    1320

gttgctgtga agtcctctca tcctccctat gactttcctg gtgttatctg ctttgactcc    1380

atcttgttct ctttctctgt gtaaaccatt atcagcttag ctgatttatt taaggattta    1440

ggacaaaaag tgtcatgagc ttggatggtt gacgtaactg aaggaactgt aagcactagc    1500

ctgaggggaa ggtgttgctt tgtgcagacg gccctgggag aggcagcccc agaacctcct    1560

ggtgggaggg atgtgggcct cctggcctct ggggtggtgg cctgtccacc tgacaaccac    1620

tacaagtagt tgttgtctat cactggccat ggccatgcta gtaccagaaa cctttcactg    1680

tgaggcccat tggttatgtc cagcacggca aagcctaaat gcacaggaac gtcctgcagg    1740

gatgcctgcc atcttccatg aggcccagca gctgcagggg tgggggtggg gggtgtctta    1800

ggcagagcag ggggcttcct gccatgcctc tgtgacctga agggcatgtg acctactctg    1860

gctcctggcc acttgaacga tggggagggg gcaccgggtg gaagccatct tggaggcccc    1920

tggtggagac ctgacctggc ccagggccag gatgtggagg gggtccagtg gttggaggta 1980
gatctgttgg ggagggtgac tttctgtgtc tttgagttgt tgcctggatt ctgtgatggg 2040
aattttgagc aagccccatc ccttccactg cctgctgaat ttgacggaaa tgtcacaata 2100
gaagagcaat tagtttgaag cttaatgtta ttgataaaga ttggctttta cattatttct 2160
caattgagtc tgatcgttac tttgccagta tttgtgtcct acagcatcct tagaatcagg 2220
gctttttttt ttcatttaat ttttaatttt atgttaagta ggctctatgc ccaatgtggg 2280
gcttgaactc acaaccctga gatcaagagt cacgtgctcc attgactgag ttagtgcagt 2340
gccccagggt caggaccttt ggaaggacac acaccatcag gaccttgtcc cgggcagcat 2400
tggaagattg tgtggctgtg gcagtgcctg aggagctatg tggctggtag ctcaccttct 2460
cgtgggcatt tccagaaatg tacacatttc ttgcctgaat gatcaatata ttcacatgat 2520
tcagaataac ataaaaggat gcctgggtgg ctcagcggtt gagcgtctgc ctttggctca 2580
ggttgtgatc cctggagtcc tgggatcaag tcccacatca gactccctgc agggagcctg 2640
cttcttcttc tgcctatgtc tctgcctctc tctgtgtctc ccatgagcaa ataaataaaa 2700
tcttaaaaaa atagcataaa aaagtgtaac aaggttcagt gagctcccct gcccctgcag 2760
tgttctgctc tccttcctcc ataccccagg cagcccctct gagtctcctg gaccctgggg 2820
ggtctttgta ccaggacagg agcagagccg catgtccatc tcatttttct cattttagag 2880
aaggtttgtt tggtcttggt gttgagagag cccctcgttc tccctatgac ctcatggtat 2940
gttgttgagt tgttgagtgt gtgttctgtg ttgactgagt ggccctgcca gtggataccg 3000
ggcagatcca gggccctggg gctgggtacg ttgctggtgt ggacacctct cttttgtata 3060
tgtgcccaat aactgtgggt cgagctcttg gaagctgcag agctcggtct gggggctagt 3120
ttgtaacatt tctcctattt gccttccatg gaggcccaac cagtgtctct ccagccggcc 3180
acacgagagg gtacctctca gggcacttaa aaatttaaag ttagcagacc ctatgtgtgt 3240
agtcattagg taataaaaat atagttttca ttaattgctt ttcctaatga atgcagttgg 3300
tcatattgag aaataaccta aaagccaatc tttatcaata aaattaagct tcagacttta 3360
gctcttttct tatgaaatca tgtagcttgc ctcaggaggt ggtgaggctc ctgtcagtgg 3420
aagaaatcaa ggccaagcaa ggttttggcg gggatgagta gagggcatgt cctggtccct 3480
ctcagcttct gggccctgtg attctggact ctgcctcttg caccccaggg tctgttctgt 3540
tggttttgtt ccctccagct ccctgagcac tgtgtctggg cctttctcag ctgctgcttt 3600
ctcagggtgc tctccttctt cccctagctc gctgtgcgtt atgtatacct ttctcagggt 3660
ccgatcctat tgtcctttgt gctgtgggta tctgtgtttg tgtgtcggtt gcccagatga 3720
gcacatgtcc tcgaggcggg gctctgtctt ggtctcctgc ctctcccagg gcctagtccc 3780
gatgctctgt gggactccag ggtgcaggtg ctgaggggaa agggtccctt gcagtccccc 3840
gctgagctga ggaccaggca atgcctggac ttcacacctt gggcggagag gcaagcagga 3900
gagatttcac tatgtggatt aaaagcaagt atctctgtta ttgcagtttt agtagagagt 3960

tactaaggat ttagtaagga ttagtttgct ttttaggctg aactaacaga gcgattcccc 4020

cctctccatg gaaaaatatg tttcctgttt gtggtgtttc ttagatgctc agtttattgg 4080

ctggtttgac tgaaagggcc tttagtgcca actctcatat aggtgttttc tcctacagaa 4140

aataaacaca tgaccctcat tgagagctgc agcctgcaat acactctgaa gccacacccg 4200

ctcctggagc ccgtgttcca tcttcttccc cagaaccttc tggtgtctgg tctgcagcac 4260

atccctggcc tcgttccagc tcatggagaa aagg 4294

<210> 3
<211> 115289
<212> DNA
<213> Canis familiaris

<220>
<221> misc_feature
<222> (10684)..(10929)
<223> n is a, c, g, or t

<220>
<221> misc_feature
<222> (93419)..(93419)
<223> 180 nucleotide deletion after nucleotide 93419

<400> 3
ttactggtac acgaccttga cgcagaaggc ttcttcattt ttgtcttcgt ggtcattgat 60

gtaaatcaga atctcctcct ccccggggct ctgccgaggt gcgaaccaca ggccgatggt 120

gtaggtctct cctcccccaa tctggaagca agcatcagat cctagcccta ctgcagccct 180

gctctccatg ccagcatggt caacacatgc aaacccaggc tgacagagca gggtaaaccg 240

aggctctggc ccccactgct gccaggcacc tgaggttctg aacacaacct ccccgtgtta 300

caggacacca ctgctctgcc cacgcctcag gggagattca cagctgaaga aacaccttcc 360

aatgacagac agactgccat ctgctgtact gtcgttaaaa tacttgcaac agagaaaagg 420

ctaacatgca ggacacttgc gaataaaatc gctgataaac tgcagggtaa attggccacc 480

agaagccctg tgacagtgcc cagatgtggg ggccctagcc gcatgggcag aactgctgag 540

agaggggtgg ccgcccctac tcccagtacc accggtgcag atcagcactg ccccctcccg 600

gggctctgga gacccctgtg cctcggggcc cctgggacat gccgaggctg ggtgtcccac 660

atgcagtgcg cacaccctgt acgcagcgct tcccgcacct ggaaggagtc ctctctgaac 720

tgcagcaggt cagggtggtc gctgtacagg tgataggtcc tctgggaggg gtaagggttg 780

gtgtaggtga tccgcttgtt ggcgcctctg cctttccctg cagccagtgt gatctcgaaa 840

gcctgtgtgg gcaggggtgt cagccttcct cagcctgctc tgcacgtaca gcccgaccta 900

tgtctccata gacatgacaa gacacccgcc gagggggctc actgtacagg agcaaccccc 960

gcccccaccc ccaggccata gactgcggag gagactcggt cacaggacca ggagacagcg 1020

aggtgacaca gcacccatac cacacacgac ccagcaggtt cccctccaga ccttggagat 1080

gagaggcggg cggcaggaga ggcacacgag ccatgaggcc accagctggt ggtagtccac 1140

gtcgaccagg ttgaggtgga tgaaccggct ccctgccctg cggggcctca caccgacatg 1200
caggtcctgc accccatgtg gtggcagcac gaacacacct ttggggtcag tctttcaaaa 1260
caacagcaga gaggcgggtc agtatgcagt ccctcatgct gtgcagaggg gttccctgaa 1320
cacctgcagc cagtgcacga gaatcccaag ggacctcagc tattcagagg cagggggcac 1380
gtggcctgca ggaagtcaga aggggaccga gtgacaatgc caggtctgaa gccagacgca 1440
gccacacagt cagggttgca gtgcatctca gtccgcatca ccacccgcat gggcaagtgc 1500
ccctccatgt ggcaacagtc cccttagcac cagagctgcc tcagcagagg gaaatcttaa 1560
caggctccag ctgtaagctt cagcaaggag tctgcattgc cctcattacc cagacgcata 1620
gttgtggcta aattaattcc aaaattctat taatatttac cttccaataa ttctaaaaat 1680
tggcctatag ggtttatatg tcactcattc catgatggca gatccaataa aaggcagttt 1740
gaatgaagac tctgccaaga gtcttaaagg agctgtgagc cgatcctcag ccccacactg 1800
gctgcagcca gtctccacac cgtccgtgcc ccaggtgggg tgaggatggg gagaggccag 1860
agaacaccca gcgacctacc ttcagctcct gagggtgtga ggtgaaagcc tgcactctcc 1920
taaccgtccg tgtccctcga aggacaaggg ataggcgtgt ccgctggcct gtgacacagg 1980
agacgtccac acgctgcaag gagtggaggt agacctgcca tatttggatg ggtgtcgcca 2040
gccaacgatc cctgtgtagg acagaaaccg tgatcaatgg tggtgaggac agcagcactg 2100
accaggaaga ggccaagaag gtggccagga caactggggc aggctggagc ccagctgcca 2160
tcatccctgc agccaccttc cccactctga aggcccactg tgactttgca ccacaacacc 2220
ctgtgtgagt ggtaatgtgg gcttctacca gcaacaaaac agtcccaaac caggtggtct 2280
gggacaaagc ctgtggaaag agtgccgtgg aagagtatgt gggttcagag gccacccaga 2340
cctgagctca caggccccgt cctagctgcc tggtgctgga ggccactggc ccttccctgg 2400
aaatggggct cgataaccct gcccctcggg cttctccaca cggccagaga ccagaaccga 2460
gcatggctac acctgcccca atcccagatg tcctattcag aagcccccag cctaccccag 2520
ttttaaaaga tcctcaaggg ggacacctgg gtggctcagc agttgagtgt gtgcctttgg 2580
ctcagggagt gatcccaggg ccctgggatc aagttccaca tcagtctccc tgcagggagc 2640
ctgcttctcc ctctgcctat gtctctgtcc ctctgtgtat ctctcatgaa taaataaata 2700
aaatctttaa aaaaaaaaaa aaaagaagat cctcaaggga ctggaagagg agacttccca 2760
gaacaaggta aaggaacagg gttatttcaa ctgagtggga gaggcaagaa ggcgatgtga 2820
tcaccatctc aggcacggag gatgaagact gccaccctcc ctgtcccctg aacggagcca 2880
ggcactgctc tggggcagac gccaccccaa gttcctttcc ttggtgggct ctccatgcct 2940
ctgcaagccg ccgggccact ctgatccgat ggcgtccttt gtgaggcccc tgccagccag 3000
ccgtggagtc tcacctcccg tggctgaagg ccccacgaca atgtgcccca tcaggaactc 3060
agcatgagcc caggcggcct ctgggatgct gtggctgcct cccagtcccg cccaccctcc 3120
agacctccca ggtttgggct cacagactca cgcgtaaacg gcgacaaaga agtctctgct 3180

ctctgggctt ggaccgctgg ccaccttcag gaacacgtcc cgaggctccc tggggccctg 3240
gcggacatgg gcgccctgag aacaccacct gcctcccccc caagcccctg gcaggtgaaa 3300
gcgggcagga actggctccc tccctcccaa caggtgttgc attttcagta ttaggcagaa 3360
ttcgtttttt tcttcactgc aaaaaagaaa atcattaact tcttgatttc atattgagtt 3420
caataattat attctgcagg agattttatc cagtttccta accagtcata acataattat 3480
tgcctttatt ctttacacaa tggcaattaa taagtggctt aattaccact cactttattc 3540
actttgcttc atgccttttt tttttttttt catttgaatt caggtaatcc aagaactgat 3600
ctgccagcag aaaagatgaa aactcatgca taaagctccc acaaggatgt caaaatagcc 3660
ccttagcacc ttagaaagac accacttaac tcaaaagccg ccgtgaagag gcctcaggag 3720
gccaggggct cagatgacca ggcactgact gggagacgct cggcagaact taccacattc 3780
tgggtctcgc agatgacgtt ggggtcgcta catcggacat agactggggg ctcctcacca 3840
ggtcttccca cgggagcacc tgctgcaggg aatatcatag caaacgtctt ctggacaggc 3900
ccgggccaca tgatgttgct ggcccatcac aggtacccca cagacaccag aagacagcaa 3960
tgccaccctt cctactgtcc cgcccacgtg ggcactggca ctcccgccct aagccacacc 4020
cgcaggcact tatccgaaga gagtgaccaa aagtaaagag cggggggggc tatggtgcag 4080
ggcgccagac acggggacgc tgctgcactc taaagcacca gaccagaagc agacaagtga 4140
cgcggcagca gaaggcgcga gcgcactcca ggcacagccc tcggggccgt gggctttagg 4200
aagtgggatt agccagccaa gggcataccc tcgcatctca gaggacgcga aaaggtcacc 4260
atttcaaacc ataagggcgg gtggcagact ttacagatgg gtcacaggtt gggataagag 4320
catcacagga gaatgtgatg atgccacttg tcaccctgag ggaaaaacct ttagccatct 4380
gtggaacctg caaaatgcca gccgcaggga cagatgggac ctgcctggaa gcgtgtgcca 4440
gggaggcagg cggatggcct tcttcaggaa ggtgagctct gggtggtaga agcggaaaac 4500
ctggtccacc acatggggct gggtctccac ggttaggcaa agcacggcga tgggcttacc 4560
gccactcccc cggaacaaga cctgccagga gagcagaggt tgccagcatc agggctggca 4620
cgtgggctca tgaagagctg cctgcaccaa gccagcatcc acagggccca gtccccccac 4680
cccccgcacc gtgacgtggg aagcacaggt gttgtcagaa atggtgggct gaatctgcag 4740
cagcaggtcc ccacagaggg gatggcaccc agcacaggga ctgcatgtgg acatgttccc 4800
gtgacacaca tgcctcaaat gtgtcagcgc tcaatttgac agccccaatg tcaaatactc 4860
cttcagccac cgacccatct cccaacatgc ctgccatgtg catgctggga agctgcccag 4920
agggtctgat gttaacctct ccaagtcctc atgggtggta gtggagcaca agctctttaa 4980
ttctgaacct aaacaagaaa ggctagagat acaaacttcc tttctaaaaa aaaaaaaaaa 5040
aagttactgg gacacctgag tggctcagtc agttaagcat ccaactcttg gtttcaactc 5100
agggtcatga gatggagccc cacggcgggc tctgcactca gcaaggagtc tgcttgagat 5160
tctctctgcc cctgcagccc tcccaagtgc atgcacatgc atgctctctc tctcctccgc 5220

ccctttctct ctctctctct ctcaaaaata aaaaatcctt ttaaaagaaa aaagaaagag 5280
agaaagggaa agaacgggta tctccctctg gccctgagcc ctccaggccc tgcatgaggc 5340
caggcatgca gcctgacacc caaggcaacc ttcccacagc aacagaaccc ggtctgcaaa 5400
ccttccacac gggtaagacg ttcttctgct ctggtacgtt ttattgtgaa acctttcaga 5460
tgtacatatg taaaaagctc tatgtataag agcatgttta agtcataaat cataacagga 5520
cagccagcag gagcccaaat ccccacttgg gatgaaccca ccatccacac tgttgagctc 5580
tgggtgcccc accagccccc caccaaggaa gccacccttc aagattctgt gggcctggtc 5640
ccatcttttc ttggctgtct tcccaccttt ggatttcacg caaggtcaca cttcacctga 5700
aaggtcttcc cccatcacat gtgctccctt ggctgggaga gaagctccca caaacacacc 5760
caccacccca tcatctgtgc cttgacactg gccacatttg caccgctagt ctgagacttc 5820
gggtctaaca ggagcacgtg tgtgttcagc tgctatgccc actgcagacc aggcttctgc 5880
cccaccccac ccacctgctc tcatggctgg gcaccccctc agtccacatg tctgctcccc 5940
ctggccactc ctggaagtgc agggaagagc caggcttggg aggacaagga ctctctggtc 6000
agcctcaccc agtgacccgc cccggtccag cagtccctcc caagtggggc ttgggtcctg 6060
gggcttattt cccacactac agacccagca ccaacaggtg acaccctcct tcagatggag 6120
tcttgggctc aaccgagtcc tctagcacaa gaaggggcag acagccactt cctgacattt 6180
ccaccacctt aaaagcttgg gagtgctggt tttacccttc cagtgacctg gttagcacgt 6240
tagcagttaa cctaggtatc agtgtcagag agcgggcagg tctagcgtcc aatgcctgtc 6300
acaggagaag cagcaaacct gaaaccacgt gttcataatg agactccaaa aacatgacct 6360
cctctggagg ttgctagggt atagtttatt atcttgaaaa ctgataaata aaatcaaaga 6420
atacaacatt gtgatgcctg ggtggctccg cagttgagca tctgcctttc actcaggtcg 6480
tgatcccagt cccaggatca agttccacat cgggctcctt gcatctccct ctgcctatgt 6540
ctctgcctct ctctgtgtct ctcatgaata aataaaatct taaaaaaaaa aaaaaaagaa 6600
tccaacatct atcctgactt tctgatataa catatatttc agggcaacca aactgctggt 6660
ggggaaattc tcattcattg ccaaatcaca gctaacaagt acagaaagaa tgaaaatagt 6720
tgaatgtccc catttcccag ggtgaaaatg agcacaggca atgaacccga ggtagaccac 6780
aaacacgggg agccagggtc agtggacccc aactcacaca caccaaacat gagaagtgag 6840
cagtggggac accaggagat tcagaaggcc tgctgctgac cagggcaggg acagcggcat 6900
gtactaggct gaaagtcctc atcggccagg gacacatcct ggggtcatta tgggtgaact 6960
catgagctct ctggggtttg ccttcagact ccagatccat atatggcaaa tgtaccaggc 7020
tctcagattc cactgatagc cctacataga gagcggacct actccttgac caaggcaaag 7080
ctgtgcctct acagtaagtg aaatacagca ggttccacta gagatggggc cgtgggcaca 7140
agctgacacc accgtgatgt gggaacacag gcctgcaatt gggaaccgtt tcttatctgg 7200
gacccactaa taaaacccat tctcaggggg cagggacaag gcaaagtctt gttgcttttg 7260

tttttacttt caaactactt cacaaaggca tttgtctacc caaaaaagag cttcagaaat 7320
tcctaaaaac aagtgtccta agctctccct caggatatcc taaaggcagg ctgcaggtgt 7380
gtgtgcaaac atgccctccc tcccaggcag taatgaaggt ttccagaagt aactgcacgc 7440
cactcccact ggtgctatgg gatgtgggat gtggtggacg gggtcagagg gctagtctag 7500
ccccctgtgt ggtggccacg tgctgggaca gctgtcctcc cagggccatg gggcaggcac 7560
gcagaccacc aaaggctaca aggcggccag tggctgggga taggcttcca tgcaggacac 7620
ccacccagag gccataccaa catcgcctga agcagtcact gccctatcag gacatgaaaa 7680
gtccaccctg gtaggaggtg caataaagga cagagggtca ggtcctaggg gcctgaggaa 7740
caacagaagg caggaatgct tcaccgggtc tgctcagagg acacctggct ctcaggcaaa 7800
accccaggag tgtctgggat cagagatctc ctacccctta ccttggcatg cttagtgggc 7860
attgcactgg atttctgggg caatccggta tccgtgtcct tctcagatcc gagctcagca 7920
gagggctgtg agaaaccaat cggtcatgag atgggagtcc gagggtgatg gccactacct 7980
gcaggacatc tctttgtagg aacagctgcc accagggcag agggctcccg tgcgccgtct 8040
catgggttct cactgggtgg ctgtctgggt gaccactgtc atgaaccaga ctcacagccc 8100
caaagaggga acaggggtgg ccatgaacta tgaaggctca ctggacatgt aactggctct 8160
gcctgaagtc aaagacctga cgtcttcact gtctttacga tctcttatcc caaaagtgca 8220
tctaaagagc ctgttgagga ttctgtgtgt ctctgagagc tcctcaagcc catttaagtg 8280
acaaccatcc tacagaactg actccaaaag ctcaggatga aatgcatgcc ctgcaccctt 8340
acccacccat cccaccagtc acacgggcat gtaccactca cacccatcac tggaaacctc 8400
cacgcctgca tttccagcag aggagcaaat gctacagaag acgtgggcac acagcattaa 8460
ctagtgaaca caaaatgcag caaagtttca acccatcatc tcttctaacg taagggagag 8520
gtcctatacc cagagcactc agaggagaca gtccagcctt tcctgaagcc caatgcatag 8580
gggctgggag agcaggtgac ctgctgaagg ctaacgggtc tgagcagcac agggggacac 8640
atggaaatgc cccactatta tttagccaaa tgggatttgc ccacgtgcct gacaagtcca 8700
tctggcattg gaagaatggc ctccccacct gcgccagggc cagttgccct acagagaagg 8760
tctggtattt gaaggggatg tgagcggtct ctcgtgggcg caggaagatc tgcggggcaa 8820
ggctgccccg cagatggaac atgtcctcct ccaccggcgt gtgcagcttg gtcgcgtctt 8880
tgaagtatct ccactcctga ctgtcctgga tgatgctaga tagcagggga gacacagcac 8940
tcccagtgag tcctccgccc gcccactcag agctggcacc taggaccagg ggtgaaagcc 9000
aggaccagtg ggaccccttc agggatgaga gcagcccggc tttcagggag aacctgtaga 9060
cacccccagg gaccctgtcg gggctgccac tcaagcctac ctgagctcag gattgtccac 9120
ctcaatggtc actgtgtgct gtgtgttgtg gggttcccta agggcaaact cgaagaactc 9180
ggcagtgccc agtgtgacgt ggattgtgtg catcgtggtg atggccaggc tcagcgcact 9240
ggtgatgctc tctgccttcg tacgctcccg ataggcagca atgacctgca ggtcacgtgc 9300

gtgctgtgcg cggatattct gctgaatctg tgggacacaa atgccaaacc aggccaccat 9360
catggggctg gctctccttc tcaagggctg tttggccacc tcccccactc ccatgctgaa 9420
taccaaattt cttctggaat caccacctcc tccttcccca gacacccggg ttggatggga 9480
ctggcccatc ctcaggtcca gaggacagta aacgggtcta cacggcactc ctggctccaa 9540
tcagccctgc agtggtcttc cctgtagaag gtggcatctg ccctgcaggc tcccaagaga 9600
ctacaaccac ccatgaggcc acctcttccc atgcctcaag gctggcatca gcatgatggg 9660
gaattagaag caaaggtttc taacaaaagg gggggaaatg acctcaaagc tactaagact 9720
agttatgtgc tcattggcca ggtccccagt gtggcgccgg gagccccgtc tgtgagcagg 9780
cgcctgtgta acctgagcca ggccacggtc cagacagctg gcacgctgcc ttagggggct 9840
cagccacagc tcagcctgcg gtccttcatc cacctccttc ctccatgccc tgattcactg 9900
ggcttggcca acacaaagtg ttctagaggc tggagactgg cttaggtttc ctgtgtgact 9960
tacactgaaa ggtgaggtcc ttggaggctg ccctgatcta ggacctagga ggggatgccc 10020
cgtgctgggg aatgagccag gccccgtgct aagaccctct gaaccctgag gaggcccaga 10080
catcagcagg cagaggatgt ctgaatgtct ctggacttca actcaagaag taaggatcca 10140
atatggtttc tgacttaaat cactttggat attgacttcc aacatccaaa actttggcct 10200
tcccctagca agagagttga ggccaagtct ttaatttctc cctggggatt tgaaaacggg 10260
atggtatttg tgcttatatt tgaggcctcc ccacacagac agagatgatt agagcttctt 10320
gatggagtgc gggtcactcc cagtttacca tggaccaggt ggtgaacagc ctcagggaga 10380
gtcaacatga tcctgggggt tgtgaagggg agactccttg cctgtcctgt gtcctgccac 10440
accacagagg aggctgtcgg gaagcctggc accccacagg acatggggtg acttctccga 10500
tatgaaccag ccagacatag ccctccctct ctcaaccctt ccttttggga tccacctgcc 10560
tggaacaccg ggtgtttgca ctgctcctca ccagtggtgg ggttagtggg aaggatcctg 10620
cctgtggagg cggcccacag ccatatggtg tcacccatgc agcttgggtc tggcatttgc 10680
tctnnnnnnn nnnnnnnnnn nnnnnnnnnn nnnnnnnnnn nnnnnnnnnn nnnnnnnnnn 10740
nnnnnnnnnn nnnnnnnnnn nnnnnnnnnn nnnnnnnnnn nnnnnnnnnn nnnnnnnnnn 10800
nnnnnnnnnn nnnnnnnnnn nnnnnnnnnn nnnnnnnnnn nnnnnnnnnn nnnnnnnnnn 10860
nnnnnnnnnn nnnnnnnnnn nnnnnnnnnn nnnnnnnnnn nnnnnnnnnn nnnnnnnnnn 10920
nnnnnnnnna cacttattgg aatcctcagg tcccaacagg cagcagagcc cagtgatcag 10980
agcagtgaga ctgctgagcc ttctgctggg atttctggaa ataaagctct gtcttctgtg 11040
gcacctggca tggaatggtg tgaagcgggg ttagtgctac tataagagac aacctggggg 11100
ctgcagggag tgggcaagtg gcagaggtag gggcatgacc ccaggggcct gaagcagggc 11160
cccaggcctg cctgctcagt accaggctcg gagtgctagg gcacctgctc caacatggaa 11220
tggctggtcc agagcccagg cccaggccct ggggaccccg tggctgcaca gacctgagtg 11280
catggggctt tgtcactgca acctcagagg acccccctcc ccgccggaca gggagatgtc 11340

ctacaagcct tctcccaaag ctgggtgggg ggtcagggtc cacagagaag gtgcccagca 11400
cacagtctcc ctatgggtgg ggctctgctg gcataggggc tgacgctgac cacagcagaa 11460
ggtgggacct gccatcatcc cgagtgccag acacagaaag cctagccacc ctcatgagaa 11520
cactgagact gctgcaaaca tatgtcgagc ccaactctgc tccaccactt ccccaagaag 11580
cccccagatc atcaccaccc ctcgagctgt tcctgactgc tgggggagtg agccacgcac 11640
agcgctcttc tctgtcttcc aatgagcagc ccccagggcc cgagaccacc agactgcacc 11700
agcatgtcag gggccaagga ccagccagag gctgagcacc acttccagct cccggagggc 11760
caactgaccg cgtaaattcc caaactctcc tgaaggctga gagagctcta ggtgcggaga 11820
aaccaccatc tcctccataa aaacaagtca ctttcacctt aaattctcca ggccgctgac 11880
ccagaatacc atgagaattt agtggaacat ttccttgtat ttagaaatcc aattacttcc 11940
caattcactg ctgtcatcag aatgagggct gcgagctgca gaatctctac caggacacat 12000
caacaggagg aagcccacca aggtaagaat cgtcaggaca ccagcaccaa actcatcaag 12060
gactggcagg gtgctgggcc caccaccctc caaggcgggg ttgctgggga gcccgggggt 12120
gggggtgggg gcagcattac ctaggggtct ctggggccac tagccccagg ggcctcaccc 12180
tcagcagctg cctggctctc tttgaggagc acctgacagc aggaagcctc cccagctgct 12240
gtgagcacga agggccgagc aggttttgcc tctattcaga tatctgcctg ccctaatgta 12300
aagtgtgatc ctcacccgtg tggcttcttg attccataaa accatcaggt acaaccaccc 12360
tctcctccca catctaagct gtaaatatgg tagggtcttc tttagttttt tagccaaaaa 12420
tgggaatttt tccagtcaga atggttactt gatttgcact gataattcat gatacggaag 12480
gcattcacag cactgacccc aaacctgggg gaccctcaga atcccctgct gagctttctt 12540
aatagcccag gtaaagaccc aggtgcagca aactggacac agaattccct ggggtagagc 12600
caagaatcgg cttttgtaaa aactctccag acttgaaaac gttgcttggg ggatgcctgg 12660
gtggctcagt ggttgagcac ctgccttcag ctcagggagt aatcctggag tcccaggatc 12720
aagtcccaca ttaggctccc tgcatggagc ctgcttctct ctctgcctat gtctctgccc 12780
gccccccccc cccccccccc cggtctcatg aatacataat ctttaaaaaa aaaaaaaaaa 12840
aaaaggaaaa cctcccttgg tggctgagag ggtccgtttc ccatacattc cacaagtagg 12900
atttgggaga gagagagcga gccacagggg aagagaagag agagcacagg ctgctttcat 12960
tcacaatgac tgtggctctg cacggggaca cccgggggca gaggggaggc ggatggagcc 13020
ctcaccaaca tgctggcccc ccagcagctg agctctcctc tggactcttg aaggcgcacg 13080
gacctcatcc gctccagctt gcgcctgcgg acggcatccc cctcacggca agcaccttgg 13140
ggccccttgc tcccatgttg catgtgggtg aacagcatgg cggccagctc acggtccacg 13200
tcagccagct tctgtgcttg gacaacattt tttactgcta gaaggttcaa agagagaaca 13260
agaggttaag gcagagtcta cattctttaa acagacctgg caacaagctt ccagccacct 13320
cacccaagga gggagctcag caggcaacct gccctttcct gcaccttcta agatgctgat 13380

aaagatggta atcctatagc ctggtctata cagtgtcccc cccctttacc agcctttcaa 13440
ataaaaggat tctgatcctt agagacaaat tcatacacca ttggataact ttccagagga 13500
aaaaaaaaaa gtcagtgttc tagaagctga tgctttctgg attcccaagg gccttgccac 13560
cctgacccag gaccactagc cctgtcaccc tcacccagga ccaccgaccg tgctcacaaa 13620
agcccccacc agcgagagcc tcctgataca ggcaccactg agcaatcagg cccacggccc 13680
caccagggcc tcagacgcac tcgtgtgctc aggtcaggag tcaggggctc tgatgagctg 13740
ctcacttggc ctgaagcccc ctgagcagct caggggggtg gggtccagac taggcctggc 13800
tcccaagctc atgcccctgt ctctgccacc agctgcctct agacagcctg gccccccggc 13860
actgactcct ctgggagact atgctcaaat cctggacaca cacaaaagcc atgtgggcaa 13920
acagaagctg aagcaggcca gacaccagca ggcagaggag ctgcattttc ttcaggtgat 13980
gaagccacac gcggcacatg gccatgtcac aagctgacac acgagattca cagaccacca 14040
ctcaagccag tgcaggcagg cagaaggtgc accccaactt agcactggcc cttcctgacc 14100
ctgaggtacc agcagtgcct acaggaaatg ggttgccgtc ctgaatctgc caaggggttg 14160
cacatggtca ccgcatctcc agcctcctac tgaaggctcc atgtggtggt aaagcagtcc 14220
ttcccccggg ctcgcccacc tcgtcctctg tcacatggaa caacaagtgc tatttccatg 14280
ttcctcacca ccattttaaa gctgttcaac tgcttttcag ataccatgag tcagtttaga 14340
caagtaaaat gccaccccaa aaagactgtt ttcacgtctc aggtgcaagc atgctgagtg 14400
ggcaaccccg cctaggttgg gtttgagagg cccgggggaa ggggccaggg caggagacaa 14460
gcaaggctag gggtaagcca aggagctcac atgtcagatt gcatcactct gctccaacaa 14520
aatcagaaat gaaatctcca cactgctcag agcctggaag cactttctaa aactctgtac 14580
aacttcctac ttgccaagcc tgccctgtgg gggaagacag gctctctgat ggagcacccc 14640
agcctctgtc tttgaccaaa cagggagaga ggaaccaatg actccagaga ctcaattctc 14700
tcaacaaacc ctcacaggtt acagcaccct gcatttcggg cagagcccct gacctgctgg 14760
gccctgaggc cgtggctttc acaaatactc cctgcctgtt cttgggacag ccagggatgg 14820
atgggccctg gccccaaagt gggaaatcca gattctcacc caagttcacc tccctctgcc 14880
acacaattag gcaagtcacc tgaccgtctc tgccttggtt tccttaacac taaacaggag 14940
aacagactct cagcctgccg ggatcatgga ccttaggccc gtctgagcat ctgagcctcc 15000
agggccatct cccagaagaa catttgagta cccacaaagt ctgggctgga gtttcagaga 15060
actctcgtgg ggtcctggag gcctcgcggc tctcaccagc cacgtccagg ggcctcccct 15120
ccctgggcac aggttcaagg acaacagtat ccgaggacca tcaacacagg aagctcgagg 15180
tagctgcaga tccacatgag atggcctgca acatggtgac actacccaaa agacccaaag 15240
ggcacacccc actttgcatc ctacctacaa ctactttggg tcatttaatg ggatgtaaat 15300
ctttggatgc cccctgctgt gaaattctcc aagcccaggc ctttatctct caggctccgt 15360
gactggttta ctcctcctca ccccaacata gggacttcaa atcggttttt aaaaaccagt 15420

gtttgttcat cctaaccttt tcttcatttt ttgctcccct gcagatgggc agctaaccaa 15480
aaaccgatcc cctcgaagga ttattgtcag cccagacagc ttccacagag cccgctctag 15540
agtgtcccgg gtatgacaca catgcttaac ccacccagcc caaggggcca ctgaggcagg 15600
gcgctgagct ggacacaaca gcccccactc ccgcccccag atcacacgtg tacctgcatg 15660
cacacagaca cacacgcagc cttccacctc ttactccacc ccacccccat gtcaatcaca 15720
gctacacatg gcccacctac cccaacccgc tccccactca catctcggac ccccgcgcga 15780
gatgagactg cctccctcga agtgtctggc tccatcattc gaaatgaccc gtgacctgga 15840
aggtggcaat gagctggaat ctctcactct ctgctcacac acatgaccta aattaaaaac 15900
atcccagtat aaggtattaa caccaaaaag aagtgtgatg ggacacatcc atgtcccctg 15960
gagggagttc tgggtttcca agggcctgtc cataccccct ggcctgctaa caccccaaaa 16020
ctgcactctg gttctgagtc tgcttggcag ttgagaggtg acgggcaggc gggtcctctc 16080
gtacatgggc agtggtgtct gctttcccac gggggttaag gctgcaggta ctcaggacac 16140
tttttcacac acactcagtt cctggtctct ggagcagcct agcagccctg cgacgtgggg 16200
cagctcacac attcccacgc cagcctttcc aatgtcaccc tgctcatgga aggcccttgg 16260
gcatgtccgc aaatgtctga ctttaaacat acttgaaggt aaaagagcct ctgcaagcta 16320
gaatcttcag gagctgtttt cacacagaag ccttggagag aagcatgcag cctagagggc 16380
caagcacaag tccagaggca gagcacacag cacacctgcc ccggctgaac gggaagcagt 16440
gtcccactaa ccagccaaca tgtgcaaggc tcaggggata agaactgttt gcctgaagaa 16500
atgcctctcc taggaggaca gaatcatacc ctcaggaccc gtgacctctc tctgtggaca 16560
tgactcccaa gagcacagct gaagtcagat tccaaaatga acccaagttc caaacacaaa 16620
tgctcatgct gtgggcttcc atgattccag caggatggtg gtgatctttc tgggagctat 16680
tagaacaggt accccttccc ctgggtgctc gtctcccgcc tctctgcgtc tcctcccacc 16740
tggcctccac tctgccctgc tcagcttcat ccgcctctgc ctctgtcctg tgggacagtc 16800
tgggtgcaag agcaagcccc ccatcacagc cgtgggctgg tcctcagcag tggtgaccaa 16860
ggtgacattt tcatatccag ctgcctagca gaaatgtctt cctcccgact ggttctgagc 16920
tgagctgggg aagggaatgc ctatgctgcc agggaggtct ctgccctgct gccaatgggg 16980
gccgcccctg cagaccccag ggaatggcag ctggagcccc ccaagacatg ggtgtgacag 17040
ctgaccgtcc cttggcattg tggaaaatgc tggattcaaa aatgagagac tgacaccacc 17100
tctggccaaa ccaagaagtg atttacttgg catggcttaa agcctggcca caaacaggtg 17160
agcagagtcc tcccctaggg acttccattc tcatcatttt caaactccct tggtcatctt 17220
gagcgttttc tgagcatctc aaatcaggct ggccttcccc cagccttcct gcaacaagag 17280
ccactcctgg ttatctgccc ttctgcctgc taaatggtgt tcacagctca gccagtagcc 17340
ctggcaagcg ccagagcatc agctgtcatc cacgctcccc gtcaggaaga ccactgtcga 17400
gggccttccc tgacacagca ccacactccg ggaatgggga agcatggagc ccaagcaccc 17460

acccaccaca ggcagagaag tcggctccaa agcccaggtt cgccatgaga ctccagatgg 17520
caggcctgtc tcctctgtag caggttcctt acctgcctgg ccactgtggc cagtgaccat 17580
cctacctggg gtctctgcct agggtaacct cggtgccagc ctggtctctt gctcttctgc 17640
ccctacctgg acgtcggctc tatatgtaca gcatacactt ggacctgcag gcccagactt 17700
ccgggcagac accctgtcgg ccactcccac ccctccctct agccctgtgt cttgagaaca 17760
agcttcttga gcccagatgt tctcagcctc tgctctgttg agaagcacgg aacacttctc 17820
cacaggggtc tgcccgccac ccacccacag ctgcttctct gctcttcccc tccagatgca 17880
ggggctgcag tgtcttctgc caaacatggt catctcagca tcacaaggta ataaagctcg 17940
tccccaagag tcgagcctgg tgttaacacc ccttcactgg gatcagctgc tcacacctcg 18000
gggcctgctt tgtcacctga agcccaagag gagcagaatt caaggcaaga caggctctca 18060
tacttgctca tccacccatt catgaacctc tcatcaccag gccaggtcac tgtggctgct 18120
gctgggtgag catcacagca ggaaccagga tgtccctgag aaagccccca ggtccctagg 18180
gggacagaca agccagtagg tggacaggca gggacagaga gcctggctaa gtgggcagga 18240
gacagccagc tagtgccagg gcccaagtcc cagcactgac actacttgct ctggtcctac 18300
cctgatttcc aggtgagagg ggaacaaagc accatctggc cccagctgca ctcagttcct 18360
gccatgagct cagagacagt ccctgctcct ggctctcagc tgcttcttcc cagtgagggc 18420
aaacagggcc tgcaagctct ccatcccacc ctgaggaccg gctccatctg tcacctggca 18480
gggggcacat accacctcca ctcactgttg gggacagcgg attcatgcca cacctgtggg 18540
aacctgggga ccctccgtag ccttccctga agcacaggca gggaggcatt caccatgtca 18600
agctgaaatg cacgcagcct acccttagca ggcaacagct ctagggggag ggacaggaac 18660
cagggaaatg gctgcagatc cggatctggt gcagacgggt gcactgccag ggcacacaac 18720
cggggtgcat ggccggcatt gtcagagccc aggctctcag gactcacggc tgtcatcgca 18780
gcatgtggga ccagcaggac catgtagtcc cctccagaca gacaagggcc atccctgagt 18840
gtcacagttt cctctaacag tatttgcact cagagcgtgg gttcgtgtgg aaggctgcac 18900
aataagctag atcagggcaa catacacctg cttactctgg gcctggatgt ggtgaaggtg 18960
ctcacttctt cagggcacca tctgcaatct caagttgctg tgggctttct tacccttcca 19020
tgacaccggg gttaagctgg ggacccacac atttgctgtg ggagcggaga ctccctccag 19080
atgagcaggg ttcctgactc agctgtgcag ctaccttcat tccacggtgg cagcagagca 19140
catcaccccg tctgacccca aacccaggct ccctgtccac accacccagc tctccttcaa 19200
ccctgagaat atggtgccag gggtcagggc agccccttgc tcccctgagg agccctggaa 19260
gggctgcaga tacaagtgac aatagctccc cgatgaacca cagccacacc gggatcccgg 19320
ggacagaaaa gcaccagaga cactgcagtc ttgtctgcag ggaccatcgc ggaacctaga 19380
agccaccagg cacaagcacc ccgccgcccc cttcctatgg cagattcatg ggagatctgg 19440
gctcagagag ggcacctcgt tctgcagcct cacacttgac agcatacact gggagtgcag 19500

agccatggag cgccgtccaa ggcaaggcca cgccaggccc atgatgctgt cctgaccctg 19560
cttctgtccc ctgaagctca cacagcctca tccaatatgc aggcctgcac ctgctgcttg 19620
gttctaaaca cccagtttta aaaaagaaat acaagacaca atgccatttt ttgttttttg 19680
tatttttttt caaaaaatgc aaaatttaca gaggaaacca gaccatggct gtatggacag 19740
tgagctgctc gcgctcgtcc tttgtcaatc cccctccgct ttggcgcctc tgggcgggtg 19800
tctctgactc tcccccgtgc gctatcgctc ccttgcaggc aaagtatgga aatgacaatt 19860
taagataacg tataatagag ctaatctttc ttctgaagat aagatttaaa gcgcggagtc 19920
actgcacatt tgtgggctgt gaataaatat aaaaataacc aatgcaatct cagaagcaga 19980
acgcttcttg cagctccctc actcatagga gcttctgaat gatgtctgct tctcacatac 20040
agttcctttt tttctccaga acctaattga gttttgctgt ttccatattg tctgttttca 20100
aggcggcctc ggatcttaca attgtcaaca agcaggcaga aacctccagc ctaacttaac 20160
gagtatgcac gcctcctgct gctgcggccg ccccagcctg cccgcctcca agctgcccgc 20220
acgagtgcat gtggcccatg gggctccagg ctttaaagcc agcctgggct ttgaggaatg 20280
gaaaattccc ccataaacca tgagaggttt gagtctcatc tagacagaca tggctccaga 20340
aacctcaaat ggcttggttt cgtcctgctt tccatttcac cccaaaaaac ctcaatcctt 20400
ccatagtctc ctcaagagag actgagtggg cttgtcccct ggccccacag gggctcactc 20460
caggccccta cccagtggga agcacaggaa gacatctcag gccccatatc ccctgtcgat 20520
caggacctcc gtggagcccc cagaatggcc agccaactcc actgtagcca cagccagccc 20580
cataccctgc cccaggcatg tgaacaacag cgcacagggc tgcactcagt gtctgaagag 20640
tgaccctcag acatcattat atgtatggtc agagtatagg ctctaaagaa gtgtgtttct 20700
ctgttccctt atgcccaagc tgcatcaaag ttgagaaaca ttttgctttc ctgtcctatg 20760
atgaagtagg ctcataatct agatgaaaat ctctcctgga accagtcatt ttatctgagg 20820
attttcatag agatgtcaca tagacaacac caagaaacaa aatgtggaca agactgagat 20880
gactaatgat tccaagtcct gatcagaaac caagaacacc aaacaactag tgccaagaca 20940
ggctcagggg tcaaggacag atctagtggt caaagacagg tccagtagtc aaggacagat 21000
gctcagtggt caaggacagg tgctcatggt caaggacaga tgctcagtgg tcaagggcag 21060
gccaagtagt gaaggacaga tgttcagtgg ccaaggacag atgccatatg atcaaggaca 21120
gatgctcagt agtcaaatag taagagtttt tctgaattcc ggttgatcat atgatgccca 21180
caataggacc actgccctga gggagctcat gcaccaactg ggaaaatgag gggccaccca 21240
ggagacagca taagaccagg taaagctgcc agggactctt acccacgttg gccaacgtca 21300
ggtgcagcca gccctttacc actgtgtaga caccgatggg cttgacacag ccaaatctgg 21360
tcacatctcc actcaccacc acatcctgtt catattcagt cgccacgacc tcaagctcat 21420
gcaggacctg cacagccggc cggccttggc ggaggagatg ctgcagagaa aaaggcacca 21480
gtcagagggt agccgaggta gagcccatct tctactgaaa atctacactc ttgatattgg 21540

gtctttccca caaattcttc aattaagtgc accccaattc acccactgag aaaatatcat 21600
ggatgcacaa acaggcctgg cagccaccct cctggaaccc cttctatggg gagaacaatg 21660
ggaaacgagc aataggagga ggctgggaat gctgagcaga ccccatctcc cagtcagtga 21720
gagcttcaag aaggaggtat gaaccaggcc ggggatgcta agggcacact aggcccaggg 21780
tacagagcag accccccaca cccggggcca ggagcatcag gcttcagcat ggctgaaaga 21840
caggtgaggc ctggttgtgg aggtccacat aaggcccagg ttggtcagag ccggcaagag 21900
cagatccagg acaggcctcc taaacccagt gctttctgct aatccagttc acccctcagt 21960
gccctgcctg agcctaagtc atagtgaggc ctgcaatggc ctcttcctgg gaggccttgg 22020
gaaaatcacc tgacctttca actagtgctt cccactcaca aatgggcatg tggaggtcag 22080
acaagcatcc caacttctgg aattcttgca cttctactac agcaaacatg cccagctctg 22140
ggagtggaag tgccccaaat gacccctctg tagtggctct ggcagcttct ggttgcttca 22200
ggaggatgag gacctggccg gactccccag ccacccaccc gcctactctg cgtgccgaac 22260
ctcccacaca ggcacacctc tgactcagaa ccacacttct gatgagttct catctgggga 22320
gggcagagag tgacggtaat aagcacaagc ttccaagtca gaactgggct aaaaggctgg 22380
gtctgctgtg gtccgcgtga ccgtgggcct gtctctcagc agcacatgaa gacagtgcag 22440
tacaactggg ctgaggacac cgtccacgaa gttcaagagc aatcagatgc cgtggcaact 22500
gtcactattg tcctaacaac acccttgtag ccttgggaat gagctgaaga ttgggcaaaa 22560
tgtacgtcca gatgttcact gcaacattgc tcatggggaa aaaaaaatca ctcagtgccc 22620
agcaacgaga gcaagtgaga cgagtcctag tgtatccata ccttccagaa aatgtgccta 22680
cccaaggcac acagctagtt cagcacctgg agttaacagg cggttctctc ttcttacctc 22740
atctcccatc tcttctatat aaattattaa ggtaaagacc ttatttttaa aaagtaaaat 22800
ttaattttag tttatccaag ttaagtgtca ataatcacaa cagcatttgt ttcaggggct 22860
ctttgtggag gacaatctag agtaacgtca tctccaggga cacagggaca ccgggctccg 22920
caagggatga ggcaggagcc acagcgggtc ccaaggtaac atgggctcct ctctgcccct 22980
gcagtgccct tcctcccccc actcctacct ccctcttccc tccactccca cttcccccac 23040
tccctcctcc ctctatccct ccccatccct cctcccccat tccttcctcc cttcactccc 23100
tccatccacc ttccctcctc tctccatccc tcctccctct atccctccac tccctcccct 23160
cattccatcc tccctccatt caccctccct cttccctcca tctctcctcc atccatccct 23220
cctcctccat ctatctctca tccctcctcc cccactccct cctccttcca tccctcctct 23280
gtttatccct ccactccctc ctcccgtttt tcctccctcc atccctcctt cctctatcct 23340
tcctctccct cctccctctt ttctctcctc cctcactccc tcctccctcc attcctcacc 23400
cttcccccca cccccatccc ctcaatgctc cttccatttg ctcattccca gctacacagc 23460
accatgccca ctgtgggggt catgactgca ctgaagatga agagcagtcc cattcctacc 23520
ctctagaact ttcaccagcc agcatcagag acagacaagc aatggccacg atgaccccct 23580

ggagttgcac ggtgacccag aaaggcccaa gggaccaggg tgaggaatga gaacatcagc 23640
caatatctac tgggtgctca ccacggggga cccctgccct cactcctcct gctcccctgg 23700
gggcatcact cctgaggggt agacactgaa tcgggtggag gagggatggt acaatccagg 23760
ttagccctca gagggaagag gggaagctgt gtcctctccc acctccgtcc cacagccggg 23820
acatcgcgtc cccagtccct agcccaggag ctgcggtgta attacactga ccctctgggt 23880
agaagcccca ggaagaatct taaattaaca tatggattta ggttttagtg taggaggaag 23940
atggaaagca caattagaga aagaattaga ttctaattac aattagaaaa gcagagttga 24000
agtaacagat agcaggcacc aggtgatctc tgcacaccca atcgatgact catcctaccc 24060
tctgtgggcc aggcccctgc tctcactggt gggggcagaa ggcagaaggg gactgcacct 24120
gtggtcagtc tgtaggagga gctgatgact gaggaataat ctcgcattac acagctgaga 24180
ctctgggaaa atcttttcct ttggggaagg acggacttta tgaacaaaga catcagatga 24240
cagcccacac tggacgagaa ccagcaactg agcagacccc tccccccccc aggagggaca 24300
ccccacccta accagcccgc tgcagagcac tgccaccttc atctggacgg ccgcagatcc 24360
cacgagcagc aaggagtctc catcccagat gtcgatctgg agggtctgag cagccatgta 24420
gtgggtgaac cagcgctgtt ctccgggttt caggaaccca ggttccacca ggtacttgag 24480
ttgaaagcca ggtgtccctg ttcagagagg ccccgaatga gcccaaaagt gatttgagtc 24540
attatcatct tcacattttg aagagactcc tgctgtggtc acaatcacta atttctgctt 24600
ccgtcataac tacagtttaa taaacacatt aatcaaaaat atcataaaac atccacattt 24660
acaaaataca gagtcgtatg cagaagtcta catttctttc caatacatac aatgaactcc 24720
tcccagaata ccccagggaa gaatcttgtg aaacagtcta tcctgaatgg ggacagatat 24780
cctgacgggg ctgcccaggg aggtcattat tctgtgtgca gggtttaagg ggaaacatgg 24840
tctgccgtca aaagtgggac agcacacatg aaagcacgcg cacacacatg gagctcaggc 24900
taaggtcagg ctgagctgcc ctcaacgcat tcccagcggc cactaccagg tcatctctga 24960
gacagcttct ccaggtggca tcaccctcga ttctaaactg acaaaaacac cactaaaatc 25020
attagttaaa catttccccc ttcccatcaa taacctattt tcaggggcac ctgggtgcct 25080
cagcggttga gcatctgtct ttggctcagg tcatgatacc ggggtcccag gatagagtcc 25140
cacattgggc tccctgcatg gagcctgctt tttcctctgc ctatgtctct gcctctctct 25200
gtgtgtctct catgaataaa taaataaata aaatcttaaa aaaaaaatat tttcagagcc 25260
tctgacgagt agaatactac catgtttact cagcattggg aagcgcgacc caagtcagtc 25320
tctgcctcta gccacctcag cccctgggga gcggggtggg ggtggggggc acacagtcat 25380
gaacgtcccc tctcctctca agactagaac agtgcttcta gttgaaggag ggaagcgggt 25440
tgggatggga cacagcacca ctgagggcct cgcatgggcc tacggactaa agccaggacc 25500
agttgccata ggcctggctc cgaccacagt gagaattcac agggacacct cattaaggga 25560
gcccagcaat aaggaatcag gaaaaaggct cagagagggg gtggtaatga catcatgcag 25620

cggcaggcca gtccttagta tcacatatcc agtgaccaga caaagtcaca gggccaatgg 25680
aggccaagga cacctaccac ccttgcccca gtgccaccca ccagctgaga cctaaagtgg 25740
cctgtgacaa ctggtcattc tcaggacacc gcctaaataa tgggagccaa ggctggctcc 25800
ttagatgcat ggtgaggacc agacaaagac ctataaacac ccaagaccct gctcatggcc 25860
cgaatggtat ggcaccccat accaggccca cctgggctgc aggagaagga agagctcagc 25920
cactggcttc tacactggtc atacttctga ttcgaggaga cagaacaggt ttgacctctg 25980
tgggccaact tcccaaacga tgcctacaga caccagcctt gggagctcca tactccacct 26040
ccagcatcgc ccagatccac agaccctcca tccagagttc tacctgctcc ccagtgacgc 26100
atgtcccaga tggaccctcc atccttccca caccagctcc ttgttacatc gtcccaaagt 26160
acttccttgg caccagaccc ttgcaacgcc catgctaggc tggctcccgc tccaccacca 26220
ctgcatccac tcagctgcct ggccctttgg cacaccatta actcacactc ttcaaataac 26280
tgcagtgccc ccagcatgtg caccaagcac cacgcaggga taacttgaga gagaaggacg 26340
gacagggagg gtccccacct gatggagctc atggtcacag gataacacac tcagcccaga 26400
gctaatggcg tgctgagtac ctgggggagg catgtggggc tgtgggggcc atcctggtct 26460
gggcccagcc tcagggctct catgctggag gctcccagtt cctcctgcca caggcttggg 26520
cccctttact tctatccagc tctgacaacc ggctcctcag tggccttccc tgaggtccac 26580
actccctgca gtccatccca ctacacagcc aaagcatcca caaagggaac cctgtgtcct 26640
ccatcctgct cagaagtgag caatgatact gaccatctac tcacacaaga cagacctcag 26700
gggcagtggc acctccccag ggtccactca ggttcccaag cttacctccc tcaccccctc 26760
tcaaacacca agccccagcc cagctggctc cattctgagc ctcagtccag ccacaccccc 26820
acatccaagt gtggtaggta ctcttcctcc ggttggatgg tcctcagtct ccatctctcc 26880
ttcatgaccc acaaacaccc ctactctgcg aaggcatcca caactttcac agatgacagg 26940
cactttatac cactatggtc tacaatccac aatgaatcca caatgaatat acagcagctg 27000
ggaatatcta cacaccatat gacaaagcaa aaatctagag acgcaaggag aaacagataa 27060
aaacataata ataggagaga tcaactaatc actttgtttt cagggcaggc caagagggca 27120
ataaacaaca gcatggaagg ccagccaaga taatccatca catagagcgt atacatgtac 27180
atatgacacc attccctgct aacattgaat atatctttcg agtctatctg gaacattaat 27240
aaaaattaac tatatacctg gccacgaaga aaacctgaat atgctccaga aggtgaaaat 27300
aacacaaaca atattctctg aatacaatgc aacaaattag aaactggaaa caatttttta 27360
aaaactaatt tacagcttac ttttaaataa cttgatgtca aatggaaaac aaactccagg 27420
gcagcccagg tggctcagcg gtttagcacc accttcagcc cagggtgcaa ttctggagac 27480
ctggaatcga gtcccacatc agactccctg catggagcct gcttcctcct ctgcctctgc 27540
ctctgcctct ctctctgtct ctcatgaata aataaataaa atcttaaaaa aaaaatccaa 27600
acagattttc tacagaataa tgaaaatact acttatctga atcaccaaag cataaagctg 27660

taatcaaagg aaaattcata gccttgaata ctttgcatcc acaaaaacaa aagaattaaa 27720
gtaagtgaat taagcaactc aaattagaaa aataacactg aagaatacaa aaaaaaaaaa 27780
aaggcaagaa agataaagat gaattttaat tggttagaaa ataagaaaag agcaaaacct 27840
gcatgaatgt gtctttggag agtcagggaa gcaggagtga ggacatgaga ggatgcccat 27900
atccctgtcc catgtcccat tagggcagtt ctcccgaggt taacctggat tcagcagaac 27960
ccccataaag ggcaaacagt ttcacatgga aaaccaaccc agccagaatg tggggtggtg 28020
gtggggctgc agggaagaag agcatggtgg agtggaggga aagctgtaag agcaaagttg 28080
cacctctagc tccacagcac acccactgag tggacaggtg gtctaatgca gagcttggca 28140
aaccacagcc cataggctga ttcagctgta agctaagaag agctttcaca tttttaaagg 28200
gttgttaaaa aagaagattc cagagagaac atatataacc cagaaagtct gaaatattta 28260
ctatctggcc cttaacagaa atcagtctgc caacctctgg tctaatgaaa caaaatataa 28320
aatccagaaa taaatctgag ccataaaaaa agggtataag gtaagggtgg ctctcaagtc 28380
actttagaaa aagatggact ttcttctaat aaatggcact gggtcaaaag aacagacgtt 28440
tgaaaaaaaa gataaacctg gactcctatg ttccatagca caacaggaca aactacaaat 28500
ggaccagaag tctcatgtaa aaaatgaaac ctataaggcc cctggagaaa acgtgtgtgg 28560
atctctttat gaaccaagga gaacaccttc gcatccatga ctcaggaatt tagaagcaat 28620
aaaactaaag accaccacat taaatggcat aacagcaaaa ttttatgggg tacctgcaag 28680
tctcagtctc agggttgtgg ttttgagccc taccctggat ggagagatta cttaaaaata 28740
aaaatcttgg gatccctggg tgactcaaag gtttagcgct tgcgttcagc ccagggtgtg 28800
atcctggaga cctgggatcg agtcccacat ggggctccct gcatggagcc tgcttctccc 28860
tctgcctgtg tctctgcccc tctctctctc tctctctgtc tctcatgaat aaataaataa 28920
aatcttttaa aaaaaatctt taaaaataaa actttacata gcaaaacaca ccaagagcaa 28980
agtaaaaatg accaaaacag gagaacatat ttccaattta tatcacaagt aatagctaat 29040
ctccccaaga aacagacttc ccaaaataga ggacagaaca agaattggat ttaaaaaatg 29100
ggaaaaaata tgaatagagg tttgcagata gacaaatatc caagatgctc aatttcactc 29160
attataagag aaatgcaaac taaaacttca ttaagatcta tctcacatcg atcagattat 29220
acaaatcccg aagtttatgc cacattttgt gggcaaggcc ctgaggaaat aggcagcccc 29280
atgcgctgca agcggagctt ggaaggccat cgaatgacag gggagtagct gacggctaca 29340
atctgattgt tagtaacgcc ccccaaacac atgtccacac acagagaaca tataaacaag 29400
gttacaagct actacattat ctgaaactca aagcctgaaa gcagcctgaa cgtccatcag 29460
aaggaggttg acctgaatca atcacagggc acacgcccag tgccagacga tgtagctgtg 29520
aaaacagtgc agggggtccc cacatggagt caagctcagg acagaatgtt aatggaaaaa 29580
agcaaagtga taaaagagca tatttggtat gctatctatt gaataaggaa ataaggagaa 29640
ataaaaatac atttgcttac ttgctgaaac aaacagaaac atcagaagga gaagccagaa 29700

actggaagcc tggttacctc caggaggccc tgcagggggc acatggtcta gagggtacaa 29760
cggttttaga ctgtgagcca tgtaaatatt ccaagaaatt ataccagaag ggaaagaaag 29820
ccaataaaaa attgataaca aattgaaata aatgaatctg actgaatatc aaattggtaa 29880
cagctccaca gaggaaagca tgagctgcag ggattctgac acgctgaact aactatgtcc 29940
ccagcgggat gtggctccgc cctagagaat gccagaaacc tcaaccctct tcactcagca 30000
cgcgtgccct tagcagtaac actggcaatg aaattctaaa agtacattat agaaatcata 30060
aggaaagcaa gccatatgat caggaagcat gattttcagt ataagatgga aaagataaaa 30120
ataaattaag gaaaaaaccc taaatctgaa ttagaaatat caatgtgaac tcatgtttga 30180
atatatattc tccagccctg cccagggaaa ggcctggaag caaggccatg caggaccaaa 30240
agtagcccag ccctcagtct ccaaaagcaa atcccatgca ggagaagcgg ctggtccagg 30300
tcaaaggcag aggaagtcca caggagccga ggatgctggg ccacccagaa ggcaaggcca 30360
tgcccaggct cctaggttaa ccaggccacc agcagccaaa cagaggggaa gtagtagtaa 30420
tgaatatatc acagtgaata gatatcagag tgattcaaaa gccaacacaa gaaacaaaga 30480
aaaaagacat ataaaacctc aattgttacc accagagtaa ctgtcacagc ttctgactct 30540
gaaaactgga aattaaaggg aaagtatcaa acatttgctc tgttttggag gatgtataca 30600
tagttcactc ttggttgata agggaaaact ggttttttaa taaaagaatg tcagtgaata 30660
aagggaaacc aaatgacaaa ttaagaaaaa ttaccattgc aaattccaat taaagacctg 30720
gcccagggga tccctgggtg gctcagcagt ttagtgtctg cctttgtccc agggcgcgat 30780
cctgaagtcc cgggatcaag tcccacatcg ggctccctgc gtggagcctg cttctccctc 30840
tgtctgtgtc tctgcctctc tctctctgtg tctctcatga ataaataaat aaaatatttt 30900
aaaaaaaaga cctggcccag gacgtaaaga agtgggtaaa aggttgttgg gtggatattc 30960
acatggagct aatgactggc ctgagtagtc catgctgaga gccaagggga atggccccct 31020
gccccccaga gggatgaggg gtagatggag ccagtgccct gagaataaga ggcctgacct 31080
ccacactgct gatgaaatcc aggcgtgggg gagtgtcagc cctacctgcc cgtgcacaag 31140
cccttcgggg agaaaccagc agatgcacac agaatgagcg acgttctatg tgacaattgg 31200
tctggtacct tccagtggcc actgtccctg gagagagaat gcaggggact attccagatg 31260
aaaggagctt ggggaggtaa ctcccagcac aacccaggac ccagactgaa tgttggagtg 31320
aaaaagtcac tgtcacaaac atacaaggaa aaaccaggaa aggaaacagt gagatcccgc 31380
tcattgtcag tatgatggag gctggttttc aatagagcct gttgctatgt tcagaggcaa 31440
accatcacct cacctgctac tgactttcaa atgattgagg gaaaaagatg aacagttaac 31500
tctcaagcat gtccaatggc ctagacacga tctccatggc agaggacatg ggactggatg 31560
cggagatcca ccgaagaaaa agcaggcaag gcagcaccag atggacccag aggaggactg 31620
gcaagcccca gatgagactt tagcccaggg gggagggggg tctctcagat gacagctcaa 31680
tgactccagc gcccttggta ataaggaagg tcactgaaac accaggagga acaagttaga 31740

aaaatccaac tccaggcctg aaagcaaaag acacgaaagg aaggaaggta ccgtgtgtgt 31800
ggaggacggc ccactaacac ctggacccct ggaaggagca ggggcaccag cgagcatgga 31860
agccctaggg gcacacccca gtcctaagga gaggagataa caagaaacaa ttctactcac 31920
aaccaggaac accaagaaac aggagcctcg ggcagaggcc accagaccca gggcggcagg 31980
ttccagaagc ttctgttcag gaggccacca agtctcctgc tggaccagat ctactccagc 32040
agatgtgccc tgggagaaac tgcttggcaa attgagtaaa gatgaacatg tgggacccta 32100
tagcaatttc actcctgggc acacccccaa caccagtgcg ggcacatatg caccagaaga 32160
atgctcacca cagcattatt caaaagaaat aagctgggaa cagggggatc cctgggtggc 32220
acagcggttt ggcgcctgcc tttggcccag ggcgcgatcc tggaaacccg ggatcgagtc 32280
ccacgtcggg ctcccggtgc atggagcctg cttctccctc tgcctatgtc tctgccaatc 32340
tctctctctc tctccccctc tctctgtgac tatcataaat aaataaatat taaaaaaaaa 32400
aaaagaaaca agctgggaac aatacaaatg tctatggaca accaatcaga taaatggtgt 32460
gcaatactca gagacttcta tacagccaag ataaaggaca aactactgcc ccacacaaca 32520
acatgcgtgg acctcataga cacaatatcc agtggaagta agactgggaa aggcacacac 32580
aagatgatcc tatttcagtt caaaactgca ggtgcactgg tctacagggc cagaattcaa 32640
agagtggtca cccctgggga agccaccgat ggtgggaaag gagaaggctg ggagaggcct 32700
ggggatgggt ctgcggccct atctgagtgg gctggtgatc acacagatgt gttcactgtg 32760
tacaagttac agctgttata aaaattaact caaaatgggt cacggaccca gatttgaaac 32820
acaaaactta gatactcttg aaagaaagta gaggacaaaa ttccaggtgt ggtgacgagt 32880
gtttacctct aacaccaaag acacgaacga tccatgacag aaataattga taaattggac 32940
tccattaaaa ttacaaacct ctgccctccg aaagacactg gtaagaccgt gaaaaagatg 33000
agccacaagc tgggagaaga tattcgcaaa gcacacatct gataatagtg caggcaggca 33060
aaatataaga aggatctctt aaaaaataca ataagaaaat ccgttctaca aacaggtaaa 33120
atatctgaac agacacctcc ccaaagaaag actcatggca agcaagccca tgagaagatg 33180
ctccacatca cacgtcatca gggaaatgga aataaaaaca acagtgagat gtcaccatgt 33240
acatatgaga atgggcacaa tccagaacgc catcaacacc aaatgctggg aggatgtggg 33300
atagcaggga tgctcactca ctgctggcag aaacccaaca tggcacagcc actcgggaag 33360
acagttgggc agtctcctac aaaaccaaac atactcttac catacaacca tgcagccatc 33420
cttctagatg ttacccaaag gagttgaaaa tttatgtcta cacaagatcc tgagcaaaaa 33480
tgtccatgat tgctctagtc ataatcacca aaaactggaa gcaacgaaga tgcccttcag 33540
taagtaagcg aatggatgtc catctagaca gtggagtatt attcagcact aaaaagaaat 33600
gagctaccaa gccacaaaaa gacatggaag agtctcaaat gtgtactact aagtgaaaga 33660
agccagtcgg gacaagctat atactatata attccaagtg tataacatcc caaaacaatg 33720
aggacagagg agggggtgat ggggcaaaga ggtggagctc agggcatatt tgggtggtga 33780

aattattctc tacgatattg cacagggata cagatgctcg gcatttgtca aaatccatag 33840
aaggtacacc accgagaata agccttgtga actctgagct ttagctaaca acaaagcatc 33900
aacactggtt cagcaactag gggaacttct ggggcaaggg gtggatggga actctgtacc 33960
cccagctcaa tttctccata aacctgaaac tgttctaaaa agtaaaatcc ataaattttt 34020
taaaattcgg ggaactgaac actgtgttgc atgcatgtta catttcatta acaaaagaaa 34080
acacatccta agtgagactc tagtggggct aggcactgag ctatggggac ctgagctggg 34140
gctgctggag gaagcctagc cccagccagg tgcccaggca gtggggtggt ggacctttgg 34200
ctgagcctca tcaggtaaga cactgaaaag aacttgtttt ggacttcaac ttaaggtttt 34260
tggacttcga gtcttaatcc aataatgaca aaatgtgatt ttggagttta atttctttgg 34320
acttcaactt acaaggaaat ttaatttttg gactaatttc tctctgggaa attcctaatg 34380
gaaaggattt atatttgaat caggagtacc tctccatggt tggccttttg aattaaatta 34440
cctgaacact attcatttaa cctcatcaca gaacctctgg tgaaccactc cagatagagc 34500
agatagatgg ggctgggaac aggaaatgaa agaggagaca gtttctcagc aaaggtgcct 34560
gggagccttg aaatcgatgc actattctga tccatttcag agccttctga gggcatgagc 34620
taaactgtcc accaatggtc cccaggagca tcagcaaagg gaaggcagtt ggtttgggac 34680
aagtggtccc aggattgtta ggaaacccag ggagttgaga aaagaaaacc tagagtattg 34740
caagagagca tccttagctg gccacacact tcatgtagtg ggacccccct acagtcaccc 34800
tctgcccagc acatgcctct cccagaccaa aagcagctca tgggctggag ggtttgggag 34860
aaggaacagg gcctgagctg tgcccccatc tcctctctct ttccagattt gtctgcagga 34920
atcgagggct ctaccccagg gtctgtggcc aggtggtcct acctctgtcg gcagcaaagc 34980
ccatctgatg aggtgagcac cagcaagcac acccaactct tgggctgacc agtgcctgca 35040
atagctctga ccctcttgac tgattctgac cttggacaag ggacagcagc ataacctgct 35100
gtacttatga ctcccctacc ccaagcccag acaaactctg tctactacct gttttctaag 35160
catttatgct tattttaaaa tttctctcct tctacttata ttgaatttgt tctggttgtt 35220
ttcttcaaac ttctggagtt gaacaattag atcagaattt ttaaaaatac tttcttcttt 35280
cccaatacaa gcacttaaag ctgtaaattt ccctataaat aacattttac ctggatctca 35340
tttgttttgc aatatttgca acatgatttt gtgataccgt tttcaataac cattcttatt 35400
ttgatttctt cattgaccca cagaataaat aaaagtatgc tttaaaatta aaaaaaaaaa 35460
actagcagtt cggagccttt ttaggggagg gagtggttga tttctgacat gtggtattac 35520
agtcagagaa tgtcatgcca ttcagatcag atactggtct cctggaacat ggtgatgcct 35580
gcacggtggc ctaaaacatg cacacgtata cgtgactgat cagttcttgt aaattagtct 35640
caagcgtgct tggaaagaat gcacatcctc taaattggtt ctataggttt tataagatta 35700
tttttttaaa ttgtatctta ttacatcctt gattattttc tgcttgatgc gtcaattcac 35760
ttggaggttc atcaccttcc acaaccatag atttgtcaat ttccccttct aaagtctgtc 35820

aatgtgtcaa catttctttt gtgttttgaa gctgcttgtt ggcagttcac aggttcagaa 35880
ttatttattt tcctagtgat tattctcttg attatattta ataggcttct ttatcctcat 35940
taccgcattt gccttttgtc tcttttgtct gatgctaatg tcaccaaact actttcactt 36000
gattatcatt ttaatatggc atttcccacc tgttctttct actgaaggag cagcaagggt 36060
tcccagctct gcccctcaaa aaccctgcca ctggccaaag aggggtaact catcattgat 36120
aaaaacaata tatttggtat gttttgatcc attgcagtgt ggtttataat gatgccagaa 36180
acaaaagccc ttggccccac tttcaaacaa tgctagggaa ctggctcatt attttgaaaa 36240
tttgtaaata aatgggaaga aatgaacacc attctgcctt tcctacatgg actacacctc 36300
aaagtaaccc catagttgag gagggcaagt ttctctatat ggaaggattc cagttgagaa 36360
acaaaaaggt gttccaggtg gggatccctg ggtggctcag cagtttagcg cctgcctttg 36420
gcccagggcg cgatcctgga gtcccgggat tgagtcccgg gatcgagtcc cgcgtcgggc 36480
tcccagcatg gagcctgctt ctccctctgc ctgtgtctct gcctctctct ctctttctct 36540
ctacgtctat cataaataaa aaaaaaaaaa aaaaaaaaaa ggtgttccag aagtacaaca 36600
tcaccactgt tcaaccttga gcaaaataac cagcctatgg gcaaccatcc taacagccac 36660
tgactatcca cagaaagaag ggaccagccc ctgcacagcc ccagccccag agagttcctg 36720
cccaaaacaa aaacatccct ctgaccgtgc atagagatct ccaatgtcca ggaaaaacag 36780
gtgacagaga agggcactgt aaatgtcacc actgggaggc aaaacccaag tgtagatggt 36840
gcgaagctct gaaggatgaa gcaatgtggc tacttcaaca aataacacaa caaccaaggc 36900
caggggcggc aggggagctg cagacacaca cactggagag tcatgtcacc gcaactcaca 36960
ccttcccaag atcttgatct gaacaaacta ttaaaaactg tatcctccat aaaaacaaac 37020
aaacaaaaat ccttcttcca tcaagttcga aaaagttttt tataaagttc ctgggaaaat 37080
ttgaacagta gctaaatatt tgataacatt taagggatta ttattaattt taagcaggat 37140
aatactgagt ttatgtcctc tcaaacaaga ctattttttt tcagatatct tttttttttc 37200
ttttcttttc ttttctaatt tgttcaagat gtttcctctc tctggcatcc cactaccctc 37260
tatttcttct tcttcttttt tttataaatt tattttttat tggtgttcaa tttgccaaca 37320
tatagaataa tacccagtgc ttatcccgtc aagtgcccac ctcagtgccc atcacccagg 37380
catccccacc cccctcccac ctccccttcc accaccccta gttcgtttcc cagagttgac 37440
tctttacttt ttaaaaatac ataaggaaat agtcacagat tttgcttcga gggatctagg 37500
gtctgggggg gcggggcaca gttgacagct gctggcaccc actggcagaa gcactggttc 37560
attacactat tccttcaaat tctgcacgta gggatccctg ggtggcgcag cggtttggcg 37620
cctgcctttg gcccagggcg caatcctgga gacccaggat cgaatcccac gtcgggctcc 37680
aggtgcatgg agcctgcttc tccctctgcc tgtgtctctg cctctctctc tctctctgtg 37740
actatcataa ataaaaaaaa attaaaaaaa aaaattctgc acgtgtttga atattttcac 37800
catacaatat tttgtaagta atacaattta taaacaaggc tcaatgctgg atttctcaca 37860

tctttgccag acacataagc acttaccagc atcagaggag ccatctttgt cagtgagaac 37920
caggagatac aacagggaac cagagcctgt cctgcccgtc tcgtccagcc tgatcagctg 37980
caagcgtggc gttgtcacgg gtgggaagcg atagaactgg aaggtgaaat acacggtctt 38040
tggccacggt gctccctggc agtcctggga cactctgaat ccaaaaaaag ccccaaatta 38100
atcaagtatc catagcagag aacctgggtt ttcaagagcc cagcaccaca ggcaagacac 38160
tgcctcatcc acccacatga gcagctctac tgggatctcg gggagcccgt cagacagcaa 38220
cccatttcag ccccagctgg agaaccctgc tctcagagct acatgaatac caagaattgg 38280
ccaacaacac tgtccatgtc aatgacagaa gttcaggcaa agccagtttc acagcatcta 38340
gagatggctg tgtgagcacc ctaaccaaat aaaaacaaaa acacatagca ttccttaatc 38400
caggaagacc tcagcataga gagtcctgcc aactcagagc catacagagg ggtttcttca 38460
atgcaaggaa agtgaaagcc aggtaaagag taacataaat agaatattta agatcgcacc 38520
tgctaaaggc aagaaaatgc agcactatct cattgctttg tagacaatct gattcttcct 38580
tttgggggtt gaatttcacg ggatctgtag gatttacagc ctcggctggt tgcttattgg 38640
catccagaat ctcaggaaag ccgcaggctt gcaggagcac cattgaggct cgtgagagct 38700
tacttccaga gctaaaacag aaaagatcat ggtggccggc ctccagaaaa gggccggcaa 38760
agaggtagga cctgccgaca acccctctct ggggaaagcc tgttgttcag agtcagtctt 38820
cccagcacag catgccaggg gcctcagggc cacctctcac ctcctggtct gggctcccac 38880
aacaatcggg gtgtgcaccg gcgtaaaagg cagctcctgc agctgttcag caacagaggc 38940
tcccaggacc aaggacgcct ggctcagctg ggcctccagg tgagagatcc tgccctccga 39000
ggggggctcc tgctgagcaa aatgcggccc caagtgaggc caacaaggat cccagcctgt 39060
aacaactgag gaggctgaga tggggggggt gttcattgat gagccacctg tcggactgct 39120
agcttcagca aaggaaaata caggacgccc aagtgcctct tacactttag gcaaacaatg 39180
aataattatg gtataatgta agtatgtccc atgaaatatc tggaacttac actaaaaagt 39240
caccacttat ctgaaattct aatttatctg ggccaattcg tattttaggt gagtcaaaaa 39300
gcaatgaggg aggtatcatc aattttatga actggcacca ttgagagatc tgctgtctga 39360
gaaggtcaat taacataaaa accacattga tagatcagaa aaatgaaccc tccttcaacg 39420
ctgcaagaca gaccactact cagctcccag ctcgcctgta tagacatttc tctgatccca 39480
tcacctgaat attctagtca ttcctttgta ttccaccttc cacatccctc cagagcagaa 39540
gacgtgtcta aatgaagaga ctgtttggaa atcagaatgc tctctttcac accagctcac 39600
ctagctttct gtttcagctc aaaaggaata ccacaaaacc agcgataact ccgacatgtc 39660
tcagctgcct ataataagaa atgcaggctt ttcataaata cgtgagccct agacatgttc 39720
tagaataagg catctcacgg tactgttttc aagtaaataa tttaatggcg ctatttttcc 39780
tcaatagact tccagtacag gactgaagat acagtttgca ggtgagccag ccaacttttg 39840
aaaccaaact ctatgtgaat tccccaaggt cagtggaaac acaggcctgt tttccccact 39900

agatgcatcc aaatccagga cggtgggctt cactacttct ccttcagctg ctgccaacaa 39960
ggagagtgtc ctggagcgct ggaggcttgg atcagcctct atttagaact ctaacaaaac 40020
tttccttccc ccttcatgta cagagatctg aggggggtct aactttgtct tgtcaggcag 40080
agcaaaagcc taagtaagcc tcatagcagg gagagctccc acaccaaatt cccaggtctg 40140
caggacagaa aggtggcaag tacctggggc atggaagtaa gccatgccca cccactcccc 40200
ctgccaccct gtccctacac ccatgtcaca gagaacccac gaaggtctca gccacctccc 40260
caaggaccag cacagctcac aaagtcaaag actccagcag atgtcaggag ttcagagcca 40320
ttccctggaa ggaggaaggg tggccatgct tacctcctgg gccggcgggg gcttagagct 40380
gtcgggttgc cgtggagggg gtgtggctga gcgggagcgg gtggtaggct gtggggaagc 40440
tgcgagctgg gaaagggaca actagagggg gaggaagagt gggtgagagg tggaacacag 40500
cccccctaca ttccagcgcc cccacacaca cacatacact cacactggca taaacccagg 40560
agacatagca cagaaggcag tgaggtctgc aaccgcttgc tgggtggctg ctcctcgttc 40620
ctttactagt tactaagagg caagattgtg gtccctacca cgtggtaaca ctggcataaa 40680
aacaatagta acaaggaaca gcaggtagaa aagaggaagg gccgaggaag acaggttctc 40740
tgccacttat gggctgtggg ggcagctggt ctttgaactt ctctggattt cacatccagc 40800
cagggactga acaggtgcaa ggtcccttcc aggtctcacc accgctgtgc ctgggacaca 40860
atcccgtcac tgaggcagtg actggaatgg gagccgacat gagctctagg gacagctgct 40920
gagactgaat ccccacacca ctattactgt gtgactgagg gcaggccatt gagtcctttc 40980
ttttcctctg tctcctcatc tgcaaaatgg gggtgaaaat agtacctaaa tcacaggact 41040
atcacaggaa ttaaatgaga agtcgtggaa agggcttggc cagaacctgg tgcatgtaag 41100
cctcctgtca cgtcgcagat gtctggaaag tgaatgcagc tgtaaggagg gcctacgctc 41160
catggaagac ctgcctctca aatcttggac tcactttatg acatatacag ttagctggcc 41220
cagcacagta ctgggacaga ggaacccaac acgagacaga tcaaaggagt ggctgggata 41280
ggcatgtggc aatgggggac cccagcagca gccccaaggg cagagaagag ctgtggggtc 41340
tgttcagctc cattcagacc cacaagctgt tttgagactt tcctgcctca ggaagtgggc 41400
agcactgttc tgtggccctg aacacttagg gtgacatgga aactgctgta acgggccagt 41460
agggaagctt gggaggctgt ctccagacct gtgtcttcag gcaaataaaa cgttcacttc 41520
tgagcgagtc tccaagctgc ttggtacacg ttaaacccac ttccttgaga cagactttga 41580
tagtgagtta ttcgactttt ctagataagt cattttccag ggtgatgctg gcaaagtcat 41640
caggtagaca agccaactct gaccctgaac aaggtgatgc cggaaccact ggcaagcact 41700
gtgacaagct tggggttaca aaggggtgga ctcaatggcc agccaacaac cagcacctct 41760
cttcctatgc aggagaccct cgcacacaga actatctgct gtctgacagg cggtggccca 41820
tctttaccag caccagctgt tcagcctatg ggcctggctt tggccccact gcccagggca 41880
gtggtcagtg ccaggcaccc tctcctggta gagacaagac aggaggaaat gaagaccaca 41940

gggcagcctg ggtggctcag cggtttagcg ccaccttcag tccagggcgt gatcctggag 42000
acctgggatc gagtcccaca tcgggctccc tgcatggagc ctgcttctcc ctctgcctgt 42060
gtctgtcttt ctctctctct ctctctctgt ttctcatgaa taaataaaat cttttaaaaa 42120
actttaaaaa ccgaacttaa aaaaaaaaaa aaaaaaagga aatgaagacc acactgtggc 42180
acctcagaaa aaaacaaagt ctactcggcc ctgaaagatt ctcagctatc agacataact 42240
gccagagaga gcacatcatt ttttaaaagt actgaatgtg tcccagcaaa acaaaagaga 42300
caccactgtt cttaatgatg cagagaatgt tctgcccctg gggcctttaa ttaatggatt 42360
ttggaattct gaaacaggcc tctcagccca gacttacccc tggtcccacc ggcgagtcct 42420
ggagggcagc gagcccctgg ggcggcagtg ctggcaggtc tgggggggaa gcagacagag 42480
gtggggaggg gcactcagga gccttgggag gcctgactca gggcctgacc catctgcccc 42540
cggctgcgac tggtgttgcc gctggcgttg ggcagtccaa tcactctgca gtaaggtcca 42600
gagccgctcc cccaggtcac agcctcagag gacggagctg agctcgggct gctgtcactc 42660
cagttcccct acccctggcc accccacctg gcctcatact ttctgactgt gtggtgtcac 42720
caagaatggg cttcatgacc catgaagtcc tcacacaccc atcctgtgcc tttccatctt 42780
cccatctgct catactcccg actctggaga gctggggccc tgggtcagct ggcctggcac 42840
ccccactcct ctgaccaggg tggacttgct gttgtgctaa gtaagcaact ttgaagcaga 42900
tgcacatgcc gcaccctgct gttcctgcct ccacccccgc agatgcccag cgcacacaac 42960
ctgtcctgag tccagcgtac tctagatgtg ggcatgctgg gcccaggagg ggccggtggc 43020
catgcatgtc cttgaccttg gtgatgccaa ggctggctcc tgcaggctcc aaggtgccat 43080
cctcctgcca agtccagggg ccagccccat cctcacaagc caagtcaggg cgcttcccag 43140
aaactgagaa acactctggc ctgcaagcct tcacccccga aaccagctca cagcagatca 43200
tgaatccagc tgtgctccca agaaaggtgt catctcacca gaagcaaagt gtggaggagg 43260
attccagcag ccaagaacct ctccctccac ttgagagtgc tggcggcagc tcactgccca 43320
tcccaaggcg gaaagcatta cagagccact gagtcctccg tatcccaccc tgataccggg 43380
actctggaaa gccccacaga ctgactctgg tcacaaggat gaaactgcag gaaatgggaa 43440
gggaccagac cagtggctcc cccaccacca gggctaatgc tgtgcggaca ggtccccact 43500
gactgcctga gacaaaggag cagctcacat ttctgctcca gcaagattca ccacctgtct 43560
gaagacactc tgttactccc ccatcagcct caactcgctg aaaatcacag tcaacagaag 43620
ctcaatcgtc caagcctccc tcctgtcaat tcagcttcta gaagcccaaa ctgcctccac 43680
tgacacctgg gctatctgtg tcccacaggg tcacataaag acctgtgacc aggtgttttc 43740
tctgctggga gggcacacct gcagcatcac acagatggga aacatcaaca cggcccagaa 43800
agcactttgg tgaggggacc agacaggatc atagaggata taaagacaag tgcacaggtg 43860
aacagaaggc caggcagtgg tcttcctgga gaaaactccc aggctggagg gcacagaagt 43920
ccccccagaa gcactgggga gcccacagta caacttcctc cctgcccatc atcaccaacc 43980

atgggccaga cctgagtagc atggagcact gaagcccttt gtcccaaatg ccagctatct 44040
ccctaggagt gcttctctga aacatggctg tgcattctgg ggagaaactg aggcaggcct 44100
gcggaagggt gctcacaagt ctgcctaggt ctagctgcag gtcaggcctt cactggcaga 44160
agcctcatcc tgggagcaag gggtctggcg atcttagagg atgggggaac agctgcccca 44220
aaaggcagag ggaagaaggg ctcatcgttg ctcaagacag agtcactgtg acttaaagaa 44280
agatggggaa aaataagtgt cccactgggc cctgaaaata aggctttaat cactatttaa 44340
aggttcttta gacactaaga actcatatgg caaatccacc tgaagaccct catttctaac 44400
tatttggggg taaaaagcca tcctccctaa gacactgatc acaggaaatg agggacataa 44460
acctaatctg tgtagagata aagggacagg cagctcagtg acggggtggc catgggcctc 44520
gccgcagaag ttgctcagag tgaaggtggc tgaggccctc cagcacacag gatgccgggt 44580
ggcccagggc actagggggc tgtagctgga gaggggctgg acttgcacac agaagagccc 44640
aggcctgcct gcagcatcca cacctccagc tgtaccagcc ccaccacacc ggccacctgt 44700
tgctcaggca ggcagggcag gcagggacag tgaggaacat gtgtcccaag cacacctgcg 44760
tgtcccaagg tcccatcgct gcccacacca gccgcttcca gaatactgcg aagttaggcc 44820
cagaacaata tgcactctgg cacctgggaa atggcattgc cctctggacc tggggggtgc 44880
atggggctct ttggggcaag ggatgcgtgc acctgataaa gctgctgctg tacccagatg 44940
tttcctaagc agaggacaca ggagaagatg cacgtcccag gtgctgataa gggagcgtca 45000
ctacaaaggt gtcatggttc tgtccatgct gctgctggca gtactataaa acccagacat 45060
agggcagccc ctgtggcgca gcagtttagt gccgcctgca gcctggagtg tgatcctgga 45120
gacccgggat cgagtcccac atcgggctcc ctgcatggag cctgcttctc cctctggctg 45180
tgtctctgcc tctctctctc tctctctcta tgaatgaatg aatgaatgaa tgaatgaatg 45240
aatgaatgaa taaataaata aataaatcta tctttaaaaa taaaaataaa aataaaaata 45300
aaaataaaaa taaaaataaa aataaaaata aaataaaacc cagacatgca aggtgccctg 45360
cctcggcgaa ctaggcccca gaggagtggg gaatcccatg gggcacccaa agcctaaggc 45420
tggctgaagc agtgactcgt ccccatcaag gcttctgctc tgagccactc actaacatcc 45480
tacagcagag cacacacacc ctgggatccc tttaaatctg cccattcaca tggctccagc 45540
tgggcggttc aaactcccgg tcctgcatga ctgcctgggt gacacgactg ccccgggggc 45600
agggagcctt ccattggaga ggacgtcccc gcagcatcga cacgcagaag gtcacaacca 45660
tgccaagaac ctgactcagg atattccggg aaaggccatg gtgacatatt tccctcgacc 45720
cagctatttg tcaaatctac tgaagcaaac ataccaacat tctgacacca ccagaacact 45780
gaagagcaaa atatgggtca gggaaattct gagtttccaa aaaccaagac cgagtgccct 45840
ctagtggctc tgtgcactgc ttcctggagg gtgaggagtg gacctctgct ccctcattaa 45900
ttctagagag gacataaaga tcattcatga cagagagccg aagtccctca cttaacatga 45960
cagcctcatg tcatcaagat ttccaacaga accgactaag ttggtaaata tcaaaggaga 46020

aaaagtttcc atcaccaatt aaacacgaat gaacctggga tgaaggcaat cacatcaatg 46080
taacacaaat gcagcaataa aatccaaatt aaataaagca aatgcaatca ggatgggtcg 46140
gtgcagacct gctggcccca aatggcagtg gagaatggag gacgtcggta ctagatcctg 46200
atccctccag caacctgtca atgggtggaa tggcaagtct ctttgcaaag aactcaagct 46260
ctgcacaggg gagacacact ctccccccaa ccatccaggg agacacctgt cccgctggcc 46320
aatggatgga tctggagccc aaagtgagct gtctttgtgg ggaggggtcc ccttccccct 46380
gaacctccac aacatgccct ggagcctaag aatgtctggc ctcccatggg cgctgctctg 46440
tgcccagctg cccacgtgga gctggaggtg gccagcttcc aggggacagg ccagctctcc 46500
ctggtctgca tcctgatcat acagcacagg aggggcatga agtgttggta tctgcccgtt 46560
tgagcatctc aagatgcagg agcagctgcc aagcctgggt gccaccacac acagaagcag 46620
cagagtgcca gggatgacct tggacttggg agtcccgagg tcaggcactc aatccacggc 46680
tgacacatct gctagctcca tgccctgact ccacactctc tcccgagagc agcagcaatg 46740
gcctccagtt ggagaagcac cctcccagct cttaaccaca cgatgaatgg tgggggacac 46800
agcagcgtga aggggctcca tctcaagacg ttcatggaac acctgaacag gacttctctc 46860
tgctgggctt cttaagcccc agggtgacct cagagctcct ggggtcgcta tggggagaaa 46920
cccttcctgg aaggaagcta acaatgggag agctggatgg taaggggacc gggtgtggaa 46980
ggcagacaga gagacagaat ccgagtgcca ggacacagcc agcctcacat cagctctgca 47040
aacatgtgcc cagtgctttc ctggcccagg accccggtgg gcaatagaaa gaggagtaca 47100
cacagccccc attcgtgaga tgctcctagg acagtgctca gggttggaca agtgcccaca 47160
ccaaagatct gcacctcttc accatcaggg cacaaattag ggcacttgcc actagatgga 47220
gccacccggc tcactctcag ccaggagctg ggaggggtcc tgtgtgtcac ttccatgcca 47280
cagcaactga ctgtggggca ggtacctccg acatgtccct ttcctcgtgg tgctatggcc 47340
agcagaactc agactacgcc ggccatctgt gaccacgaaa gacagagttg ttaccaacct 47400
gcaggggatg tggagcaaga acaggaaata aaagcctttt gttttaaagc ggtgagatct 47460
ggggattatg tgtcaccatg gcaaactgga ctgattcatt ctgagggcaa cagaggaagg 47520
aggagacaca caacagtatg gaataaacaa caggtgtccc aagatcagag agttgtggaa 47580
gagcacacta tgcctgggag atgcctgcgc cgggctccaa acatgagcag tttactctgg 47640
gtatttcaat gcaagactat tttttctaaa cttctaaatt caaggtcaaa ggaaaataga 47700
aattgaaacc aataatttag aaaataagaa cactgtagat caatatgtca gaaatagagt 47760
caaagaagta cttggaaaga aaccacagga cctgaaatgt tttattaaac agacaaatgg 47820
agggacagtg actccatggt tgagctgcct ttggctcagg ttgggatccc agggtcctgg 47880
gatcgagtcc cgcattgagc tctccacagg gagtctgctt ctccctctgc ctatgtctct 47940
gtctttctct ctctgtgtgt gtcatgaata aataaataaa atttttttaa aaaggcaaat 48000
gaaaataagc taagcattca acttctttaa aactttaaaa aaaatgaagg gattaataaa 48060

ggcaaaagca tataaaatta aattataatt aattacatta aaattaaaga cacaattaga 48120
aacttcaaaa cagcacagga attaatgaag gtatgcaaga gccgactccc tacagaagat 48180
aaaaggtaaa cacctctgat gaatctgccc aagagaagga gacactcacc aggagaccag 48240
gaatggcaaa ggaatccaca cagagataga aattaggata tttacattac atacaagttt 48300
atcccaggat ccattcatct atagactata tatatcccca aagttaacct gagctgagat 48360
agaaaaccta aatagagcca taaccataat aaaaacttga aggtagggga tccctgggtg 48420
gctcagctat ttggcacctg ccttcagccc agggtgtgat cctggagccc cgggatcaag 48480
tcccacgtcg ggctccctac atggagcctg cttctccctc tgcctgtgtc tctgcttctc 48540
tctctctctc tctctctctc tctctctcga tctctcatga ataaataaaa aaccttaaaa 48600
aaaagaaact cgaaggtggt gaaaacgagc taaggaaacc ccactgagct gggagaagga 48660
gtggagggtc agcaggtgcc atcccccacc gggtcccatc tgggtaccag cagggaccca 48720
gaaccaatga gaagccgcca catggagttc tcttttcctc acgaactcct gttcaaaacc 48780
atcccttcca gcctcctcct tccctctatc ccctctatta gtggaccctt gctctctggt 48840
ctctcaactg gcctattggt tcaccatggt ctgcacatct cctttttttt ttttaatttt 48900
tatttattta tgatagtcac agagagagag agagagagag agaggcagag acataggcag 48960
agggagaagc aggctccatg caccgggagc ctgacgtggg atttgatcct gggtctccag 49020
gatcacgccc tgggccaaag gcaggcgcta aaccactgcg ccacccaggg atccctccac 49080
atctcctttt gtaattcctc tgccgttctg gaataaacca agttgctact caataacttc 49140
gttgttttca aggttggcat gatcagaaaa tccttcccaa aggcactgag ttcctgatgg 49200
ttttatagac aagttttacc aaaccttcaa gaaacaagtc ttgccttttg ttgaaattat 49260
tttaaagccc agaaaaagag agaaagcatc ctaactcact gtgatgatta atttttttta 49320
agattttatt tatttattca tgagaaacac agggagagag gcagagacac aggcagaggg 49380
agaagcaggc tccatgcagg cagcccaacg tgggactcga tcccaggtct ccaggatcag 49440
gccctgggct gaaggcggca ctaaaccact gatgattaat tttatgtgtc aggtagacta 49500
gatgaatcac aggggcccag acaggtggcc aaccatcatt ctgagcgtgt ctgtgaagat 49560
gattccagat gaaattccta tttgaatact ccccctgagt gacgcgctct gcccccccca 49620
accctgcccc cgtgtgggtg ggcatcatct catgtgttga gggcttgagg aacaccagag 49680
gggaggaagg aagaactggg cgttcctgcc tgtcaatggg ggatgcacat ctccttccct 49740
ccatgctcct gctccgcagg ccttcagacc cagactggaa cctgcatggt ggctttcccc 49800
cactctcagg cctctggatt tggactaatt ccccagcagc tttcctgggc ctcttctcag 49860
cctccactga tggtgtgagc caatttctta taataaatct cttcctacat ggatggccca 49920
ctggttctgt ttctctagag agccctagct acgcactcat tttgcaaaac gagcactcca 49980
aataccaaag cctgataaag acatgacaaa acagaaagct atagatgagc gatctgcagg 50040
tgatggccca ctcatcaaat tccacccaca acctgtgttt gtgagtaagc ttttattgta 50100

acacagccaa gctcattcat atggtttcca ctacacagca ctggagatga ggagctacaa 50160
cagggcatcc ggcccacaaa gcctaaaata tttactatct ggccttaaag tcagctgacc 50220
tctgccacag atgcagctca attatggaga tgagacagaa atcctgaaca caggttaatc 50280
aaatcaagac agacatgatt ataaagcttc ctaaaagtca ttccctctgt ttcttttctt 50340
aaacagactt tatttttagg gcagttttat tatattcata gcaatattga acagaaagta 50400
cagagaattc ccatgtactc cctgccccag atatgcatgg cttccccagt atctacatcc 50460
cgcaccaggg tgctacacta ttctttttgc tacctaaaat atttggtagt tttttattgt 50520
ctctttttaa aatcaatctt ttaggtgcct gggtggctca gtcagttaag catctatctg 50580
ccttccactc gggtgatggt cccagggtcc tgggatcaaa tcccacatca ggctccctgc 50640
tcaggggaga gtgtgcttct ccctctgctc gctgctcccc atgctggtgg gtatacatgc 50700
tctctctctc tctctctcta ataaataaaa tcaaaattaa ctaataaatt aaattaaagc 50760
ataagctctg tgagaacagg agcctaaaaa gtacctggca catcacagag tcccagtaag 50820
gatttgcaga atggatgagg gagggaggag agaaagagag ccactaagca aaccctatct 50880
tagtaagtgt tgccttaatt tctggctaat gtaaaatgat agaaacaaac aaaaataatg 50940
gtagaaacag agttaacact gaatgtgcgg ggcccaatga ggacccactt ccctggatgg 51000
tgtcatgggc actggtatgt gtccactgaa gactctgggg tcaaactctc catggagatg 51060
tgctgcattt gcacctgccc tggggaccat cttgtttcac tgggccaacg gctaaaaggg 51120
gaatgagcag gtggcactgc ccagcgatgg tgccagccag agatgacagc ggcagccgtg 51180
aagcgtgccc agtgccaggc catcctgagc gcatccacag atgtttgccc actcaatcct 51240
ccctacaacc tctggtggac catgtctgtg tttctttcca gttgtttcct tcttactgat 51300
ctgcaggagc tcttcctata atattgcttc tttctgatat tctaattcct tcaattaact 51360
tctcatggtt gtaggcactg taactgtctt tcaatttaag gtttgtctat cgacttgttt 51420
gtggagtttt ctctcttaca aaagttttat attttaatat aatcctattt cttagtcttc 51480
tttgtttatg cattgtctag cttgtttaag aaagctttcc taggtggctc agtcagctga 51540
gtgtctgact tttggtttca actcaggtca tgatctcggg gttgagatcc agtcccaagc 51600
agggctcagt gctcagtggg gagtctgctt gtctctctcc ctctgccctc cctggatcat 51660
gcaaatgtgc tctctcactc tctctctcta aaataaataa ataaaatatt taaaaagaag 51720
aaagaaagaa agaaagaaag aaagaaagaa agaaagaaag aaagaaagaa agaaagaaag 51780
gaaagaaaga aaaagaaaag aaagctttcc taaccccaaa gtcacaaaaa tatcctctaa 51840
ttttttttcg aaggcttttc aagttttatt ctttcctatt cagttctttg ttgcatccca 51900
ttttacagct gaaaaaattg aggaagagag acagtcagca aggggtccgg ggtcatccaa 51960
ttagcacaca tgcaccagtc aatgacacat gcaactgttc tcaaagttac tgaaagccgc 52020
cacgttgtac acttccttcc agaacttgtc ctcactcctc acatggtctt gaagattcac 52080
ccctgaggac atgtaaagac cttcttggtt cattctgact gctatacggc atctggtcat 52140

acaaacatac cagctcgttt atccaccccc agggggagga gtgagcaggc caccatgtac 52200
ccaggaacca gctgctctca acaacaggtg tctagaaaca gcacttctga gtagcagggt 52260
atgaactaaa tattatgaaa ttgctcttca aagcagctgt ccatatagcg ttcctgcttg 52320
cgtggtaaga cccttcttcc ccatgagcat cctcatgaac cttgtatatg aggcagctac 52380
atttctccag ttgtgaagta tgagatgatt cctcatttta ttgtgcattt ctctgatcac 52440
gactgatact gagccctgtc tcatgcttac gggcccctct ggcttcttcc atagaaatcc 52500
cctattcatg tgttttgccc atttttcttt cctgttaccg ttatttctta ttgatttgga 52560
acagctattt ctgtaacctt tcttctttct aatcttctaa ttctagagtt ttctaaaatt 52620
tctaaagttt tatttcataa tccacctaca atttattttt gcacaagata tgaaattagg 52680
atctaatttt atatgttcct tatggataag cattcctgca ctgttaagtg actggtccat 52740
ccctattcct ggtggtaagc agaacgatgt ttcctacccc agaaataccc acatcctaac 52800
ccccagagcc tggcaatgtg atgttgcact gccggtaatc atggctgctg atcggagaga 52860
ccatcctaga tgtttgggtg ggcccaatgc tatcacagca gtcatagcgg tcctggtaag 52920
tgccagggag ccaggaggat cagagtcaga gggagatttg gaaactgcac tgctgacttc 52980
gaagagaaga gaatgagcca aaggctacta gaggctggaa aggcaaggag gtggagcctg 53040
ccctggagtc tccggaagga accagcccca ccaaaacctc taccttagcc tgtgagaccc 53100
cttttggact tctgacctcc agaactgtgg gacagcaagc tcatgctatt ttaagccatt 53160
ttacatttat ggtaatttgt tacaacaata agagtaaagt aatacactta tctataatgt 53220
gtccagtcat cacactctgt cttcttatct gtccatttct gccccgatgc cacactgttc 53280
tagttactat aactataaat ctgccaatta ggaaaactga agcctctttt ctacttcaaa 53340
attgccttgg ctattcttga ccatgtaatt ttttaattaa aaaaaaaaaa gaatctattt 53400
caaagtaaaa taaatgcagt gcacgaggga ctacactgca gcatagagca tgagcccaac 53460
tcagaagtca tgaaaacaca caacatagct ggtgaatcac agtagcacag agcagccagg 53520
aaaactttca aaaccactag accatcaggc ctgggtggag gatctcagca gcccaccgac 53580
atcaggcaaa gacatctgtc tcccaccaac gtagagtaag caaataggga ccagactgac 53640
cgccccatga aacaaatttt tttaaaggga caaaatatat gaaacaactg gcctcaagac 53700
attagaattc aggcaacaaa ggacagtgat cctcagggac aggaaacaaa agaagtgggc 53760
cctgaaaccg ccccaactct cagcagctaa tgagtctgtg ggccacagta cagggagacg 53820
gggcaccagg ggcccccagt ggagaaaaca gagctgaatg tccaagaaga ccgaggaagc 53880
tcccatttgc agggccaagt gccagacaag acaaggtgca cagaaagaga gctctggtca 53940
tttacagagg ctcccctcta gaattcagta ggtgactggt caggaaagta cccaatgctg 54000
gggccagagc cattggaaca gtccccagtg cttacatggg gcagaaatag acttactgca 54060
cagacaggcc aaactggcaa acctcataat tcaggagatg tcaggcagag cacacaaagg 54120
ggtcttgtcg tggggaataa ttagccctgg actaaaagct ggtctggtcc cacctaacaa 54180

cccttaaggg caagagccca aaggatcaaa ctgtttccaa agctcaagag tattcatcaa 54240
atacaaacct atttagcact ctcaatcaaa gaaaattcac aatatctgca atccaataaa 54300
aaatactgat ggcagataaa caggaaaata caacccaaat taggataaaa tgactcaatc 54360
aaaactgagc cagaattggg gcacctggtt ggctcagtta agtgtctgac tcttgatttt 54420
ggttcaggtc atgatctcag ggtcctggga ttgagttcca agtcgggctc cctgctcagc 54480
ggagggtctg cttctctctc tctccctctg cccaccgcct acttatgggt gcactgcaca 54540
ctcctattct ctccctctca aataaataaa atctttttaa aataacttaa aaataaaata 54600
aaaataaaaa taaattaaca taattctggg aagcagagca agcagcagac tgggtttaaa 54660
gcccagatct gctcacaaca gtcaaggcaa ttgggaaaat gagacccaag cctgcagacc 54720
gcagagccac agcgggtaca gccggtgttc gccccaagca gccaagtgat aaggcaattc 54780
catcattgtt tagaattaat ttgattagaa ggtcttcaaa caaaccagct gcttgcttcc 54840
agcaccacag cggcaccata aactaaccct tgaaaactcc tttccaggaa aagtcagtat 54900
atcttcccca taaaagctac ctgtacaggc atgtccccca aggacatgat ctattctggc 54960
atttccacag acacaggtga actacacata ctacatttca ctagtgtcct ttttgagtag 55020
cttaatccaa aatatttaat cataacattt acctattttt gtaatctgga atactatctt 55080
tcatatttac ctttctcaga gcttactatt tgttgtattt ttatttttat ttttttaaag 55140
attttattta tttatttatt tatttcagaa agagagagag caaaagcagg gtgaggggca 55200
gggggagaag cagacgcccc actgagcaga aagcccaatg aggggctcaa tcccaggacc 55260
tgggattatg ctctgactca aaggcagatg cttaaccgac tgagctgcct aggtacccct 55320
tgttttattt tttaaatgta actaagaata tatgtgaagg aagaagagac gctagattta 55380
caggagagaa tgagtgggaa ataaattgta ccctttgttt agtattttta agcgatatgt 55440
tctatgcctt caatgcattc gactttatta agtaaaactg gaaacactct aaacaaaccc 55500
tgagagaaaa tccatgtctt ccgcccacca ctgcagggac tccatcagat atcctggcaa 55560
cagcctcggg gctgtggtgc tggacgtttt cagggctaca ctggtaagag tggttattag 55620
cggacacact ggtggtaaac agcaatccca agagcaatgt gacaagcaca atattcaaaa 55680
attgaaagat caaaacaact aaaaccatca aatgcactgc ctttttgaga agaaggaaaa 55740
tactcaatga tgtggtttgt cccaaagcta cggattgttt ggcacctgaa tatgttccga 55800
tacctcagaa aaccttggaa gatcatgaag acagtggcca gcatgtccag gtccaggcca 55860
aagaactaag gggactgctt ccaccaacag taaattaaat tcaacaatac caccttccat 55920
taactagatt ctcaactcag aaaaaatcag tcccaaatga gtcccagacc cactagtgcc 55980
tccgctccag cagccaggac ctcagtaagt cctccctggg gacagagaag cacccatggg 56040
gaagatggat aggattaagc tccccactgt gggcttatac attagttcag tccctacctt 56100
tctaactttc taagagacta acttcctata tctagaacat tcttccagct ggagcagtct 56160
ctagaagcct caggtgcctg aggctcagtg ccctgccaag tgaacttcac caacacccct 56220

ctgacatcag ccctgcaggg acctgaccca tgggcttctc agtcagatgc tggcacaggg 56280
gtccttaggg gtccttatgg tccttagggg tggtgaggca gaagccaaga ggccctgacc 56340
agcaggtgct aggccagtca gacacaaggc tgacagcaac gttcgctgct ccgccacctg 56400
cacacatgca gacacatcac tgcccccagg aagatgttgc tctgagggta gccctcactc 56460
tccccaaagt cgccagctga gatcagacac cccattcttg gaatgcttca agggagtaaa 56520
aagcaccttt tctaaacaac aaatgtacag cacgttaaca acaacaccca gagatggcag 56580
cccatggctc tcggtcaaac tcttactctc aacgctgcgt ctataggact cccatgccaa 56640
gtgcccatta ggtgtcactg gcagagcctt ccacaagcct ctagtcaggg ggctgcaaga 56700
cacaggtgac aacacacttg gccaccctct gtcactgtcc aattctacat ccccgtatta 56760
agcaaaagct caaccaactg actccaatta aagcaatctg tagcaattat ctgataattg 56820
ttattgtact ggactaagta tttttcctta agaacagatg gaagtcatgc tatgtgctca 56880
aaaagtcagt tctctgcaaa cacttacttg caggagatgt gggtggcttc ctggagggct 56940
gccgctccgc ctcagggctg tggaccagcc ccgtgggtgt gtccaggtgt ccctcagggc 57000
tcagcgagaa gtggaactgg acagtccctg actccacttg ctgcacctac aatcaaaaca 57060
agaacaatgg cagctgcctc ctttcatcgg tgcccttcta ttacaatgca accagcccca 57120
aagtctacat aaaaccaggg attcctgaag ccccgtgttc tcagatcata atcaagtcac 57180
tttctgagca gaaacaatca gccgtggcat tgaaatccca gaagaccaca ttagcacaga 57240
atggctctta ctcaaggaga aattacattt ccccagtttt gcacgttttt aaacaaacct 57300
atgcaaaact gaccttttaa atgttggtat gtagttctgt tcaacacatg tactcactac 57360
cggagttggg gtgcagcgtg gctccatcac ccaaacaatc cctcgtgttg ccccagtctg 57420
actcccccaa ccccctgttt ctgagctgct cctgtcatgg cagcctcggc tctccagaat 57480
gccacagaca cggaacccaa ggatatataa ttaagcctag cttcttacac tccacataat 57540
gctgatgaga ctcatcccag taagtagtac gtatcaatag ttggttcctt ttcctgctga 57600
gcagtattct atggtaccat gtgcatggtt tattcatcca tttaagcaca tttaggctcc 57660
tttcagtttg ggtggttaca aacaccgggg tacatgtgtc catgtgaacc taagtcttca 57720
tatctctagg aacaggattg ctgggtcacg tggtacgtgt atatttaatc ctataggaca 57780
ccatggttta cttctagagt actctgctcc caatttatat gccaatcccg cagcaacagc 57840
accttacagt ggttgtcgca gctctactcc aagtgtcaca ctgtggggaa ggattcctcc 57900
aacatcactg ctatcgtcac agttctttgg actcttctaa ttcctgggac tttctgtata 57960
aattttagga tcagtttctc tacatgtaga ccaaaaaaaa aaaaaatcct actggaattt 58020
tgaataaaat tacattatct gtacatgaat taggggagaa tttctatctt caacacactg 58080
aatattccca tgcataaaga tgctgtgtct ctccatttac ttagagtcct ctgtgatctc 58140
acccaagagt aaccacacga gaactgtgac atgcaccaaa gggaatatgc tacagttcac 58200
aacagacaga tcctgtacac actgtgttag atttatctta ctgtgggaag gccttgataa 58260

gtaatactgt ttttttttta ttttgttcat caactgccct ttcttaatat acagaaaaga 58320
ttgatttttc tatcttgatc tcctattgtg tcttgcaacc tcaataaagt cactaattca 58380
ttctagcagg tttttgtaga tcccttggga ttttccacac aatcatgctg cctgcaagta 58440
agtacagttc tgtttctctc tttccaatct atgtgtcttt tattttgtct tcttgcctta 58500
acgccctggc taggactttg agtatgatgc tgaacactga gtgcacatcc ctcacttatt 58560
cctgatcttg gggcgggggg gcacccaaac tcttaccact ccataggctg ctcactgatg 58620
ggtttgtgtg gatgcccccc aagcaggaag agggactgct cttctactgt tagtatgctc 58680
acagttttta tcataagtca gtgttaaata aatcttatca aatgcctttc atgtgtcata 58740
cagttcatct cccttaggct attactatgg tgaattatat ttattgattt cttgcatttc 58800
tgggataaac ccccacttgg tcatggccta gtcttccttc cacattgttg ggttctattt 58860
gctagcattt tgttggttgt tttgcaactg tgtccatgaa ggacacatgt ccatggtctt 58920
cttatgctgt ctctggtttt attatcaggg cagcacaagc ctcattacat gaattaggag 58980
atgttcccat ccttctattt cctgggggag accgtattaa actactcttc tgtctcttcc 59040
ttaagtcatt ctgtagaatt cacagtgaat aggcctggat gttctcaaca gtgttttagt 59100
catgaattca gtttctataa tggtcatagg actactcagg ttgtctacat catcctgagt 59160
gagttttgat catttctgat ttaagataaa ctgatccatt tcatcgaaat tgccaagttt 59220
acaggcacag aattgctcac agtgttcctt tgctgtctgt ttgatgcctg tggggccagt 59280
agtgctggtc cttcttccat tcctaacatc agcagtttga gtcctcctct cctgttgtcg 59340
ttcttgctag acatttatca attttattga tctttccaaa gcctcagttt ttggtttcat 59400
tgatttcctc tgttttcgtt ctctatttgt tctgttctgt ttcttctgct ggatttaggg 59460
ttattttgtt cttctttttt ttaattttat ttatttattc atgagagaca caaagagaga 59520
gaggcagaga cacaggcagg gggagaagcg ggctccatgc gggcagcccg atgtgggact 59580
tgatcccagg actccaggat catgccctga gctgaaggca gacccttaac tgctgagcca 59640
cccaggcgtc cctgtttctc tttctcaagc tcctaaaagt agaagataag attactgaat 59700
gaagaccatt cttctgttcc aacacaagcc cttaatgcta tcaatttccc tggaagcaca 59760
tgtcaagtgc atcctccacc ttttgatgta ttttcatttt tgttcagtcc aaaacatcct 59820
ctatttcctc ctgagtcttc ctctttgatc tatgggttat ttagaaatgt attatttact 59880
tttcaaatgc ctatactttt taatcttatc tttgctaccg acttctggtt aaattctgtt 59940
atgaccgaaa aatgtaccct atataattcc aattctttag aaatcatgaa gatttgtttt 60000
atggcccagg atgtggtctg gctgagtgtt ccctgtgccc tagaaaagac tatgtgttct 60060
gctgctgttg ggtgcagagt tctgtagacg tcatgttgat ccagacggtt catgatgtcc 60120
tttagctgtt tcatgtcctc agtgattttc tctatgttcc ctggtttact tagagcgatg 60180
ttaaagtctc caactataac tatgagtttg tctatttgtc ctttaacttc tagtaattag 60240
tttttttgct ttatgtattt tgaagctctg cctatactgg atgcacacac ctttaggact 60300

gctatgtctt cttggttaat tgactttttt atcatatgtc atatccttct taattcctga 60360
aaatttcctt tgctccaaag tccactctgt ctggtgttga tacagccact cctgttttct 60420
tttggttttt gttttcgttt ttttttaaag atttatttat tcatgagaga cacacacaca 60480
gagagagagg cagagacaca ggcagaggga gaagcagact ccatgcagga agcccgatgt 60540
gggactcaat cccggggctc caggatcaca ccctgggtca aaggcagcgc taaaccgctg 60600
agccacccgg gctgccttcc tgttttcttt tgattggtgt ttacattgaa tgttttggtg 60660
tctgttgagt gtcttttctc tgaaagttga gattttcctg cctctttgca tctcaattat 60720
gtttagatta tatcacagac attttgaata ctatgttagg agactgtgag tctcatttaa 60780
attctgtgga gaatggtgat atttttgttt cgtcacgaat cagctcaatg ggttcaggcc 60840
acagtttcca accagccttc tatgcattgt cagcccctgt gccatgcttt tcagatccct 60900
cctgcatgtg atccacccag ggaaactctg gaacggggct ggaggcctag ccacagtcag 60960
ttctcagggt ctttggaact gtagttcagg gtcagactca ggcagcctga gggttaaccc 61020
agcatttctt aatgttatgg gattgctttc caagcctctc cctctccata gcctccccaa 61080
acctgtctgg gactttacaa tcctccagcc agaccccact gcgctgcaca cttctgggaa 61140
caaacagtcc ctgaagccaa gacccctcag ggggaggaca gagtagtttc ttaaaagagc 61200
tcaggagtgt atcaagcaag ttctcattta tcattattgg aagtcaatat ataatgtcta 61260
aagttgataa acctaagaaa tagcagaagc ataatatttg gaaacaaagt aaatatcata 61320
agtcagagca aagagttgaa agtggctgcc tctggagata gaaatttttt ttaaaagaca 61380
attatttgct caaaggaaaa atacaggagt taagaaagaa aacataatca taatgtacta 61440
cttagcttca cagtaacatc aaaataataa cagaaacccc cagatttacg tagccaaaaa 61500
ttatgggatg tttctaatat ttatactttt ctctgattag tatctgccaa ttccccttaa 61560
ctgtaagttc acaggatcgg ggatcttttt ccaagggtat aagtgctcag taaaagttca 61620
ctgaatgggt aagtaagaca cagaatgacc tggcataggg gcagagacaa ggtgcacctc 61680
actcaggggc atctctgccc ctaagcctcc acactacctg cccagacatg gcactgctgt 61740
agaagacaca atgggaagaa gcagaggagg aagcagaatt aagtttatgg ccagttttag 61800
ttcattattt taattagtta tgtattttag aatagtgatc ctcgactaga ggcaattttg 61860
cccttgagag acatttggca acgtctgcag acaactcagt ctatcatagt tgggggcacg 61920
ctactggcat ccagcagaca gaggccaggg ctggtaacga acatcctcca atgcacagga 61980
caacccccac agcaaagaat tatctggccc aaaatgtcag cagtgctgag ggtgagataa 62040
cccaattaga agtgatattc cttgtgaata atggcagaat ttgatatatt tgtgtcctca 62100
agatagtcaa gccttccata ctcatcccca atgcagttta cacactcagg gagtgaagga 62160
gatgtagtgg catgacaagg ctaaagttaa atagaataga tatatattta aatacataca 62220
catatacatc cagagaggga aagacagagg caatggctcc aggatctctc acggcagttt 62280
tataataagg gctcagacta gtccatgggg gaaggcagca aaatgtgctc ccataatgag 62340

atatccttat aagaagggaa gctgcacaat cagctgttcc tgatccaggt ccctaaagca 62400
tcacccacat cataaataac attgaaaata acatttgtgg cgagagtcag cacacggatg 62460
atgcatggaa taccagggat ggtcctttca cctgagaaaa atgagacaac tgagctgtag 62520
acggctccca gccacactgc tgaaacagca gagtgtggta caggagtgca actgggagaa 62580
ccaagcaccc ttgcgtgaca ctggcgtcag gatgggccag ccagagcagt gctttcctgc 62640
gcggggtacc tgcagctccc tggcaggtga cccctcactc acacttcaac tggccctgcc 62700
accgtttacc ggtcttctcc accggggttt tggagatagc ttcattccaa acatggattt 62760
taaaagctcc tgctgtggcc cgccctccat gcaaagctgg aaggagccaa gggccagtgg 62820
gcaagagtaa caagtcacag agatgcaccg ggctcaacac caactgccag gcactgacca 62880
caaccctggt accaccaaag ctcattctcc atagcaaaag gttccaagag tcctttttga 62940
aaagacacat atagcacaga agcagatgct gtttgcacag tgcatacaaa taaaagccac 63000
aggccccaca ggccaccttg ttgagactta tgtgtgaatg gggatgtcct aagccccgag 63060
ataaaacctg cctcagtgag ttcacagcca actatgactc agcaatcatg cttgtccttt 63120
aagggcacgt ccatgacatg aaaaaaattc tgttcatcac atatccacaa acatatctgt 63180
caacttctgg ttttgaaaat gtgtgaactt gctggaagct cacaggcaaa gattcatact 63240
tataaagtat ctgaacaact tacctcttca gagctcatgc tggcaggggt tactttatag 63300
accaaacagt gtgaggggtt gggccggatc ccaccctgca gaggtaagat caccttccca 63360
gagccggatt ccagcaaggg gttccaaaca gcccagcgaa ctatgtgcat acacgccaca 63420
ctggacaaag aggtgaccga ggctgcctaa gaagggaaat gaggcacatt ggtacatgac 63480
ttaccaccaa acccaccgtg acttaccacc aaaccaaccc tgcaaacgtc actagggagc 63540
agtgcagcca agcagggatc acacagaagg aaccagacca ggaggagggc aacatggaag 63600
cttggggcag gggcagggga agcagcatcc aacctaacca gtaccctttc ctatactgat 63660
ctttcttctg gctcaaggaa gtagcagctt ttcttttaaa gaccaacacc atccttctga 63720
tccctcctgt tcccccgagg ctcttgtgca gatctataag catgctgtat cttgactaca 63780
tctgcccaca tccattagga aaatactagg aatgacactt ggtgttagtg acaggaaact 63840
ggaaaataat gaagaatgcc catggaagtt caatttggta ttatttgcac acattttctc 63900
caaccacctt ttgctcataa tgaatggtac atttcatata cgtgactctc catggatctg 63960
ggaagttggc caggtccaat tttctgcacc gtcccttctt ccgccttctc tatcactgac 64020
caagtgaaag caatgaggac actaaggaaa ccggtttcag ggctcaactc agcagtttta 64080
atggcaccac cttcatccat tcacccagaa agaattaaga ataaagtcac actccaggcc 64140
tggaacccgt gctcagctgg gcagtgagac ttgcgattgc cgccttgggg gaggcaacat 64200
cagagacccc agctctgact gacagatgcc gcaggttcct tgggaggcag cacgtcagga 64260
ccccaggaag aggccatggg gcacttggca agagtccagg caggtgtttt ccagcttcag 64320
agcacagctg tcctgaccag agtcacattg aaactatcca gcccaaggag gcagcagcca 64380

cacctgtcac cctgactccc acctcagtgg tgatgtggga ggaggaaaac ctgaaggtgt 64440
aggcagtact tggagcatgc acacagcatt ccacctctta atgccctctt cacaggtggc 64500
agacagctgt ctgtgcccct ctgggatgag gacagcaaag gaactgggtg actccgtgtg 64560
gtatccaggt ggctgaggga ccaaagccct ctgatgctct aaaccaccag ccacatctgt 64620
gaacccagac agccctctca atgggggcca caggctaggg aggctgggca gcctacaagg 64680
agggggcctc gagctcttag aaaggttgga agcccatacc tcatctgatg gtaaaggaca 64740
tcatccccaa ggagaagaga gctgtatcag aagccacaga gtaaggcaag actccagcag 64800
agctgctaag gctcccaaca gaccccgctc acttgctggg cgtgggtgag ctgccgtgcc 64860
attgtggcac ggacttgtgg catgggcagg actgactgag cttgagtcca aggcagactg 64920
tggcacacat acacaaaagg actctggcca gaacccaatg cagatggttc aatggtcagc 64980
caggccacac cccagccgag catatccatg agaagcatcc aacatggaaa aggaagcaca 65040
ttcatctcac ccaagtcaga ctatccagac tgttccttag tcaggtagtc tcccagcctg 65100
ctgtatccct agtcacatgg ggataaatgt ccagagtcag tgtgtggtct tgaattaatc 65160
cctttcctga agccccattg caagtggccc aacctcactt cagcagggcc tgttctcatt 65220
ctttactgga gcccaacttc agatcatagg ctaggctccc atcacatatg cctgggtcac 65280
aggggtaacc aggtccctgc cagttcaatg acaaaaaaca ccaatcctga aagtacaaag 65340
aaccagcaaa gtaagaagcc taagcatatg tctctgccac acttcaaacg tgtcaaatac 65400
gtagccaatg cctgccgctc ttcatccggt tagctgatga cagtgacata aaatggtact 65460
gagcaactgt gcaaagttga ctataaactc actgctcttt aaaaacaaaa ttactataaa 65520
tgtattggca aaactggaca ctaaaaaacc atgtgattcg accatttctg gactactttt 65580
ataaacagcc atctcctata aaatccagtt tattactgga tcaaaaataa aatggcacac 65640
ctgggtggct cagcggttag gcatttgcct tccagctcag ggcatgatcc tggaatccca 65700
ggatcaagtc ccacatcggg ctccctgcat ggagcctgct tctctctctg cctttctctc 65760
tctgtgtctc taataaataa ataaaatctt tttaaaattt tttaaattta aaaaaaataa 65820
taagataaag tatgtatcca agcataacac atgctattcg tgcaaatctt ctggtttagt 65880
ctttcaaatt aggcgattct gatatgcagt tttccaattt cacagtccct ctggtttcct 65940
agttctcaat cgataatggc tctcccctac gcggagtacc tgtcagttat acacataccc 66000
atttcccctc actttcgtct ctaacagcta caacaagaag agatggctag cgagtcactg 66060
atcactcctt tgcaaagatt tctgtgacaa ttaggacttg ttggcaattt atcagcctgt 66120
gccctcttct gccaggctgg ggtgacacct gcaggtgaga acctcctcaa tctcacacac 66180
ctcgaatccc cccagctagg tccctggctc tgagcaggtc aggtgcatcc aagcaggtgg 66240
gcagaagaaa agcaactatt taactggtca acaaaacaag gctcttcctg tcagtgaatc 66300
tagtggttga gataaaatca gttgctttct aacatcttat ccgcccaatt gggtttggct 66360
tcaagaaaag aaatacaaac tgaaaagaga aaaaaaatat tgacttgttc cccatttaga 66420

cttttattta taacaccaca taatatacaa gaggccaaaa gagtacacct gggttatata 66480
taaaaagcca ggttcttcag gaacagaaga actgcaccaa cagtcaccag gcaaagccca 66540
aacagacaag ggaccctgac ctctgggaat gctgtaaacc aggttcccta gggtgggtgc 66600
catggtggct tcaagaaggc aagtcctcag cctaatccct ggaccctttg tgacctgatt 66660
tggacaaagg gtccacgtaa acgtaattaa gtattatgag attaggagac ctagattatc 66720
caggtaggcc tcaaatccaa cgacaagtgt ccttagaaag caggatagag atttgagaca 66780
gagggaaggc cctgtgaatg agaaggcaga agctgaagca acatggccat gaacagaggg 66840
aggacagcag gcgccagaac ctgttagagg caggagggac cctcactgga gcctctggag 66900
ggaaccccac cctgtaggcc cactgctttc agcccccatt tgcagctgat tgtcaatctg 66960
tggcctccag agctgagaga ataaatgtgg attgctttaa gacacatcca gttcatatca 67020
atgaacagca gacactcatg caccacgacg aggcaccgaa accacagtgg catgcgggat 67080
ctccccagta agtgaccctg ggcggcagga tggcaccaac gcgctgagca taaaggtgaa 67140
agtggacaca atgaacccct gcaggaacag gatgggctct cctaggacac ctcaaatcga 67200
gacataacag acatcaaggc aggcagatct gcctacagga aaaatggtgc tgaggtggcc 67260
aagacaccct gggccaaggc ccatgggtga aaaccctgag ttccagtggg acaccacttg 67320
cggtggggtg gggggtgcct ctgggaggtg acacaaatcc gatgaaagtg ttggggggtg 67380
tttttagtgt ccttgcaaga aggggaggag gggagagaga cctcccgcct tccacacaca 67440
cacaccaaca cactcctaca cacccctaca cacacacacc agggcagcag ctgcaaggtg 67500
gggccccact gggaacccat ctgcggctcc agcacctgga gggataggag tctgacgttc 67560
acatggcatt tccttcggct gccacagcag ataaagacaa ggccaaacac aggggtgtgg 67620
gtcaaaggca gaccgcagag caggaagagg tgtcacacac tgtcccgagg gagaacaggt 67680
accctcactc acaaaaatca ccacagaaaa acgggttaaa gaagtgacca gacagacaac 67740
ccacaacccg actgcacatt ccacttcacg aactgtccct caccatgagg ccatttctcc 67800
cctccaagta gcagaaccca aagtatgaga ggccactcct gcaaaggatc atgctcttgc 67860
ccctggaccc agcatctcct tggggcgcac gcgcacagcc ctcaatccag cacacccttg 67920
gggcacacac ccctcgatcc cgcatgccct caggcgcatg ctcacacccc tcaatctagc 67980
atgccctcaa ggtgcatgcc tacacccctt gatccagctc accctcgggg cacatgctca 68040
cacccctcaa tcccgcacac cctcagggta caggctcaca ctcctcgatg tggcatgccc 68100
tccgcgtgtg tgcctacacc cctcgatcca ggacctcctc agggcgcaca gccatacccc 68160
tggacccagc acccctcaga gtgcatgctc acacctctca atccagcaca cccttggggt 68220
gcatgctcac accccttgat cccacatgcc ctcaaggcac atgctcacac ccctcaatct 68280
ggcaccccct cagggcacat gctcacaccc cctcaatctg gcacctcctg ggggcgcacc 68340
ctggagacac cccagagcac aaggtgagcc aacacacaga cagttccacc cacacctggt 68400
ccacctatag gccctgcaaa ttgctgagct atccaaccac aggaaatgca tgcacaagct 68460

ggggggcaca agcagagtac atgccgttgg aaaaggagga gacacgctct gtggccagcc 68520
ccgcctgatg gccagggcac cccaagagtc acccacgcac atggccactt ccctccctca 68580
ccccaagaag cagcccagga gggatgcact gggagcaaca gcagcggctg gctccagaat 68640
gaacagtgga agggacacag aggagcaatg cccttctgaa accagctttg ggtagaattt 68700
ttactgaaaa gcctatcagt gtcctacatg ttccggaagt gaaatgaaat cactaggatg 68760
ggagggaaat aactctgaac tagagcacag ataaaaacag gatggtgaaa gactcctaac 68820
tctgggaaac aaactagggg tggtggaagg ggaggtgggc gaggggtggg ggtgactggg 68880
tgacgggcac tgaggtgggc acttgacggg ctgagcactg ggtgttattc tatatgttgg 68940
caaattgaac accaataaaa aataaattta taataaaaac aggatggccc actgtgaccg 69000
agtgagcagt gaggctacac cattcgggcc aggcctaggg gctctggaca ctcttcccct 69060
ccatctgggt ggccagagcc ctgaacggct gggagggcca tctggcagca gcagtgtggg 69120
tgaggcaacc ctgcgctgag gctgatggtg ccctgggctt ctccctgggg aaggaggctc 69180
ggagagcctt gccagtcggc tgggatcggc aagaactggg tttttttttt gttttgtttt 69240
gtttaagaac tgggttctta aggtcaatct gtgtacatgg caagtgcaca tgtgtgtgct 69300
cacatgtgtg catatgcgca tatgtgtttc tagaggtaag tctcccagct ctgtccactg 69360
acatcagatc tgggaaacca aaacacacca gcacccagat ctggattcaa atacatcccc 69420
tttaaaaaga accagggtgg gagcagccca ggtgacgcag cggtttggcg ctgccttcag 69480
cccagggcct gatcctggag accctggatc aagtcctgcg tcgggctccc ggcatggagc 69540
ctgcttctcc ctctgcctgt gtctctgcct ctctctctct ctctgtctct tatgaataaa 69600
taaaatcttt aaaaaaaaaa aaaaaaagaa ccagggttca tcaaaaacaa ggaaagtcag 69660
aaactgtcac agtccagagg tgtgtgagaa gacacgatga ctacatgatg catgacatcc 69720
caggtggggt cctagaacag aagaggactt taggaaagga aattcaaata aagcatggac 69780
ttaggttggt aacaatatat cgatactggt tcattagttg tgacaaatgt accatagtaa 69840
ggaggctaat aagaatggaa ctggggacag gtatgtggga tctctgtatt atccacacag 69900
tttttctgta aatctaaaac ttttctgaaa taaaaagttt attgagaaaa ggcaggagtg 69960
gggagaccac agctccccac agaaatggct gatgtcgagg ctggaatggg gaagctgtga 70020
gatgagcctg gggcaccttg ttctaccaga atgtaagaaa ctgctcggaa aacagaacac 70080
catggaggat gtcacaagga cacagcctga agggactacg gacaactcca ggacaatctg 70140
agcactaaaa taatcaggaa cagtagattg gtttttttaa aaggtgtcct tgagtccatc 70200
ccaatgtaga gctggaaagc ctcactcaga ggcagcaagg atgccaaccc cggcagatgt 70260
ctgtgtggcc tgaagggtct ccccacaaat ttcttgtaag gacaaaatca gttactgtcc 70320
agtggggaca tcacatgaca cccgactggt gatcaaaaca ggccaaaaag gtgggagggc 70380
tgccacgcac atctgatgtg ggactctgcg ggacacagcc caccccactg cagtgttcta 70440
gcagagatgc agaggctgac cccattggga ggaagcatca ctaaatccaa caggggaaca 70500

tgtactttaa aacgttttta ccaaaatcta taaagaactt attaaattca acacccaaaa 70560
aacaaagaat ccagtcaaga aatgggcaga agacaagaac agacatttct ccaaagaaga 70620
catacaaatg gccaaaagac acatgaaatc agggaaatac agatcaaaac cacgagatac 70680
cacctcacac tggtgagaat ggctaaaatt aacaaatcag gaaacaacag atgttggtaa 70740
ggacgtggag aaagaggaac cctcttacgc tgttggtggg aatccagact ggtttagcca 70800
ttctggaaaa cagcgtgggg gttgctcaaa aagttaaaaa cagggttacc ctataatcca 70860
gcaactgtac tgccctaggt atttacccta aggatacaaa cattgtgtgg cctgaagggg 70920
cacctggacc cgaatcttta tagcagccat gtccataata gccaaccaac agaaagagcc 70980
cagatgccca tcgacaatga atggataaag aagatgtggt gtatatatag aatggaatac 71040
taatcagcca tcaaaaaatg aaatcttacc atttgcaatg acatggatgg aaccagaggg 71100
tattatgtta ggtgaaataa gtcagtcaga gaaagacaat tatatgattt cactcatatg 71160
tggaatttag gaaacaaaac aggggatcat aagggaaggg agggaaaaat aaaataagat 71220
gaaatcagag gggtagacaa accataaaag actcttaact ctaagaaaca aactgaagaa 71280
cactggaggg aaggggggat aggcattaag aagggcatgt gatgaaaaaa aaaaaaaaag 71340
aagggcacgt gatggaacga gcactgggta ttatacatgc aactgatgaa tccctgaact 71400
ctatctgtaa cactaaaaaa taaattaatt aaaaataaaa tgtttccaaa aggagggaaa 71460
atgtcctata aaaataaaat cagtaaatcc aaaaaaaggc tgaggagctg ctccagatca 71520
gaggagaccc tgaaggcagg aaccgtacca ccatgagccc tgcactggag agaaatgcca 71580
ccaaggacac accaggtcgc tggcaggtct ggattacagt ggagattatg gtcagactca 71640
gtgctgagcc aggcaggctg ctgtgtgtgc ccatggaagg gcatctcagc tctgcagaaa 71700
tgcacactga agcatgtgaa gtcaggggct gtgacgtctg taagttaccc ccaaagagct 71760
cacaaaagat aattgtatgg atctgcacac acacgtacac atgcacagag gacaggtggg 71820
aggaagaaat actggccaga aggtatggat aaagcaagtg tggcaggtgc gaacatctga 71880
atctgggtta agaatagacc agtagtccta gtaattttgt aactggtctg taagtttaaa 71940
atcatgtcta aataaaaagt ttttaaagaa attatgcaat atctactatg ccttttcttt 72000
ttccaagaac tatgttaata tgaaaaggca agtgacagac catggaaaat attcacaatc 72060
tatatatctg atgcaagatt tatagcaaaa atatataaag gaaattccca tctatccaat 72120
aataaaaaga cacacaatcc aaaacatgga ctggccactt catgaaggaa gaataggaat 72180
ggccaacaaa catgggaaca tatattcttg cctggtcatt aaggaaaagc aaattaagac 72240
cacattcaca cccactagaa agactgaagt caaaaaatag aaaccaaaag ctggtgagga 72300
tacggagaaa ctgtaagctt caaacagtgc tggtgcaagc accttgggaa aatatttaac 72360
atgtatttac ctaggaccca gaacatggtt ccatagcact caataacaag aaatgaaaat 72420
gtatgtccac aaagacttgc ccaagaatgc tgaaagcagc tttatttatt agctggaaaa 72480
gcctaccaat ggtaaaatga aaaaacaaat ttgcaaccca cattatctga taagagatta 72540

acatccaggg acacctggat ggctcagtgg ttgaacatct gcctttggct caggtcatga 72600
tcctggggtc ctgggattga atcccatatc acacttactg cggagagcct gcttctctgt 72660
ctgcccatgt ctctgtgtct ctcatatata aataaaatat taaaaaaaaa ttaacatcca 72720
gaacacagag agaacttcct aagcctgcaa caaaacaaca aaccacctga ttcaaaacca 72780
ggcaaaggac tcaaacagac atatctccta tgaagataca ggccaacaag catgcaaaaa 72840
gatgctcagc atcattgacc atcagggaaa tgcaaatcaa aaccacaggg aaaccccacc 72900
tcacacccat tcactgacta ttatcaaaaa acaaacaaac aaacaatgag tgatggcaag 72960
aacgtggaga caccagaacc ctggtgtact atcggtagga acacaaaatg gcacggctgt 73020
ggtggaaaac agtgtggggg ttcctcaaaa aaattaaaaa tagaatcacc acatgattca 73080
ataattcttc tggtatatac cccaaagaac tgaaagtagg atctcgaagg gatatgtgta 73140
tacacatgtt catgttaaca ttattcacaa tgtttgaaat gtggaagcca cccaaatcca 73200
tcaatcgatg actaaatgag caaaacgcag catatccatt cctcagtcat aagaaggaag 73260
gaaattccta aaatgcaaca acgtggatgg gccatgagca cacaattctg agtgaaagaa 73320
accagccaca aaaggacaaa tactatatga ttccacatac atgaggtcct tagagttgtc 73380
ataaccataa agctgtcaaa tatacggtgg gagccagggg ctgggggtga ggaatgagga 73440
gtcagtgctt catggggcca gagttccaga ttcacatgat gtgaattctg gggacagacg 73500
gtggtgacag ctgcgtaaca aatgtaaatg tccttgaagc caccaaattg cacacctaaa 73560
aatagttaag atggtaaact ttgtgttatg tgtattttta caagaaaaaa gagacatgaa 73620
aaaaaaaaac aaaggtaagt cagaatgcca ctaatctata cactttaaaa ttattctagg 73680
ccttgtgggt ttcttagtaa aataaactac agttgaaaaa gataaaccaa aaaaaaaaac 73740
aattttcaaa aagtgagttg agggcagcac cggtggccca gcggtttggc gccgcctgca 73800
gcccagggtg tgatcctgga gacctgggat cgagtcccac gtcaggctcc ctgcatggag 73860
cctgcttctg tctctgcctc tctctctctc tctgtctctg tctctgtctc tcatgaataa 73920
ataaataaaa tctttaaaaa aaaaagtgag ttgatatctt tcttaaagct ataaggggag 73980
cctaagtctg aaagacttcc agaaaaccct atagatatct atatttctaa gttttgtctt 74040
tttaaaaacg tttttttaag attttattta tttattcatg agagacacag agagagagag 74100
gcagagacat agacagaggg acaaacaggc ttcaatgcag ggagtctgat atgggactca 74160
atcccaggtc tccaggatca ggccctgggc tgaaggcggt gctaaaccgc tgagccaccc 74220
gggctcctct atttccaagt tttacaagtt taactttaca tcattttcag aataaatcga 74280
gacataactc ttatgtgcca aatatgcaac atcaaaatat tcagttctga ggattcccag 74340
gcacctggca agcactcaga tctttgttat atggacttac attgtccaaa gaatgctaga 74400
aaatacataa ataataaatt agttaatctt caccttctag agatctggga gttattttgc 74460
tatgtgactc cgaaaaaaag gactgttttg accgctagca gccctgtggg gatttgcact 74520
tattctgaca taaaaatgca gcagaagaca ccgggcctgg ttatttccac agctaccgtc 74580

attctgtgtc actctcttct gctccaggga atctgcttta tcaactttca caatcagtca 74640
atgctgatgg agcccacacc tctgtggctt ccagcatttt caggcctcct aatgcaggtg 74700
cactgtcccc atgtattcaa attcctggga aggaagctat ctggatggga agaaatactt 74760
tctctgtctc tttacagttc acccagagac aaattacaaa tggctttagt tgttctaagt 74820
aaaagatgca aacattacca acagattacc aacaaggagc atgtcttctc aaaatggtct 74880
ttctagtttg ttttccatga ctaccaaaat actcaggacc aaaaaagttc cctcagactc 74940
aggtaagcag gtctaaatgg gatacactca acctggatgg tttctcccat ttaccactca 75000
tcggtgacca atctcacaca gggccccagg acactgaggc ttttggctcc tagtccaccc 75060
cgtggacaag gcctggccca ttctgtgtct ccaagcgtta ggcacaaaag aacacaagac 75120
ataaggaaca tggcaaggtc atcctgagaa caggtcatgg cacaagaggc atccgtgagg 75180
gagcatacag ctgccccact taggaagatt ggttcagaaa tcatttttca aagaaaagta 75240
catttacttt ttttggctct tctgattctg tacttgagta cttgagaaac atccaggcag 75300
gctgactaaa tactgggtga cactggacaa atctgtgtca gtattttagg tgtgactatg 75360
gtactgcagc agcacgctta agccccattg ttagagacac agagggaccc gttcacagag 75420
gtggcagcgg gctgtctggg actgacccag ctggactggg gaggagtggg ggtgggcacc 75480
tccttctctc tatcttcatt tgaaatttta agtaacaaaa aggttaaaag aagaaaagaa 75540
cctatgtgcc acaatgttca cagtaatcaa agcagaaaat gactggaatt tgcttcaaaa 75600
taaatcaaaa tgggggatcc ctgggtggcg cagcagtttg gcgcctgcct ttggcccagg 75660
gcgtgatcct ggagacccgg gatcgaatcc catgtcgggc tcccggtgca tggagcctgc 75720
ttctccctct gcctgtgtct ctgcctctct ctctctgtgt gactatcatg aataaataaa 75780
taaaatcttt aataaaaaaa aaaataaaaa taaaaaataa ataaataaat caaaatgggt 75840
cgtgggagat ggtacagaca atataggcct gcacatgcag ttggtaactg ctgagccagc 75900
tggttcattt tactacacgg tctgccttgg tttacctctg aaatgtccat cgtatgaagc 75960
tctgacacag ggaacaaagg ggagaaagac tggcaggcgg gatgcaagca aagagtgcgc 76020
tccacggctc tcctatctgc caagggatcg gggtgtgctg gctcagcctg tccccacacc 76080
ccgggctgcg cccccacaac acacattcct ggggaaggat gggggcccag ccagcagcac 76140
ccctgtgccc tcccctctaa tgtggagacc tgtctgtgat gggagtgccc tagtcaggcc 76200
agccccatgc ccacagcagg cagcctgggc ggatcaccac ctcacagcca ggaccctcct 76260
tcccagactg cacccaagaa agtgcaccca atggcagtcc agtagaatcc agtattctgt 76320
gtgaagagaa acctccccac cactaagaat cgatgagcta gataagccct gtttcgtaag 76380
gcagcaacac acagtaaaac cacctgcagg ttaagcactg cagggaagag ttaaaaaaat 76440
aataaagcat catcaatgaa acgccgggac acctgcaccc cgctgtttat agcagcaatg 76500
tctacaatag ccaaactgtg gaaggagcct tggtgtccat caaaatgcat ggataaagaa 76560
gatgtggttt atgtatacga tggaatatta ctcagccatt agaaatgaca aatatccacc 76620

atttgcttca acatggatgg aactggaggg tattatgctg agtgaaataa gtcaatcgga 76680
gaaggacaaa catatatggt ctcattcatt tggggaatat aaataatagt gaaagggaat 76740
agaagggaag ggagaagaaa tgggtaggaa atatcagaaa gggagacaga acatgaagac 76800
tcctaactct gggaaacgaa ctaggggtgg tggaagggga ggagggcggg gggtgggggt 76860
gaatgggtga caggcactga gaggggcact tgacgggatg agcactggat gttattctgt 76920
atgttggcaa attgaacacc aataaaaaat aaatttatta ttaaataaat aaataataat 76980
aataataata ataaagtgtc atgtgagaca caccagtttc tgtatacata ctctgtgatc 77040
accagcagac cgtaattcaa cattctagag tggatgctaa tgacacgtcc acagacgcag 77100
agagcgccaa acgcacacag caacttggcc ggctccctcc ctgtctcctt ccaagggcag 77160
cacagggggc caccccagga gccccctccc tcctggatga gtcccccacc caagcccaga 77220
gtgccctccc cacgcagcca ggtgccttca catacagttg catttcagtg gcaatttctg 77280
gtaataaata aagcagagct ggaaacgacg gcttgattaa aacaactgtg accaaaaaat 77340
aaataaataa aacaactgtg accatgtgtt cagccataat cagaacacct gcttctagat 77400
ttgttcagct gatcaggact ctgaattata ctgacctgag gtgtggacgg cagagagcag 77460
atgagaaaat aaaacagcac ctccactcag cacccccttc accttcctaa ggctcccctc 77520
aagaccatac ttggtcctgg tcctccagca gcaaggctgc aatgctggca gaccgactgc 77580
catggaaggt gccttgtcat ccttttattt ctaatttaca tgttaaattg atatttactt 77640
attcaaacag aaaattgaat cttaagggta gtatgatttg agtcttctaa agactctcca 77700
tcagtgatac aatgtctaat aaataaataa ataaaattaa aaaaaaaaaa aaaccaatgt 77760
ctatatgcta tacagggacc catgaataac ccggggctga aggacagcaa cccccacgca 77820
gtcaaaagcc tgcatgtaac ttctgacttc tccaaaaact taactactaa caccattcag 77880
ttgaccagag gccttaccac taacagcaac agtcagtgaa cacatatttc gtatgtcgta 77940
tgtattatat gctgtgttct caccataaag tcagccagag gttagaaaat gtcattaaga 78000
aaaccatgag aagaaaaata cacttacagt actgtatcta acaaaaaaaa aaaaaaaaaa 78060
tctgttgtaa gtggccccac gcagttcaaa cctatgttgc tcaagggtca accacatttc 78120
ttctaagtga atcttacaaa gtcttagcta tgatacaggc acacacaaag ttcactgtaa 78180
gaaaacattt accacttagt aatggtaaaa acagtaaagt gcatagtaaa gctttactat 78240
ccttaacatc atacgaagaa tacattaaaa tagactcatt tttcacaatt atcaacttga 78300
tatttgtgaa ttaatgctat tatgcatgcc tattgaacta ctgattacca tcggccagca 78360
gaacattcca tcaccagaaa gaggggcaat gattgcaaca aagccacaac caggaagaag 78420
cgttttctat aatctcattc tacacccgtt atgtacactg ctgataatca cgctgctctg 78480
aacagaaaca atgttagtgg tgactttgtt tctccttggg gcacggagcc cagggccttc 78540
tcaccttgct gtccactcca gaagggctgg tgaacacgta ctccagttga aagactatgg 78600
caaacgcagg gtgatggacc atctcaggga gtcggaggcg gcttctcaaa actagagcct 78660

ggtttccaga gctgggtcaa gaacacagag cagcagcaca tcaggcagtg ttcctgccac 78720
cctgcacccc ccagggtgcc aggccctgct gctgtggcag gttcccccag caatgctgca 78780
gagctctgca ctgtgtctgg cccccaaggt agatcctaaa ccagagcctc tcatgcttca 78840
gtcgacactc attccaaatc gccagagctt acatcaggag gacagaacca catggccagg 78900
tctccgagga ggctcgtcct ccagggcacc cctcggagag gtctctgatc ctgacttggg 78960
aatagaggac tgaacagaat ggcagaactt cctgcctgct gcttctccac cacgtgagag 79020
actgatcaca gacacagtgg gggtataaag tgtgctaggg tcccctcaga tcctaagcag 79080
ccacacctgg agacacctat aatgttagct ccagaactgt cacagcacag ttcaggattt 79140
ttccaacctg ataacactga ggtctgctcc taacagatat aaatcagcaa gaggaaacaa 79200
aggcaccagg gaagaagtcc aggtggacaa gtgcatactt cacatgcaaa accagctcca 79260
tttccctttg atccccagta atgtgcaccc tagtacctcc taattccacg aggctggaca 79320
tcacgaccct agggagaata cctggtcttc gaagaggagc caactttcct gctgaaactg 79380
gcagagcgcg tcaaggccac atccatctcg ggcaccagca ccaccacgtg tggcctctgc 79440
acgaagccca gaccattgtg cacgcccaca cacaggcggc gctccaagat ctccagcgtg 79500
ctgccatcta gtggactgtc cccctagaag agcacatgtc acacacagta aatccctgtg 79560
cagaagggca gaagctccca gtggagaggc aaacaaagag aagaaactgt aactgaagtc 79620
tcaggcattt ccagatcaga tggtgctcag tgaaaatgaa ttcaggcaaa taatcctatg 79680
agaaccgcca gcctcagggc attttctagg tagttctggg tctttacaca tagcacggtc 79740
ttctcagagc aatatcaccc tccccctaga agatacctga atcaccaaag tcttactcag 79800
gtttccatgg ctgagctcaa ttcttaggat gcagcaaatg agcaaccagg gccctagcgc 79860
cccaaggcag agaagcccac tgcccccaag gtgcctctat cctcccccag actcagacac 79920
cagaggcttg gggtggaagg ggcagcagga aaagggagca catttatgca gtaagtcata 79980
aaccaataaa cacagaatct gccccaaagt gatcgctgga tattaaagtt tttcatctta 80040
acaaaatatg tacaggcact ttaacaaatg agtcaggata gtcctccagg ctattactgt 80100
ttattaaatt acgtgatcgt ggtgaaatga ttcctgaatt ttcaagtttt cttcactgcc 80160
cattaatcaa gctgaacaga cctaaattca gcctattagt ctctgagtaa tgaaaacaga 80220
gcgatggagg aaacagagta gctaccagtt ggataaggac aaaaatacaa agtagtaaaa 80280
atatgtaata aagtgatttt ggggggatac acaaaattca acaatgatac tcctaaagca 80340
tctctaccct tatgtgaccc actctgagca ataagaacac agcaggaaac acagtaagaa 80400
aatgtgctcc gtcaatcctc ttttctgtgt ttctacttca catgagacat cgtatagaac 80460
taccttgtca agaacgggca gcaagcaaaa aaaagtccat ctcatctatg tgtgctgtgc 80520
acatgcatct atgcatacat gtgcatgtgt gcatgtatgt gtgtgcttgt gtgcatgtgt 80580
gtgcagctaa gactttgctg acttcccaga ctgctaggat gggcacctac cagtggaact 80640
gctcgttgtc acgatggaga gatagcaaag gcctgtgaca ggctgacact gaagcacctt 80700

gggcacagca gagaacccaa ccacatccct gttttcctgg tgcaaatact ccagacagac 80760
aggcaacaga gcaaggaata ggtaaggaac ccaggtgctc gtgggcaggg ccaagcaccc 80820
ggagaaagga agcaggtgac atacttacag agacttgggg ggtgggcctc atcagcacat 80880
tccagtggcc tgcaggactg tcgagtttgc acagagacct gaagcctaag gggagccatg 80940
caagggtcta gacgcagagc atccaggaaa atgagctacc agcccaaagg ccacagtggg 81000
gacacactgt gacctgtgac caccaagact tcatggcagc acatcagatg ggtaggcaga 81060
cctggaccct gtgaggggag tacctttcac agaaagtggg tcggccggga taaaatgact 81120
aagacttcta gtcgtctcct tggaatgagc caaatatctt gaaaatgtcc tttataaatg 81180
ctgataattt tattggtctc cctaagctca tttggtgatt taatttactt ttacatgaag 81240
actgctttaa gatacaacaa aactaacatt agggtctcac ggtaatagca ccatcatcgc 81300
accaaagtta aaacaacggg ttagacatcc tcttctgcca ctaaaagagg aagcacgcaa 81360
gactgtggct ggaccgtcca gcctggtggt gtcagagacc ggcagcgtcc ccacctctgc 81420
cgtccccacc tctgccctgc ccacatctct actgttcaga ttaagctcca ccatgtagga 81480
cagtacctcc cggaagttat cactgatgag gagttccaga agctcttcct caaacttctc 81540
aagggaagga tatagcgtga agaataaatc atccaagtac catgtgacag acttctgaag 81600
gcgaggcttt cggagggtgt cccctgaaaa aatgaggggg ttcgtgtaga gtatccgtgg 81660
ggacaggacc accagaagca cctagggcct ctcacccacc taggtcaagg ccatggtgcc 81720
aggccctcca ggcagagaag aaaccacagg cacagcactc agagccctaa atcagaagcc 81780
acccccattc acaccaagac gccaagccca gatttcaatt acaacccaat ttctgccccc 81840
accctcctcc aggaacacat tttataattc agcaatccaa agaacacaga tcactgaatg 81900
cagggtaaca tctgttccta tcagcttggt ttccctttat cgctaacttc ctctctagca 81960
agaacacagc cagtgatgcc gtttcttatt tttgtacgtt taccttgaac catttggaaa 82020
catgaagaaa tactcatgag cacattaagc caacaagcaa aaacaccgtc tgggctggca 82080
ccacaagaaa agtccccatc tgggtggggg agctgccctg gactgtgagc tccatgagga 82140
cagaaggggc ggtagttcct ggcacacagc tgggtgcccg gcacagagca ggtgcatcaa 82200
ccctgggctg tgaaaggcat ggtccagtct gagcagagac ctcccctcca actcgggcac 82260
gaaggccaag ccaaccacct ccacaccagt acacaccccg cgctctgccc tcccatcccg 82320
tcactgccct attgccctgt ctcttcccca gctttcaccc gtaagaacta aggagcaaag 82380
ccttagattt taagaactat ttccaaagtt agagaaagga aacatcagcg gaagactcag 82440
cgcagaggga actgtcatgg taagagccct tggtcgtcct gctaggaggt caatcagcac 82500
agccagaggc gatcccatct tcgcctctgt gtgcactgag acccttcaga atctcctcag 82560
ccgctctctg ccccactaat tccacatttt gaacctattc agcaaaatta atcgaatgtg 82620
ggagcaaagc tttatgcgca agaatactta ttaaacatta tttataacag aaaaaaataa 82680
gaaacaacca aatgctccct aggatgggga atgttaaaca agttaccatg cctccgtata 82740

aaacaggaat gaaactccta ttgtcaaaga tcacttaatg attgaaaaat aatcataata 82800
ttgcaggtaa aaacgatgtg tgtaacactg cgtatgagat catttaaagc caattttatt 82860
tgaaaatcta catttacatg catacatgga tacgtatgag aagtaatcac agatgacttt 82920
atttccttct ttatggtttt ctatcccttc ctcatcacaa atatgtatta gattctcata 82980
tttcacaatt tcttatgctt ctaagaaacg ttgcacgttc ctttctgaat ttaaaaagaa 83040
gtgttgaagc tgctctgtgg aggaatcaga acccacgcaa agtataggta tagttattca 83100
ccatggccaa gggttaaaga caacccaagt gaccagcaac agatgaaaat aaggaaaatg 83160
tggtccatcc acacacagaa tcttattcag cattaaaaag gaaggaaatt gacacctgga 83220
tggacctgga agacatggcg ctcggtgaaa tgagatagtc accgaaaggg caaatcctag 83280
acaatctact cacaagaagc cctaaggtgg taaaatcaga gtcagaagat ggaagggcgg 83340
gtgccagggg ctggggaggg gctgggcact agtgtctcat ggggctgagc tgcagttttt 83400
gtgagatgag acattctgga ggcaaacggt agtggtggcc gcagactgca tggtttacat 83460
ggttcaagtg gcaagtgtga cactacatct atgttaccac aatttttctt taaagagcaa 83520
aacatagagt ccaaactttc atgaaatggc caacaagtgt ctgaggcatg tcctgaattg 83580
agatcctcca ccagagaaag agaagggacg cctgggtggc ccaggggttg agcatctgcc 83640
ttcggctcag ggcgtgatcc tggagtccca ggatcgagtc ccacatcggg ctccctgcat 83700
gcagcctgct tctcccactg cctatgtctc tgcctctctg tgtgtgtgtg tctgtcttga 83760
acaaataaat aaaatcttaa aaaaaaaaaa aaggagaaag aggaccgaca aggaaagaga 83820
ggaaggagct aaagcagcac acttccacca ccagtgccaa gggcagcaag ccaggtcctc 83880
catcatgggc ctccctagca acaggtcaca gggcacacag cagcatgggg gtgctcatgg 83940
aggattttgg ggatgacgag acagggcagc tgtggccctc tgggcacaga gaaggagcga 84000
gggaccatca tcaaaccagg gcccatcagc aggaagaggc caggcccgag ccaacaccac 84060
tgcctgctgg gggtgctgag tacatcctac ccgcaggtag aaggcgtgtg tcctgaaaca 84120
agggaagaca acgggtggtc aggcaccctg gggccagggc ctaggggcca accccagttt 84180
ctaaacctgt gtcctcagct gtggaagcca ggtgacagcc ccccactcag gacagtgctg 84240
tggggacaga gctaatacca ttcacagcca tgaacacagg gtcatagctc ccgaggaaag 84300
gcacacacat gtacacacat agctcatcag actaatctta aagcctcggc atggctcatc 84360
cctgtactgg ctcatccctg tacgttctag tctctacttt acaagagaaa cagggctcag 84420
acattctaag tgacttgcct gaggatacca cacccacaga atgtgagtga gggagaccca 84480
aatgttgaag ctgctaatac acatccccaa atgaccagga aagtactgtg agaacttcag 84540
ggagtgaata gattcacaga tacagaaccc aagagaaaca aagacgggct gctctgggag 84600
caggggactc aggtgggacc ccaggggcgc cagccaatgc cagcccttcc agagatgggg 84660
cagcagcaga gacagacttg ccggtatgct ggggccactg cctgtccagc agggaagcca 84720
cccgcccctc gccaatctcc actactgtgc tgttgcctgc ccctctagac aacagtacag 84780

taggatgagc aaatctgggc atccagaagg tggcggagtc cagggcttct ggaagaggct 84840
cagtcctggc aggaagcagg ctctgcccag ctccccattg acaggtggcc cccaggccca 84900
cagccattag caaaaaggag ccttgactct agtctcacag agatgtgagg aagggagccc 84960
cacacacaga agtcagacag ggagaggaaa gatggttcct ccaccaggag atcagactaa 85020
ggagccacat atgcacaggg cagaaccttc cagatcctga ggggcccagg aggcagggca 85080
gggccaggtt gcatctggcc ggctttctcc aggccatgtc ctgtgaaaat cacaggcact 85140
ggagaatggc tctcactcag aggacagagc aggcctccca ggatgctcca ctgcagagga 85200
gcggccaggg tgaggacagt atcccatgaa taaagaacaa aggtcctggg gccggtgctt 85260
cctaagttct cagtgctgag ctccatcggc ttacttcttt gctcctcaca tgcatctcca 85320
ggggcaggtc ccaagaaagt ccccatttta tgcaggaaag ggcagcagca gaggctggga 85380
gaggttagga gcctggaggc ccactccgta ctcagaacct cttccctgct ccctcctgca 85440
tccccctcac aacagcttca ccatctgaag tgtcaccctg aaatgtcacc tcattcactc 85500
tgaagggtca ccccgttcac tctaccaccc aacctcagca accggggttg gggaactggg 85560
gtgaggcggg ggacggggac tcatggcccg cacactctgt gactggggct atttttcata 85620
tggttgttgc caaggctctg ggcaggaatg caatccccaa aaacagcaat gttccttcag 85680
gaatccagac tccctgccct cagggatccc cacacatgca ctgccgagaa accagggatt 85740
acaggcgcag acaggcaaga actccctgta gtgacatcct taagagaagc gttcagaaat 85800
gccctccagg tgcccaacaa gctctccacg aggtcctaac agggtcccac agcctcccca 85860
ggacacagct ttcctggaag ggcagtggat tccccagaat gcaccccacc aaacagtgtt 85920
gcaagaggaa caacagaatg agaccagtta gagggtgacc caggcaggag cggaaagagt 85980
ctgagccatc cacattgcta aaacacctgt tgcctttgat ggctgtggca agacgtgatg 86040
ttgcccttgc ttccatctgt ccaagacagg agcactaaga gccccatgct gtgccctcac 86100
cctggctcag tgtctgtgca cacggatcca gccaatgagc cttccgcgga tgaatcatgg 86160
acaaacccaa gcatcggtga acaagggaga ggataaaagg atacaccgaa gtcggagcac 86220
tgctttctcc agggaggttc cccggggagc aagcctggaa ggaggacttt cagcaaagat 86280
gacctctgtc gtattaaaac tggagctcac acagggcagg agtcagctct cctcttgtca 86340
ccccaccacc acccctgcct acccccacct acccacaacc cacagtaacg tggcacaatc 86400
cagaaggtga atcccagcaa ggaccgagcg gcccagagaa gcctaggaga aggcccacag 86460
ccactgagat acttggatgg agggtgggta gggtggggga agcagcattt ttaaggagca 86520
cctcaggaga ttcctgagca gggacttggg aggaccatgt gggataccac cagagtgggc 86580
cacctcgtgg cagactggcg ggcatgaggt tctgacaccc cacttctcag ctctaccgag 86640
ctctggtgta acagatgagg aggcagacct ggaaaggaga gcccacggca aaggccgctt 86700
tgtctgcttc agacgcctgc gtggtgtcag ccagctcagg ggagtgtgag gccctctgct 86760
tcctccagcc ctacagcact gtgagaagtg ggcccaggag ccctgcactc cctgagcacc 86820

cagtcaggaa gtctcgctca ggactccagg cagtcctggc attcacctag ccttcagggc 86880
cactgttaga aaacaggctt ccaacttata tttacaacga agcctccagt gcactcagct 86940
actttatgca aagctcgcac ccactccagg gcaggatgag caatctgagc cttcccccaa 87000
acgacagaca tctttctggt cctgatctcc tgagaccatc cccccaatga cagaggcagc 87060
atgcagatcc gcaaccaagg agcctccgtg cggagactca ctctgagcca ggccacggga 87120
gctctccttg aaagaagcag ctttcaggag ccacggcatg tgtgcaacga ccctggtgct 87180
gacacagctg tccaggtctg tcaaaccaaa gcaagtgaaa cggtcagaac caggccgagg 87240
agaccacgag agggagggtg cgtggatttc caagatgcat tggcaggacc tgctaatctg 87300
gccagatgct gagggcctcc ctctgagagc cacccgaggg gaaaggcctg cctcccttct 87360
ggagagtgac aaagagccac cagcacaaca cagctaagac cttcatttcc agccattccc 87420
aggctgaaaa tcactcttcc aaaatcagcc ttcacagaac tgagtgagtc aaagtggaag 87480
acaggaggaa ggttggagac tgtgtcaaca tcggtatcct ggtcgtgact ttgtgctaca 87540
atttggggga aaccaggcaa aggacacatg gagtctctct gcgttattaa aaccacatgt 87600
gaatctagac tgacctcaat ataaatttca gttaacaact caatcttcct gtattgtgtg 87660
atattttaca aatgaacaaa tatccacatc cccatatggc accaaaataa aatcagaccc 87720
tctccaaatt gcacgtgaat tccaccactg aagctggtag cagtcactgc cttggccctc 87780
tcagggctat aaaatattat tcagcaaaga aacacttttc caagagtgag tcacaaaagc 87840
ccagaagacc ctgggcagga tttataacac agacaagaac ggcgtcagaa agcagggaca 87900
gtgcctgcat ggctgagagc acagatcagc ctggcgtcct gcacatccag ggaaggtgga 87960
gcaaaaccag ttccttttct cagatggtca ccggaggact tggtgagaag ctgtgttccg 88020
gtcctttctc cccaagaaga gggagtatgc tcctgcccac ccgggcactt tccggctcca 88080
cttctagggt ccctggtcat tagcttttat gcaaaatatt agggacacct aggtggctca 88140
gcagtttggc acctgccttc agcccagggc atgatccagg agacccggga tcaagtctca 88200
cgtcaggctc cctgcatgga gcctgcttct ctctctctgc ctctctctct gcctgtgtct 88260
ctgcctctct ctgtatctct catgaataaa taaaatcttt tataaataaa taaataaata 88320
aataaataaa taaataaata aataaataaa taaaaattaa caacagatta aaacaacagc 88380
aatttcccag acgctgcctg cacccagtaa gtgtcccagt gaccaactgg ccgctctaca 88440
ggagggggct cagatggctt ctgaagacat gacccttcag aatgaggagc tggagtatgc 88500
cccactctgc agctggcccc tctgatgccc caggctgaca caagacactg agaataaact 88560
cggggccacc agagctgccc attgtcacca caggaatgac ataggaccag gctccagatg 88620
agcttcggca ggtccaggtt ttatctgcag ctctactgac acccactggc aagtcataga 88680
gtgagggaca cacctgagcc cccaccaaca ctctgctcac ccagaagccc aggtcagaag 88740
gactcatgta agctgagttt taggcaggtg tgcacctagg ttcttgacat caggacatag 88800
cacagtggca caatgggccc tctctgatcg agagggccga ggcctcagag tcacacgcgg 88860

attgtcatgt tttcaatctc gaaggcagtg accacgcaac attctatctc cctgcttgac 88920
cccgtcttct ctttcgagag gacataagca aattctggag ggtgaaaagg cttcctatat 88980
ggggctggtg gcagggggca gggggttggg ggggctgggg gagggtggtt gcttggtaag 89040
ccatggcaaa tccagaggtg ggaccctcct cagacttgct cctgttcata gctatcattt 89100
ctggtgtgtc atcatctctt cttagcaaca ctggaaaaat agtgaccttg taatggcctg 89160
accaccatgg gcccatccca gaaatttacc aactcagata acaatgtctg ggccccagta 89220
ttgactagaa cactgagcca tgtgcaggga cgttgttact gaaaacattc ccatactgga 89280
gagctgagct acttcttttt tggggggtct tgagtattcc tattctaagt caaaactcgg 89340
cccttccagc tcacgctcat gcccaatcct gctctcccca gaacagaaca gggagccagc 89400
ccaccccaca caagaggttc cagcagcacc aggtggggaa ggatgggtgt ggcaccttct 89460
cctggatacc tcagacagcc agacggagag cctccagaag tgcctggaag gagcatgaag 89520
ttcagatcga ggcccgtcag cagaacccag gagcagagca tatctctgca gagacctcct 89580
ggctaatcca ccactggaga acctagttct aaacacacca agacagagcc cggaggcttt 89640
cacgtggagt tcggttttca aaaatactca agccatggct cacccgcatt cacagcagca 89700
ttatgcacga cagtcaaaaa gcatcctgtc cattgaaagg taaacagaga aacaaaatgt 89760
ggtccatccc tgcaatggaa tattattcag ccttacaaag gaaggaaatt ccgacacatg 89820
acccaacaag gatggacctt gaggacacga tgccaagtgg aataacccag ccacagaagg 89880
gcaaatatgg actatttccc tcagaggagg agcctggagg agtcaaattc acagaggcag 89940
gaagcagggc agtggggtgg ccagggctgg ggaggggaca cggggagtca ttattgaggg 90000
attgacagag tttcagtcag gggacatgag aaagttctgg agtggatggt ggtggtggtg 90060
ggtgtacaac aagatgaaca tccttaatgc ccctgaactg catacttaca aacggttaga 90120
acagtagatt tatgttatgt ctattttgcc acaatttaaa gagaacgata atcaagcctc 90180
tgatatggca gcccagcaag ggtgggaact aacaacagtg gttttcccgt tttcccgacc 90240
ttggtcatga aaatgcgaac gatggatctt atgacagcat ttcccaagta aggacacaga 90300
agcaacttga gggatgagag acaccgctct gaagggacct gtgccctgcc ctcctccctc 90360
ccctccctcc cttcccctct cctccctcct ctcctcctcc cttcctcctc tcctctctct 90420
cctcccattt gctaagggta cctagccaca cccagcccac ctcatagcac agagcctgat 90480
ccctgcaagg tctgaccacc aaactaatac agactcacca aatagaggga gacagagcaa 90540
cccttaccct tttctccatg agctggaacg aggccaggga tgtgctgcag accagacacc 90600
agaaggttct ggggaagaag atggaacacg ggctccagga gcgggtgtgg cttcagagtg 90660
tattgcaggc tgcagctctc aatgagggtc atgtgtttat tttctgtagg agaaaacacc 90720
tatatgagag ttggcactaa aggccctttc agtcaaacca gccaataaac tgagcatcta 90780
agaaacacca caaacaggaa acatattttt ccatggagag gggggaatcg ctctgttagt 90840
tcagcctaaa aagcaaacta atccttacta aatccttagt aactctctac taaaactgca 90900

ataacagaga tacttgcttt taatccacat agtgaaatct ctcctgcttg cctctccgcc 90960
caaggtgtga agtccaggca ttgcctggtc ctcagctcag cgggggactg caagggaccc 91020
tttcccctca gcacctgcac cctggagtcc cacagagcat cgggactagg ccctgggaga 91080
ggcaggagac caagacagag ccccgcctcg aggacatgtg ctcatctggg caaccgacac 91140
acaaacacag atacccacag cacaaaggac aataggatcg gaccctgaga aaggtataca 91200
taacgcacag cgagctaggg gaagaaggag agcaccctga gaaagcagca gctgagaaag 91260
gcccagacac agtgctcagg gagctggagg gaacaaaacc aacagaacag accctggggt 91320
gcaagaggca gagtccagaa tcacagggcc cagaagctga gagggaccag gacatgccct 91380
ctactcatcc ccgccaaaac cttgcttggc cttgatttct tccactgaca ggagcctcac 91440
cacctcctga ggcaagctac atgatttcat aagaaaagag ctaaagtctg aagcttaatt 91500
ttattgataa agattggctt ttaggttatt tctcaatatg accaactgca ttcattagga 91560
aaagcaatta atgaaaacta tatttttatt acctaatgac tacacacata gggtctgcta 91620
actttaaatt tttaagtgcc ctgagaggta ccctctcgtg tggccggctg gagagacact 91680
ggttgggcct ccatggaagg caaataggag aaatgttaca aactagcccc cagaccgagc 91740
tctgcagctt ccaagagctc gacccacagt tattgggcac atatacaaaa gagaggtgtc 91800
cacaccagca acgtacccag ccccagggcc ctggatctgc ccggtatcca ctggcagggc 91860
cactcagtca acacagaaca cacactcaac aactcaacaa cataccatga ggtcataggg 91920
agaacgaggg gctctctcaa caccaagacc aaacaaacct tctctaaaat gagaaaaatg 91980
agatggacat gcggctctgc tcctgtcctg gtacaaagac cccccagggt ccaggagact 92040
cagaggggct gcctggggta tggaggaagg agagcagaac actgcagggg caggggagct 92100
cactgaacct tgttacactt ttttatgcta ttttttaag atttatttta tttgctcatg 92160
ggagacacag agagaggcag agacataggc agaagaagaa gcaggctccc tgcagggagt 92220
ctgatgtggg acttgatccc aggactccag ggatcacaac ctgagccaaa ggcagacgct 92280
caaccgctga gccacccagg catcctttta tgttattctg aatcatgtga atatattgat 92340
cattcaggca agaaatgtgt acatttctgg aaatgcccac gagaaggtga gctaccagcc 92400
acatagctcc tcaggcactg ccacagccac acaatcttcc aatgctgccc gggacaaggt 92460
cctgatggtg tgtgtccttc caaaggtcct gaccctgggg cactgcacta actcagtcaa 92520
tggagcacgt gactcttgat ctcagggttg tgagttcaag ccccacattg ggcatagagc 92580
ctacttaaca taaaattaaa aattaaatga aaaaaaaaag ccctgattct aaggatgctg 92640
taggacacaa atactggcaa agtaacgatc agactcaatt gagaaataat gtaaaagcca 92700
atctttatca ataacattaa gcttcaaact aattgctctt ctattgtgac atttccgtca 92760
aattcagcag gcagtggaag ggatggggct tgctcaaaat tcccatcaca gaatccaggc 92820
aacaactcaa agacacagaa agtcaccctc cccaacagat ctacctccaa ccactggacc 92880
ccctccacat cctggccctg ggccaggtca ggtctccacc aggggcctcc aagatggctt 92940

ccacccggtg ccccctcccc atcgttcaag tggccaggag ccagagtagg tcacatgccc 93000
ttcaggtcac agaggcatgg caggaagccc cctgctctgc ctaagacacc ccccaccccc 93060
acccctgcag ctgctgggcc tcatggaaga tggcaggcat ccctgcagga cgttcctgtg 93120
catttaggct ttgccgtgct ggacataacc aatgggcctc acagtgaaag gtttctggta 93180
ctagcatggc catggccagt gatagacaac aactacttgt agtggttgtc aggtggacag 93240
gccaccaccc cagaggccag gaggcccaca tccctcccac caggaggttc tggggctgcc 93300
tctcccaggg ccgtctgcac aaagcaacac cttcccctca ggctagtgct tacagttcct 93360
tcagttacgt caaccatcca agctcatgac actttttgtc ctaatccctt aaataaatcc 93420
cgtgatacag cctcaacctg caaagcaaga ccacagagcg catgtcaacc ctccgcatcc 93480
tgcatttagc caagaaacat cagtgatgct tctcactcca cacctactag agctgtgtca 93540
acaccataca atcacatcct ctgcaggaga gacaggtgag caaagcagca gccccccatg 93600
cccccgggcc acatgcttcc aacacacaga cagtactcct cccgacacac gcaagaccca 93660
ggcacctaga aaaagtacac cagggaaatt tctaatatct gaaatgtccc aagaaagaca 93720
gcatttgagg aatcttcatg gttgtgtatc tagagcactg ttctcaacca gggatgatac 93780
catctccaag aggatatctg gtaatgtcca gagatatttt tgattgtcat ggctaggggt 93840
gggtgcagcg gggagagggc tttggcatct ggtaggtaag gcctgggatg ctctcagaca 93900
ccccacaggg cacgaggacg gcccccacag caaagacttg tcctggcccc catgtcagca 93960
tgccaaggct gaggaaccct gtccaaagtg acagtgtaaa cttcagcact ggctgacatg 94020
gggacacact gtcttctccg tcctgctata tagccagctc ctaaacagag cccggcacaa 94080
tgtgtctgct gaacagatgc ccactgctta ctgggtgcct gttggtgaag aacatattat 94140
ttagcaccca cacaacctta tgacatgtgt agcgttctct ctcatgcccc gatgagtgaa 94200
atcagtcaag agtttgggtc actggcctaa aaccctacag gaataagtga gagctcactt 94260
catcctacat cagttcctac atcagcctga agaacacttc cttggctcac tgagaaatgg 94320
caaacagtta ccacacagca gaaaagctcc cagacaacac acggttagga agaaaccaca 94380
acacaagctg acaattgcaa agttttctgt tacagccaat gagggagcat gaaatctcca 94440
caaaatgatg ctggaaaact ccaaggatga agaagacgag gcaggtaccg cttgtcctgg 94500
gaggcagagg taggagattc cagtttgttg ccaaagatcc gaagaattcc aaagccacat 94560
gacagagcct ggagagagcc atcctgcttc cggccttcgg tgaccacctc aaccacagcc 94620
accacgttag ggtggttcaa ggacgtgtga aagtacaagg gctgcaaaac agaaaccaca 94680
acttcggtga cagactgacc atccaccagc tgcagagagc atacagaaaa ggtacaggcg 94740
ccctcgctct gccctcctat gaatgtgtgt gcccgtgtgg aacaccgcgt ccctcccagg 94800
gcaccgcagg agagaacagg catggtaaac ttgctccctc ggagtcaaga aacattccag 94860
agcacccaac acccaagaga gcagactatt tgcttcgctc ctggagcttc tgacccacag 94920
gaaatgacag atggcaggcc ctccacacac cacctgtcgc acacaccacc ccattgccca 94980

acaaggaccc agtcactgca aacacacaca cctgctgagc tacttcacag agttctcacc 95040
acgtaaaact tcaaaacaac tcgccaatat tggatctaac ccgttttgtg ggtaaccatt 95100
atgaagttcc acctaaacta gttttgttta aactaagcag gatccctaga ttttaggaga 95160
tgctttgtaa ttatacccag ttcagagccc gactaacatc tttttatgac aaaaagcgcc 95220
taacatcatt ttcttacaag atttttttct ttttgaatct aagccaagta gatagtattc 95280
aagtctctct taaaaggttt tccacctgta acgttccctc ggatgcaaat ggacccccag 95340
ctggggcggt ggaaggggtc agatccacct gcgtccagcc tccctgcgat gtgcactcct 95400
gctcaggaga gacagtgcct ctgctcagcg gtcgcttcct tcaacttcat catgcccggt 95460
ctggctgctt ttatttaaaa ctaataaacg tcgtaggcac agaagatggg gacagaggcg 95520
ctgctggggt ttgcagaaca aatctagatg gaggcactga gctgtggctc tggacacgga 95580
aggtccccag aatcgcatct gcactggggg agcatgggtg tggcacccgc ccccaacaca 95640
aagaccaccc ccctccaagc aggtagcggt aaggccgggc tgctgcgatg tgatttaaac 95700
gtggactaat cccccattaa cgagtcatta ccgtggtgtc accaggaaaa cgtttactgt 95760
aatcacagac tgtcagccca ggataacaga gagacacatt aaaacatcag ccatgagata 95820
taaatcgtta ttctattatc ttttattgca aaatgatgaa ataatgacca cttacagctg 95880
acaggtggtt ggaccaaaag agaattatgt ttctgtttct aagactcaag aactgttttg 95940
gagagcccca ctgcagagtt gggtttgtca ctgtctcacc tcttatattt atacctgtgc 96000
acatccgtgt taggaacact acccactttt cctttggcag tgaaacaaat accttaaacc 96060
gtgatgcaaa gccacagtca tctcctaccc agaggtcacc ttgaaattgc tctgcctgga 96120
ggccctggga ccagctgctt ccacacgtcc caggttcaaa tttatggctg ctccctctcc 96180
tttccgttta ccccaaaaac aactgctctg agaccaaaac aacaggagca gagccctgct 96240
gttgtccatc tgcaaagacc ccatcttcaa gtgggagggt cagtggtgga agtgccactt 96300
cctgaacacg acccgaggtg tgaggggtgt tgccctggca ctactggcca tgaagggcac 96360
atggtctctt ccaaatataa tttatggacc ctttggtgtg ggaacagcat cgtgctaaag 96420
aatttctaag gagcctgaaa acacgatccc accctcaagt tcagagttca aatgtaaagg 96480
ctagattagg cagatagaaa tggcaaattc tgctcaggac cgaccagaat cacgtggaag 96540
taaaagcctc ccacgggtga gagcacaggg cagctccaca gcttgttagg aaaacccaac 96600
aggccctata gtcacctatg ctaagcccca gtcagcaaac caagggacac agacccaact 96660
gcagttccca ctctcccggg cttggagctc tgagtgggtg gatctggaat tccctggtca 96720
gcaccagtga ggtgatctcc tggtagaccc tctgcacccc cgcacacaca ggaaggtacc 96780
ttgcctgaaa aagtttctct ttgctagaaa cttcttcctt ccccattgta tctataaaag 96840
cctttcactt tgtacagctt cctatagatc ctttctggag gctaaatggg atgctgccca 96900
aattatgagt cgtgcataaa gccaattaga tcttcaaatt tactcagttg atttttggtg 96960
ttgtgggttc atttagattt tatttatttg agataagatc gagggagcag gaggaggggt 97020

gggggtgtgg agggaaaagg aaaagcaggc tccccgctgg gcagggagcc caactcaggg 97080
ctgacatgga gcttgatccc atgaccctta aatcacaacc tgatccgaaa gcaaatgctt 97140
aacccactga cccatgcaga tgcccctgat ttttggtttt tgtctttttt tttaagattt 97200
tatttattca tgagagacac agagagaggg agagacagag acacaggcag agggagaagc 97260
aggctctatg caaggagcct gacgtgggac ttgatcctgg gactccagga tcacaccctg 97320
aaccaaaggc aggcactaaa ccacttagcc acccagggat ctccatgatt tttggttttt 97380
aacaagcttt aatcccctgg tcaccttgag gggaccgtcc ccaggagggt aaagttgggc 97440
cagtccagct cacctggtac ctaggcatat tcccacctta cagcccagga acaagctaca 97500
ctacaaatcc tgccagcatt cagctgagca aaaactgcaa ataagccctt cagtctaagg 97560
acatttaatt aacaacatgc agcaagcttt gagaggaaaa ccctatgcat caactgaaat 97620
tgcatgatta aaagtttgag tcaagcccat caaattacct gcaagagaag gcctctccag 97680
acagaagagg ggaccctcgt tcaccccagt cacatcccac tcatctacat ctgacaggtt 97740
tcctccctga caggataaac agattaataa atcatggaaa tgctcctgtc ttaatgcctt 97800
gatcttcaaa aacatggaca ccattttgcc tcggggccag gtccccattt ctcacccaca 97860
tgacagaact gaaattgggc ctctgatgat gtgcctctgt ggagagactg cagccaatgt 97920
gtccccaacg gcattgccac tcgacctgcc cccttcagtc cccgagcaga aagctgatgg 97980
cacggccagg gaagagcaga gcagagattc taacagtgcc ttccaaacaa tttttttaaa 98040
taaaccttta atagaatatc ttcaggaagg cagaaagata acacttcctg cagctcctac 98100
ctgctagctc agcctcccac ctgagggctt gggtggttcc tgtacacttc ccacctcaag 98160
actgaatgct ccaggcctgg ggaactctgc tacatgaaac tctgctgaga gatgcaaagt 98220
ctctagttcc agagtcagaa gcttcagtcc actgttaccc acaggaaaca acacgataga 98280
tccagcccca ctaggagact cgtccagccc cccatgtcag cgtgccaagg ctaaggagcc 98340
tgtccagagt gacagtgtaa acttcagccc tggctgacat ggggacacac tgtcttctcc 98400
aacctgctac agccagctcc tgaacagagc ctggcatgat gcatctgctg aacagatgcc 98460
cactgcttac tgggtgtctg ttggtgaaga acatattatt tagcacccac gtaaacctta 98520
tgacatgcgt agtgttctct ctcatgccca gatgagtgaa atcagagtca agagtttggg 98580
tcactggcct aaaaccccac aggaataagt gagagctcac ttccatccta catcaacccc 98640
tatatccaag gccagagcca atcccactcc aacctaaagg acacttcctt ggcttactca 98700
gaagagtaag caaacgcaaa taacccacag tatatattat tatgaataga caacacaagc 98760
tgacaaattt aaagctttct atcatccccc atggtagtta aatctccgta cgactaaggc 98820
ccagtgctag aaacatccag gaaagtgatc attcctccat atctggctcg gcgtcctggg 98880
ggtactttcc gtagagctgg agaggaaggg gccacggtga aggacactgc cctagggctc 98940
ctgccatggt cacatatgct tgggagctgc atagaggaca catgagaggc tggctattga 99000
gggagaacca gcacgtgttc aagaagtaag ggattaatca agtcacttgg aagagcttcc 99060

aggactttcc ttgggattac atggatattc cttaacagca tccactttta aagagggaat 99120
tacatggttc tcattactct aacaaataag aacatttgag aattgtttct catggtgtca 99180
cttaagggaa gcagaagcca cccaattagc tgtaccttga gctcaaacac ctgggttacc 99240
tactcctcta acaacacccg gggccaaggc aagctctggg taggtgcaag cctggtctgt 99300
gtggcggtgg tcagtggaga cccctaggac tcctgcccag tcactcccat aacataagag 99360
gaggaagaaa aatgagactg tcacaggaag cccgtgtgcg gttagttaag ggaaaatttt 99420
aaaggagaat tatgtccatc tcagaaactg cacggtactt tggcttccca gattattttt 99480
taaaagatac tttctgagag gctgaacaaa ttattaacca tctcagcaga agtgaaaggc 99540
aattcctctg cacgagaatg gtctggaagg acggcccaga actgcgggct gggagctgag 99600
aggctccagc aggcagcacc taggaggccg agcctgagag gggaggaatt catcattcac 99660
ggaaacatca gctcaagttc aaggcaaggt ctttctgcag gacagtgttt gccaagtaat 99720
aaaatcaggc ccacatatga cacctaacga ctcagtaaaa accctgagca caggagctgg 99780
gttcattaat ctacatttga ctggacagcc atggtgattc caaagatgca gcctgcccac 99840
ctccagtcag atctccccac gccactgctg gccagtaaga ccagaaaaca caaccaataa 99900
aatcagagag agaccttcac caaatttcag aatccccctg aaaaggggca cctggtattc 99960
acttccacgg gtatgcgggc agagcgcaca gaccctcctg tgcatcagtg ccccgtgtgc 100020
gtggaagtac ggatgtgcac ataagcttgc gtgcatgcgc cacacagggg ttttgctttc 100080
cttaactcaa ctcaagaaga acctatttcc ctgtgtgctg agcaccaagg aaaggagagt 100140
agtgctgggc tcggtgagga cgcagctgaa gggcgcggcc gaccggagct cattcctggg 100200
ccgccactgg cctctgctgc tcgccttggg catagcagct ggacggcagg gctccctgag 100260
caggtccggg ctctggcctc acctcccagc cgaagacact agggccagcc cccggcccta 100320
atgcacactc cacttcagca actccaagac tgaagggtgc agaattttcc agaaacatta 100380
aaccctgaag tcctcactaa cccagaatcc cctatcaccc tgcccagctc agcagaagac 100440
aaaatgtgag ggcagccgag gcaaagcagg gtggcacttg gcaccctgga cctgggactt 100500
ggccgtgagg gctcaggact gggagacacc tgaagtgttg tggtcccacg agtggcacgg 100560
cagccaaggt ctctgggggt agcaatgcta accctagaca agtccaggag gagcaggtct 100620
gctctgaggg ctgttttcta ccgtgactgc agttctagag ggtcaccata gccaagccac 100680
cctgggcaga ggacatgacc ctatgacagg gacgtcccac cactcaccat gggccaggga 100740
agggtgggct gaggggagga tgtagaagag gaactcattc caaaactaca accgcaaagc 100800
actcagtact gatttcagtg cacgcctccc tccctctacc cacaagactc ttccaaacgg 100860
aaagcccagg agcagacact ggtcagggga ggctgcagag caggtgcgct gccccaggca 100920
ctcccagccc cctaatgtgc tgggttactc ttcaaggaag gacatggaac accagcctct 100980
ggaggagagg ggtgggaatg gaatcacata aatgtgctgt gagccaacgc tcagcccaca 101040
gttaactccc tggtagggga cagccatgtc accatggcct tcatcattca cttggtaact 101100

gtcacaacac caggcttccc agggccagac tgtacccacg acaaagagaa atttgaacag 101160
ccatgccctt gagggccacc ctcaaaggca gcagaggctg agctgaagcc aggtgatcag 101220
ccacaccggg gccatgctgc tcctggtgaa ccctgacctg ctggatcaga ggcgtgaagg 101280
ccactcatca cctgtgctga tgaaaccctg tagggagtca ccgacaggcc cacaggtgac 101340
tcacactcgg cacggcaggc cagaggcctt tcagcagcac aacttctgtc cccaggacaa 101400
cattcctgac gtctaagggg taggagcacc ctccactacc ctgtgatggg agggctccca 101460
tcagaatcaa tccaagcccc aaccacaatc caaccataat ctggactctg ggtggcagcc 101520
aaatgctgag agcagcacat gctcagctta ctttgtttca cgtctgggcc gggtcagtcc 101580
ccagcagcaa agggctacct tcggctctgg ttacaggcag tgacacctcc aagaatggct 101640
atggtcacag ccacctccat tactcacagc actacctgcc cctcgagccc actctgccca 101700
tgagaaagat cacatcaacg gggcctcagc cctttccata cctgtgacca tcacccacat 101760
caatcaagca gaccaactgt gttgggaagt gtgccccagc gctgggctct gcccctggtg 101820
tttgcagcct gtgagcgtgc aagccctgct ccactcacag tgcctgtgct cacagacatc 101880
caaaggacta gtccctcttg ctgcaggtct ggcttcaatg ctcgctgcca gggaggaccc 101940
tccttctttg ccctcccacg tcaggtctcc ccgttcaccc ccagggacag ttctggtctc 102000
cttcagaggc tcaccacaat ttgcaatgac cctgtcacct gcctttgtgt atcacccctg 102060
ctatacacca tgagtgccac ggccatgcca cccaccatca ccactagacg ctcagtccaa 102120
gggctgagta gaggctcgta ttgggtgccg ccaaacaaag acaaaccatc tagctgcagc 102180
cagacacaac catgcgggca agaccatgtc accaagcaat gccaatgcag ccactcagga 102240
tggaaacaga aggcatgggt tcgagtccag gctctgccac ctgctacctg ggcaagccac 102300
cctccttcta agctgcaagt gctcatcttt aaaaacaaag aaaaaagcca caatgaaaga 102360
aggcagacaa ggagagtgag ggaaaacagg agaatcagat gagatcaata atttcctaac 102420
tttgttccaa gaaccccaag ggtccttgaa ggcgagaaga cctccaggag ccccctgtcc 102480
taccgcttcg accagagcca tgtgctctca tctgttcagg atgtttggcc tccacacaga 102540
ttttgtttag aaatgaagtg ccccagtgtt aaaacaagct ttgcaatgac ccatctgctg 102600
ctcagtggca tttctcgggt ctgagatgca ctggtcgtgt ccccaggtca taagccccac 102660
acacctgctc ttggccacac agctgccctg cctgccacca tggcccacct catcaaagat 102720
gatcctagac ggctgcctgg aaagctgctc agccggcctc accgcggtct tccatgtcct 102780
cccgaaaaag tgtcggtagg tgacatcaaa gagagacaca cgcagatggc agcccacctc 102840
cgaaagcacc tccagcactc cctaggagac agaagggtac aggtcctgaa gggttcttga 102900
catcctgagc catcccatgt gtgctgactg accagcttgg tagaattgga tcacttagca 102960
tcttttaggt gtcaatatcc ttggggggaa cttttctctc ccatggccag cggtcttcag 103020
gttatgcatg aaagtgcttg gccctcccgg ctacttagag cactgaagcc ccgaggcaca 103080
gacacgggca tatatgcccc tcctggggcc agctaattcc tatacagagt aaatgacttg 103140

ccctccagaa catctaggat gagcaaacca accaatccag agttcacact cccacctggc 103200
cctccatctg gctcttagac tcggggagac cactgagatt actcacacca gccatcctaa 103260
gcctggtcat ctgctcagcc tgccttgcac attcctcccc acagaaacaa caatgaaggc 103320
tttcagccac agtctctcca gctgctctgc ctcccgatcg accccatgcc accccgcgtg 103380
gccctatgtg gcctggtaca cccccacctc ttgggaatgg caataaacgg tcttctctag 103440
gttgctctca ctgggcctga acaaacaaaa tcccacacct gcctgtgtcc ctacagcagc 103500
tgtgagaggt agagggagaa gccaatgtgc catggaactc agcaggggtg cctcacaaag 103560
cacatgggtg tcaaatcccc gcactgcatg cttacatagc tctcaactat tttatccatt 103620
acacctccct ccagctacca catggaggtg actgggtgac accagtgatg ggccagatgt 103680
ggggaggtga ggaacaacag ctggaaggac ggggtggcca gctgcctcca ggtgcccagg 103740
cctcacctgg gcctagtgct gcaagtccta catccctagc gaaggatgat ggcatgtttt 103800
ctgccccggg ctgctgggaa tgaggcagtg ttgggtacag aggtgtcaga cagctgacag 103860
gtctggaaga ggcatctgag ccccgtttca atcacagagg tatgggggcc ccttgggact 103920
ttgcagacct ttctcagacc tctctcgaca cacgggctgc ctctctaccc atatgttttc 103980
tcaggaacct ccccagctca ctcccatgcc ttccctgagc actgccatct agacacctgc 104040
ccatcctctg accctggttt tttttttttt ttttttttaa gattttattt atttattatt 104100
tttttttaag attgtattta ttttttaaaa gattttattt attcatagac acacagaggc 104160
agagacacag gcagaggggg aagcaggctc ccctacaaga gcctgatgcg ggacttgatc 104220
ccagaatgac aatctgagtc gaaggcagat cctcaaccac tgagccacct ggttaaactg 104280
tggtttctgg ggccaatttc tttcagggat gtttgctatc atctaaagtt gtcaattctc 104340
aaagtgacta aactcgtaca aacattgtag gactaaactt tttcatgtgt ttcctacttg 104400
gtcttttttt tttttttttt tttgacttgg ttttccttta aatgctatct tttcactgat 104460
tttgtacgta cagctgaccc ttgaagaaca caggtttgaa ttgcacaggt ccacttctat 104520
gtggtttcat tctgttttga tgatgttttg aggatgatgg tacagaaccg tagatgtgtt 104580
ttctcttcct tacaccttaa caggattttc tttcctatag ctgactttat tataagaaca 104640
taggatatga tacatacagc ataaaaaaat acatattagt caactgttta cattcccagg 104700
aaggattctg gaataatagt aggctattat ttgttaagtt ttgggggagt caaaagttat 104760
gtgtggattt ttgactgcgt tgcggggtca gtgcccctaa cctcacattg ttcaagagtc 104820
aacaataaaa cttgtaaaat ttagggttcc tgtgttaaat cccatctgat tgtgaggtac 104880
tatcctttac gtatatcact agatctgatg tcctcatagt ttaagaactt ttgtgttcat 104940
attcatgagg gatgtctgta ctttcttgtg atatccttgc ccaaactggt tatcagttat 105000
ggcagcctca aaataacagc tgggaagcat tctctcctct gttttctgaa tgagtttatc 105060
aaaaattcaa attaattaat cttcctctta tactttataa aatccaacaa tgaagtcatc 105120
aaagtctgga gtttctttgt aggaagctgt ttaattatga attcatttat ttaacaaata 105180

taggactttt tatattttct atttctttta gtgtcacttt tggcaagtcg agcttttcaa 105240
gaaatgtgtc tatctcaact aagttatcaa atttgttggc acacaagtta ttcatatccc 105300
tcgacttttt aacatctgta ggatatatag caatgctcct tctttccttc ctaatattgg 105360
caatctgtgt gttttcttgc tcttttttca tgatcagtct tgtaggagtt tatcaatctt 105420
attcatcttt tcaaagaacc aacttctggc tttgctgatt ttctctatta tttgcccttt 105480
ctttactgat ttctgctaaa tgagcagaaa tctaccattt tcttcttcta ttctctttgg 105540
gtttaacttg ttcttctcat gtgatgctta aagaggacat agaggtcatc gaatttataa 105600
ctttcttctc ctcttacata gtcatataaa gatgtaaatt tccctacaag tactaaacta 105660
ctcacacccc ataaattttg gtctgttctt ccattactca gttcaaatta ttttctaatt 105720
tccctatgaa tccttctttg acccaaggat tagttgttag tatgctgttt aatttcctcc 105780
tgtttgagga ttttctaggt atcttcttgt tcatttctaa tttaacatca tacaagtcag 105840
tgaacatata tatactcttt ataaattcca atccttttaa atccattgag acttattttc 105900
atggcccagc atatggtcta tcttggtgaa tgtcccacat gcacttgaga aaaacgcgta 105960
ttctatttgt tggttatagt gttttagaaa tgacagtcag gtcagagtgg tggatggtgt 106020
ttcaatcttc caaatcctta tgagtgttct ggttcatttg ttctatcaat tattgagaga 106080
ggagggctaa aagtctccaa ttgtgatggt gaatatgaat ggagctccct ttagtcctac 106140
acaattttga ttcacgtgtt tggaagctgt gtcgctgggt gctgcgcaca aaggcgcaat 106200
cacagcttgc ccaaccatta tgccaaacta ttgccaaact attgtgcttt gaaaggccag 106260
tataggggca cctgggtggc tcaattggtt aagtgtctgc catcagctta ggtcatgatc 106320
tcagtgtcct gggatcaaga gccgcattgg actccctgct cagtggggac tcggcttctc 106380
cttcttcctc tacccctccc ccctccactc acacttgctc tctctcagat aaataaataa 106440
ataaaatctc ttaaaaaaaa aaaaaaaaaa gccagtatag gaggctggcc cttggatgga 106500
ggctgggcac ttagcattca ggagggttcc catcaccata ctgacaacgg caccctgtgc 106560
ctaaactgta tacagtggtt tacgctaaac acctcctttc ttgctgggag tgtggaatct 106620
ggaaacatgc taggcagagg atacccgcct gaccagtcct caaacactgt ggccactgag 106680
tgtctaacga gcttccttgc tgggaacatt tcatacgtgt tgtcatgact cgttaccgag 106740
ggaactgaat gtgtcctgtg aggacccttg aagcacatgc ctagcccccc cacactttcc 106800
cccatatgcc tttcctattg ctgatcttac tctatatcct ttcactgcag taaacctttt 106860
ctatgaggct gattatatgc tgacacatgt gaagtcttcc caaggaatca ctgagtctgg 106920
ggggtggtct cacaggtctc aacagaggta catgcacatt tagaactgtc ctatcttcct 106980
gactgcatca ttatgaaatg ccccttcatc tctggtagta ctcctcacct tgaaactgac 107040
tgtctgacat cacagcacaa aggcttctta caaggtctac atggcctgta ttttctccat 107100
cctttaactt tcataggtac agttcatttc tttctgctta aagtgtgttt ttttataagc 107160
aacctataaa tggtcttaac aataatttga aacacgtaac tctgagtcag gaactccatt 107220

tggaggaagt attccacaga catatatgta catatgtgta aatgaagtta atcactgtag 107280
ctgtgtctgt attgcaaatg actgagaaaa aatctacatg ttcatcaata aaggtctgga 107340
taaatacagg caggttcatc cagagaaata ctatgttgtc ttaagagaga ttgttcatgt 107400
cattacatga acaatctata ggacatatta ctaagaatat tttttaagca aaataataca 107460
ggtatattta cctgctgtgt ttgcttagag tttccagaaa gttacacaaa aaagcaggtc 107520
acactagctg agtctagggt gggacactgc tgccttaggg attctgtgta attatacact 107580
ctcctcacgt accctagtga attctgaaca ctgataatta ccttttcaaa agtaaataaa 107640
tacaaataaa caaaataaaa taggaaaata aataaaattt ccttcctaaa attcctaaga 107700
gagaggacag cactgtccac tactctgtga gctcctatta ggtggaagtc actggcgctg 107760
ggtgaccagg caggagccca gctggccacc tggattgggg caccccacgc acccggctgc 107820
agtcccctca ggctgtttcc cgctctgtga aatccagtag cgacggaccc cacttcagag 107880
ggctgctgca aggagcaggc caactggaac aggaaagtgt tgggctacta gtaaggctgg 107940
aagagcagca gccaccatgg ggcaagcaca gtggccacac acagcagtcg ctggattaga 108000
gggcttacaa gcgaatgcta tagggtgtga gtgatatcaa agaagtgagc tcttacagat 108060
ccaggccccc aacaactaaa tacatggaga atcacttgtc tatctgcaga ggagaacata 108120
gccttgccaa gccaaaccct atgaaagtca catgttcaca ctggcaggtc tggatccttc 108180
tcatgcgtcc cactgggtgt cccactgggt ctcactagaa ccatccaatt acatgggtgc 108240
tctgtgggca gcgcagaagt accagcttct ctagcagttt tcctgcccct gaaggacaca 108300
gatgaagcca gagccacaga gcaagggcct gagtgtgcag gacgctatgg cacatccagg 108360
cacacagctt cagccagagc tctgcccttg tggttctaac tgccaggctg caagcaggct 108420
gcttgagtcg tgcatgagcc cacctcctgc aaaccaccca gctcagaaat aatgcaggaa 108480
atcatattac ctggatttta aaatggcctc actgtgggca gaaagaaacc ttcacccagt 108540
gtcaaaagtg ctgaccgagt tgctgaatca cgcatgtgtt ctctttgctg atgagccact 108600
ttgcctccaa ataatgccaa ggcccaaggt aatgggtatg gtggtaagac tcaaataaat 108660
aagtgaaaat caataccctc tcagctgaga gcaggagcaa catatatagg aaaataaata 108720
gcaaatttct atacctgctt aattagtggc ccgtcaagcc acttcaggac acactgaaat 108780
gctgttgatt cctttaaaag ctggcgtgct ctctgtgggt ggggagggac cagcacgttt 108840
tggtcaaaaa tcctgagcca gtcattcatt ctgccagtcc cgagggtcac ttcggcctct 108900
aggagtacct gcacaatttg tgttcaaata aataaaggag ttacgaaggc catagtgcac 108960
agagcaattc tgaaagtttc ctacctcccc ttgcatccca gaggaaccac catccccaaa 109020
acccacactt tgcaattcct cccctgcatc cccagtgcag cacactggta tctgtgccac 109080
cttcccctgg taaacacacc catcttgctc cagatcatct ctctacccct gctttcagga 109140
gccaactaca gcatgaccat gctggcctca gtgactagtg cagaaatagg tatatgcagg 109200
gctgctttgt acagctgcag aggttgagca ctacatgaca gtgccaggcc ttggggactg 109260

gggctatctt ggaagggact aggaagcatg acccaatcag agccaaggag atgcaaatct 109320
cacagtttct gctggctctg ctgagggaag ggaatactct tctgcagccc tcgaaagaga 109380
ggtcatcagt atgaagctgt ccggaaccac cgtgggagca gcttccttga gacacacatg 109440
aaaaaagcaa gcagagcaaa agcacaagca agggtcagtg gatctaggtg tcacagttag 109500
agccctggcc tgaggagacc ctaaagcacc ccaggactgc tcagctacat taatacagtc 109560
tctcgttgcc aaagctggtt tggaacagat tttgagtgtt atgggctttt tggtgacctg 109620
gctcactgag gacatcatcc accttcccac cagatagaga tctgtacctt ctctagcctc 109680
ttttcggctg acatacaatc ttaagatctt ggctttgata ggaggctgaa ttctgcacac 109740
taaaatgaca agcaagataa ggcagcagat ttggtctcct aaaacagagg tactttaaat 109800
gggttcccca gactctgcgg ttccccggag ttgtcaaaag ctcaatgtca aagctatttt 109860
cataatattc agatgttatt tgcctttttc accgtgtcga tgtttgggct gatgaagcaa 109920
agggaagagt gggcacactg gcctcagtgc accgaggcaa acctctggcc gtcgtgcatt 109980
cttcaccact cacagcaagg gcagagtccc cagtgaagct gcaaaaacta tgaattttat 110040
tacatcttga tccttgagac tggcattttt aatactctgt gatgagaagg gaaagacagg 110100
gtagccctgg tggcgcagtg gtttagcgcc gcctgcagcc cagggcgtga tcctggagcc 110160
ccgggatcaa gtcccacgtc gggctctctg catggagcct gcttctccct ctgcctgtgt 110220
ctctgcctct ctctctagct gtgtctctat gaataaataa acttataaaa aaaaaaaaaa 110280
gaaagaaaag aaaaaaagag aagggaaaga cacagcaagc attgctgctg caaaatgaag 110340
ggtactggtc atctcagggt gaggctgctg tgagcctctg tggcacactc aggacccact 110400
gctcctgttg tctggtgatg ctatcttttt tttttttttt tttaagattt tatttgagaa 110460
agagagagag agagagagag agcatgagca agggaagggg cagagaaaga agcagacgcc 110520
ccacagagca gggagtccga ttcaggactc aatcccagga ccctggaatc atgacctgaa 110580
ccaaaggcag acacttgact gagccaccca ggtgcccctg gcaataccat ctttactaaa 110640
agagtagctg agagacagac tgagcttact caaactcggg cactttgcag tctctcaaac 110700
gtgaactaaa tgagtctgtc actttcaagg aaaactggta ataagaattg ggctttcagg 110760
caaaaatcag aattgggaaa atctgtatcc tccactgtga ttctgacagc ttcctcctcc 110820
ttaaagacgt gcttgacaag atggatggtg actgtgacaa aagtgatctt ctgaaactac 110880
gattaaatgc acactatttg gaagatcagc ataccttaga agcagtgttc tccaagtgag 110940
cagtgcatca tgatataaaa aagcctgcat gagtaaaaga tccatccaca gtgcaggaca 111000
gggcagccct ggtggtgcag cggtttggcg ccgcctgcag cctggggtgt gatcctggag 111060
acccggaatc gagtcccaca tcgggttccc tgagtggagc ctgcttctcc ctctccctgt 111120
gtctctgcct ccctctgtgt gtctatgaat aaataaataa aatctttaaa aaaaaaaaaa 111180
aagtgcagga cagaccaaag gactttaaag taccggagca cgcaaacttc actggcaggg 111240
tgccaaactc cacactgcaa caaactttta agaaactacc actcgcagtg gcgctggcgt 111300

tgcatttgga tgaagaatca aaggagaatg ccacaattac ctgagactct gctcccttct 111360
ccgcatgagg ctgtttcttt caaatacttc aaccaaaaca actttgctca acaggttaaa 111420
aacagaagcc taagggcgcc tggatggctt agatgagtat cagactcttg atttcagctt 111480
aggtcacaat ctcagggttg cactcagtgg ggaatctgct tgagagtatc tccctcttcc 111540
tctccctctg cccctcccat tgtgagtgct ctcaaataaa taaatcttaa aaacaaaaag 111600
aaagaagcct atatgagacc cagctgtcca ttaagccaga cattacagaa atgtgcaaaa 111660
atgcaaaacc accccttttc ctgctaattt ttttattctg gaaaacagct ggttttcata 111720
aaaaatacat tttatgctaa catgtaacag gtctattgtt atctctacat gagcaataac 111780
attgctaaca tctctcagtt ttcatttcca atacagttaa agctggtaga cataataatt 111840
aaagtaaaca aaaggtctct ggagtcctcg gcaatttttt aaagggtaaa aggtacgaga 111900
ccacaaagtg tgagaaaggc tattctaaca ggacccaaaa gatgagggct ttgtacagcc 111960
aggccaccct ccacctttgc aggctgtgtg gggagcggag aggatccccc agagcacgga 112020
catggctggc gtgaaaagct ctcccacacc cagcatgccc aggcacaaag gcccagcacc 112080
cacaccccac agcccctgca ggcaggcatg cagtgcagca tccagatcta gactgtcacc 112140
atgtgagcag tgaggcacac atgcattgcg ttttctgttc tgagcagtta cgtctgctca 112200
gtatcccctt gttttaagga atgaccctac aatccagtgt gggaaggctg tccacaaatc 112260
taggcagagc actgtagtga tgggctcaag atgaacacgt gacccacact gtgccaatca 112320
tcatctcccc caggactcca gccgcggttc cctctgaaga ggctgtctct gctgagaggg 112380
caggagctgc tgggccagcc tgaattccag gggctaccct gccacttcag ggagagcctg 112440
aggtcaaagg gtgtcctgcc cacagagccc agacccctag ggggagagag cctgatggaa 112500
ggcagccccc actcccatag cagccgaggt gtagcagcca agaaaagccc atggaggctt 112560
tccctggtgg ctaaatggcc cccaaagaag tcccggtcct catccccagg atctgtgaat 112620
atgggacttc gcatggcaaa agggactctg ggagtgtgat taagtgaagg gtcttaagga 112680
ggggaatgga tgacaggagc cacctagatc cacctggatg acacaggtgg gcctgatgta 112740
gtcacaagga tcctcagaag agggaggcag gagggtcaca gaacaggtcg agtgaccacg 112800
aaacagacag agaagggagg gagggagggg tcacaggcca atgaccagct agaaaaggca 112860
aggaaggctt ctcccctgga gctttcataa ggaacacagc cctgcccaca ccttgatctg 112920
agcccagtga tattgaattt ggacctctga cctccagacc tggaagaggg taactgtgtt 112980
gtctcaagct gcccagattg tggtatcatt acaaaagcca caggcactca cacaggtgtc 113040
taccactagt aagcagcaga ggcctgatac ccagcaggag ggccacatgc ccaggagagc 113100
ttcttggagg ccctgggggc cagtgactgg gcccacctgg cccctgtgct tcccatggtt 113160
gcagcactcg ccacctgtcc tgtgagggtc tgcctgctgg ccaatcactt gcacgagggg 113220
atctattttg ttgtgtcctt ggtacctggc tcaccatctg aactcaacaa attttcataa 113280
aatcaaagtc tcaataaaat tgactcccaa gcatttttta aagagaaatt tttgggatcc 113340

ctgggtggcg cagcggttta gcgcctgcct ttggcccagg gcgcgatcct ggagatccgg 113400
gatcgagtcc cacatcgggc acctggtgca tggaggctgc ttctccctct gcctatgtct 113460
ctgcctctct ctctctctgt gtgtgtgtga ctatcataaa taaatttttt taaaaaattt 113520
taaaaaattt taaagagaaa tttttaaaag aaatgttttc tcattgactt tcattaaaaa 113580
aaaaaggggg ggggtgttaa ttcatacaaa gtgagtgtca ctacggattg aaagtccctg 113640
tcaggaagtc cccctgccta gggagcaccg tagccaagca tcacctctga aaggggcctc 113700
tgtgcaaggc ccgcactgtt tccccggctc caggactggg tctcttttgc aggcccttcc 113760
agactttgct cattgggccc agattcctga gccagcacct acctccccac cccctggcta 113820
ccacacttgg ccagcagatg ctgaaggacg ctcctgttct agtccaccta gcagaggaag 113880
catgtccaca ggactgcccc aggctgtacc cagtccccat aggatgcacg ctgggaagat 113940
aggcctccac tctgaatggg acagtcaccc aaccaaaaca gtgagccagg cagatggagg 114000
cgggtagcaa cagtctgagt gggcgataga gaagagggca cccgccacat aggctcctgg 114060
gagtgggaga cctagtgggc acagagcatt tccctcccaa cctcccaacc cccacctaac 114120
actctgtcct ctctgtggcc actgtgcccc cgctcggccc ctgctccaat tcctcctgga 114180
caccatgccc cctaggatcc tgtcacagaa agtcaccaca attgctcagg ctccccattt 114240
ctgttgtcct gcgagaatga gctcagccag gtcagggcta gatcctcagt gaaaatcttt 114300
tgatgactac gtaaataatt aaattatgct gtgaaggagc taagactgct acagagagag 114360
agcagaaggc ctgtagggct agatgaacag ggagaaaaca gtgaggcccc atgcccaaca 114420
caaagggctc tccaggggag aaacaacatg agaaaacaca ccagctccca cagagagcct 114480
gctgcgagct gggtgggcac tcacagaccc attcatgtgt attagtccaa caaatgtgca 114540
gataagtatc acagacgagg acaaggagac taaggctaaa cagccaggcc aaagccacat 114600
ggctgctcat gaccaggctc aaagccagga gaggctccgg agcctacagc atgacactgg 114660
ctggagcacc caggctaagc cccactctcc tcagacttca gggaagtctc ctgccctccc 114720
tgaatctgct cccacctaca ggatgagagg ggactgtgag gtctcccaga gggctctgct 114780
ctgcacacct cttctgttcc actgtgtgca atggtgaaca atgccaaagg ccaggtcttt 114840
aagaatctaa aaatccagtg gggaaaagac aagggttcga gtgtgagcag cacacagaag 114900
acaagcataa ttcagtctcc tttttgggag gatgcggggc agagagaaga ggaaatcccg 114960
gcactgaaga agcaagctgg gataaaatct caacaacagt ggaacaccag agaaagaggg 115020
gggcaggagc agctgtgcag gcagggggat gtttccttcc aagaagtgaa caagatctgg 115080
gatgcctgag accgaaaagc tttgggaact ttactgggag taagtggcag ttatctcaac 115140
cccgcctcga agtttttggg tctctcccac cccccccccc cccccccctc cgagcaagca 115200
atcttctttg cacacctacc aggtgccagg acctggagaa accatcctca acatagcaga 115260
caagctccct gccctcaggg aatggatct 115289

<210> 4
<211> 4819
<212> DNA
<213> Canis familiaris

<400> 4
agatccattc cctgagggca gggagcttgt ctgctatgtt gaggatggtt tctccaggcc 60

ctggcacctg gtaatcctag aggccgaagt gaccctcggg actggcagaa tgaatgactg 120

gctcaggatt tttgaccaaa acgtgctggt ccctccccac ccacagagag cacgccagct 180

tttaaaggaa tcaacagcat ttcagtgtgt cctgaagtgg cttgacgggc cactaattaa 240

gcagggagtg ctggaggtgc tttcggaggt gggctgccat ctgcgtgtgt ctctctttga 300

tgtcacctac cgacactttt tcgggaggac atggaagacc gcggtgaggc cggctgagca 360

gctttccagg cagccgtcta ggatcatctt tgatgagccc ttgtactttc acacgtcctt 420

gaaccaccct aacgtggtgg ctgtggttga ggtggtcacc gaaggccgga agcaggatgg 480

ctctctccag gctctgtcat gtggctttgg aattcttcgg atctttggca acaaactgga 540

atctcctacc tctgcctccc aggacaagcg gttgaggctg tatcacggca ctccccgagc 600

cctcctgcac ccactgctcc aggacccttt cgaacaaaat aaacacatga ccctcattga 660

gagctgcagc ctgcaataca ctctgaagcc acacccgctc ctggagcccg tgttccatct 720

tcttccccag aaccttctgg tgtctggtct gcagcacatc cctggcctcg ttccagctca 780

tggagaaaag ggggacaccc tccgaaagcc tcgccttcag aagtctgtca catggtactt 840

ggatgattta ttcttcacgc tatatccttc ccttgagaag tttgaggaag agcttctgga 900

actcctcatc agtgataact tccgggaggg ggacagtcca ctagatggca gcacgctgga 960

gatcttggag cgccgcctgt gtgtgggcgt gcacaatggt ctgggcttcg tgcagaggcc 1020

acacgtggtg gtgctggtgc ccgagatgga tgtggccttg acgcgctctg ccagtttcag 1080

caggaaagtt ggctcctctt cgaagaccag ctctggaaac caggctctag ttttgagaag 1140

ccgcctccga ctccctgaga tggtccatca ccctgcgttt gccatagtct ttcaactgga 1200

gtacgtgttc accagccctt ctggagtgga cagcaaggca gcctcggtca cctctttgtc 1260

cagtgtggcg tgtatgcaca tagttcgctg ggctgtttgg aaccccttgc tggaatccgg 1320

ctctgggaag gtgatcttac ctctgcaggg tgggatccgg cccaacccct cacactgttt 1380

ggtctataaa gtaacccctg ccagcatgag ctctgaagag gtgcagcaag tggagtcagg 1440

gactgtccag ttccacttct cgctgagccc tgagggacac ctggacacac ccacggggct 1500

ggtccacagc cctgaggcgg agcggcagcc ctccaggaag ccacccacat ctcctgcaaa 1560

cctgccagca ctgccgcccc aggggctcgc tgccctccag gactcgccgg tgggaccagg 1620

gttgtccctt tcccagctcg cagcttcccc acagcctacc acccgctccc gctcagccac 1680

accccctcca cggcaacccg acagctctaa gcccccgccg gcccaggagg agcccccctc 1740

ggagggcagg atctctcacc tggaggccca gctgagccag gcgtccttgg tcctgggagc 1800

ctctgttgct gaacagctgc aggagctgcc ttttacgccg gtgcacaccc cgattgttgt 1860

gggagcccag accaggagct ctggaagtaa gctctcacga gcctcaatgg tgctcctgca 1920
agcctgcggc tttcctgaga ttctggatgc caataagcaa ccagccgagg ctgtaaatcc 1980
tacagatccc gtgaaattca acccccaaaa ggaagaatca gattgtctac aaagcaatga 2040
gatagtgctg cattttcttg cctttagcag agtgtcccag gactgccagg gagcaccgtg 2100
gccaaagacc gtgtatttca ccttccagtt ctatcgcttc ccacccgtga caacgccacg 2160
cttgcagctg atcaggctgg acgagacggg caggacaggc tctggttccc tgttgtatct 2220
cctggttctc actgacaaag atggctcctc tgatgctggg acacctggct ttcaactcaa 2280
gtacctggtg gaacctgggt tcctgaaacc cggagaacag cgctggttca cccactacat 2340
ggctgctcag accctccaga tcgacatctg ggatggagac tccttgctgc tcgtgggatc 2400
tgcggccgtc cagatgaagc atctcctccg ccaaggccgg ccggctgtgc aggtcctgca 2460
tgagcttgag gtcgtggcga ctgaatatga acaggatgtg gtggtgagtg gagatgtgac 2520
cagatttggc tgtgtcaagc ccatcggtgt ctacacagtg gtaaagggct ggctgcacct 2580
gacgttggcc aacgtgggtc atgtgtgtga gcagagagtg agagattcca gctcattgcc 2640
accttccagg tcacgggtca tttcgaatga tggagccaga cacttcgagg gaggcagtct 2700
catctcgcgc gggggtccga gatcagtaaa aaatgttgtc caagcacaga agctggctga 2760
cgtggaccgt gagctggccg ccatgctgtt cacccacatg caacatggga gcaaggggcc 2820
ccaaggtgct tgccgtgagg gggatgccgt ccgcaggcgc aagctggagc ggatgaggtc 2880
cgtgcgcctt caagagtcca gaggagagct cagctgctgg ggggccagca tgttgattca 2940
gcagaatatc cgcgcacagc acgcacgtga cctgcaggtc attgctgcct atcgggagcg 3000
tacgaaggca gagagcatca ccagtgcgct gagcctggcc atcaccacga tgcacacaat 3060
ccacgtcaca ctgggcactg ccgagttctt cgagtttgcc cttaggaacc cccacaacac 3120
acagcacaca gtgaccattg aggtggacaa tcctgagctc agcatcatcc aggacagtca 3180
ggagtggaga tacttcaaag acgcgaccaa gctgcacacg ccggtggagg aggacatgtt 3240
ccatctgcgg ggcagccttg ccccgcagat cttcctgcgc ccacgagaga ccgctcacat 3300
ccccttcaaa taccagacct tctctgtagg gcaactggcc ctggcgcagc cctctgctga 3360
gctcggatct gagaaggaca cggataccgg attgccccag aaatccagtg caatgcccac 3420
taagcatgcc aaggtcttgt tccgggggag tggcggtaag cccatcgccg tgctttgcct 3480
aaccgtggag acccagcccc atgtggtgga ccaggttttc cgcttctacc acccagagct 3540
caccttcctg aagaaggcca tccgcctgcc tccctggcac acgcttccag gtgctcccgt 3600
gggaagacct ggtgaggagc ccccagtcta tgtccgatgt agcgacccca acgtcatctg 3660
cgagacccag aatgtgggcc ccagggagcc tcgggacgtg ttcctgaagg tggccagcgg 3720
tccaagccca gagagcagag acttctttgt cgccgtttac gcggatcgtt ggctggcgac 3780
acccatccaa atatggcagg tctacctcca ctccttgcag cgtgtggacg tctcctgtgt 3840
cacaggccag cggacacgcc tatcccttgt ccttcgaggg acacggacgg ttaggagagt 3900

gcaggctttc acctcacacc ctcaggagct gaagactgac cccaaaggtg tgttcgtgct 3960

gccaccacat ggggtgcagg acctgcatgt cggtgtgagg ccccgcaggg cagggagccg 4020

gttcatccac ctcaacctgg tcgacgtgga ctaccaccag ctggtggcct catggctcgt 4080

gtgcctctcc tgccgcccgc ctctcatctc caaggctttc gagatcacac tggctgcagg 4140

gaaaggcaga ggcgccaaca agcggatcac ctacaccaac ccttacccct cccagaggac 4200

ctatcacctg tacagcgacc accctgacct gctgcagttc agagaggact ccttccagat 4260

tgggggagga gagacctaca ccatcggcct gtggttcgca cctcggcaga gccccgggga 4320

ggaggagatt ctgatttaca tcaatgacca cgaagacaaa aatgaagaag ccttctgcgt 4380

caaggtcgtg taccagtaaa agagcagccg gaggtgctgg tggggcttcc tgtcctgggg 4440

gctctgtagc cttttgtccc ctcgatgacc tgctggggct gctggtgagc atggccgagg 4500

gtggtgtgcg cgccgtgccc ctgcccagtc ctcctgccca agtaagcgct gctgtcacca 4560

gctgtgccac gagccccgcg ggcgtcctca gggtacgagg ctcgtttctt ctgctgcact 4620

ctggctgttg tcactgtcta tgacatgttg atttactttt taggaatata tattttatga 4680

atcatcttaa gtgataaaat tatgtattct tactggattt tgtacaaaca ttatttgcta 4740

tgcaatattt tatattggtg tcctttcata tcttttttta agttacgtat gaaatactaa 4800

acttttacct gttttctct 4819

<210> 5
<211> 4754
<212> DNA
<213> Canis familiaris

<400> 5
agatccattc cctgagggca gggagcttgt ctgctatgtt gaggatggtt tctccaggcc 60

ctggcacctg gtaatcctag aggccgaagt gaccctcggg actggcagaa tgaatgactg 120

gctcaggatt tttgaccaaa acatgctggt ccctccccac ccacagagag cacgccagct 180

tttaaaggaa tcaacagcat ttcagtgtgt cctgaagtgg cttgacgggc cactaattaa 240

gcagggagtg ctggaggtgc tttcggaggt gggctgccat ctgcgtgtgt ctctctttga 300

tgtcacctac cgacactttt tcgggaggac atggaagacc gcggtgaggc cggctgagca 360

gctttccagg cagccgtcta ggatcatctt tgatgagccc ttgtactttc acacgtcctt 420

gaaccaccct aacgtggtgg ctgtggttga ggtggtcacc gaaggccgga agcaggatgg 480

ctctctccag gctctgtcat gtggctttgg aattcttcgg atctttggca acaaactgga 540

atctcctacc tctgcctccc aggacaagcg aaaataaaca catgaccctc attgagagct 600

gcagcctgca atacactctg aagccacacc cgctcctgga gcccgtgttc catcttcttc 660

cccagaacct tctggtgtct ggtctgcagc acatccctgg cctcgttcca gctcatggag 720

aaaaggggga caccctccga aagcctcgcc ttcagaagtc tgtcacatgg tacttggatg 780

atttattctt cacgctatat ccttcccttg agaagtttga ggaagagctt ctggaactcc 840

tcatcagtga taacttccgg gagggggaca gtccactaga tggcagcacg ctggagatct 900

tggagcgccg cctgtgtgtg ggcgtgcaca atggtctggg cttcgtgcag aggccacacg 960
tggtggtgct ggtgcccgag atggatgtgg ccttgacgcg ctctgccagt ttcagcagga 1020
aagttggctc ctcttcgaag accagctctg gaaaccaggc tctagttttg agaagccgcc 1080
tccgactccc tgagatggtc catcaccctg cgtttgccat agtctttcaa ctggagtacg 1140
tgttcaccag cccttctgga gtggacagca aggcagcctc ggtcacctct ttgtccagtg 1200
tggcgtgtat gcacatagtt cgctgggctg tttggaaccc cttgctggaa tccggctctg 1260
ggaaggtgat cttacctctg cagggtggga tccggcccaa cccctcacac tgtttggtct 1320
ataaagtaac ccctgccagc atgagctctg aagaggtgca gcaagtggag tcagggactg 1380
tccagttcca cttctcgctg agccctgagg gacacctgga cacacccacg gggctggtcc 1440
acagccctga ggcggagcgg cagccctcca ggaagccacc cacatctcct gcaaacctgc 1500
cagcactgcc gccccagggg ctcgctgccc tccaggactc gccggtggga ccagggttgt 1560
ccctttccca gctcgcagct tccccacagc ctaccacccg ctcccgctca gccacacccc 1620
ctccacggca acccgacagc tctaagcccc cgccggccca ggaggagctc ccctcggagg 1680
gcaggatctc tcacctggag gcccagctga gccaggcgtc cttggtcctg ggagcctctg 1740
ttgctgaaca gctgcaggag ctgcctttta cgccggtgca caccccgatt gttgtgggag 1800
cccagaccag gagctctgga agtaagctct cacgagcctc aatggtgctc ctgcaagcct 1860
gcggctttcc tgagattctg gatgccaata agcaaccagc cgaggctgta aatcctacag 1920
atcccgtgaa attcaacccc caaaaggaag aatcagattg tctacaaagc aatgagatag 1980
tgctgcattt tcttgccttt agcagagtgt cccaggactg ccagggagca ccgtggccaa 2040
agaccgtgta tttcaccttc cagttctatc gcttcccacc cgtgacaacg ccacgcttgc 2100
agctgatcag gctggacgag acgggcagga caggctctgg ttccctgttg tatctcctgg 2160
ttctcactga caaagatggc tcctctgatg ctgggacacc tggctttcaa ctcaagtacc 2220
tggtggaacc tgggttcctg aaacccggag aacagcgctg gttcacccac tacatggctg 2280
ctcagaccct ccagatcgac atctgggatg gagactcctt gctgctcgtg ggatctgcgg 2340
ccgtccagat gaagcatctc ctccgccaag gccggccggc tgtgcaggtc ctgcatgagc 2400
ttgaggtcgt ggcgactgaa tatgaacagg atgtggtggt gagtggagat gtgaccagat 2460
ttggctgtgt caagcccatc ggtgtctaca cagtggtaaa gggctggctg cacctgacgt 2520
tggccaacgt gggtcatgtg tgtgagcaga gagtgagaga ttccagctca ttgccacctt 2580
ccaggtcacg ggtcatttcg aatgatggag ccagacactt cgagggaggc agtctcatct 2640
cgcgcggggg tccgagatca gtaaaaaatg ttgtccaagc acagaagctg gctgacgtgg 2700
accgtgagct ggccgccatg ctgttcaccc acatgcaaca tgggagcaag gggccccaag 2760
gtgcttgccg tgagggggat gccgtccgca ggcgcaagct ggagcggatg aggtccgtgc 2820
gccttcaaga gtccagagga gagctcagct gctggggggc cagcatgttg attcagcaga 2880
atatccgcgc acagcacgca cgtgacctgc aggtcattgc tgcctatcgg gagcgtacga 2940

aggcagagag catcaccagt gcgctgagcc tggccatcac cacgatgcac acaatccacg 3000
tcacactggg cactgccgag ttcttcgagt ttgcccttag gaacccccac aacacacagc 3060
acacagtgac cattgaggtg gacaatcctg agctcagcat catccaggac agtcaggagt 3120
ggagatactt caaagacgcg accaagctgc acacgccggt ggaggaggac atgttccatc 3180
tgcggggcag ccttgccccg cagatcttcc tgcgcccacg agagaccgct cacatcccct 3240
tcaaatacca gaccttctct gtagggcaac tggccctggc gcagccctct gctgagctcg 3300
gatctgagaa ggacacggat accggattgc cccagaaatc cagtgcaatg cccactaagc 3360
atgccaaggt cttgttccgg gggagtggcg gtaagcccat cgccgtgctt tgcctaaccg 3420
tggagaccca gccccatgtg gtggaccagg ttttccgctt ctaccaccca gagctcacct 3480
tcctgaagaa ggccatccgc ctgcctccct ggcacacgct tccaggtgct cccgtgggaa 3540
gacctggtga ggagccccca gtctatgtcc gatgtagcga ccccaacgtc atctgcgaga 3600
cccagaatgt gggccccagg gagcctcggg acgtgttcct gaaggtggcc agcggtccaa 3660
gcccagagag cagagacttc tttgtcgccg tttacgcgga tcgttggctg gcgacaccca 3720
tccaaatatg gcaggtctac ctccactcct tgcagcgtgt ggacgtctcc tgtgtcacag 3780
gccagcggac acgcctatcc cttgtccttc gagggacacg gacggttagg agagtgcagg 3840
ctttcacctc acaccctcag gagctgaaga ctgaccccaa aggtgtgttc gtgctgccac 3900
cacatggggt gcaggacctg catgtcggtg tgaggccccg cagggcaggg agccggttca 3960
tccacctcaa cctggtcgac gtggactacc accagctggt ggcctcatgg ctcgtgtgcc 4020
tctcctgccg cccgcctctc atctccaagg ctttcgagat cacactggct gcagggaaag 4080
gcagaggcgc caacaagcgg atcacctaca ccaaccctta cccctcccag aggacctatc 4140
acctgtacag cgaccaccct gacctgctgc agttcagaga ggactccttc cagattgggg 4200
gaggagagac ctacaccatc ggcctgtggt tcgcacctcg gcagagcccc ggggaggagg 4260
agattctgat ttacatcaat gaccacgaag acaaaaatga agaagccttc tgcgtcaagg 4320
tcgtgtacca gtaaaagagc agccggaggt gctggtgggg cttcctgtcc tgggggctct 4380
gtagcctttt gtcccctcga tgacctgctg gggctgctgg tgagcatggc cgagggtggt 4440
gtgcgcgccg tgcccctgcc cagtcctcct gcccaagtaa gcgctgctgt caccagctgt 4500
gccacgagcc ccgcgggcgt cctcagggta cgaggctcgt ttcttctgct gcactctggc 4560
tgttgtcact gtctatgaca tgttgattta ctttttagga atatatattt tatgaatcat 4620
cttaagtgat aaaattatgt attcttactg gattttgtac aaacattatt tgctatgcaa 4680
tattttatat tggtgtcctt tcatatcttt ttttaagtta cgtatgaaat actaaacttt 4740
tacctgtttt ctct 4754

<210> 6
<211> 1429
<212> PRT
<213> Canis familiaris

<400> 6

Met Asn Asp Trp Leu Arg Ile Phe Asp Gln Asn Val Leu Val Pro Pro
1               5                   10                  15

His Pro Gln Arg Ala Arg Gln Leu Leu Lys Glu Ser Thr Ala Phe Gln
            20                  25                  30

Cys Val Leu Lys Trp Leu Asp Gly Pro Leu Ile Lys Gln Gly Val Leu
        35                  40                  45

Glu Val Leu Ser Glu Val Gly Cys His Leu Arg Val Ser Leu Phe Asp
    50                  55                  60

Val Thr Tyr Arg His Phe Phe Gly Arg Thr Trp Lys Thr Ala Val Arg
65                  70                  75                  80

Pro Ala Glu Gln Leu Ser Arg Gln Pro Ser Arg Ile Ile Phe Asp Glu
                85                  90                  95

Pro Leu Tyr Phe His Thr Ser Leu Asn His Pro Asn Val Val Ala Val
            100                 105                 110

Val Glu Val Val Thr Glu Gly Arg Lys Gln Asp Gly Ser Leu Gln Ala
        115                 120                 125

Leu Ser Cys Gly Phe Gly Ile Leu Arg Ile Phe Gly Asn Lys Leu Glu
    130                 135                 140

Ser Pro Thr Ser Ala Ser Gln Asp Lys Arg Leu Arg Leu Tyr His Gly
145                 150                 155                 160

Thr Pro Arg Ala Leu Leu His Pro Leu Leu Gln Asp Pro Phe Glu Gln
                165                 170                 175

Asn Lys His Met Thr Leu Ile Glu Ser Cys Ser Leu Gln Tyr Thr Leu
            180                 185                 190

Lys Pro His Pro Leu Leu Glu Pro Val Phe His Leu Leu Pro Gln Asn
        195                 200                 205

Leu Leu Val Ser Gly Leu Gln His Ile Pro Gly Leu Val Pro Ala His
    210                 215                 220

Gly Glu Lys Gly Asp Thr Leu Arg Lys Pro Arg Leu Gln Lys Ser Val
225                 230                 235                 240

Thr Trp Tyr Leu Asp Asp Leu Phe Phe Thr Leu Tyr Pro Ser Leu Glu
                245                 250                 255

Lys Phe Glu Glu Glu Leu Leu Glu Leu Leu Ile Ser Asp Asn Phe Arg

            260                 265                 270

Glu Gly Asp Ser Pro Leu Asp Gly Ser Thr Leu Glu Ile Leu Glu Arg
        275                 280                 285

Arg Leu Cys Val Gly Val His Asn Gly Leu Gly Phe Val Gln Arg Pro
    290                 295                 300

His Val Val Val Leu Val Pro Glu Met Asp Val Ala Leu Thr Arg Ser
305                 310                 315                 320

Ala Ser Phe Ser Arg Lys Val Gly Ser Ser Ser Lys Thr Ser Ser Gly
                325                 330                 335

Asn Gln Ala Leu Val Leu Arg Ser Arg Leu Arg Leu Pro Glu Met Val
            340                 345                 350

His His Pro Ala Phe Ala Ile Val Phe Gln Leu Glu Tyr Val Phe Thr
        355                 360                 365

Ser Pro Ser Gly Val Asp Ser Lys Ala Ala Ser Val Thr Ser Leu Ser
    370                 375                 380

Ser Val Ala Cys Met His Ile Val Arg Trp Ala Val Trp Asn Pro Leu
385                 390                 395                 400

Leu Glu Ser Gly Ser Gly Lys Val Ile Leu Pro Leu Gln Gly Gly Ile
                405                 410                 415

Arg Pro Asn Pro Ser His Cys Leu Val Tyr Lys Val Thr Pro Ala Ser
            420                 425                 430

Met Ser Ser Glu Glu Val Gln Gln Val Glu Ser Gly Thr Val Gln Phe
        435                 440                 445

His Phe Ser Leu Ser Pro Glu Gly His Leu Asp Thr Pro Thr Gly Leu
    450                 455                 460

Val His Ser Pro Glu Ala Glu Arg Gln Pro Ser Arg Lys Pro Pro Thr
465                 470                 475                 480

Ser Pro Ala Asn Leu Pro Ala Leu Pro Pro Gln Gly Leu Ala Ala Leu
                485                 490                 495

Gln Asp Ser Pro Val Gly Pro Gly Leu Ser Leu Ser Gln Leu Ala Ala
            500                 505                 510

Ser Pro Gln Pro Thr Thr Arg Ser Arg Ser Ala Thr Pro Pro Pro Arg
        515                 520                 525

Gln Pro Asp Ser Ser Lys Pro Pro Pro Ala Gln Glu Glu Pro Pro Ser

530                 535                 540

Glu Gly Arg Ile Ser His Leu Glu Ala Gln Leu Ser Gln Ala Ser Leu
545                 550                 555                 560

Val Leu Gly Ala Ser Val Ala Glu Gln Leu Gln Glu Leu Pro Phe Thr
                565                 570                 575

Pro Val His Thr Pro Ile Val Val Gly Ala Gln Thr Arg Ser Ser Gly
            580                 585                 590

Ser Lys Leu Ser Arg Ala Ser Met Val Leu Leu Gln Ala Cys Gly Phe
        595                 600                 605

Pro Glu Ile Leu Asp Ala Asn Lys Gln Pro Ala Glu Ala Val Asn Pro
    610                 615                 620

Thr Asp Pro Val Lys Phe Asn Pro Gln Lys Glu Glu Ser Asp Cys Leu
625                 630                 635                 640

Gln Ser Asn Glu Ile Val Leu His Phe Leu Ala Phe Ser Arg Val Ser
                645                 650                 655

Gln Asp Cys Gln Gly Ala Pro Trp Pro Lys Thr Val Tyr Phe Thr Phe
            660                 665                 670

Gln Phe Tyr Arg Phe Pro Pro Val Thr Thr Pro Arg Leu Gln Leu Ile
        675                 680                 685

Arg Leu Asp Glu Thr Gly Arg Thr Gly Ser Gly Ser Leu Leu Tyr Leu
    690                 695                 700

Leu Val Leu Thr Asp Lys Asp Gly Ser Ser Asp Ala Gly Thr Pro Gly
705                 710                 715                 720

Phe Gln Leu Lys Tyr Leu Val Glu Pro Gly Phe Leu Lys Pro Gly Glu
                725                 730                 735

Gln Arg Trp Phe Thr His Tyr Met Ala Ala Gln Thr Leu Gln Ile Asp
            740                 745                 750

Ile Trp Asp Gly Asp Ser Leu Leu Leu Val Gly Ser Ala Ala Val Gln
        755                 760                 765

Met Lys His Leu Leu Arg Gln Gly Arg Pro Ala Val Gln Val Leu His
    770                 775                 780

Glu Leu Glu Val Val Ala Thr Glu Tyr Glu Gln Asp Val Val Val Ser
785                 790                 795                 800

Gly Asp Val Thr Arg Phe Gly Cys Val Lys Pro Ile Gly Val Tyr Thr

                805                 810                 815

Val Val Lys Gly Trp Leu His Leu Thr Leu Ala Asn Val Gly His Val
            820                 825                 830

Cys Glu Gln Arg Val Arg Asp Ser Ser Ser Leu Pro Pro Ser Arg Ser
        835                 840                 845

Arg Val Ile Ser Asn Asp Gly Ala Arg His Phe Glu Gly Gly Ser Leu
    850                 855                 860

Ile Ser Arg Gly Gly Pro Arg Ser Val Lys Asn Val Val Gln Ala Gln
865                 870                 875                 880

Lys Leu Ala Asp Val Asp Arg Glu Leu Ala Ala Met Leu Phe Thr His
                885                 890                 895

Met Gln His Gly Ser Lys Gly Pro Gln Gly Ala Cys Arg Glu Gly Asp
            900                 905                 910

Ala Val Arg Arg Arg Lys Leu Glu Arg Met Arg Ser Val Arg Leu Gln
        915                 920                 925

Glu Ser Arg Gly Glu Leu Ser Cys Trp Gly Ala Ser Met Leu Ile Gln
    930                 935                 940

Gln Asn Ile Arg Ala Gln His Ala Arg Asp Leu Gln Val Ile Ala Ala
945                 950                 955                 960

Tyr Arg Glu Arg Thr Lys Ala Glu Ser Ile Thr Ser Ala Leu Ser Leu
                965                 970                 975

Ala Ile Thr Thr Met His Thr Ile His Val Thr Leu Gly Thr Ala Glu
            980                 985                 990

Phe Phe Glu Phe Ala Leu Arg Asn Pro His Asn Thr Gln His Thr Val
        995                 1000                1005

Thr Ile Glu Val Asp Asn Pro Glu Leu Ser Ile Ile Gln Asp Ser
    1010                1015                1020

Gln Glu Trp Arg Tyr Phe Lys Asp Ala Thr Lys Leu His Thr Pro
    1025                1030                1035

Val Glu Glu Asp Met Phe His Leu Arg Gly Ser Leu Ala Pro Gln
    1040                1045                1050

Ile Phe Leu Arg Pro Arg Glu Thr Ala His Ile Pro Phe Lys Tyr
    1055                1060                1065

Gln Thr Phe Ser Val Gly Gln Leu Ala Leu Ala Gln Pro Ser Ala

1070                    1075                    1080

Glu Leu Gly Ser Glu Lys Asp Thr Asp Thr Gly Leu Pro Gln Lys
    1085                    1090                    1095

Ser Ser Ala Met Pro Thr Lys His Ala Lys Val Leu Phe Arg Gly
    1100                    1105                    1110

Ser Gly Gly Lys Pro Ile Ala Val Leu Cys Leu Thr Val Glu Thr
    1115                    1120                    1125

Gln Pro His Val Val Asp Gln Val Phe Arg Phe Tyr His Pro Glu
    1130                    1135                    1140

Leu Thr Phe Leu Lys Lys Ala Ile Arg Leu Pro Pro Trp His Thr
    1145                    1150                    1155

Leu Pro Gly Ala Pro Val Gly Arg Pro Gly Glu Glu Pro Pro Val
    1160                    1165                    1170

Tyr Val Arg Cys Ser Asp Pro Asn Val Ile Cys Glu Thr Gln Asn
    1175                    1180                    1185

Val Gly Pro Arg Glu Pro Arg Asp Val Phe Leu Lys Val Ala Ser
    1190                    1195                    1200

Gly Pro Ser Pro Glu Ser Arg Asp Phe Phe Val Ala Val Tyr Ala
    1205                    1210                    1215

Asp Arg Trp Leu Ala Thr Pro Ile Gln Ile Trp Gln Val Tyr Leu
    1220                    1225                    1230

His Ser Leu Gln Arg Val Asp Val Ser Cys Val Thr Gly Gln Arg
    1235                    1240                    1245

Thr Arg Leu Ser Leu Val Leu Arg Gly Thr Arg Thr Val Arg Arg
    1250                    1255                    1260

Val Gln Ala Phe Thr Ser His Pro Gln Glu Leu Lys Thr Asp Pro
    1265                    1270                    1275

Lys Gly Val Phe Val Leu Pro Pro His Gly Val Gln Asp Leu His
    1280                    1285                    1290

Val Gly Val Arg Pro Arg Arg Ala Gly Ser Arg Phe Ile His Leu
    1295                    1300                    1305

Asn Leu Val Asp Val Asp Tyr His Gln Leu Val Ala Ser Trp Leu
    1310                    1315                    1320

Val Cys Leu Ser Cys Arg Pro Pro Leu Ile Ser Lys Ala Phe Glu

1325                 1330                 1335

Ile Thr Leu Ala Ala Gly Lys Gly Arg Gly Ala Asn Lys Arg Ile
    1340                 1345                 1350

Thr Tyr Thr Asn Pro Tyr Pro Ser Gln Arg Thr Tyr His Leu Tyr
    1355                 1360                 1365

Ser Asp His Pro Asp Leu Leu Gln Phe Arg Glu Asp Ser Phe Gln
    1370                 1375                 1380

Ile Gly Gly Gly Glu Thr Tyr Thr Ile Gly Leu Trp Phe Ala Pro
    1385                 1390                 1395

Arg Gln Ser Pro Gly Glu Glu Glu Ile Leu Ile Tyr Ile Asn Asp
    1400                 1405                 1410

His Glu Asp Lys Asn Glu Glu Ala Phe Cys Val Lys Val Val Tyr
    1415                 1420                 1425

Gln

<210>  7
<211>  155
<212>  PRT
<213>  Canis familiaris

<400>  7

Met Asn Asp Trp Leu Arg Ile Phe Asp Gln Asn Met Leu Val Pro Pro
1               5                   10                  15

His Pro Gln Arg Ala Arg Gln Leu Leu Lys Glu Ser Thr Ala Phe Gln
            20                  25                  30

Cys Val Leu Lys Trp Leu Asp Gly Pro Leu Ile Lys Gln Gly Val Leu
        35                  40                  45

Glu Val Leu Ser Glu Val Gly Cys His Leu Arg Val Ser Leu Phe Asp
    50                  55                  60

Val Thr Tyr Arg His Phe Phe Gly Arg Thr Trp Lys Thr Ala Val Arg
65                  70                  75                  80

Pro Ala Glu Gln Leu Ser Arg Gln Pro Ser Arg Ile Ile Phe Asp Glu
                85                  90                  95

Pro Leu Tyr Phe His Thr Ser Leu Asn His Pro Asn Val Val Ala Val
            100                 105                 110

Val Glu Val Val Thr Glu Gly Arg Lys Gln Asp Gly Ser Leu Gln Ala
        115                 120                 125

Leu Ser Cys Gly Phe Gly Ile Leu Arg Ile Phe Gly Asn Lys Leu Glu
    130                 135                 140

Ser Pro Thr Ser Ala Ser Gln Asp Lys Arg Lys
145                 150                 155

<210>  8
<211>  23
<212>  PRT
<213>  Canis familiaris

<400>  8

Arg Leu Arg Leu Tyr His Gly Thr Pro Arg Ala Leu Leu His Pro Leu
1               5                   10                  15

Leu Gln Asp Pro Phe Glu Gln
            20

<210>  9
<211>  295
<212>  DNA
<213>  Canis familiaris

<220>
<221>  misc_feature
<222>  (132)..(132)
<223>  Exon 5 deletion is after nucleotide 132

<400>  9
gctaagctga taatggttta cacagagaaa gagaacaaga tggagtcaaa gcagataaca      60

ccaggaaagt catagggagg atgagaggac ttcacagcaa cacctcaggc caggccaggc     120

cttgtctctt acctgcaaag caagaccaca gagcgcatgt caaccctccg catcctgcat     180

ttagccaaga aacatcagtg atgcttctca ctccacacct actagagctg tgtcaacacc     240

atacaatcac atcctctgca ggagagacag gtgagcaaag cagcagcccc ccatg          295

<210>  10
<211>  4290
<212>  DNA
<213>  Canis familiaris

<400>  10
atgaatgact ggctcaggat ttttgaccaa aacgtgctgg tccctcccca cccacagaga      60

gcacgccagc ttttaaagga atcaacagca tttcagtgtg tcctgaagtg gcttgacggg     120

ccactaatta agcagggagt gctggaggtg ctttcggagg tgggctgcca tctgcgtgtg     180

tctctctttg atgtcaccta ccgacacttt ttcgggagga catggaagac cgcggtgagg     240

ccggctgagc agctttccag gcagccgtct aggatcatct ttgatgagcc cttgtacttt     300

cacacgtcct tgaaccaccc taacgtggtg gctgtggttg aggtggtcac cgaaggccgg     360

aagcaggatg gctctctcca ggctctgtca tgtggctttg gaattcttcg gatctttggc     420

aacaaactgg aatctcctac ctctgcctcc caggacaagc ggttgaggct gtatcacggc 480
actccccgag ccctcctgca cccactgctc caggaccctt tcgaacaaaa taaacacatg 540
accctcattg agagctgcag cctgcaatac actctgaagc cacacccgct cctggagccc 600
gtgttccatc ttcttcccca gaaccttctg gtgtctggtc tgcagcacat ccctggcctc 660
gttccagctc atggagaaaa gggggacacc ctccgaaagc ctcgccttca gaagtctgtc 720
acatggtact tggatgattt attcttcacg ctatatcctt cccttgagaa gtttgaggaa 780
gagcttctgg aactcctcat cagtgataac ttccgggagg gggacagtcc actagatggc 840
agcacgctgg agatcttgga gcgccgcctg tgtgtgggcg tgcacaatgg tctgggcttc 900
gtgcagaggc cacacgtggt ggtgctggtg cccgagatgg atgtggcctt gacgcgctct 960
gccagtttca gcaggaaagt tggctcctct tcgaagacca gctctggaaa ccaggctcta 1020
gttttgagaa gccgcctccg actccctgag atggtccatc accctgcgtt tgccatagtc 1080
tttcaactgg agtacgtgtt caccagccct tctggagtgg acagcaaggc agcctcggtc 1140
acctctttgt ccagtgtggc gtgtatgcac atagttcgct gggctgtttg gaaccccttg 1200
ctggaatccg gctctgggaa ggtgatctta cctctgcagg gtgggatccg gcccaacccc 1260
tcacactgtt tggtctataa agtaacccct gccagcatga gctctgaaga ggtgcagcaa 1320
gtggagtcag ggactgtcca gttccacttc tcgctgagcc ctgagggaca cctggacaca 1380
cccacggggc tggtccacag ccctgaggcg gagcggcagc cctccaggaa gccacccaca 1440
tctcctgcaa acctgccagc actgccgccc caggggctcg ctgccctcca ggactcgccg 1500
gtgggaccag ggttgtccct ttcccagctc gcagcttccc cacagcctac cacccgctcc 1560
cgctcagcca caccccctcc acggcaaccc gacagctcta agcccccgcc ggcccaggag 1620
gagcccccct cggagggcag gatctctcac ctggaggccc agctgagcca ggcgtccttg 1680
gtcctgggag cctctgttgc tgaacagctg caggagctgc cttttacgcc ggtgcacacc 1740
ccgattgttg tgggagccca gaccaggagc tctggaagta agctctcacg agcctcaatg 1800
gtgctcctgc aagcctgcgg ctttcctgag attctggatg ccaataagca accagccgag 1860
gctgtaaatc ctacagatcc cgtgaaattc aacccccaaa aggaagaatc agattgtcta 1920
caaagcaatg agatagtgct gcattttctt gcctttagca gagtgtccca ggactgccag 1980
ggagcaccgt ggccaaagac cgtgtatttc accttccagt tctatcgctt cccacccgtg 2040
acaacgccac gcttgcagct gatcaggctg gacgagacgg gcaggacagg ctctggttcc 2100
ctgttgtatc tcctggttct cactgacaaa gatggctcct ctgatgctgg gacacctggc 2160
tttcaactca agtacctggt ggaacctggg ttcctgaaac ccggagaaca gcgctggttc 2220
acccactaca tggctgctca gaccctccag atcgacatct gggatggaga ctccttgctg 2280
ctcgtgggat ctgcggccgt ccagatgaag catctcctcc gccaaggccg gccggctgtg 2340
caggtcctgc atgagcttga ggtcgtggcg actgaatatg aacaggatgt ggtggtgagt 2400
ggagatgtga ccagatttgg ctgtgtcaag cccatcggtg tctacacagt ggtaaagggc 2460

tggctgcacc tgacgttggc caacgtgggt catgtgtgtg agcagagagt gagagattcc 2520
agctcattgc caccttccag gtcacgggtc atttcgaatg atggagccag acacttcgag 2580
ggaggcagtc tcatctcgcg cgggggtccg agatcagtaa aaaatgttgt ccaagcacag 2640
aagctggctg acgtggaccg tgagctggcc gccatgctgt tcacccacat gcaacatggg 2700
agcaaggggc cccaaggtgc ttgccgtgag ggggatgccg tccgcaggcg caagctggag 2760
cggatgaggt ccgtgcgcct tcaagagtcc agaggagagc tcagctgctg gggggccagc 2820
atgttgattc agcagaatat ccgcgcacag cacgcacgtg acctgcaggt cattgctgcc 2880
tatcgggagc gtacgaaggc agagagcatc accagtgcgc tgagcctggc catcaccacg 2940
atgcacacaa tccacgtcac actgggcact gccgagttct tcgagtttgc ccttaggaac 3000
ccccacaaca cacagcacac agtgaccatt gaggtggaca atcctgagct cagcatcatc 3060
caggacagtc aggagtggag atacttcaaa gacgcgacca agctgcacac gccggtggag 3120
gaggacatgt tccatctgcg gggcagcctt gccccgcaga tcttcctgcg cccacgagag 3180
accgctcaca tccccttcaa ataccagacc ttctctgtag ggcaactggc cctggcgcag 3240
ccctctgctg agctcggatc tgagaaggac acggataccg gattgcccca gaaatccagt 3300
gcaatgccca ctaagcatgc caaggtcttg ttccggggga gtggcggtaa gcccatcgcc 3360
gtgctttgcc taaccgtgga gacccagccc catgtggtgg accaggtttt ccgcttctac 3420
cacccagagc tcaccttcct gaagaaggcc atccgcctgc ctccctggca cacgcttcca 3480
ggtgctcccg tgggaagacc tggtgaggag cccccagtct atgtccgatg tagcgacccc 3540
aacgtcatct gcgagaccca gaatgtgggc cccagggagc ctcgggacgt gttcctgaag 3600
gtggccagcg gtccaagccc agagagcaga gacttctttg tcgccgttta cgcggatcgt 3660
tggctggcga cacccatcca aatatggcag gtctacctcc actccttgca gcgtgtggac 3720
gtctcctgtg tcacaggcca gcggacacgc ctatcccttg tccttcgagg gacacggacg 3780
gttaggagag tgcaggcttt cacctcacac cctcaggagc tgaagactga ccccaaaggt 3840
gtgttcgtgc tgccaccaca tggggtgcag gacctgcatg tcggtgtgag gccccgcagg 3900
gcagggagcc ggttcatcca cctcaacctg gtcgacgtgg actaccacca gctggtggcc 3960
tcatggctcg tgtgcctctc ctgccgcccg cctctcatct ccaaggcttt cgagatcaca 4020
ctggctgcag ggaaaggcag aggcgccaac aagcggatca cctacaccaa cccttacccc 4080
tcccagagga cctatcacct gtacagcgac caccctgacc tgctgcagtt cagagaggac 4140
tccttccaga ttgggggagg agagacctac accatcggcc tgtggttcgc acctcggcag 4200
agccccgggg aggaggagat tctgatttac atcaatgacc acgaagacaa aaatgaagaa 4260
gccttctgcg tcaaggtcgt gtaccagtaa 4290

<210> 11
<211> 465
<212> DNA
<213> Canis familiaris

<400>  11
atgaatgact ggctcaggat ttttgaccaa aacatgctgg tccctcccca cccacagaga      60

gcacgccagc ttttaaagga atcaacagca tttcagtgtg tcctgaagtg gcttgacggg     120

ccactaatta agcagggagt gctggaggtg ctttcggagg tgggctgcca tctgcgtgtg     180

tctctctttg atgtcaccta ccgacacttt ttcgggagga catggaagac cgcggtgagg     240

ccggctgagc agctttccag gcagccgtct aggatcatct ttgatgagcc cttgtacttt     300

cacacgtcct tgaaccaccc taacgtggtg gctgtggttg aggtggtcac cgaaggccgg     360

aagcaggatg gctctctcca ggctctgtca tgtggctttg gaattcttcg gatctttggc     420

aacaaactgg aatctcctac ctctgcctcc caggacaagc gaaaa                     465


<210>  12
<211>  69
<212>  DNA
<213>  Canis familiaris

<400>  12
cggttgaggc tgtatcacgg cactccccga gccctcctgc acccactgct ccaggaccct      60

ttcgaacaa                                                              69


<210>  13
<211>  22
<212>  DNA
<213>  Artificial

<220>
<223>  PCR primer

<400>  13
gcatgctaat atgtgaggca ct                                               22


<210>  14
<211>  20
<212>  DNA
<213>  Artificial

<220>
<223>  PCR primer

<400>  14
caatgtggcc atcacttgac                                                  20


<210>  15
<211>  20
<212>  DNA
<213>  Artificial

<220>
<223>  PCR primer

<400>  15
aggaatccca acaaggttcc                                                  20


<210>  16
<211>  20
<212>  DNA

<213>  Artificial

<220>
<223>  PCR primer

<400>  16
cctagccaca tcccactgtt                                                    20


<210>  17
<211>  20
<212>  DNA
<213>  Artificial

<220>
<223>  PCR primer

<400>  17
cccttgaatg agtcccctaa                                                    20


<210>  18
<211>  20
<212>  DNA
<213>  Artificial

<220>
<223>  PCR primer

<400>  18
tcgtcaagaa ggcagattca                                                    20


<210>  19
<211>  20
<212>  DNA
<213>  Artificial

<220>
<223>  PCR primer

<400>  19
ctgtcttgcc tttgcctttc                                                    20


<210>  20
<211>  20
<212>  DNA
<213>  Artificial

<220>
<223>  PCR primer

<400>  20
aactcttccc cctttgcact                                                    20


<210>  21
<211>  20
<212>  DNA
<213>  Artificial

<220>
<223>  PCR primer

<400>  21
caccctctgt gttgcctttt                                                    20

<210>  22
<211>  20
<212>  DNA
<213>  Artificial

<220>
<223>  PCR primer

<400>  22
tccagctcac actcatgctc                                                    20


<210>  23
<211>  20
<212>  DNA
<213>  Artificial

<220>
<223>  PCR primer

<400>  23
atttgcccca ccttccttta                                                    20


<210>  24
<211>  20
<212>  DNA
<213>  Artificial

<220>
<223>  PCR primer

<400>  24
aggtgcctgc cttcagtcta                                                    20


<210>  25
<211>  20
<212>  DNA
<213>  Artificial

<220>
<223>  PCR primer

<400>  25
ggaggctctg agttggagaa                                                    20


<210>  26
<211>  20
<212>  DNA
<213>  Artificial

<220>
<223>  PCR primer

<400>  26
ctgcataggg cttcttgctc                                                    20


<210>  27
<211>  20
<212>  DNA
<213>  Artificial

<220>
<223>  PCR primer

<400>  27
gggtggaggg tagattccat                                                    20


<210>  28
<211>  20
<212>  DNA
<213>  Artificial

<220>
<223>  PCR primer

<400>  28
cagcctccct gaaaatgagt                                                    20


<210>  29
<211>  20
<212>  DNA
<213>  Artificial

<220>
<223>  PCR primer

<400>  29
aagcagccag tgccactatt                                                    20


<210>  30
<211>  20
<212>  DNA
<213>  Artificial

<220>
<223>  PCR primer

<400>  30
ccctctgcct atgtctctgc                                                    20


<210>  31
<211>  20
<212>  DNA
<213>  Artificial

<220>
<223>  PCR primer

<400>  31
ggctgccctc tttaggttct                                                    20


<210>  32
<211>  20
<212>  DNA
<213>  Artificial

<220>
<223>  PCR primer

<400>  32
gacacaggca gagggagaag                                                    20


<210>  33
<211>  20
<212>  DNA

<213>  Artificial

<220>
<223>  PCR primer

<400>  33
ggcgctgtat tctgttggtt                                                    20


<210>  34
<211>  19
<212>  DNA
<213>  Artificial

<220>
<223>  PCR primer

<400>  34
gccagtgaca gctctctgc                                                     19


<210>  35
<211>  20
<212>  DNA
<213>  Artificial

<220>
<223>  PCR primer

<400>  35
caaaatcatc tgggcaggtc                                                    20


<210>  36
<211>  21
<212>  DNA
<213>  Artificial

<220>
<223>  PCR primer

<400>  36
acactcctgg ctggtctcat c                                                  21


<210>  37
<211>  20
<212>  DNA
<213>  Artificial

<220>
<223>  PCR primer

<400>  37
catgacaact gcccattttg                                                    20


<210>  38
<211>  20
<212>  DNA
<213>  Artificial

<220>
<223>  PCR primer

<400>  38
catccaacca tccaaaccat                                                    20

<210> 39
<211> 20
<212> DNA
<213> Artificial

<220>
<223> PCR primer

<400> 39
tgcccgaaaa taaggatttg 20


<210> 40
<211> 20
<212> DNA
<213> Artificial

<220>
<223> PCR primer

<400> 40
catctgagtt ccctgcatga 20


<210> 41
<211> 20
<212> DNA
<213> Artificial

<220>
<223> PCR primer

<400> 41
gacacaggca gagggagaag 20


<210> 42
<211> 20
<212> DNA
<213> Artificial

<220>
<223> PCR primer

<400> 42
ctcttgtaac cctgggagca 20


<210> 43
<211> 20
<212> DNA
<213> Artificial

<220>
<223> PCR primer

<400> 43
ggacgccaaa tgctattgtt 20


<210> 44
<211> 20
<212> DNA
<213> Artificial

<220>
<223> PCR primer

<400>  44
tacccccatc cacaaggata                                                    20


<210>  45
<211>  20
<212>  DNA
<213>  Artificial

<220>
<223>  PCR primer

<400>  45
ccattggtgc agagtaagca                                                    20


<210>  46
<211>  19
<212>  DNA
<213>  Artificial

<220>
<223>  PCR primer

<400>  46
gatggtggat ggatggaca                                                     19


<210>  47
<211>  20
<212>  DNA
<213>  Artificial

<220>
<223>  PCR primer

<400>  47
agttccatcc acatcattgc                                                    20


<210>  48
<211>  20
<212>  DNA
<213>  Artificial

<220>
<223>  PCR primer

<400>  48
cctggcaaag gtcatgtttt                                                    20


<210>  49
<211>  20
<212>  DNA
<213>  Artificial

<220>
<223>  PCR primer

<400>  49
tgccatgaaa atttgctgaa                                                    20


<210>  50
<211>  20
<212>  DNA

<213>  Artificial

<220>
<223>  PCR primer

<400>  50
gccatccaaa tcagaaagga                                                    20


<210>  51
<211>  20
<212>  DNA
<213>  Artificial

<220>
<223>  PCR primer

<400>  51
ttgatggcct atggctgaat                                                    20


<210>  52
<211>  20
<212>  DNA
<213>  Artificial

<220>
<223>  PCR primer

<400>  52
caggtgcaga agcacaaaaa                                                    20


<210>  53
<211>  20
<212>  DNA
<213>  Artificial

<220>
<223>  PCR primer

<400>  53
aggagtgctg tgcaggagat                                                    20


<210>  54
<211>  20
<212>  DNA
<213>  Artificial

<220>
<223>  PCR primer

<400>  54
cgtatcctgt ctccctctgc                                                    20


<210>  55
<211>  20
<212>  DNA
<213>  Artificial

<220>
<223>  PCR primer

<400>  55
ccatgaaggc aaagagtgct                                                    20

<210>  56
<211>  20
<212>  DNA
<213>  Artificial

<220>
<223>  PCR primer

<400>  56
caaaaggcag aggagtcagg                                                    20


<210>  57
<211>  20
<212>  DNA
<213>  Artificial

<220>
<223>  PCR primer

<400>  57
tgtcatggtt ctgtccatgc                                                    20


<210>  58
<211>  20
<212>  DNA
<213>  Artificial

<220>
<223>  PCR primer

<400>  58
actcctctgg ggcctagttc                                                    20


<210>  59
<211>  20
<212>  DNA
<213>  Artificial

<220>
<223>  PCR primer

<400>  59
gttgagatcc agtcccaagc                                                    20


<210>  60
<211>  20
<212>  DNA
<213>  Artificial

<220>
<223>  PCR primer

<400>  60
tgactggtgc atgtgtgcta                                                    20


<210>  61
<211>  20
<212>  DNA
<213>  Artificial

<220>
<223>  PCR primer

<400>  61
ggtcctttct ccccaagaag                                                    20

<210>  62
<211>  20
<212>  DNA
<213>  Artificial

<220>
<223>  PCR primer

<400>  62
atctgagccc cctcctgtag                                                    20

<210>  63
<211>  20
<212>  DNA
<213>  Artificial

<220>
<223>  PCR primer

<400>  63
tggggaagta tgcatgaggt                                                    20

<210>  64
<211>  20
<212>  DNA
<213>  Artificial

<220>
<223>  PCR primer

<400>  64
cactgatggg atgtcagtgc                                                    20

<210>  65
<211>  20
<212>  DNA
<213>  Artificial

<220>
<223>  PCR primer

<400>  65
ctctccctgc tgtgctctct                                                    20

<210>  66
<211>  20
<212>  DNA
<213>  Artificial

<220>
<223>  PCR primer

<400>  66
gatctctggc ttgctccttg                                                    20

<210>  67
<211>  20
<212>  DNA

<213>  Artificial

<220>
<223>  PCR primer

<400>  67
atgagagaca cggagggaga                                                    20


<210>  68
<211>  20
<212>  DNA
<213>  Artificial

<220>
<223>  PCR primer

<400>  68
tgcaccaaaa gcaatttcaa                                                    20


<210>  69
<211>  20
<212>  DNA
<213>  Artificial

<220>
<223>  PCR primer

<400>  69
tatggctgaa tggtgttcca                                                    20


<210>  70
<211>  20
<212>  DNA
<213>  Artificial

<220>
<223>  PCR primer

<400>  70
agcagccact gtggaaaagt                                                    20


<210>  71
<211>  20
<212>  DNA
<213>  Artificial

<220>
<223>  PCR primer

<400>  71
caggcaagga tgttgctttt                                                    20


<210>  72
<211>  20
<212>  DNA
<213>  Artificial

<220>
<223>  PCR primer

<400>  72
ggagaaggag ggaccatgag                                                    20

<210>  73
<211>  20
<212>  DNA
<213>  Artificial

<220>
<223>  PCR primer

<400>  73
ctcccttagc ctggaatggt                                                    20


<210>  74
<211>  19
<212>  DNA
<213>  Artificial

<220>
<223>  PCR primer

<400>  74
gagactcggg gtcaagtcc                                                     19


<210>  75
<211>  20
<212>  DNA
<213>  Artificial

<220>
<223>  PCR primer

<400>  75
gaaattgcgg tttccttgaa                                                    20


<210>  76
<211>  20
<212>  DNA
<213>  Artificial

<220>
<223>  PCR primer

<400>  76
caaactcacg accccaaaat                                                    20


<210>  77
<211>  20
<212>  DNA
<213>  Artificial

<220>
<223>  PCR primer

<400>  77
agttggaccc ctaaccgact                                                    20


<210>  78
<211>  19
<212>  DNA
<213>  Artificial

<220>
<223>  PCR primer

<400>  78
gagcctgctt ctccctctg                                                    19


<210>  79
<211>  20
<212>  DNA
<213>  Artificial

<220>
<223>  PCR primer

<400>  79
agacactgtg ccaggtgcta                                                   20


<210>  80
<211>  20
<212>  DNA
<213>  Artificial

<220>
<223>  PCR primer

<400>  80
tggccctctt ctcagtttgt                                                   20


<210>  81
<211>  20
<212>  DNA
<213>  Artificial

<220>
<223>  PCR primer

<400>  81
cacttccaga ggctttcagc                                                   20


<210>  82
<211>  20
<212>  DNA
<213>  Artificial

<220>
<223>  PCR primer

<400>  82
agtccactat ggctgggatg                                                   20


<210>  83
<211>  20
<212>  DNA
<213>  Artificial

<220>
<223>  PCR primer

<400>  83
gtggacaatc tgctctgcaa                                                   20


<210>  84
<211>  20
<212>  DNA

<213>  Artificial

<220>
<223>  PCR primer

<400>  84
cttctccctc tccctctgct                                                    20


<210>  85
<211>  25
<212>  DNA
<213>  Artificial

<220>
<223>  PCR primer

<400>  85
gaacaattca atatgctttc aaaaa                                              25


<210>  86
<211>  20
<212>  DNA
<213>  Artificial

<220>
<223>  PCR primer

<400>  86
ccattctcca ggtgatggac                                                    20


<210>  87
<211>  24
<212>  DNA
<213>  Artificial

<220>
<223>  PCR primer

<400>  87
tgaatgttta tcaccaaagt gtca                                               24


<210>  88
<211>  20
<212>  DNA
<213>  Artificial

<220>
<223>  PCR primer

<400>  88
gctcttgatc ttggctcagg                                                    20


<210>  89
<211>  20
<212>  DNA
<213>  Artificial

<220>
<223>  PCR primer

<400>  89
atcaggatgc ccactttctg                                                    20

<210>  90
<211>  20
<212>  DNA
<213>  Artificial

<220>
<223>  PCR primer

<400>  90
cctgcggaat ctgatgctat                                                    20


<210>  91
<211>  18
<212>  DNA
<213>  Artificial

<220>
<223>  PCR primer

<400>  91
gcctgcttct ccctcctc                                                      18


<210>  92
<211>  24
<212>  DNA
<213>  Artificial

<220>
<223>  PCR primer

<400>  92
cacagacaac cgctaataca tctc                                               24


<210>  93
<211>  21
<212>  DNA
<213>  Artificial

<220>
<223>  PCR primer

<400>  93
gagtactcaa gggccaaagt t                                                  21


<210>  94
<211>  20
<212>  DNA
<213>  Artificial

<220>
<223>  PCR primer

<400>  94
atatgctggg tgtggagctt                                                    20


<210>  95
<211>  20
<212>  DNA
<213>  Artificial

<220>
<223>  Primer used in amplifying exon of DAB1 gene

<400>  95
ggctgaaggt ggcactaaac                                                    20


<210>  96
<211>  20
<212>  DNA
<213>  Artificial

<220>
<223>  Primer used in amplifying exon of DAB1 gene

<400>  96
ggcataccag gaagggtttt                                                    20


<210>  97
<211>  21
<212>  DNA
<213>  Artificial

<220>
<223>  Primer used in amplifying exon of DAB1 gene

<400>  97
ctcggtccaa tgatgtgact t                                                  21


<210>  98
<211>  20
<212>  DNA
<213>  Artificial

<220>
<223>  Primer used in amplifying exon of DAB1 gene

<400>  98
ccacacagta ggcgctcaat                                                    20


<210>  99
<211>  20
<212>  DNA
<213>  Artificial

<220>
<223>  Primer used in amplifying exon of DAB1 gene

<400>  99
aggagaggca aacacaccag                                                    20


<210>  100
<211>  20
<212>  DNA
<213>  Artificial

<220>
<223>  Primer used in amplifying exon of DAB1 gene

<400>  100
cggggactgt ttgacacttt                                                    20


<210>  101
<211>  20
<212>  DNA

<213>  Artificial

<220>
<223>  Primer used in amplifying exon of DAB1 gene

<400>  101
gctcgttttc cttccatctg                                                    20


<210>  102
<211>  20
<212>  DNA
<213>  Artificial

<220>
<223>  Primer used in amplifying exon of DAB1 gene

<400>  102
tccgaggcta tttgtgctct                                                    20


<210>  103
<211>  20
<212>  DNA
<213>  Artificial

<220>
<223>  Primer used in amplifying exon of DAB1 gene

<400>  103
taggaaaagg aagggctgtg                                                    20


<210>  104
<211>  20
<212>  DNA
<213>  Artificial

<220>
<223>  Primer used in amplifying exon of DAB1 gene

<400>  104
tctgttctcc tccccagaaa                                                    20


<210>  105
<211>  20
<212>  DNA
<213>  Artificial

<220>
<223>  Primer used in amplifying exon of DAB1 gene

<400>  105
tggggaggag aacagaaaga                                                    20


<210>  106
<211>  20
<212>  DNA
<213>  Artificial

<220>
<223>  Primer used in amplifying exon of DAB1 gene

<400>  106
gatcccgtgt tatggatgct                                                    20

<210>  107
<211>  20
<212>  DNA
<213>  Artificial

<220>
<223>  Primer used in amplifying exon of DAB1 gene

<400>  107
agaaaagtca gggggagcat                                                    20


<210>  108
<211>  20
<212>  DNA
<213>  Artificial

<220>
<223>  Primer used in amplifying exon of DAB1 gene

<400>  108
gccattgaac atttccacaa                                                    20


<210>  109
<211>  20
<212>  DNA
<213>  Artificial

<220>
<223>  Primer used in amplifying exon of DAB1 gene

<400>  109
ctgctgcccc tataattcca                                                    20


<210>  110
<211>  20
<212>  DNA
<213>  Artificial

<220>
<223>  Primer used in amplifying exon of DAB1 gene

<400>  110
ccttgatcca gactggcact                                                    20


<210>  111
<211>  21
<212>  DNA
<213>  Artificial

<220>
<223>  Primer used in amplifying exon of DAB1 gene

<400>  111
cttaaccact gctggctttc c                                                  21


<210>  112
<211>  20
<212>  DNA
<213>  Artificial

<220>
<223>  Primer used in amplifying exon of DAB1 gene

<400>  112
caggctgggt gaggagaata                                                    20


<210>  113
<211>  20
<212>  DNA
<213>  Artificial

<220>
<223>  Primer used in amplifying exon of DAB1 gene

<400>  113
agcccagata aactgccaga                                                    20


<210>  114
<211>  20
<212>  DNA
<213>  Artificial

<220>
<223>  Primer used in amplifying exon of DAB1 gene

<400>  114
attgctgatg catggtttca                                                    20


<210>  115
<211>  19
<212>  DNA
<213>  Artificial

<220>
<223>  Primer used in amplifying exon of DAB1 gene

<400>  115
accagcttgt ggtctgcac                                                     19


<210>  116
<211>  20
<212>  DNA
<213>  Artificial

<220>
<223>  Primer used in amplifying exon of DAB1 gene

<400>  116
ggcagcctct caaagtcatc                                                    20


<210>  117
<211>  20
<212>  DNA
<213>  Artificial

<220>
<223>  Primer used in amplifying exon of DAB1 gene

<400>  117
tggctctgga aggaacactc                                                    20


<210>  118
<211>  20
<212>  DNA

<213>  Artificial

<220>
<223>  Primer used in amplifying exon of DAB1 gene

<400>  118
gcctgctgaa tgtgaactga                                                    20


<210>  119
<211>  20
<212>  DNA
<213>  Artificial

<220>
<223>  Primer used in amplifying exon of DAB1 gene

<400>  119
gtgtgcaccc actctttcct                                                    20


<210>  120
<211>  20
<212>  DNA
<213>  Artificial

<220>
<223>  Primer used in amplifying exon of DAB1 gene

<400>  120
agcagggcat attttcatgc                                                    20


<210>  121
<211>  20
<212>  DNA
<213>  Artificial

<220>
<223>  Primer used in amplifying exon of DAB1 gene

<400>  121
ggcactagag gggctttctt                                                    20


<210>  122
<211>  20
<212>  DNA
<213>  Artificial

<220>
<223>  Primer used in amplifying exon of DAB1 gene

<400>  122
ttctccactc ctcaccccta                                                    20


<210>  123
<211>  20
<212>  DNA
<213>  Artificial

<220>
<223>  Primer used in amplifying exon of DAB1 gene

<400>  123
ttgaaaacca agggcaaaac                                                    20

<210> 124
<211> 20
<212> DNA
<213> Artificial

<220>
<223> Primer used in amplifying exon of DAB1 gene

<400> 124
cggtcaacag gtcgaaaaat 20


<210> 125
<211> 20
<212> DNA
<213> Artificial

<220>
<223> Primer used in amplifying exon of AJAP1 gene

<400> 125
gtgcttctga gatgctgacg 20


<210> 126
<211> 18
<212> DNA
<213> Artificial

<220>
<223> Primer used in amplifying exon of AJAP1 gene

<400> 126
ggggacgagg aggaggag 18


<210> 127
<211> 20
<212> DNA
<213> Artificial

<220>
<223> Primer used in amplifying exon of AJAP1 gene

<400> 127
gagtctgaag agcggacagg 20


<210> 128
<211> 20
<212> DNA
<213> Artificial

<220>
<223> Primer used in amplifying exon of AJAP1 gene

<400> 128
ccagaaaaga ggcggttgta 20


<210> 129
<211> 18
<212> DNA
<213> Artificial

<220>
<223> Primer used in amplifying exon of AJAP1 gene

<400>  129
cggagacgga gttcatcg                                                    18


<210>  130
<211>  20
<212>  DNA
<213>  Artificial

<220>
<223>  Primer used in amplifying exon of AJAP1 gene

<400>  130
ggacacacaa gtccctggat                                                  20


<210>  131
<211>  19
<212>  DNA
<213>  Artificial

<220>
<223>  Primer used in amplifying exon of AJAP1 gene

<400>  131
atgctgaggc tgggactct                                                   19


<210>  132
<211>  20
<212>  DNA
<213>  Artificial

<220>
<223>  Primer used in amplifying exon of AJAP1 gene

<400>  132
ctgcacacaa acccacattc                                                  20


<210>  133
<211>  20
<212>  DNA
<213>  Artificial

<220>
<223>  Primer used in amplifying exon of AJAP1 gene

<400>  133
gagagggagc gaactgattg                                                  20


<210>  134
<211>  20
<212>  DNA
<213>  Artificial

<220>
<223>  Primer used in amplifying exon of AJAP1 gene

<400>  134
gccgcaacac cttctatgtt                                                  20


<210>  135
<211>  18
<212>  DNA

<213>  Artificial

<220>
<223>  Primer used in amplifying exon of AJAP1 gene

<400>  135
acacggagct tggccttc                                                    18


<210>  136
<211>  20
<212>  DNA
<213>  Artificial

<220>
<223>  Primer used in amplifying exon of AJAP1 gene

<400>  136
tggacagcag cactattgga                                                  20


<210>  137
<211>  20
<212>  DNA
<213>  Artificial

<220>
<223>  Primer used in amplifying exon of VAMP3 gene

<400>  137
ttcacttgag accctgctca                                                  20


<210>  138
<211>  20
<212>  DNA
<213>  Artificial

<220>
<223>  Primer used in amplifying exon of VAMP3 gene

<400>  138
ctcgggagga gtatttgtgg                                                  20


<210>  139
<211>  19
<212>  DNA
<213>  Artificial

<220>
<223>  Primer used in amplifying exon of VAMP3 gene

<400>  139
cctgccccat cctttctaa                                                   19


<210>  140
<211>  20
<212>  DNA
<213>  Artificial

<220>
<223>  Primer used in amplifying exon of VAMP3 gene

<400>  140
atgtgcagca cgtcaaactc                                                  20

<210> 141
<211> 20
<212> DNA
<213> Artificial

<220>
<223> Primer used in amplifying exon of NPHP4 gene

<400> 141
taatgggctc cacttccaac 20


<210> 142
<211> 20
<212> DNA
<213> Artificial

<220>
<223> Primer used in amplifying exon of NPHP4 gene

<400> 142
tcaacaacag tggaacacca 20


<210> 143
<211> 20
<212> DNA
<213> Artificial

<220>
<223> Primer used in amplifying exon of NPHP4 gene

<400> 143
ggagcaagat gggtgtgttt 20


<210> 144
<211> 20
<212> DNA
<213> Artificial

<220>
<223> Primer used in amplifying exon of NPHP4 gene

<400> 144
tttgctgatg agccactttg 20


<210> 145
<211> 20
<212> DNA
<213> Artificial

<220>
<223> Primer used in amplifying exon of NPHP4 gene

<400> 145
ttctggaggg caagtcattt 20


<210> 146
<211> 20
<212> DNA
<213> Artificial

<220>
<223> Primer used in amplifying exon of NPHP4 gene

<400>  146
tttgttccaa gaaccccaag                                                    20


<210>  147
<211>  20
<212>  DNA
<213>  Artificial

<220>
<223>  Primer used in amplifying exon of NPHP4 gene

<400>  147
ttcctgtggg tcagaagctc                                                    20


<210>  148
<211>  20
<212>  DNA
<213>  Artificial

<220>
<223>  Primer used in amplifying exon of NPHP4 gene

<400>  148
agcagaaaag ctcccagaca                                                    20


<210>  149
<211>  20
<212>  DNA
<213>  Artificial

<220>
<223>  Primer used in amplifying exon of NPHP4 gene

<400>  149
ttctaggtgc ctgggtcttg                                                    20


<210>  150
<211>  20
<212>  DNA
<213>  Artificial

<220>
<223>  Primer used in amplifying exon of NPHP4 gene

<400>  150
tcccctcagg ctagtgctta                                                    20


<210>  151
<211>  20
<212>  DNA
<213>  Artificial

<220>
<223>  Primer used in amplifying exon of NPHP4 gene

<400>  151
atgcctggac ttcacacctt                                                    20


<210>  152
<211>  20
<212>  DNA

<213>  Artificial

<220>
<223>  Primer used in amplifying exon of NPHP4 gene

<400>  152
tcccttcctc ctctcctctc                                                    20


<210>  153
<211>  20
<212>  DNA
<213>  Artificial

<220>
<223>  Primer used in amplifying exon of NPHP4 gene

<400>  153
gggcagaaat tgggttgtaa                                                    20


<210>  154
<211>  20
<212>  DNA
<213>  Artificial

<220>
<223>  Primer used in amplifying exon of NPHP4 gene

<400>  154
accatcatcg caccaaagtt                                                    20


<210>  155
<211>  20
<212>  DNA
<213>  Artificial

<220>
<223>  Primer used in amplifying exon of NPHP4 gene

<400>  155
tcttctaggg ggagggtgat                                                    20


<210>  156
<211>  20
<212>  DNA
<213>  Artificial

<220>
<223>  Primer used in amplifying exon of NPHP4 gene

<400>  156
aaaggcacca gggaagaagt                                                    20


<210>  157
<211>  20
<212>  DNA
<213>  Artificial

<220>
<223>  Primer used in amplifying exon of NPHP4 gene

<400>  157
gcaggaagtt ctgccattct                                                    20

<210>  158
<211>  20
<212>  DNA
<213>  Artificial

<220>
<223>  Primer used in amplifying exon of NPHP4 gene

<400>  158
atgattgcaa caaagccaca                                                    20


<210>  159
<211>  20
<212>  DNA
<213>  Artificial

<220>
<223>  Primer used in amplifying exon of NPHP4 gene

<400>  159
ttctgtgtga tccctgcttg                                                    20


<210>  160
<211>  20
<212>  DNA
<213>  Artificial

<220>
<223>  Primer used in amplifying exon of NPHP4 gene

<400>  160
tgaatgggga tgtcctaagc                                                    20


<210>  161
<211>  20
<212>  DNA
<213>  Artificial

<220>
<223>  Primer used in amplifying exon of NPHP4 gene

<400>  161
gtgcaaaact ggggaaatgt                                                    20


<210>  162
<211>  20
<212>  DNA
<213>  Artificial

<220>
<223>  Primer used in amplifying exon of NPHP4 gene

<400>  162
caccctctgt cactgtccaa                                                    20


<210>  163
<211>  20
<212>  DNA
<213>  Artificial

<220>
<223>  Primer used in amplifying exon of NPHP4 gene

<400>  163
tgtgaggact tcatgggtca                                                    20

<210>  164
<211>  20
<212>  DNA
<213>  Artificial

<220>
<223>  Primer used in amplifying exon of NPHP4 gene

<400>  164
cactgtggca cctcagaaaa                                                    20

<210>  165
<211>  20
<212>  DNA
<213>  Artificial

<220>
<223>  Primer used in amplifying exon of NPHP4 gene

<400>  165
acctcactgc cttctgtgct                                                    20

<210>  166
<211>  20
<212>  DNA
<213>  Artificial

<220>
<223>  Primer used in amplifying exon of NPHP4 gene

<400>  166
aaactttcct tccccttca                                                     20

<210>  167
<211>  20
<212>  DNA
<213>  Artificial

<220>
<223>  Primer used in amplifying exon of NPHP4 gene

<400>  167
cacttgggcg tcctgtattt                                                    20

<210>  168
<211>  20
<212>  DNA
<213>  Artificial

<220>
<223>  Primer used in amplifying exon of NPHP4 gene

<400>  168
attcttcctt ttgggggttg                                                    20

<210>  169
<211>  20
<212>  DNA

<213>  Artificial

<220>
<223>  Primer used in amplifying exon of NPHP4 gene

<400>  169
ctgcaggagc tgccttttac                                                    20


<210>  170
<211>  20
<212>  DNA
<213>  Artificial

<220>
<223>  Primer used in amplifying exon of NPHP4 gene

<400>  170
tgtgtgagca ccctaaccaa                                                    20


<210>  171
<211>  20
<212>  DNA
<213>  Artificial

<220>
<223>  Primer used in amplifying exon of NPHP4 gene

<400>  171
tgaaatgggt tgctgtctga                                                    20


<210>  172
<211>  20
<212>  DNA
<213>  Artificial

<220>
<223>  Primer used in amplifying exon of NPHP4 gene

<400>  172
ctggagaccc aggatcgaat                                                    20


<210>  173
<211>  20
<212>  DNA
<213>  Artificial

<220>
<223>  Primer used in amplifying exon of NPHP4 gene

<400>  173
ggggtattct gggaggagtt                                                    20


<210>  174
<211>  20
<212>  DNA
<213>  Artificial

<220>
<223>  Primer used in amplifying exon of NPHP4 gene

<400>  174
ggaggagctg atgactgagg                                                    20

<210>  175
<211>  20
<212>  DNA
<213>  Artificial

<220>
<223>  Primer used in amplifying exon of NPHP4 gene

<400>  175
ggtgaattgg ggtgcactta                                                    20


<210>  176
<211>  19
<212>  DNA
<213>  Artificial

<220>
<223>  Primer used in amplifying exon of NPHP4 gene

<400>  176
aagggcaggc caagtagtg                                                     19


<210>  177
<211>  20
<212>  DNA
<213>  Artificial

<220>
<223>  Primer used in amplifying exon of NPHP4 gene

<400>  177
tgctccagag accaggaact                                                    20


<210>  178
<211>  20
<212>  DNA
<213>  Artificial

<220>
<223>  Primer used in amplifying exon of NPHP4 gene

<400>  178
ctgcatgcac acagacacac                                                    20


<210>  179
<211>  19
<212>  DNA
<213>  Artificial

<220>
<223>  Primer used in amplifying exon of NPHP4 gene

<400>  179
aggtggctgg aagcttgtt                                                     19


<210>  180
<211>  20
<212>  DNA
<213>  Artificial

<220>
<223>  Primer used in amplifying exon of NPHP4 gene

<400>  180
ccaggatcaa gtcccacatt                                                    20


<210>  181
<211>  20
<212>  DNA
<213>  Artificial

<220>
<223>  Primer used in amplifying exon of NPHP4 gene

<400>  181
cccgtttact gtcctctgga                                                    20


<210>  182
<211>  20
<212>  DNA
<213>  Artificial

<220>
<223>  Primer used in amplifying exon of NPHP4 gene

<400>  182
ggagacacag cactcccagt                                                    20


<210>  183
<211>  20
<212>  DNA
<213>  Artificial

<220>
<223>  Primer used in amplifying exon of NPHP4 gene

<400>  183
gcacacagtg accattgagg                                                    20


<210>  184
<211>  20
<212>  DNA
<213>  Artificial

<220>
<223>  Primer used in amplifying exon of NPHP4 gene

<400>  184
aggtgacctg ctgaaggcta                                                    20


<210>  185
<211>  20
<212>  DNA
<213>  Artificial

<220>
<223>  Primer used in amplifying exon of NPHP4 gene

<400>  185
ttcaggcaga gccagttaca                                                    20


<210>  186
<211>  20
<212>  DNA

<213>  Artificial

<220>
<223>  Primer used in amplifying exon of NPHP4 gene

<400>  186
ataccaacat cgcctgaagc                                                    20


<210>  187
<211>  20
<212>  DNA
<213>  Artificial

<220>
<223>  Primer used in amplifying exon of NPHP4 gene

<400>  187
caaattgagc gctgacacat                                                    20


<210>  188
<211>  20
<212>  DNA
<213>  Artificial

<220>
<223>  Primer used in amplifying exon of NPHP4 gene

<400>  188
atgggtcaca ggttgggata                                                    20


<210>  189
<211>  20
<212>  DNA
<213>  Artificial

<220>
<223>  Primer used in amplifying exon of NPHP4 gene

<400>  189
tgtctgcttc tggtctggtg                                                    20


<210>  190
<211>  21
<212>  DNA
<213>  Artificial

<220>
<223>  Primer used in amplifying exon of NPHP4 gene

<400>  190
tcactttgct tcatgccttt t                                                  21


<210>  191
<211>  21
<212>  DNA
<213>  Artificial

<220>
<223>  Primer used in amplifying exon of NPHP4 gene

<400>  191
aaaggcatga agcaaagtga a                                                  21

<210> 192
<211> 19
<212> DNA
<213> Artificial

<220>
<223> Primer used in amplifying exon of NPHP4 gene

<400> 192
agccgtggag tctcacctc 19


<210> 193
<211> 20
<212> DNA
<213> Artificial

<220>
<223> Primer used in amplifying exon of NPHP4 gene

<400> 193
aagtcacagt gggccttcag 20


<210> 194
<211> 20
<212> DNA
<213> Artificial

<220>
<223> Primer used in amplifying exon of NPHP4 gene

<400> 194
acccagacgc atagttgtgg 20


<210> 195
<211> 20
<212> DNA
<213> Artificial

<220>
<223> Primer used in amplifying exon of NPHP4 gene

<400> 195
actcggtccc cttctgactt 20


<210> 196
<211> 20
<212> DNA
<213> Artificial

<220>
<223> Primer used in amplifying exon of NPHP4 gene

<400> 196
tgatctcgaa agcctgtgtg 20


<210> 197
<211> 20
<212> DNA
<213> Artificial

<220>
<223> Primer used in amplifying exon of NPHP4 gene

<400>  197
gtctcctccg cagtctatgg                                                    20


<210>  198
<211>  20
<212>  DNA
<213>  Artificial

<220>
<223>  Primer used in amplifying exon of NPHP4 gene

<400>  198
ctgtgacagt gcccagatgt                                                    20


<210>  199
<211>  20
<212>  DNA
<213>  Artificial

<220>
<223>  Primer used in amplifying exon of NPHP4 gene

<400>  199
agctgtgaat ctcccctgag                                                    20


<210>  200
<211>  20
<212>  DNA
<213>  Artificial

<220>
<223>  Primer used in amplifying exon of NPHP4 gene

<400>  200
gtgacagcag cgcttacttg                                                    20


<210>  201
<211>  20
<212>  DNA
<213>  Artificial

<220>
<223>  Primer used in amplifying exon of NPHP4 gene

<400>  201
cggatctttg gcaacaaact                                                    20


<210>  202
<211>  21
<212>  DNA
<213>  Artificial

<220>
<223>  Primer used in amplifying exon of NPHP4 gene

<400>  202
ctgatgagga gttccagaag c                                                  21


<210>  203
<211>  20
<212>  DNA

<213>  Artificial

<220>
<223>  Primer used in amplifying exon of NPHP4 gene

<400>  203
agctgtgaat ctcccctgag                                                    20


<210>  204
<211>  20
<212>  DNA
<213>  Artificial

<220>
<223>  Primer used in amplifying exon of NPHP4 gene

<400>  204
gtgacagcag cgcttacttg                                                    20


<210>  205
<211>  240
<212>  DNA
<213>  Canis familiaris

<400>  205
ctctctccag gctctgtcat gtggctttgg aattcttcgg atctttggga acaaactgga      60

atctcctacc tctgcctccc aggacaagcg gttgaggctg tatcacggca ctccccgagc     120

cctcctgcac ccactgctcc aggacccttt cgaacaaaat aaacacatga ccctcattga     180

gagctgcagc ctgcaataca ctctgaagcc acacccgctc ctggagcccg tcttccatct     240


<210>  206
<211>  175
<212>  DNA
<213>  Canis familiaris

<400>  206
ctctctccag gctctgtcat gtggctttgg aattcttcgg atctttggga acaaactgga      60

atctcctacc tctgcctccc aggacaagcg aaaataaaca catgaccctc attgagagct     120

gcagcctgca atacactctg aagccacacc cgctcctgga gcccgtcttc catct          175


<210>  207
<211>  301
<212>  DNA
<213>  Canis familiaris

<400>  207
gcgctctgtg gtcttgcttt gcaggttgag gctgtatcac ggcactcccc gagccctcct      60

gcacccactg ctccaggacc ctttcgaacg taagagacaa ggcctggcct ggcctgaggt     120

gttgctgtga agtcctctca tcctccctat gactttcctg gtgttatctg ctttgactcc     180

atcttgttct ctttctcctg tgtaaaccat tatcagctta ggtgatttat ttaagggatt     240

aggacaaaaa gtgtcatgag cttggatggt tgacgtaact gaaggaactg taagcactag     300

c                                                                       301

<210>  208
<211>  119
<212>  DNA
<213>  Canis familiaris

<400>  208
gcgctctgtg gtcttgcttt gcaggttgag gctgtatcac gggatttatt taagggatta      60

ggacaaaaag tgtcatgagc ttggatggtt gacgtaactg aaggaactgt aagcactag      119


<210>  209
<211>  269
<212>  DNA
<213>  Canis familiaris

<400>  209
gttgaggctg tatcacggca ctccccgagc cctcctgcac ccactgctcc aggacccttt      60

cgaacgtaag agacaaggcc tggcctggcc tgaggtgttg ctgtgaagtc ctctcatcct      120

ccctatgact ttcctggtgt tatctgcttt gactccatct tgttctcttt ctctgtgtaa      180

accattatca gcttagctga tttatttaag ggattaggac aaaaagtgtc atgagcttgg      240

atggttgacg taactgaagg aactgtaag                                        269